# EUROPEAN PATENT SPECIFICATION

(11) **EP 1 278 878 B1**
(45) Date of publication and mention of the grant of the patent: **31.03.2010**
(21) Application number: 01921651.4
(22) Date of filing: 18.04.2001
(51) Int. Cl.: C12N 15/86, C12N 5/10, C07K 14/16, C07K 14/555, C12N 7/04, A61K 48/00

(54) **CODON OPTIMIZED EIAV VECTORS**
KODON-OPTIMIERTE EIAV VEKTOREN
VECTEURS DU EIAV À CODONS OPTIMISÉS

(30) Priority: 19.04.2000 GB 0009760
(43) Date of publication of application: 29.01.2003
(73) Proprietor: Oxford Biomedica (UK) Limited, Oxford 0X4 4GA (GB)
(72) Inventor: KINGSMAN, Alan John, c/o Oxford Biomedica (UK) Limited, Oxford OX4 4GA (GB); KIM, Narry The Howard Hughes Medical Institute, 415 Curie Blvd. Phila., PA 19104-6148 (US); KOTSOPOULOU, Ekaterini Enders 850, Boston, MA 02115 (US); ROHLL, Jonathan, c/o Oxford Biomedica (UK) Limited, Oxford OX4 4GA (GB); MITROPHANOUS, Kyriacos Andreou, c/o Oxford Biomedica (UK) Limited, Oxford OX4 4GA (GB)
(74) Representative: Mallalieu, Catherine Louise
(86) International application number: PCT/GB2001/001784
(87) International publication number: WO 2001/079518

(56) References cited:
- WO-A-00/15819
- WO-A-00/39302
- WO-A-00/65076
- WO-A-99/32646
- WO-A-99/41397
- WO-A1-00/55341
- WO-A1-01/25466
- CHUNG ET AL.: "Development of new safer retrovirus packaging sequences and cell lines" J. CELL. BIOL. SUPPLEMENT, vol. 0, no. 21A, 1995, page 404 XP001064207
- G. BUCHSCHACHER ET AL.: "Development of lentiviral vectors for gene therapy of human diseases" BLOOD, vol. 95, no. 6, 2000, pages 2499-2504, XP000916373
- J. ZUR MEGEDE ET AL.: "Increased expression and immunogenicity of sequence-modified HIV-1 gag gene" J. VIROL., vol. 74, no. 6, 2000, pages 2628-2635, XP000980650
- E. KOTSOPOLOU ET AL.: "A rev-independent HIV 1-based vector that exploits a codon optimised HIV-1 gag-pol gene" J. VIROL., vol. 74, no. 10, 2000, pages 4839-4852, XP002140792
- R. WAGNER AT AL.: "Rev-idependent expression of synthetic gag-pol genes of HIV-1 and SIV: implications for the safety of lentiviral vectors" HUMAN GENE THERAPY, vol. 11, 2000, pages 2403-2413, XP001064159

## Description

### Field of the Invention

The present invention relates to uses for the safety of retroviral vectors capable of delivering therapeutic genes for use in gene therapy, and to novel nucleotide sequences for such use.

### Background to the Invention

Retroviral vectors are now widely used as vehicles to deliver genes into cells. Their popularity stems from the fact that they are easy to produce and mediate stable integration of the gene that they carry into the genome of the target cell. This enables long-term expression of the delivered gene (1).

There has been considerable interest, for some time, in the development of retroviral vector systems based on lentiviruses. Lentiviruses are a small subgroup of complex retroviruses. They contain, in addition to the common retroviral genes (*gag, pol* and *env*), genes which enable them to regulate their life cycle and to infect non-dividing cells (2). Vector systems based thereon are therefore of interest because of their potential use in the transfer of a gene of interest to non-dividing cells such as neurones. In addition, lentiviral vectors enable very stable long-term expression of the gene of interest. This has been shown to be at least three months for transduced rat neuronal cells while MLV based vectors were only able to express the gene of interest for six weeks.

The most commonly used lentivirus is the Human Immunodeficiency Virus (HIV), the etiologic agent of AIDS (acquired immune deficiency syndrome). HIV-based vectors have been shown to efficiently transduce non-diving cells (3) and can be used, for example, to target anti-HIV therapeutic genes to HIV susceptible cells.

However, HIV vectors have a number of significant disadvantages that may limit their therapeutic application to certain diseases. In particular, HIV-1 is a human pathogen carrying potentially oncogenic proteins and sequences. There is the risk that introduction of vector particles produced in packaging cells which express HIV gag-pol will introduce these proteins into the patient leading to seroconversion.

Emphasis has therefore been placed on the safety of these vectors. One strategy looks at the design of production systems for retroviral vectors. A retrovirus vector system basically consists of two elements, a packaging cell line and a vector genome. The simplest packaging line consists of a provirus in which the ψ sequence (a determinant of RNA packaging reporting in HIV as lying between U5 and *gag*) has been deleted. When stably transfected into a cell, virus particles containing reverse transcriptase will be produced but virion RNA will not become packaged within these particles. The complementing component in a retrovirus vector system is the genome vector itself. The genome vector needs to contain a packaging sequence but much of the structural coding regions can be deleted. Often a selectable marker gene, or other nucleotide sequence of interest, is incorporated into the vector. Vector stocks of the packaging line can then be used to infect target cells. Provided the cell is successfully infected by the viral particle, the genome vector sequence will be reverse transcribed and integrated by the retroviral machinery. However, infection is an end process so no further replication or spread of the vector should occur.

As indicated above, however, problems are encountered in the design of safe and effective retroviral vectors. These include the possibility that recombination between the packaging vector and the packaging sequence can lead to the generation of wild type replication competent virus. Consequently efforts have been directed at improving the safety of packaging cell constructs.

In second generation packaging cell lines, in addition to deletion of the packaging sequence, the 3' LTR was also deleted so that two recombinations are necessary to generate a wild type virus.

In third generation packaging lines the *gag-pol* genes and *env* gene are placed on separate constructs that are sequentially introduced into the packaging cells to prevent recombination during transfection.

With regard to the packaging signal, EP 0 368 882A (Sodroski) discloses that in HIV it corresponds to the region between the 5' major splice donor and the *gag* initiation codon, and particularly corresponds to a segment just downstream of the 5' major splice donor, and about 14 bases upstream of the *gag* initiation codon. It is this region which Sodroski teaches should be deleted from the *gag-pol* cassette. WO97/12622 (Verma) describes that in HIV-1 a 39 bp internal deletion in the ψ sequence can be made between the 5' splice donor site and the starting codon of the *gag* gene.

Codon wobbling can be used to reduce recombination frequency while maintaining the primary protein sequence of the constructs, c.f. (4) in which the region of overlap between the *gag-pol* and *env* expression constructs was reduced to 61 bp extending over the common region between *pol* and *env* which are in different reading frames. Transversion mutations were introduced into the final 20 codons of *pol,* retaining the integrity of the coding region while reducing the homology with *env* to 55% in the overlap region. Similarly wobble mutations were introduced into the 3' of *env* and all sequences downstream of the *env* stop codon were deleted.

Efficient vectors usually contain part of *gag* on the genome vector to increase virion titre. Unlike the packaging sequence which can be in any position within a sequence to effect packaging, the *gag* sequence must be in its native position adjacent to ψ to have any effect.

It will be appreciated that whilst significant improvements in packaging cell and vector design have been made there is still scope for further refinement of current packaging lines.

### Summary of the Invention

It is therefore an aim of the invention to provide retroviral particles, in particular lentiviral particles, and particularly those which carry nucleotide constructs encoding therapeutic proteins, that have improved safety over the corresponding wild type viral particle. In our WO99/41397 we describe codon optimisation of the *gag-pol* genes as a means of overcoming the Rev/RRE requirement for export and to enhance RNA stability. We have now found however that the codon optimised *gag*-*pol* sequence overcomes potential recombination problems with vector genomes which carry part of a *gag* sequence with the aim of increasing titre. This strategy also avoids the need to use *gag* regions from different viruses in the packaging and vector genome constructs.
Another significant advantage provided by the invention is that the codon optimisation disrupts RNA secondary structures, such as the packaging signal, thus rendering the *gag-pol* mRNA non-packagable. Thus, the present invention allows retroviral sequence upstream of the *gag* initiation codon to be retained, in contrast to Sodroski and Verma, without significantly compromising safety.

### Statements of the Invention

Accordingly in one aspect the present invention provides use of a nucleotide sequence coding for retroviral gag and pol proteins, capable of assembly of a retroviral vector genome into a retroviral particle in a producer cell, to generate a replication defective retrovirus in a target cell, wherein the nucleotide sequence is codon optimised for expression in the producer cell, and wherein the nucleotide sequence comprises the sequence shown in SEQ ID NO: 16. In another aspect the present invention provides use of a nucleotide sequence coding for retroviral gag and pol proteins capable of assembly of a retroviral vector genome into a retroviral particle in a producer cell to prevent packaging of the retroviral vector genome in a target cell, wherein the nucleotide sequence is codon optimised for expression in the producer cell, and wherein the retroviral particle is substantially derived from EIAV; and the nucleotide sequence comprises the sequence shown in SEQ ID NO: 16.

In another aspect the present invention provides use of a nucleotide sequence coding for retroviral gag and pol proteins capable of assembly of a retroviral vector genome, comprising at least part of a gag nucleotide sequence, into a retroviral particle in a producer cell to prevent recombination between said nucleotide sequence coding for retroviral gag and pol proteins and the at least part of a gag nucleotide sequence, wherein the nucleotide sequence coding for retroviral gag and pol proteins is codon optimised for expression in the producer cell, and wherein the nucleotide sequence comprises the sequence shown in SEQ ID NO: 16.

Put another way the present invention provides a method of producing a replication defective retrovirus comprising transfecting a producer cell with the following:
i) a retroviral genome;
ii) a nucleotide sequence coding for retroviral gag and pol proteins; and
iii) nucleotide sequences encoding other essential viral packaging components not encoded by the nucleotide sequence of (ii);
characterised in that the nucleotide sequence coding for retroviral gag and pol proteins is codon optimised for expression in the producer cell, and in that the codons optimised nucleotide sequence comprises the sequence shown in SEQ ID NO: 16.

Thus in one aspect the present invention provides a method of preventing packaging of a retroviral genome in a target cell comprising the steps of:
a. transfecting a producer cell with the following to produce retroviral particles:
   i) a retroviral genome;
   ii) a nucleotide sequence coding for retroviral gag and pol proteins; and
   iii) nucleotide sequences encoding other essential viral packaging components not encoded by one or more of the nucleotide sequences of (ii); and
b. transacting a target cell with retroviral particles of step (a);
characterised in that the nucleotide sequence coding for retroviral gag and pol proteins is codon optimised for expression in the producer cell, and in that the codon optimised nucleotide sequence comprises the sequence shown in SEQ ID NO: 16.

In another aspect the present invention provides a method to prevent recombination between a retroviral vector genomes and a nucleotide sequence encoding a viral polypeptide required for the assembly of the viral genome into retroviral particles comprising transfecting a producer cell with the following:
(i) a retroviral genome comprising at least part of a gag nucleotide sequence;
(ii) a nucleotide sequence coding for retroviral gag and pol proteins; and
(iii) nucleotide sequences encoding other essential viral packaging components not encoded by the nucleotide sequence of (ii);
characterised in that the nucleotide sequence coding for retroviral gag and pol proteins is codon optimised for expression in the producer cell, and in that the codon optimised nucleotide sequence comprises the sequence shown in SEQ ID NO: 16.

We also describe a codon optimised sequence as shown in SEQ ID NOS: 15.

The present invention further provides a retroviral particle produced using the sequence of the present invention, and production methods for so doing.

The present invention also provides a pharmaceutical composition comprising a viral particle according to the present invention, together with a pharmaceutically acceptable diluent or carrier.

By "reducing we mean that the chance of an event occurring is reduced compared to a comparable population having the wild-type *gag-pol* sequence. Within a population the chance of an event occurring may be prevented for an individual retrovirus vector or particle.

### Detailed Description of the Invention

Various preferred features and embodiments of the present invention will now be described by way of non limiting example.

The present invention employs the concept of codon optimisation.

Codon optimisation has previously been described in our WO99/41397 as a means of overcoming the Rev/RRE requirement for export and to enhance RNA stability. The alterations to the coding sequence for the viral components improve the sequences for codon usage in the mammalian cells or other cells which are to act as the producer cells for retroviral vector particle production. This improvement in codon usage is referred to as "codons optimisation". Many viruses, including HIV and other lentiviruses, use a large number of rare codons and by changing these to correspond to commonly used mammalian codons, increased expression of the packaging components in mammalian producer cells can be achieved. Codon usage tables are known in the art for mammalian cells, as well as for a variety of other organisms.

By virtue of alterations in their sequences, the nucleotide sequences encoding the packaging components of the viral particles required for assembly of viral particles in the producer cells/packaging cells have RNA instability sequences (INS) eliminated from them. At the same time, the amino acid coding sequence for the packaging components is retained so that the viral components encoded by the sequences remain the same, or at least sufficiently similar that the function of the packaging components is not compromised.

The term "viral polypeptide required for the assembly of viral particles" means a polypeptide normally encoded by the viral genome to be packaged into viral particles, in the absence of which the viral genome cannot be packaged. For example, in the context of retroviruses such polypeptides would include *gag-pol* and *env.* The term "packaging component" is also included within this definition.

As discussed in our WO99/32646, the sequence requirements for packaging HIV vector genomes are complex. The HIV-1 packaging signal encompasses the splice donor site and contains a portion of the 5'-untranslated region of the *gag* gene, which has a putative secondary structure containing 4 short stem-loops. However, additional sequences elsewhere in the genome are also known to be important for efficient encapsidation of HIV. For example, the first 350 bps of the *gag* protein coding sequence may contribute to efficient packaging. Thus, for construction of HIV-1 vectors capable of expressing heterologous genes, a packaging signal extending to 350 bps of the *gag* protein-coding region has been used on the vector genome. We have now found that codon optimisation of the *gag* coding region on the packaging vector, at least in the region into which the packaging signal extends, also has the effect of disrupting packaging of the vector genome. Thus codon optimisation is a novel method of obtaining a replication defective viral particle.

Also as disclosed in WO99/32646, the structure of the packaging signal in equine lentiviruses is different from that of HIV. Instead of a short sequence of 4 stem loops together with a packaging signal extending to 350 bps of the *gag* protein-coding region, we have found that in equine lentiviruses the packaging signal may not extend as far into the *gag* protein-coding region as may have been thought.

In one example only codons relating to the packaging signal are codon optimised. Thus, codon optimisation extends to at least the first 350 bps of the *gag* protein coding region. In equine lentiviruses, at least, codon optimisation extends to at least nucleotide 300 of the gag coding region, more preferably to at least nucleotide 150 of the gag coding region. Although not optimal, codon optimisation could extend to, say, only the first 109 nucleotides of the *gag* coding region. It may also be possible for codon optimisation to extend to only the first codon of the *gag* coding region.

However, the sequences are codon optimised in their entirety, with the exception of the sequence encompassing the frameshift site.

The *gag-pol* gene comprises two overlapping reading frames encoding *gag* and *pol* proteins respectively. The expression of both proteins depends on a frameshift during translation. This frameshift occurs as a result of ribosome "slippage" during translation. This slippage is thought to be caused at least in part by ribosome-stalling RNA secondary structures. Such secondary structures exist downstream of the frameshift site in the *gag-pol* gene. For HIV, the region of overlap extends from nucleotide 1222 downstream of the beginning of *gag* (wherein nucleotide 1 is the A of the *gag* ATG) to the end of *gag* (nt 1503). Consequently, a 281 bp fragment spanning the frameshift site and the overlapping region of the two reading frames is preferably not codon optimised. Retaining this fragment will enable more efficient expression of the gag-pol proteins.

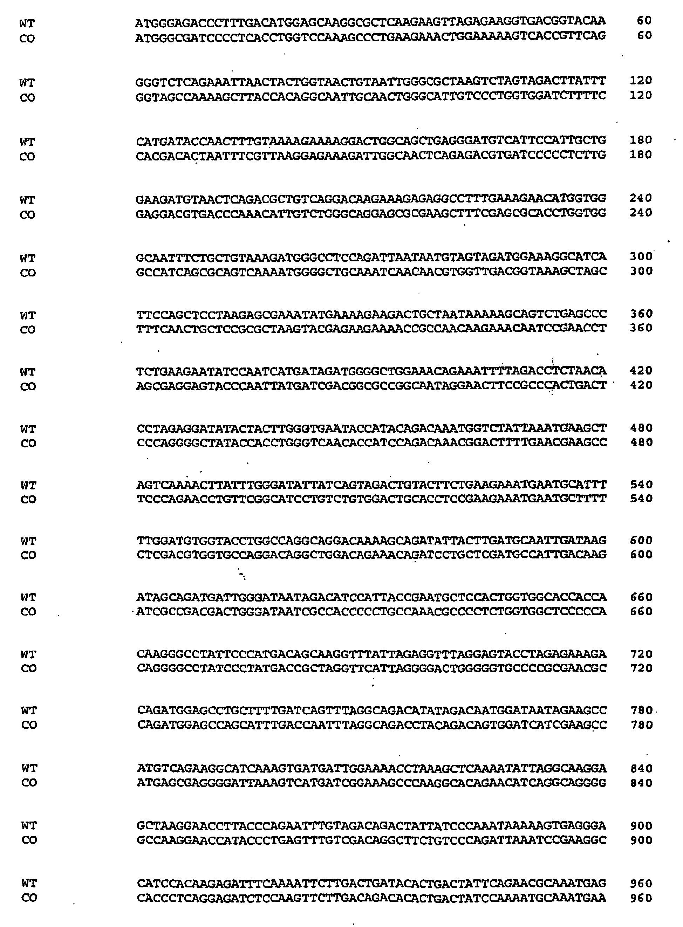

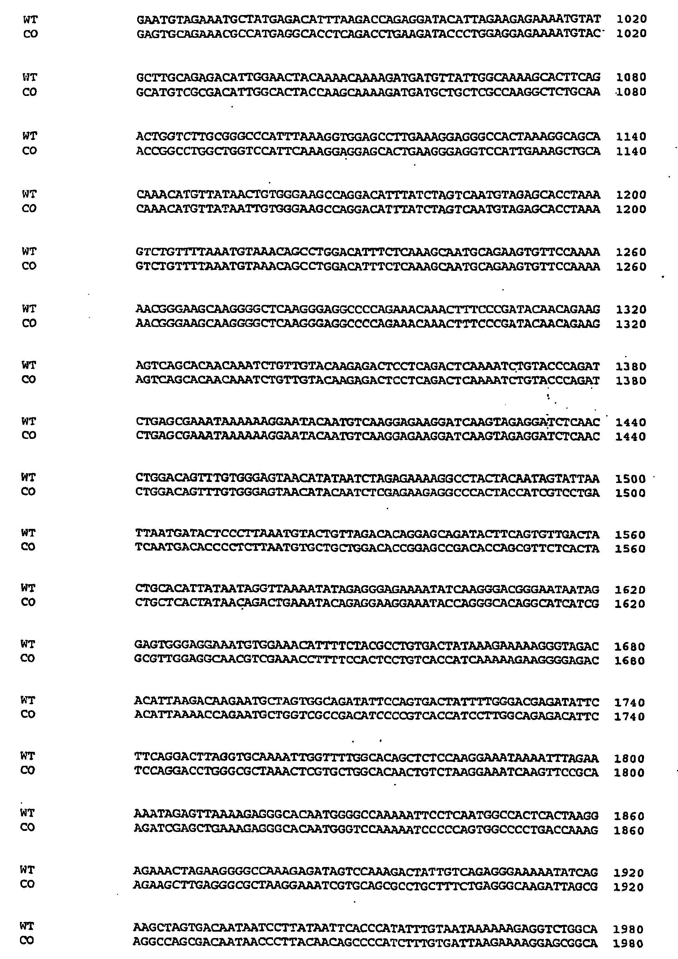

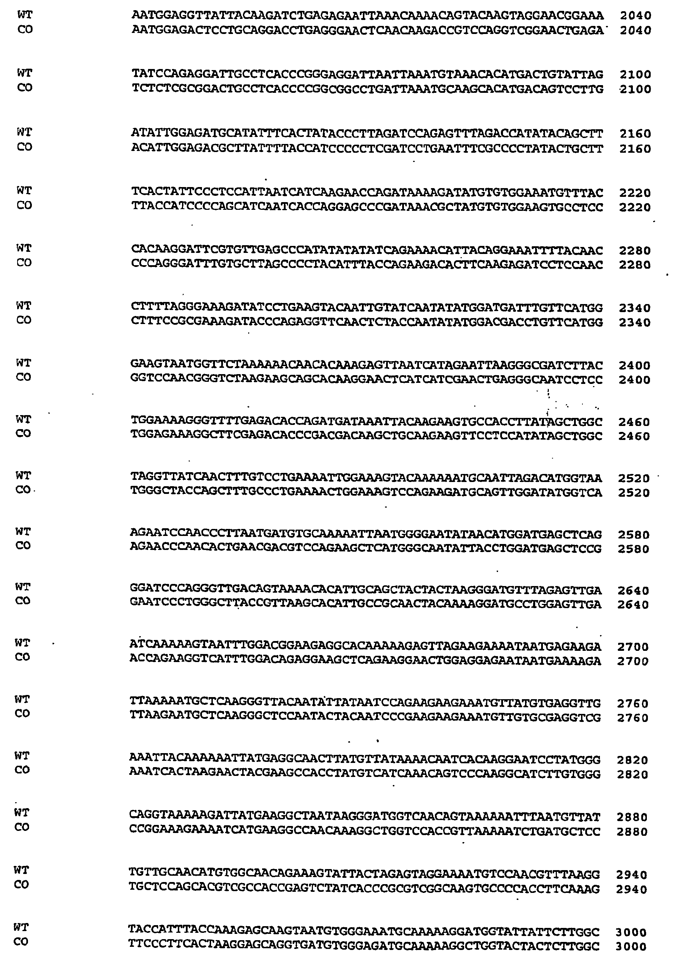

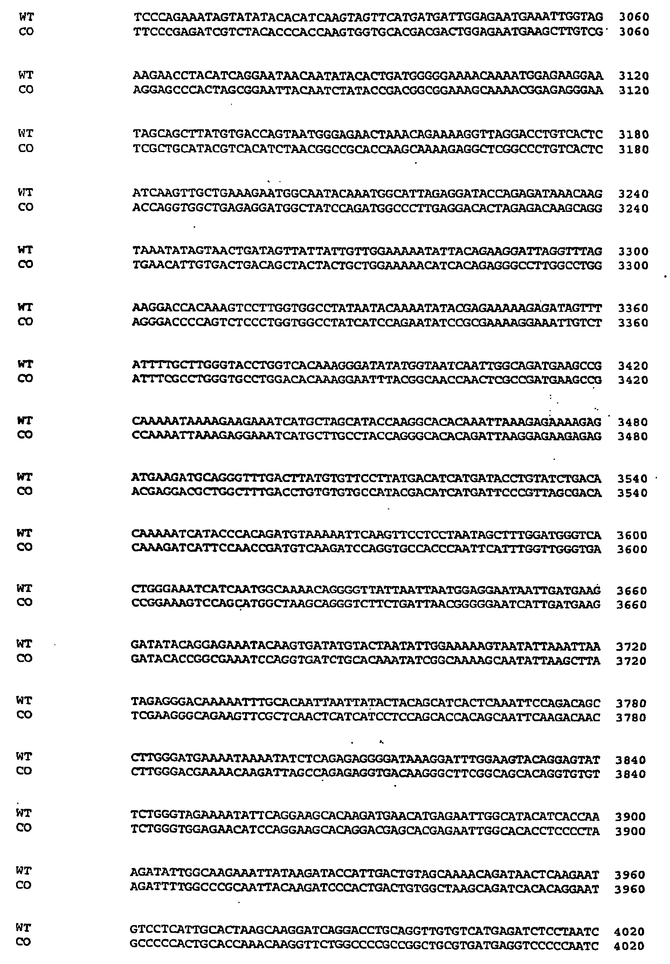

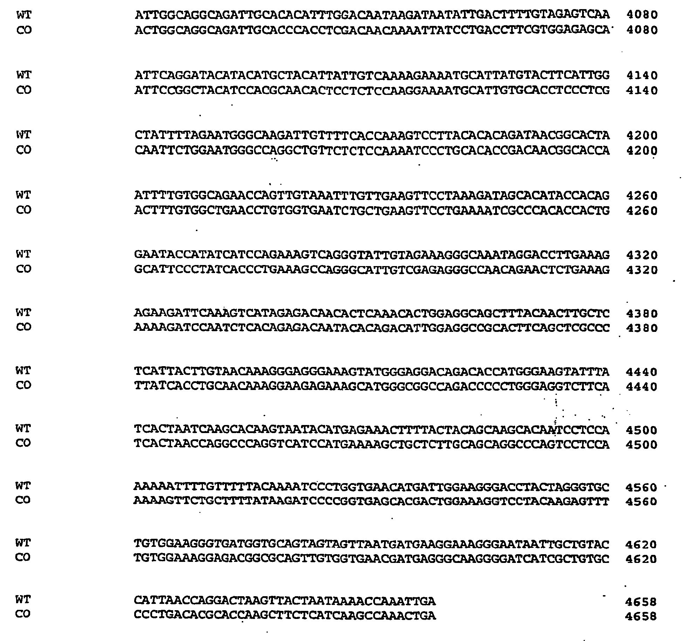

For EIAV the beginning of the overlap has been taken to be nt 1262 (where nucleotide 1 is the A of the *gag* ATG). The end of the overlap is at 1461 bp. In order to ensure that the frameshift site and the *gag, gag-pol* overlap the wild type sequence has been retained from nt 1156 to 1465. This can be seen in Figure 9b.

Derivations from optimal codon usage may be made, for example, in order to accommodate convenient restriction sites, and conservative amino acid changes may be introduced into the *gag-pol* proteins.

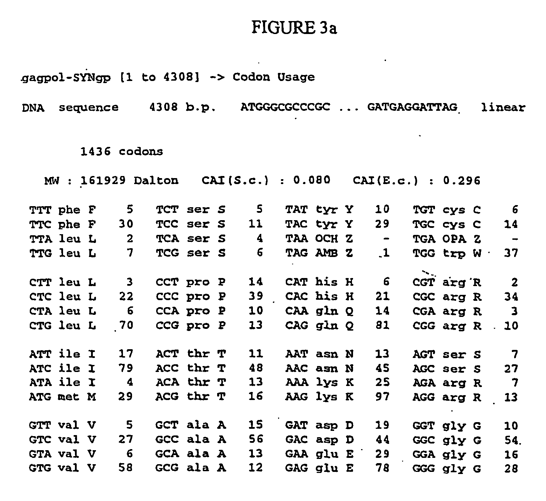

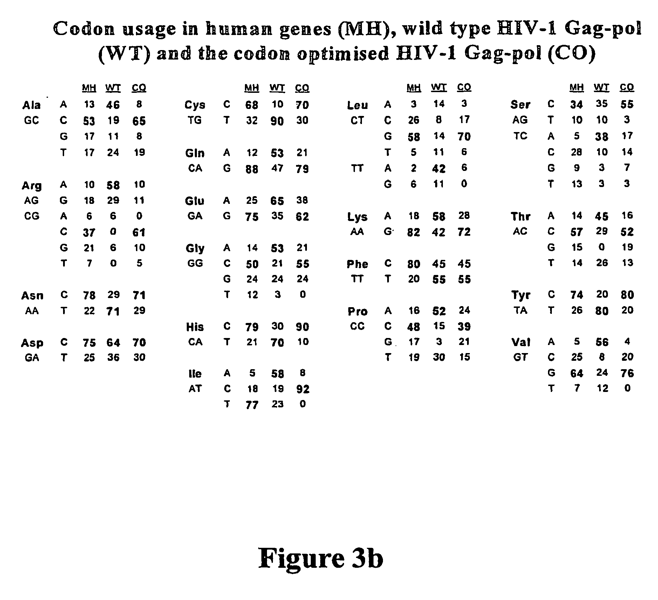

Codon optimisation was based on lightly expressed mammalian genes. The third and sometimes the second and third base may be changed. An example of a codon usage table is given in Figure 3b.

Due to the degenerate nature of the Genetic Code, it will be appreciated that numerous *gag-pol* sequences can be achieved by a skilled worker. Also there are many retroviral variants described and which can be used as a starting point for generating a codon optimised *gag-pol* sequence. Lentiviral genomes can be quite variable. For example there are many quasi-species of HIV-1 which are still functional. This is also the case for EIAV. These variants may be used to enhance particular parts of the transduction process. Examples of HTV-1 variants may be found at http://hiv-web.lanl.gov. Details of EIAV clones may be found at the NCBI database: http://www.ncbi.nlm.nih.gov.

The strategy for codon optimised *gag-pol* sequences can be used in relation to any retrovirus. This would apply to all the lentiviruses, including EIAV, FIV, BIV, CAEV, VMR, SIV, HIV-1 and HIV-2. In addition this method could be used to increase expression of genes from HTLV-1, HTLV-2, HFV, HSRV and human endogenous retroviruses (HERV).

As codon optimisation may result in disruption of RNA secondary structures such as the packaging signal, it will be appreciated that any endogenous packaging signal upstream of the *gag* initiation codon could be retained without compromising safety.

An additional advantage of codon optimising packaging components is that this can increase gene expression. In particular, it can render *gag-pol* expression Rev independent. In order to enable the use of anti-rev or RRE factors in the retroviral vector, however, it would be necessary to render the viral vector generation system totally Rev/RRE independent (5). Thus, the genome also needs to be modified. This is achieved by optimising vector genome components. Advantageously, these modifications also lead to the production of a safer system absent of all accessory proteins both in the producer and in the transduced cell, and are described below.

As described above, the packaging components for a retroviral vector include expression products of *gag, pol* and *env* genes. In addition, efficient packaging depends on a short sequence of 4 stem loops followed by a partial sequence from *gag* and *env* (the "packaging signal"). Thus, inclusion of a deleted *gag* sequence in the retroviral vector genome (in addition to the full *gag* sequence on the packaging construct) will optimise vector titre. To date efficient packaging has been reported to require from 255 to 360 nucleotides of *gag* in vectors that still retain *env* sequences, or about 40 nucleotides of *gag* in a particular combination of splice donor mutation, *gag* and *env* deletions. We have surprisingly found that a deletion of up to 360 nucleotides in *gag* leads to an increase in vector titre. Further deletions resulted in lower titres. Additional mutations at the major splice donor site upstream of *gag* were found to disrupt packaging signal secondary structure and therefore lead to decreased vector titre. Thus, preferably, the retroviral vector genome includes a *gag* sequence from which up to 360 nucleotides have been removed.

We therefore allow the preparation of a so-called "minimal" system in which all of the accessory genes may be removed. In HIV these accessory genes are vpr, *vif, tat, nef, vpu* and *rev*. Similarly, in other lentiviruses the analogous accessory genes normally present in the lentivirus may be removed. For the avoidance of doubt, however, we would mention that the present invention also extends to systems, particles and vectors in which one or more of these accessory genes is present and in any combination.

The term "viral vector" refers to a nucleotide construct comprising a viral genome capable of being transcribed in a host cell, which genome comprises sufficient viral genetic information to allow packaging of the viral RNA genome, in the presence of packaging components, into a viral particle capable of infecting a target cell. Infection of the target cell includes reverse transcription and integration into the target cell genome, where appropriate for particular viruses. The viral vector in use typically carries heterologous coding sequences (nucleotides of interest or "NOIs") which are to be delivered by the vector to the target cell, for example a first nucleotide sequence encoding a ribozyme. By "replication defective" we mean that a viral vector is incapable of independent replication to produce infectious viral particles within the final target cell.

The term " viral vector system" is intended to mean a kit of parts which can be used when combined with other necessary components for viral particle production to produce viral particles in host cells. For example, an NOI may typically be present in a plasmid vector construct suitable for cloning the NOI into a viral genome vector construct. When combined in a kit with a further nucleotide sequence, which will also typically be present in a separate plasmid vector construct, the resulting combination of plasmid containing the NOI and plasmid containing the further nucleotide sequence comprises the essential elements of the invention. Such a kit may then be used by the skilled person in the production of suitable viral vector genome constructs which when transfected into a host cell together with the plasmid containing the further nucleotide sequence, and optionally nucleic acid constructs encoding other components required for viral assembly, will lead to the production of infectious viral particles.

Alternatively, the further nucleotide sequence may be stably present within a packaging cell line that is included in the kit.

The kit may include the other components needed to produce viral particles, such as host cells and other plasmids encoding essential viral polypeptides required for viral assembly. By way of example, the kit may contain (i) a plasmid containing an NOI and (ii) a plasmid containing a further nucleotide sequence encoding a modified retroviral *gag-pol* construct which has been codon optimised for expression in a producer of choice. Optional components would then be (a) a retroviral genome construct with suitable restriction enzyme recognition sites for cloning the NOI into the viral genome, optionally with at least a partial *gag* sequence; (b) a plasmid encoding a VSV-G env protein. Alternatively, nucleotide sequence encoding viral polypeptides required for assembly of viral particles may be provided in the kit as packaging cell lines comprising the nucleotide sequences, for example a VSV-G expressing cell line.

The term "viral vector production system" refers to the viral vector system described above wherein the NOI has already been inserted into a suitable viral vector genome.

In the present disclosure, several terms are used interchangeably. Thus, "virion", "virus", "viral particle", "retroviral particle", "retrovirus", and "vector particle" mean virus and virus-like particles that are capable of introducing a nucleic acid into a cell through a viral-like entry mechanism. Such vector particles can, under certain circumstances, mediate the transfer of NOIs into the cells they infect. A retrovirus is capable of reverse transcribing its genetic material into DNA and incorporating this genetic material into a target cell's DNA upon transduction. Such cells are designated herein as "target cells".

As used herein the term "target cell" simply refers to a cell which the regulated retroviral vector, whether native or targeted, is capable of infecting or transducing.

A lentiviral vector particle will be capable of transducing cells which are slowly-dividing, and which non-lentiviruses such as MLV would not be able to efficiently transduce. Slowly-dividing cells divide once in about every three to four days including certain tumour cells. Although tumours contain rapidly dividing cells, some tumour cells especially those in the centre of the tumour, divide infrequently.

Alternatively the target cell may be a growth-arrested cell capable of undergoing cell division such as a cell in a central portion of a tumour mass or a stem cell such as a haematopoietic stem cell or a CD34-positive cell.

As a further alternative, the target cell may be a precursor of a differentiated cell such as a monocyte precursor, a CD33-positive cell, or a myeloid precursor.

As a further alternative, the target cell may be a differentiated cell such as a neuron, astrocyte, glial cell, microglial cell, macrophage, monocyte, epithelial cell, endothelial cell, hepatocyte, spermatocyte, spermatid or spermatozoa.

Target cells may be transduced either *in vitro* after isolation from a human individual or may be transduced directly *in vivo.*

Viral vectors are retroviral vectors, in particular lentiviral vectors such as HIV and EIAV vectors. The retroviral vector may be derived from or may be derivable from any suitable retrovirus. A large number of different retroviruses have been identified. Examples include: murine leukemia virus (MLV), human immunodeficiency virus (HIV), simian immunodeficiency virus, human T-cell leukemia virus (HTLV). equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV); Avian myelocytomatosis virus-29 (MC29), and Avian erythroblastosis virus (AEV). A detailed list of retroviruses may be found in Coffin et al., 1997, "Retroviruses", Cold Spring Harbour Laboratory Press Eds: JM Coffin, SM Hughes, HE Varmus pp 758-763.

The term "derivable" is used in its normal sense as meaning a nucleotide sequence such as an LTR or a part thereof which need not necessarily be obtained from a vector such as a retroviral vector but instead could be derived therefrom. By way of example, the sequence may be prepared synthetically or by use of recombinant DNA techniques.

Details on the genomic structure of some retroviruses may be found in the art. By way of example, details on HIV and Mo-MLV may be found from the NCBI Genbank (Genome Accession Nos. AF033819 and AF033811, respectively). Details of HIV variants may also be found at http://hiv-web.lanl.gov. Details of EIAV variants may be found through http://www.ncbi.nlm.nih.gov.

The lentivirus group can be split even further into "primate" and "non-primate". Examples of primate lentiviruses include human immunodeficiency virus (HIV), the causative agent of human auto-immunodeficiency syndrome (AIDS), and simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

The basic structure of a retrovirus genome is a 5' LTR and a 3' LTR, between or within which are located a packaging signal to enable the genome to be packaged, a primer binding site, integration sites to enable integration into a host cell genome and *gag, pol* and *env* genes encoding the packaging components - these are polypeptides required for the assembly of viral particles. More complex retroviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration, and transcription. LTRs also serve as enhancer-promoter sequences and can control the expression of the viral genes. Encapsidation of the retroviral RNAs occurs by virtue of a *psi* sequence, which it has been disclosed in respect of HIV, at least, is located at the 5' end of the viral genome.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

In a defective retroviral vector genome *gag, pol* and *env* may be absent or not functional. The R regions at both ends of the RNA are repeated sequences. U5 and U3 represent unique sequences at the 5' and 3' ends of the RNA genome respectively.

As discussed above, in a typical retroviral vector for use in gene therapy, at least part of one or more of the *gag, pol* and *env* protein coding regions essential for replication may be removed from the viral vector. This makes the retroviral vector replication-defective. The removed portions may even be replaced by a nucleotide sequence of interest (NOI), as in the present invention, to generate a virus capable of integrating its genome into a host genome but wherein the modified viral genome is unable to propagate itself due to a lack of structural proteins. When integrated in the host genome, expression of the NOI occurs - resulting in, for example, a therapeutic and/or a diagnostic effect. Thus, the transfer of an NOI into a site of interest is typically achieved by: integrating the NOI into the recombinant viral vector; packaging the modified viral vector into a virion coat; and allowing transduction of a site of interest - such as a targeted cell or a targeted cell population.

A minimal retroviral genome for use in the present invention may therefore comprise (5') R - U5 - one or more NOIs - U3-R (3'). However, the plasmid vector used to produce the retroviral genome within a host cell/packaging cell will also include transcriptional regulatory control sequences operably linked to the retroviral genome to direct transcription of the genome in a host cell/packaging cell. These regulatory sequences may be the natural sequences associated with the transcribed retroviral sequence, i.e. the 5' U3 region, or they may be a heterologous promoter such as another viral promoter, for example the CMV promoter.

Some retroviral genomes require additional sequences for efficient virus production. For example, in the case of HIV, rev and RRE sequence should be included. However, we have found that the requirement for *rev* and RRE can be reduced or eliminated by codon optimisation. As expression of the codon optimised *gag-pol* is REV independent, RRE can be removed from the *gag-pol* expression cassette, thus removing any potential for recombination with any RRE contained on the vector genome.

Once the retroviral vector NOIs sequences need to be expressed. In a retrovirus, the promoter is located in the 5' LTR U3 region of the provirus. In retroviral vectors, the promoter driving expression of a therapeutic gene may be the native retroviral promoter in the 5' U3 region, or an alternative promoter engineered into the vector. The alternative promoter may physically replace the 5' U3 promoter native to the retrovirus, or it may be incorporated at a different place within the vector genome such as between the LTR.

Thus, the NOI will also be operably linked to a transcriptional regulatory control sequence to allow transcription of the first nucleotide sequence to occur in the target cell. The control sequence will typically be active in mammalian cells. The control sequence may, for example, be a viral promoter such as the natural viral promoter or a CMV promoter or it may be a mammalian promoter. It is particularly preferred to use a promoter that is preferentially active in a particular cell type or tissue type in which the virus to be treated primarily infects. Thus, in one embodiment, a tissue-specific regulatory sequences may be used. The regulatory control sequences driving expression of the one or more first nucleotide sequences may be constitutive or regulated promoters.

The term "operably linked" denotes a relationship between a regulatory region (typically a promoter element, but may include an enhancer element) and the coding region of a gene, whereby the transcription of the coding region is under the control of the regulatory region.

As used herein, the term "enhancer" includes a DNA sequence which binds other protein components of the transcription initiation complex and thus facilitates the initiation of transcription directed by its associated promoter.

In one preferred example, the enhancer is an ischaemic like response element (ILRE).

The term "ischaemia like response element" - otherwise written as ILRE - includes an element that is responsive to or is active under conditions of ischaemia or conditions that are like ischaemia or are caused by ischaemia. By way of example, conditions that are like ischaemia or are caused by ischaemia include hypoxia and/or low glucose concentration(s).

The term "hypoxia" means a condition under which a particular organ or tissue receives an inadequate supply of oxygen.

Ischaemia can be an insufficient supply of blood to a specific organ or tissue. A consequence of decreased blood supply is an inadequate supply of oxygen to the organ or tissue (hypoxia). Prolonged hypoxia may result in injury to the affected organ or tissue.

A preferred ILRE is a hypoxia response element (HRE).

In one preferred aspect, there is hypoxia or ischaemia regulatable expression of the retroviral vector components. In this regard, hypoxia is a powerful regulator of gene expression in a wide range of different cell types and acts by the induction of the activity of hypoxia-inducible transcription factors such as hypoxia inducible factor-1 (HIF-1; 6), which bind to cognate DNA recognition sites, the hypoxia-responsive elements (HREs) on various gene promoters. Dachs *et al* (7) have used a multimeric form of the HRE from the mouse phosphoglycerate kinase-1 (PGK-1) gene (8) to control expression of both marker and therapeutic genes by human fibrosarcoma cells in response to hypoxia *in vitro* and within solid tumours *in vivo* (7 *ibid).*

Hypoxia response enhancer elements (HREEs) have also been found in association with a number of genes including the erythropoietin (EPO) gene (9; 10). Other HREEs have been isolated from regulatory regions of both the muscle glycolytic enzyme pyruvate kinase (PKM) gene (11), the human muscle-specific β-enolase gene (ENO3; 12) and the endothelin-1 (ET-1) gene (13).

Preferably the HRE is selected from, for example, the erythropoietin HRE element (HREE1), muscle pyruvate kinase (PKM), HRE element, phosphoglycerate kinase (PGK) HRE, B-enolase (enolase 3; ENO3) HRE element, endothelin-1 (ET-1)HRE element and metallothionein II (MTII) HRE element.

Preferably the ILRE is used in combination with a transcriptional regulatory element, such as a promoter, which transcriptional regulatory element is preferably active in one or more selected cell type(s), preferably being only active in one cell type.

As outlined above, this combination aspect is called a responsive element.

Preferably the responsive element comprises at least the ILRE as herein defined.

Non-limiting examples of such a responsive element are presented as OBHRE1 and XiaMac. Another non-limiting example includes the ILRE in use in conjunction with an MLV promoter and/or a tissue restricted ischaemic responsive promoter. These responsive elements are disclosed in WO99/15684.

Other examples of suitable tissue restricted promoters/enhancers are those which arc highly active in tumour cells such as a promoter/enhancer from a *MUC*1 gene, a *CEA* gene or a *5T4* antigen gene. The alpha-fetoprotein (AFP) promoter is also a tumour-specific promoter. One preferred promoter-enhancer combination is a human cytomegalovirus (hCMV) major immediate early (MIE) promoter/enhancer combination.

The term "promoter" is used in the normal sense of the art, e.g. an RNA polymerase binding site.

The promoter may be located in the retroviral 5' LTR to control the expression of a cDNA encoding an NOI, and/or gag-pol proteins.

Preferably the NOI and/or gag-pol proteins are capable of being expressed from the retrovirus genome such as from endogenous retroviral promoters in the long terminal repeat (LTR).

Preferably the NOI and/or gag-pol proteins are expressed from a heterologous promoter to which the heterologous gene or sequence, and/or codon optimised *gag-pol* sequence is operably linked.

Alternatively, the promoter may be an internal promoter.

Preferably the NOI is expressed from an internal promoter.

Vectors containing internal promoters have also been widely used to express multiple genes. An internal promoter makes it possible to exploit promoter/enhancer combinations other than those found in the viral LTR for driving gene expression. Multiple internal promoters can be included in a retroviral vector and it has proved possible to express at least three different cDNAs each from its own promoter (14). Internal ribosomal entry site (IRES) elements have also been used to allow translation of multiple coding regions from either a single mRNA or from fusion proteins that can then be expressed from an open reading frame.

The promoter of the present invention may be constitutively efficient, or may be tissue or temporally restricted in their activity.

Preferably the promoter is a constitutive promoter such as CMV.

Preferably the promoters of the present invention are tissue specific. That is, they are capable of driving transcription of a NOI or NOI(s) in one tissue while remaining largely "silent" in other tissue types.

The term "tissue specific" means a promoter which is not restricted in activity to a single tissue type but which nevertheless shows selectivity in that they may be active in one group of tissues and less active or silent in another group.

The level of expression of an NOI or NOIs under the control of a particular promoter may be modulated by manipulating the promoter region. For example, different domains within a promoter region may possess different gene regulatory activities. The roles of these different regions are typically assessed using vector constructs having different variants of the promoter with specific regions deleted (that is, deletion analysis). This approach may be used to identify, for example, the smallest region capable of conferring tissue specificity or the smallest region conferring hypoxia sensitivity.

A number of tissue specific promoters, described above, may be particularly advantageous. In most instances, these promoters may be isolated as convenient restriction digestion fragments suitable for cloning in a selected vector. Alternatively, promoter fragments may be isolated using the polymerase chain reaction. Cloning of the amplified fragments may be facilitated by incorporating restriction sites at the 5' end of the primers.

The NOI or NOIs may be under the expression control of an expression regulatory element, such as a promoter and enhancer.

Preferably the ischaemic responsive promoter is a tissue restricted ischaemic responsive promoter.

Preferably the tissue restricted ischaemic responsive promoter is a macrophage specific promoter restricted by repression.

Preferably the tissue restricted ischaemic responsive promoter is an endothelium specific promoter.

Preferably the regulated retroviral vector is an ILRE regulated retroviral vector.

Preferably the regulated retroviral vector is an ILRE regulated lentiviral vector.

Preferably the regulated retroviral vector is an autoregulated hypoxia responsive lentiviral vector.

Preferably the regulated retroviral vector is regulated by glucose concentration.

For example, the glucose-regulated proteins (grp's) such as grp78 and grp94 are highly conserved proteins known to be induced by glucose deprivation (15). The grp 78 gene is expressed at low levels in most normal healthy tissues under the influence of basal level promoter elements but has at least two critical "stress inducible regulatory elements" upstream of the TATA element (15 *ibid;* 16). Attachment to a truncated 632 base pair sequence of the 5'end of the grp78 promoter confers high inducibility to glucose deprivation on reporter genes *in vitro* (16 *ibid*). Furthermore, this promoter sequence in retroviral vectors was capable of driving a high level expression of a reporter gene in tumour cells in murine fibrosarcomas, particularly in central relatively ischaemic/fibrotic sites (16 *ibid).*

Preferably the regulated retroviral vector is a self-inactivating (SIN) vector.

By way of example, self-inactivating retroviral vectors have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive provirus (17; 18; 19; 20). However, any promoter(s) internal to the LTRs in such vectors will still be transcriptionally active. This strategy has been employed to eliminate effects of the enhancers and promoters in the viral LTRs on transcription from internally placed genes. Such effects include increased transcription (21) or suppression of transcription (22). This strategy can also be used to eliminate downstream transcription from the 3' LTR into genomic DNA (23). This is of particular concern in human gene therapy where it is of critical importance to prevent the adventitious activation of an endogenous oncogene.

As discussed above, replication-defective retroviral vectors are typically propagated, for example to prepare suitable titres of the retroviral vector for subsequent transduction, by using a combination of a packaging or helper cell line and the recombinant vector. That is to say, that the three packaging proteins can be provided *in trans.*

In general a "packaging cell line" contains one or more of the retroviral *gag, pol* and *env* genes. In the present invention it contains codon optimised *gag-pol* genes, and optionally an *env* gene. The packaging cell line produces the proteins required for packaging retroviral DNA but it cannot bring about encapsidation. Conventionally this has been achieved through lack of a *psi* region. However, when a recombinant vector carrying an NOI and a *psi* region is introduced into the packaging cell line, the helper proteins can package the psi-positive recombinant vector to produce the recombinant virus stock. This virus stock can be used to transduce cells to introduce the NOI into the genome of the target cells. Conventionally a *psi* packaging signal, called *psi* plus, has been used that contains additional sequences spanning from upstream of the splice donor to downstream of the *gag* start codon (24) since this has been shown to increase viral titres.

The recombinant virus whose genome lacks all genes required to make viral proteins can tranduce only once and cannot propagate. These viral vectors which are only capable of a single round of transduction of target cells are known as replication defective vectors. Hence, the NOI is introduced into the host/target cell genome without the generation of potentially harmful retrovirus. A summary of the available packaging lines is presented in Coffin *et al.,* 1997 (*ibid*).

The retroviral packaging cell line is preferably in the form of a transiently transfected cell line. Transient transfections may advantageously be used to measure levels of vector production when vectors are being developed. In this regard, transient transfection avoids the longer time required to generate stable vector-producing cell lines and may also be used if the vector or retroviral packaging components are toxic to cells. Components typically used to generate retroviral vectors include a plasmid encoding the gag-pol proteins, a plasmid encoding the env protein and a plasmid containing an NOI. Vector production involves transient transfection of one or more of these components into cells containing the other required components. If the vector encodes toxic genes or genes that interfere with the replication of the host cell, such as inhibitors of the cell cycle or genes that induce apotosis, it may be difficult to generate stable vector-producing cell lines, but transient transfection can be used to produce the vector before the cells die. Also, cell lines have been developed using transient transfection that produce vector titre levels that are comparable to the levels obtained from stable vector-producing cell lines (25).

Producer cells/packaging cells can be of any suitable cell type. Producer cells are generally mammalian cells but can be, for example, insect cells. A producer cell may be a packaging cell containing the virus structural genes, normally integrated into its genome into which the regulated retroviral vectors of the present invention are introduced. Alternatively the producer cell may be transfected with nucleic acid sequences encoding structural components, such as codon optimised *gag-pol* and *env* on one or more vectors such as plasmids, adenovirus vectors, herpes viral vectors or any method known to deliver functional DNA into target cells. The vectors are then introduced into the packaging cell.

As used herein, the term "producer cell" or "vector producing cell" refers to a cell which contains all the elements necessary for production of regulated retroviral vector particles and regulated retroviral delivery systems.

Preferably, the producer cell is obtainable from a stable producer cell line.

Preferably, the producer cell is obtainable from a derived stable producer cell line.

Preferably, the producer cell is obtainable from a derived producer cell line

As used herein, the term "derived producer cell line" is a transduced producer cell line which has been screened and selected for high expression of a marker gene. Such cell lines contain retroviral insertions in integration sites that support high level expression from the retroviral genome. The term "derived producer cell line" is used interchangeably with the term "derived stable producer cell line" and the term "stable producer cell line

Preferably the derived producer cell line includes but is not limited to a retroviral and/or a lentiviral producer cell.

Preferably the derived producer cell line is an HIV or EIAV producer cell line, more preferably an EIAV producer cell line.

Preferably the envelope protein sequences, and nucleocapsid sequences are all stably integrated in the producer and/or packaging cell. However, one or more of these sequences could also exist in episomal form and gene expression could occur from the episome.

As used herein, the term "packaging cell" refers to a cell which contains those elements necessary for production of infectious recombinant virus which are lacking in a recombinant viral vector. Typically, such packaging cells contain one or more vectors which are capable of expressing viral structural proteins (such as codon optimised *gag-pol* and *env*) but they do not contain a packaging signal.

The term "packaging signal" which is referred to interchangeably as "packaging sequence" or *"psi"* is used in reference to the non-coding, *cis*-acting sequence required for encapsidation of retroviral RNA strands during viral particle formation. In HIV-1, this sequence has been mapped to loci extending from upstream of the major splice donor site (SD) to at least the *gag* start codon.

Packaging cell lines suitable for use with the above-described vector constructs may be readily prepared (see also WO 92/05266), and utilised to create producer cell lines for the production of retroviral vector particles. As already mentioned, a summary of the available packaging lines is presented in "Retroviruses" (1997 Cold Spring Harbour Laboratory Press Eds: JM Coffin, SM Hughes, HE Varmus pp 449).

Also as discussed above, simple packaging cell lines, comprising a provirus in which the packaging signal has been deleted, have been found to lead to the rapid production of undesirable replication competent viruses through recombination. In order to improve safety, second generation cell lines have been produced wherein the 3'LTR of the provirus is deleted. In such cells, two recombinations would be necessary to produce a wild type virus. A further improvement involves the introduction of the *gag-pol* genes and the *env* gene on separate constructs so-called third generation packaging cell lines. These constructs are introduced sequentially to prevent recombination during transfection (26; 27).

Preferably, the packaging cell lines are second generation packaging cell lines.

Preferably, the packaging cell lines are third generation packaging cell lines.

In these split-construct, third generation cell lines, a further reduction in recombination may be achieved by "codon wobbling". This technique, based on the redundancy of the genetic code, aims to reduce homology between the separate constructs, for example between the regions of overlap in the *gag-pol* and *env* open reading frames.

The packaging cell lines are useful for providing the gene products necessary to encapsidate and provide a membrane protein for a high titre regulated retrovirus vector and regulated nucleic gene delivery vehicle production. When regulated retrovirus sequences are introduced into the packaging cell lines, such sequences are encapsidated with the nucleocapsid (*gag-pol*) proteins and these units then bud through the cell membrane to become surrounded in cell membrane and to contain the envelope protein produced in the packaging cell line. These infectious regulated retroviruses are useful as infectious units *per se* or as gene delivery vectors.

The packaging cell may be a cell cultured *in vitro* such as a tissue culture cell line. Suitable cell lines include but are not limited to mammalian cells such as murine fibroblast derived cell lines or human cell lines. Preferably the packaging cell line is a human cell line, such as for example: HEK293, 293-T, TE671, HT1080.

Alternatively, the packaging cell may be a cell derived from the individual to be treated such as a monocyte, macrophage, blood cell or fibroblast. The cell may be isolated from an individual and the packaging and vector components administered *ex vivo* followed by re-administration of the autologous packaging cells.

It is highly desirable to use high-titre virus preparations in both experimental and practical applications. Techniques for increasing viral titre include using a *psi* plus packaging signal as discussed above and concentration of viral stocks. In addition, the use of different envelope proteins, such as the G protein from vesicular-stomatitis virus has improved titres following concentration to 10⁹ per ml (28). However, typically the envelope protein will be chosen such that the viral particle will preferentially infect cells that are infected with the virus which it desired to treat. For example where an HIV vector is being used to treat HIV infection, the *env* protein used will be the HIV *env* protein.

The process of producing a retroviral vector in which the envelope protein is not the native envelope of the retrovirus is known as "pseudotyping". Certain envelope proteins, such as MLV envelope protein and vesicular stomatitis virus G (VSV-G) protein, pseudotype retroviruses very well. Pseudotyping is not a new phenomenon and examples may be found in WO-A-98/05759, WO-A-98/05754, WO-A-97/17457, WO-A-96/09400, WO-A-91/00047 and (29).

As used herein, the term "high titre" means an effective amount of a retroviral vector or particle which is capable of transducing a target site such as a cell.

As used herein, the term "effective amount" means an amount of a regulated retroviral or lentiviral vector or vector particle which is sufficient to induce expression of an NOI at a target site.

Preferably the titre is from at least 10⁶ retrovirus particles per ml, such as from 10⁶ to 10⁷ per ml, more preferably at least 10⁷ retrovirus particles per ml.

It is possible to manipulate the viral genome or the regulated retroviral vector nucleotide sequence, so that viral genes are replaced or supplemented with one or more NOIs which may be heterologous NOIs.

The term "heterologous" refers to a nucleic acid sequence or protein sequence linked to a nucleic acid or protein sequence which it is not naturally linked.

The term NOI (i.e. nucleotide sequence of interest) includes any suitable nucleotide sequence, which need not necessarily be a complete naturally occurring DNA sequence. Thus, the DNA sequence can be, for example, a synthetic DNA sequence, a recombinant DNA sequence (i.e. prepared by use of recombinant DNA techniques), a cDNA sequence or a partial genomic DNA sequence, including combinations thereof. The DNA sequence need not be a coding region. If it is a coding region, it need not be an entire coding region. In addition, the DNA sequence can be in a sense orientation or in an anti-sense orientation. Preferably, it is in a sense orientation. Preferably, the DNA is or comprises cDNA.

The NOI(s) may be any one or more of selection gene(s), marker gene(s) and therapeutic gene(s).

As used herein, the term "selection gene" refers to the use of a NOI which encodes a selectable marker which may have an enzymatic activity that confers resistance to an antibiotic or drug upon the cell in which the selectable marker is expressed.

Many different selectable markers have been used successfully in retroviral vectors. These are reviewed in "Retroviruses" (1997 Cold Spring Harbour Laboratory Press Eds: JM Coffin, SM Hughes, HE Varmus pp 444) and include, but are not limited to, the bacterial neomycin (neo) and hygromycin phosphotransferase genes which confer resistance to G418 and hygromycin respectively; a mutant mouse dihydrofolate reductase gene which confers resistance to methotrexate; the bacterial gpt gene which allows cells to grow in medium containing mycophenolic acid, xanthine and aminopterin; the bacterial *hisD* gene which allows cells to grow in medium without histidine but containing histidinol; the multidrug resistance gene (*mdr*) which confers resistance to a variety of drugs; and the bacterial genes which confer resistance to puromycin or phleomycin. All of these markers are dominant selectable and allow chemical selection of most cells expressing these genes. Other selectable markers are not dominant in that their use must be in conjunction with a cell line that lacks the relevant enzyme activity. Examples of non-dominant selectable markers include the thymidine kinase (tk) gene which is used in conjunction with tk cell lines.

Particularly preferred markers are blasticidin and neomycin, optionally operably linked to a thymidine kinase coding sequence typically under the transcriptional control of a strong viral promoter such the SV40 promoter.

Suitable NOI sequences include those that are of therapeutic and/or diagnostic application such as, but are not limited to: sequences encoding cytokines, chemokines, hormones, antibodies, engineered immunoglobulin-like molecules, a single chain antibody, fusion proteins, enzymes, immune co-stimulatory molecules, immunomodulatory molecules, anti-sense RNA, a transdominant negative mutant of a target protein, a toxin, a conditional toxin, an antigen, a tumour suppressor protein and growth factors, membrane proteins, vasoactive proteins and peptides, anti-viral proteins and ribozymes, and derivatives therof (such as with an associated reporter group).

When included, such coding sequences may be typically operatively linked to a suitable promoter, which may be a promoter driving expression of a ribozyme(s), or a different promoter or promoters, such as in one or more specific cell types.

Suitable NOIs in the treatment or prophylaxis of cancer include NOIs encoding proteins which: destroy the target cell (for example a ribosomal toxin), act as: tumour suppressors (such as wild-type p53); activators of anti-tumour immune mechanisms (such as cytokines, co-stimulatory molecules and immunoglobulins); inhibitors of angiogenesis; or which provide enhanced drug sensitivity (such as pro-drug activation enzymes); indirectly stimulate destruction of target cell by natural effector cells (for example, strong antigen to stimulate the immune system or convert a precursor substance to a toxic substance which destroys the target cell (for example a prodrug activating enzyme).

Examples of prodrugs include but are not limited to etoposide phosphate (used with alkaline phosphatase; 5-fluorocytosine (with cytosine deaminase); Doxorubin-N-p-hydroxyphenoxyacetamide (with Penicillin-V-Amidase); Para-N-bis (2-chloroethyl)aminobenzoyl glutamate (with Carboxypeptidase G2); Cephalosporin nitrogen mustard carbamates (with B-lactamase); SR4233 (with p450 reductase); Ganciclovir (with HSV thymidine kinase); mustard pro-drugs with nitroreductase and cyclophosphamide or ifosfamide (with cytochrome p450).

Suitable NOIs for use in the treatment or prevention of ischaemic heart disease include NOIs encoding plasminogen activators. Suitable NOIs for the treatment or prevention of rheumatoid arthritis or cerebral malaria include genes encoding anti-inflammatory proteins, antibodies directed against tumour necrosis factor (TNF) alpha, and anti-adhesion molecules (such as antibody molecules or receptors specific for adhesion molecules).

The expression products encoded by the NOIs may be proteins which are secreted from the cell. Alternatively the NOI expression products are not secreted and are active within the cell. In either event, it is preferred for the NOI expression product to demonstrate a bystander effect or a distant bystander effect; that is the production of the expression product in one cell leading to the killing of additional, related cells, either neighbouring or distant (e.g. metastatic), which possess a common phenotype. Encoded proteins could also destroy bystander tumour cells (for example with secreted antitumour antibody-ribosomal toxin fusion protein), indirectly stimulated destruction of bystander tumour cells (for example cytokines to stimulate the immune system or procoagulant proteins causing local vascular occlusion) or convert a precursor substance to a toxic substance which destroys bystander tumour cells (eg an enzyme which activates a prodrug to a diffusible drug). Also, the delivery of NOI(s) encoding antisense transcripts or ribozymes which interfere with expression of cellular genes for tumour persistence (for example against aberrant *myc* transcripts in Burkitts lymphoma or against *bcr-abl* transcripts in chronic myeloid leukemia. The use of combinations of such NOIs is also envisaged.

The NOI or NOIs may also comprise one or more cytokine-encoding NOIs. Suitable cytokines and growth factors include but are not limited to: ApoE, Apo-SAA, BDNF, Cardiotrophin-1, EGF, ENA-78, Eotaxin, Eotaxin-2, Exodus-2, FGF-acidic, FGF-basic, fibroblast growth factor-10 (30). FLT3 ligand, Fractalkine (CX3C), GDNF, G-CSF, GM-CSF, GF-β1, insulin, IFN-γ, IGF-I, IGF-II, IL-1α, IL-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein (30 *ibid),* M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MEP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNIL-1, TPO, VEGF, GCP-2, GRO/MGSA, GRO-β, GRO-γ, HCC1, 1-309.

The NOI or NOIs may be under the expression control of an expression regulatory element, such as a promoter and/or a promoter enhancer as known as "responsive elements" in the present disclosure.

When the regulated retroviral vector particles are used to transfer NOIs into cells which they transduce, such vector particles also designated "viral delivery systems" or "retroviral delivery systems". Viral vectors, including retroviral vectors, have been used to transfer NOIs efficiently by exploiting the viral transduction process. NOIs cloned into the retroviral genome can be delivered efficiently to cells susceptible to transduction by a retrovirus. Through other genetic manipulations, the replicative capacity of the retroviral genome can be destroyed. The vectors introduce new genetic material into a cell but are unable to replicate.

The regulated retroviral vector can be delivered by viral or non-viral techniques. Non-viral delivery systems include but are not limted to DNA transfection methods. Here, transfection includes a process using a non-viral vector to deliver a gene to a target mammalian cell.

Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (31), multivalent cations such as spermine, cationic lipids or polylysine, 1, 2,-bis (oleoyloxy)-3-(trimethylammonio) propane (DOTAP)-cholesterol complexes (32) and combinations thereof.

Viral delivery systems include but are not limited to adenovirus vector, an adeno-associated viral (AAV) vector, a herpes viral vector, a retroviral vector, a lentiviral vector, or a baculoviral vector. These viral delivery systems may be configured as a split-intron vector. A split intron vector is described in WO 99/15653.

Other examples of vectors include *ex vivo* delivery systems, which include but are not limited to DNA transfection methods such as electroporation, DNA biolistics, lipid-mediated transfection, compacted DNA-mediated transfection.

The vector may be a plasmid DNA vector. Alternatively, the vector may be a recombinant viral vector. Suitable recombinant viral vectors include adenovirus vectors, adeno-associated viral (AAV) vectors, Herpes-virus vectors, or retroviral vectors, lentiviral vectors or a combination of adenoviral and lentiviral vectors. In the case of viral vectors, gene delivery is mediated by viral infection of a target cell.

If the features of adenoviruses are combined with the genetic stability of retro/lentiviruses then essentially the adenovirus can be used to transduce target cells to become transient retroviral producer cells that could stably infect neighbouring cells.

The present disclosure also provides a pharmaceutical composition for treating an individual by gene therapy, wherein the composition comprises a therapeutically effective amount of a regulated retroviral vector according to the present invention. The pharmaceutical composition may be for human or animal usage. Typically, a physician will determine the actual dosage which will be most suitable for an individual subject and it will vary with the age, weight and response of the particular patient.

The composition may optionally comprise a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system).

Where appropriate, the pharmaceutical compositions can be administered by any one or more of: minipumps, inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

The present composition is believed to have a wide therapeutic applicability - depending on *inter alia* the selection of the one or more NOIs.

For example, it may be useful in the treatment of the disorders listed in WO-A-98/05635. For ease of reference, part of that list is now provided: cancer, inflammation or inflammatory disease, dermatological disorders, fever, cardiovascular effects, haemorrhage, coagulation and acute phase response, cachexia, anorexia, acute infection, HIV infection, shock states, graft-versus-host reactions, autoimmune disease, reperfusion injury, meningitis, migraine and aspirin-dependent anti-thrombosis; tumour growth, invasion and spread, angiogenesis, metastases, malignant, ascites and malignant pleural effusion; cerebral ischaemia, ischaemic heart disease, osteoarthritis, rheumatoid arthritis, osteoporosis, asthma, multiple sclerosis, neurodegeneration, Alzheimer's disease, atherosclerosis, stroke, vasculitis, Crohn's disease and ulcerative colitis; periodontitis, gingivitis; psoriasis, atopic dermatitis, chronic ulcers, epidermolysis bullosa; corneal ulceration, retinopathy and surgical wound healing; rhinitis, allergic conjunctivitis, eczema, anaphylaxis; restenosis, congestive heart failure, endometriosis, atherosclerosis or endosclerosis.

In addition, or in the alternative, it may be useful in the treatment of disorders listed in WO-A-98/07859. For ease of reference, part of that list is now provided: cytokine and cell proliferation/differentiation activity; immunosuppressant or immunostimulant activity (e.g. for treating immune deficiency, including infection with human immune deficiency virus; regulation of lymphocyte growth; treating cancer and many autoimmune diseases, and to prevent transplant rejection or induce tumour immunity); regulation of haematopoiesis, e.g. treatment of myeloid or lymphoid diseases; promoting growth of bone, cartilage, tendon, ligament and nerve tissue, e.g. for healing wounds, treatment of burns, ulcers and periodontal disease and neurodegeneration; inhibition or activation of follicle-stimulating hormone (modulation of fertility); chemotactic/chemokinetic activity (e.g. for mobilising specific cell types to sites of injury or infection); haemostatic and thrombolytic activity (e.g. for treating haemophilia and stroke); antiinflammatory activity (for treating e.g. septic shock or Crohn's disease); as antimicrobials; modulators of e.g. metabolism or behaviour; as analgesics; treating specific deficiency disorders; in treatment of e.g. psoriasis, in human or veterinary medicine.

In addition, or in the alternative, it may be useful in the treatment of disorders listed in WO-A-98/09985. For ease of reference, part of that list is now provided: macrophage inhibitory and/or T cell inhibitory activity and thus, anti-inflammatory activity; anti-immune activity, i.e. inhibitory effects against a cellular and/or humoral immune response, including a response not associated with inflammation; inhibit the ability of macrophages and T cells to adhere to extracellular matrix components and fibronectin, as well as up-regulated fas receptor expression in T cells; inhibit unwanted immune reaction and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery, bone marrow transplantation or other transplantation complications and/or side effects, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

The invention will now be further described by way of Examples. The Examples refer to the Figures. In the Figures:

### Description of the Figures

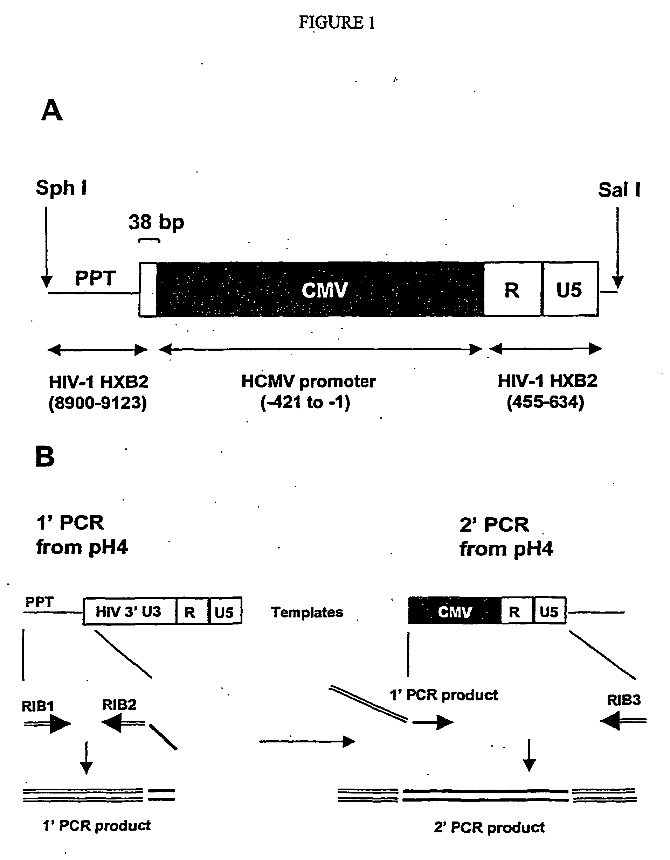

Figure 1 shows schematically how to create a suitable 3' LTR by PCR;

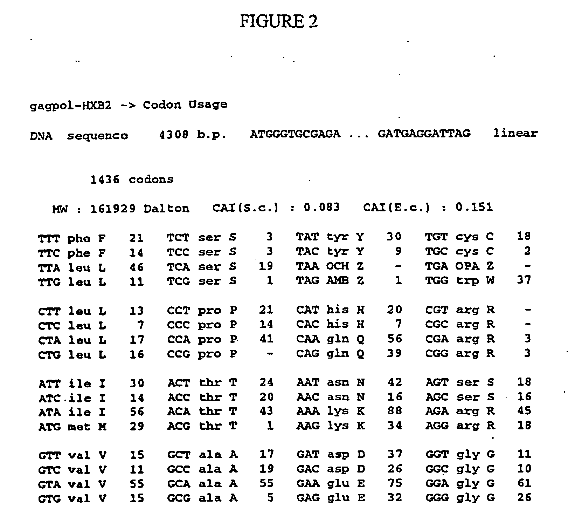

Figure 2 shows the codon usage table for wild type HIV *gag-pol* of strain. HXB2 (accession number: K03455);
Figure 3a shows the codon usage table of the codon optimised sequence designated gagpol-SYNgp. Figure 3b shows a comparative codon usage table;

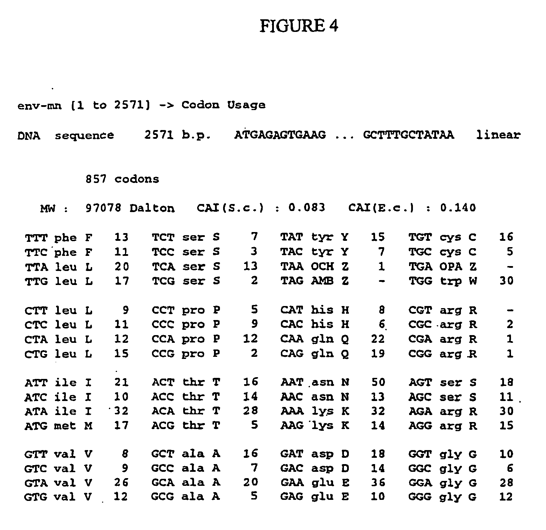

Figure 4 shows the codon usage table of the wild type HIV *env* called env-mn;

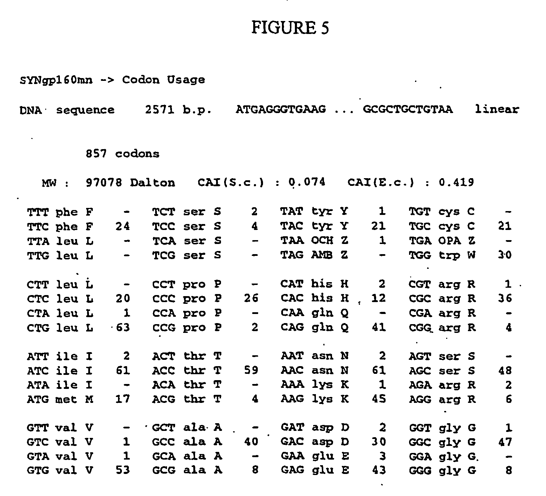

Figure 5 shows the codon usage table of the codon optimised sequence of HIV *env* designated SYNgp160mn;

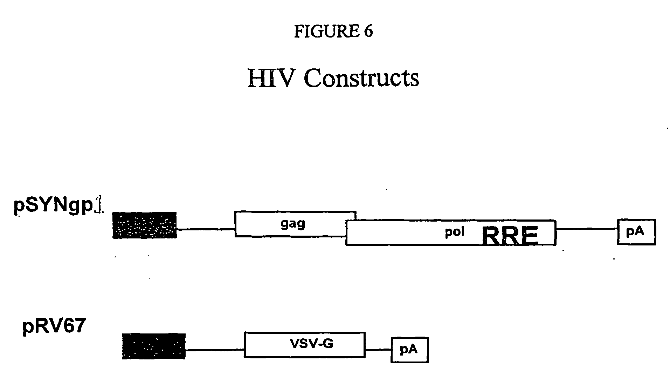

Figure 6 shows two plasmid constructs for use in the invention;

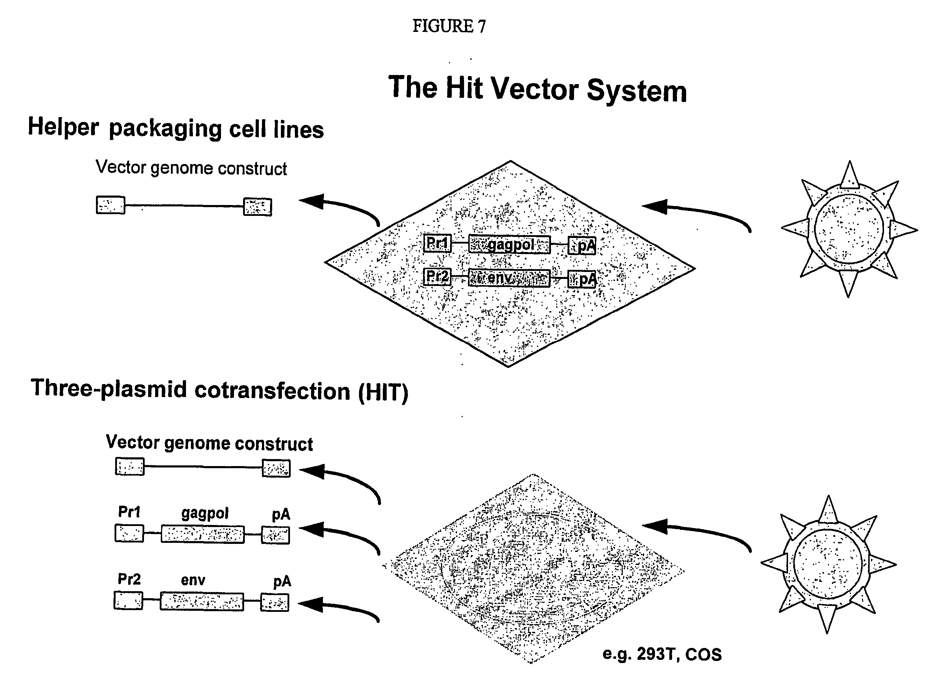

Figure 7 shows the principle behind two systems for producing retroviral vector particles;
Figure 8 shows a sequence comparison between the wild type HIV *gag-pol* sequence (pGP-RRE3) and the codon optimised *gag-pol* sequence (pSYNGP);
Figure 9 shows a sequence comparison between the wild type EIAV *gag-pol* sequence (WT) and the codon optimised *gag-pol* sequence (CO);
Figure 10 shows Rev independence of protein expression particle formation;
Figure 11 shows translation rates of wild-type (WT) and codon optimised *gag-pol;*
Figure 12 shows *gag-pol* mRNA levels in total and cytoplasmic fractions;
Figure 13 shows the effect of insertion of WT *gag* downstream of the codon optimised gene on RNA and protein levels;
Figure 14 shows the plasmids used to study the effect of HIV-1 *gag* on the codon optimised gene;
Figure 15 shows the effect on cytoplasmic RNA of insertion of HIV-1 *gag* upstream of the codon optimised gene;
Figure 16 shows the effect of Leptomycin B (LMB) on protein production;
Figure 17 shows the cytoplasmic RNA levels of the vector genomes;
Figure 18 shows transduction efficiency at MOI 1;
Figure 19 shows a schematic representation of pGP-RRE3;
Figure 20 shows a schematic representation of pSYNGP;
Figure 21 shows vector titres generated with different *gag-pol* constructs;
Figure 22 shows vector titres from the Rev/RRE (-) and (+) genomes;
Figure 23 shows vector titres from the pHS series of vector genomes;
Figure 24 shows vector titres for the pHS series of vector genomes in the presence or absence of Rev/RRE;
Figure 25 shows an analysis of *gag-pol* constructs;
Figure 26 shows a Western blot of 293T extracts;
Figure 27 is a schematic representation of pESYNGP;
Figure 28 is a schematic representation of LpESYNGP;
Figure 29 is a schematic representation of LpESYNGPRRE;
Figure 30 is a schematic representation of pESYNGPRRE;
Figure 31 is a schematic representation ofpONY4.0Z;
Figure 32 is a schematic representation of pONY8.0Z;
Figure 33 is a schematic representation of pONY8.1Z;
Figure 34 is a schematic representation ofpONY3.1;
Figure 35 is a schematic representation of pCIneoERev;
Figure 36 is a schematic representation of pESYNREV;
Figures 37 and 38 show the effect of different vector constructs on viral vector titres;
Figures 39 and 40 show the effect of different vector constructs on RT activity;
Figure 41 shows the effect of the 5' leader sequence on viral vector titre;
Figure 42 shows viral vector titres when using pONY8.1Z;
Figure 43 shows a comparison between the sequences of pONY3.1 and codon optimised pONY3.2OPTI in the first 372 nucelotides of *gag;*
Figure 44 is a schematic representation of pIRES1hygESYNGP;
Figure 45 and 46 show the results of experiments to confirm that codon optimised *gag-pol* can be used in the production of packaging and producer cell lines;
Figures 47 and 48 show the results of experiments which confirm that RNA from codon optimised *gag-pol* is packaged less efficiently than that from the wild-type gene;
Figure 49 shows the results of an experiment which confirms that expression from pESYNGP and pESDSYNGP are similar;
Figure 50 is a schematic representation of pESDSYNGP; and
Figure 51 shows the results of an experiment which confirms that the efficiency of encapsidating *gag-pol* RNA in PEV-17 cells and B-241 cells in similar.

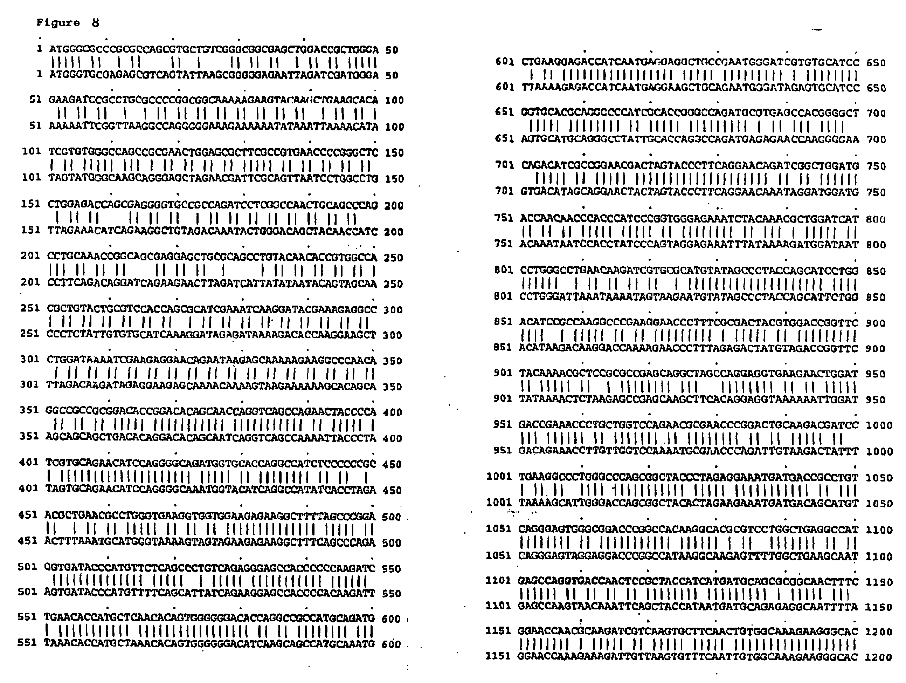

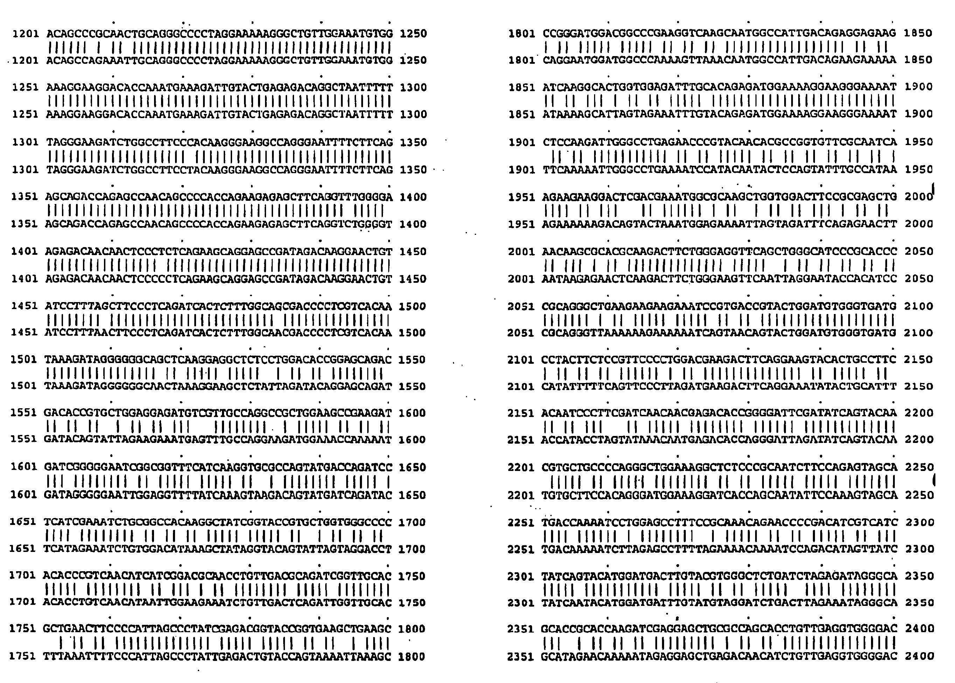

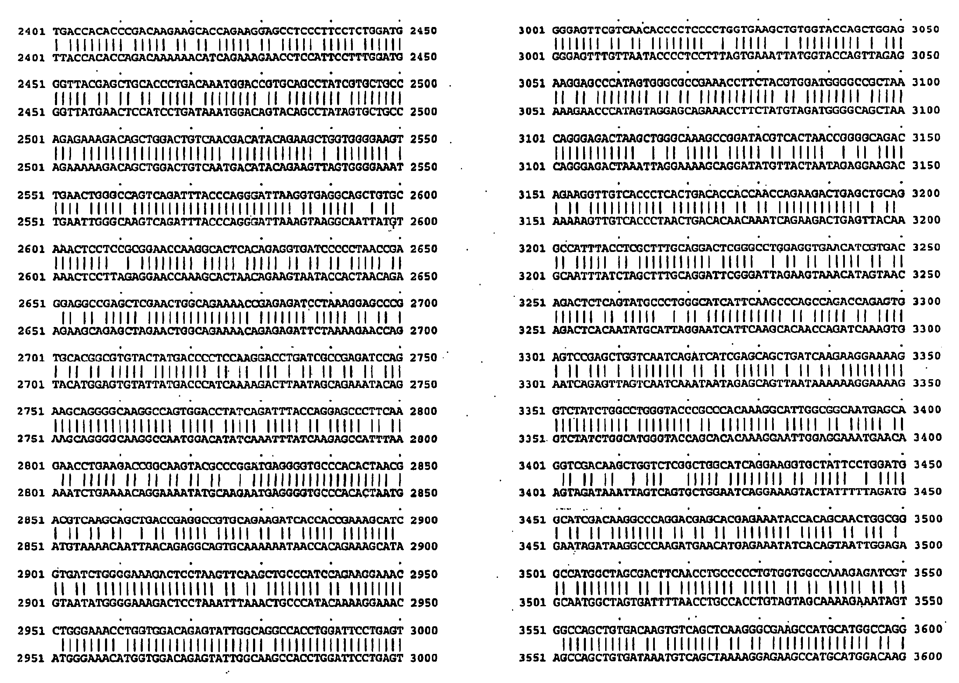

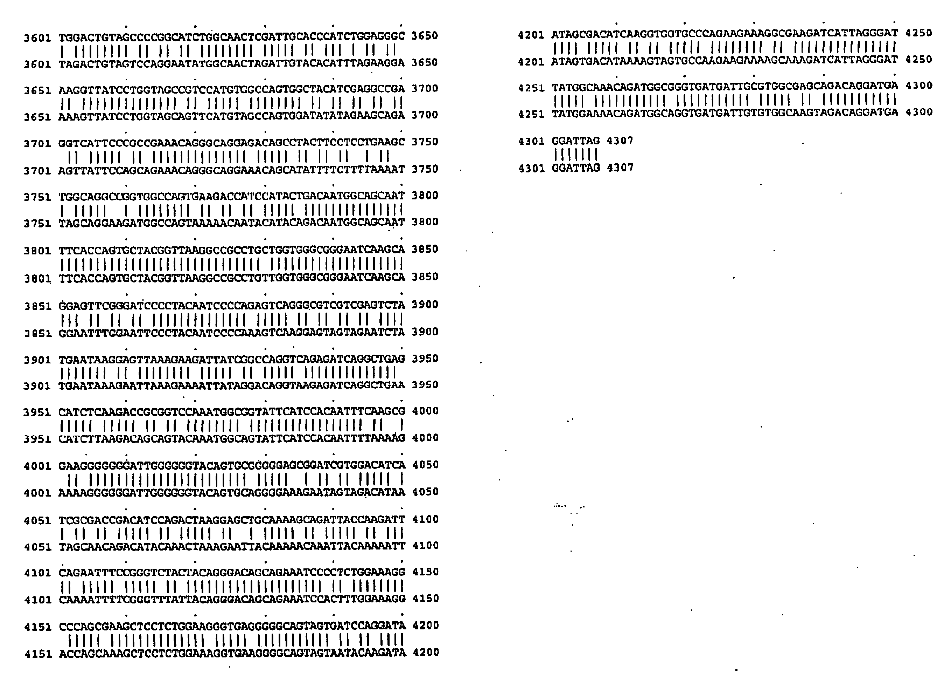

In more detail, Figure 8 shows a sequence comparison between the wild type HIV *gag-pol* sequence (pGP-RRE3) and the codon optimised *gag-pol* sequence (pSYNGP) wherein the upper sequence represents pSYNGP and the lower sequence represents pGP-RRE3.

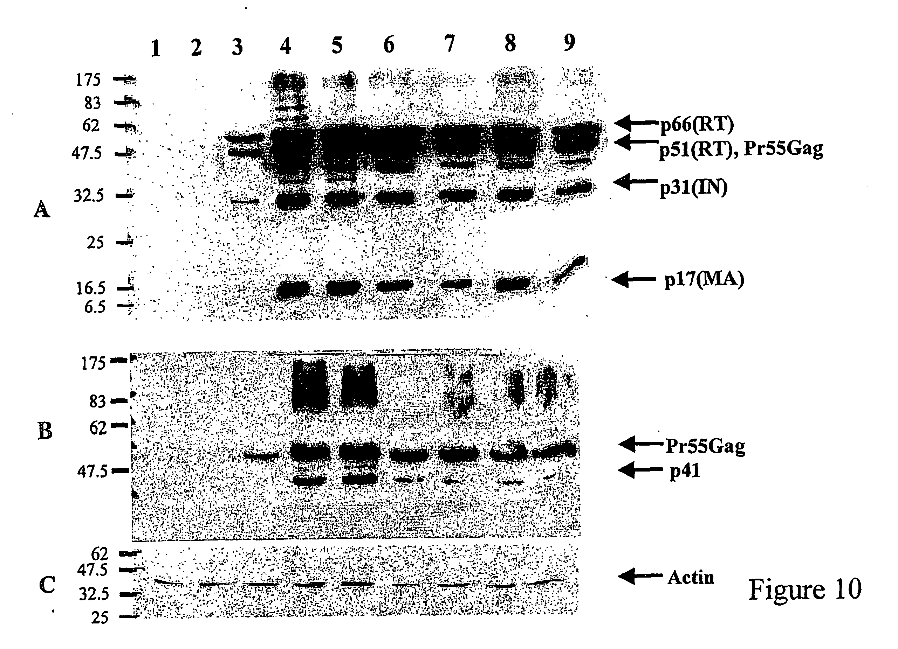

Figure 10 shows Rev independence of protein expression particle formation. 5µg of the *gag-pol* expression plasmids were transfected into 293T cells in the presence or absence of Rev (pCMV-Rev, 1µg) and protein levels were determined 48 hours post transfection in culture supernatants (A) and cell lysates (B). HIV-1 positive human serum was used to detect the gag-pol proteins. The blots were re-probed with an anti-actin antibody, as an internal control (C). The protein marker (New England Biolabs) sizes (in kDa) are shown on the side of the gel. Lanes: 1. Mock transfected 293T cells, 2. pGP-RRE3, 3. pGP-RRE3 + pCMV-Rev, 4. pSYNGP, 5. pSYNGP + pCMV-Rev, 6. pSYNGP-RRE, 7. pSYNGP-RRE +pCMV-Rev, 8. pSYNGP-ERR, 9. pSYNGP-ERR + pCMV-Rev.

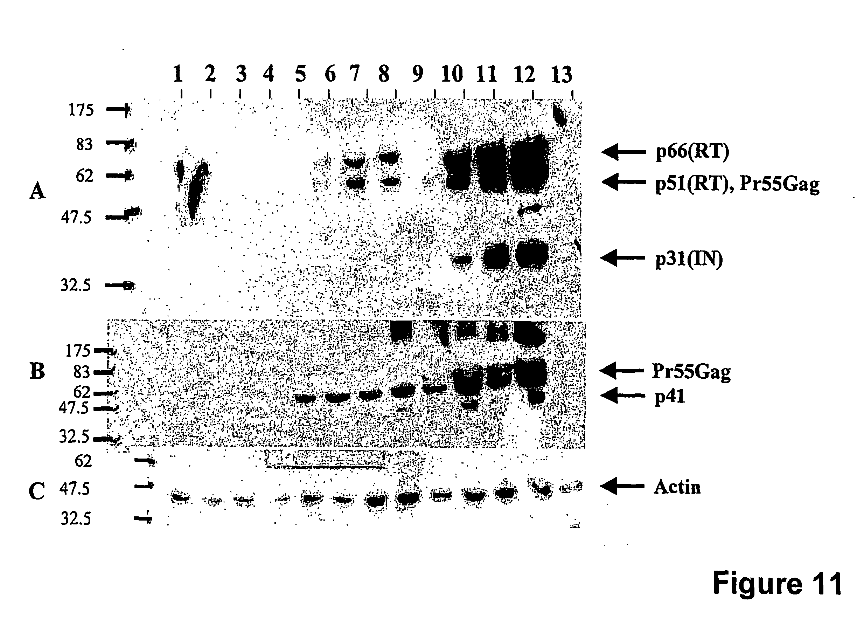

Figure 11 shows translation rates of WT and codon optimised *gag-pol.* 293T cells were transfected with 2µg pGP-RRE3 (+/- 1µg pCMV-Rev) or 2µg pSYNGP. Protein samples from culture supernatants (A) and cell extracts (B) were analysed by Western blotting 12, 25, 37 and 48 hours post-transfection. HIV-1 positive human serum was used to detect gag-pol proteins (A, B) and an anti-actin antibody was used as an internal control (C). The protein marker sizes are shown on the side of the gel (in kD). A Phosphorimager was used for quantification of the results. Lanes: 1. pGP-RRE3 12h, 2. pGP-RRE3 25h, 3. pGP-RRE3 37h, 4. pGP-RRE3 48h, 5. pGP-RRE3 + pCMV-Rev 12h, 6. pGP-RRE3 +pCMV-Rev 25h, 7. pGP-RRE3 + pCMV-Rev 37h, 8. pGP-RRE3 + pCMV-Rev 48h, 9. pSYNGP 12h, 10. pSYNGP 25h, 11. pSYNGP 37h, 12. pSYNGP 48h, 13. Mock transfected 293T cells.

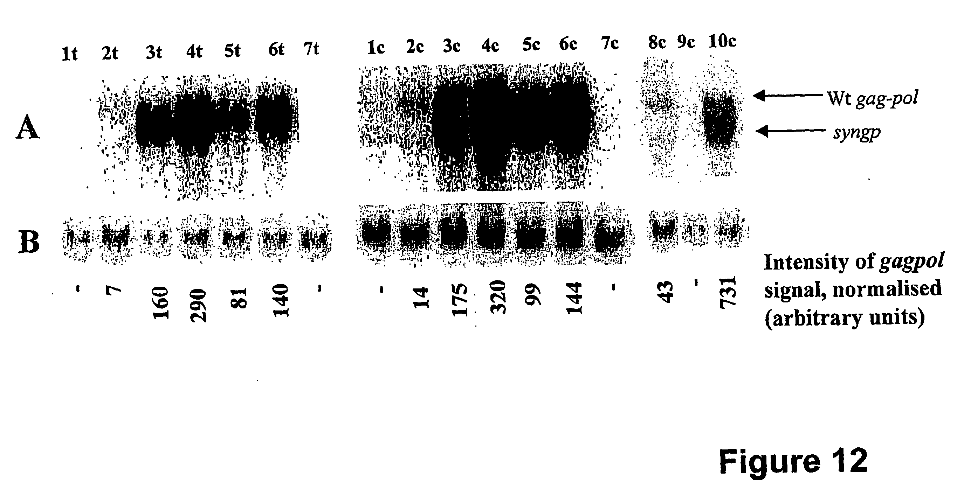

Figure 12 shows *gag-pol* mRNA levels in total and cytoplasmic fractions. Total and cytoplasmic RNA was extracted from 293T cells 36 hours after transfection with 5µg of the *gag-pol* expression plasmid (+/- 1µg pCMV-Rev) and mRNA levels were estimated by Northern blot analysis. A probe complementary to nt 1222-1503 of both the wild type and codon optimised gene was used. Panel A shows the band corresponding to the HIV-1 *gag-pol* The sizes of the mRNAs are 4.4 kb for the codon optimised and 6 kb for the wild type gene. Panel B shows the band corresponding to human ubiquitin (internal control for normalisation of results). Quantification was performed using a Phosphorimager. Lane numbering: c indicates cytoplasmic fraction and t indicates total RNA fraction. Lanes: 1. pGP-RRE3 , 2. pGP-RRE3 + pCMV-Rev, 3. pSYNGP, 4. pSYNGP + pCMV-Rev, 5. pSYNGP-RRE, 6. pSYNGP-RRE + pCMV-Rev, 7. Mock transfected 293T cells, 8. pGP-RRE3 + pCMV-Rev, 9. Mock transfected 293T cells, 10. pSYNGP.

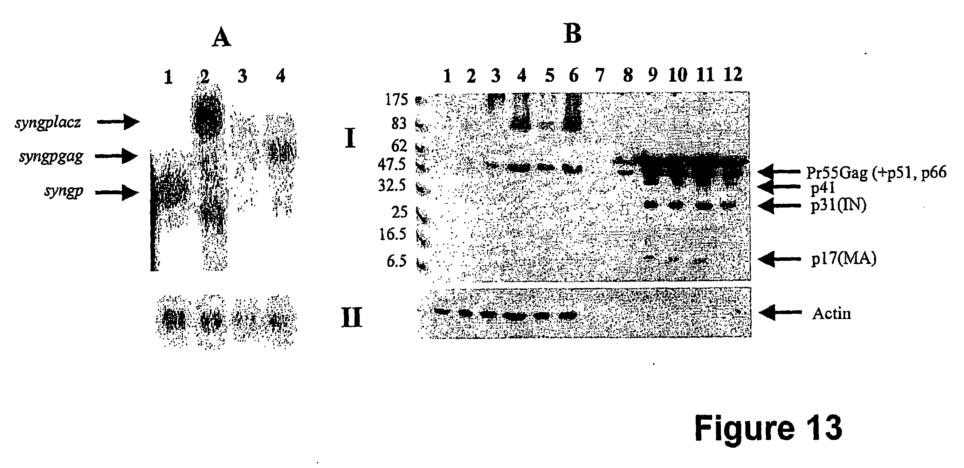

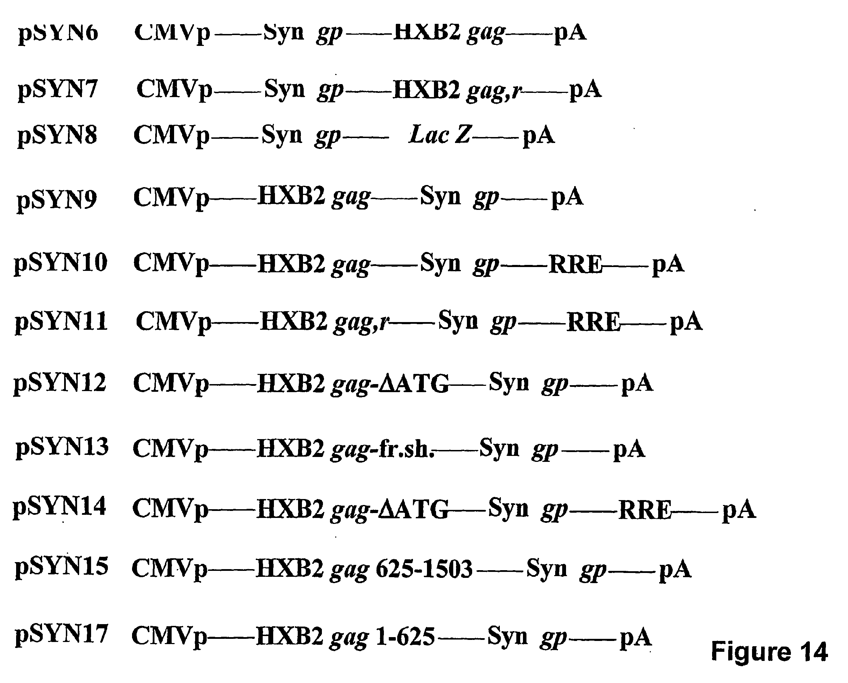

Figure 13 shows the effect of insertion of WT *gag* downstream of the codon optimised gene on RNA and protein levels. The wt *gag* sequence was inserted downstream of the codon optimised gene in both orientations *(Not*I site), resulting in plasmids pSYN6 (correct orientation, see Figure 14) and pSYN7 (reverse orientation, see Figure 14). The gene encoding for β-galactosidase (LacZ) was also inserted in the same site and the correct orientation (plasmid pSYN8, see Figure 14). 293T cells were transfected with 5 µg of each plasmid and 48 hours post transfection mRNA and protein levels were determined as previously described by means of Northern and Western blot analysis respectively.

Northern blot analysis in cytoplasmic RNA fractions. The blot was probed with a probe complementary to nt 1510-2290 of the codon optimised gene (I) and was re-probed with a probe specific for human ubiquitin (II). Lanes: 1. pSYNGP, 2. pSYN8, 3. pSYN7, 4. pSYN6

Western blot analysis: HIV-1 positive human serum was used to detect the gag-pol proteins (I) and an anti-actin antibody was used as an internal control (II). Lanes: Cell lysates: 1. Mock transfected 293T cells, 2. pGP-RRE3 + pCMV-Rev, 3. pSYNGP, 4. pSYN6, 5. pSYN7, 6. pSYN8. Supernatants: 7. Mock transfected 293T cells, 8. pGP-RRE3 + pCMV-Rev, 9. pSYNGP, 10. pSYN6, 11. pSYN7, 12. pSYN8. The protein marker (New England Biolabs) sizes are shown on the side of the gel.

Figure 14 shows the plasmids used to study the effect of HIV-1 *gag* on the codon optimised gene. The backbone for all constructs was pCI-Neo. Syn gp: The codon optimised HIV-1 *gag-pol* gene. HXB2 *gag:* The wild type HIV-1 *gag* gene. HXB2 *gag*,r: The wild type HIV-1 *gag* gene in the reverse orientation. HXB2 *gag*ΔATG: The wild type HIV-1 *gag* gene without the *gag* ATG. HXB2 *gag*-fr.sh.: The wild type HIV-1 *gag* gene with a frameshift mutation. HXB2 *gag* 625-1503: Nucleotides 625-1503 of the wild type HIV-1 *gag* gene. HXB2 *gag* 1-625: Nucleotides 1-625 of the wild type HIV-1 *gag* gene.

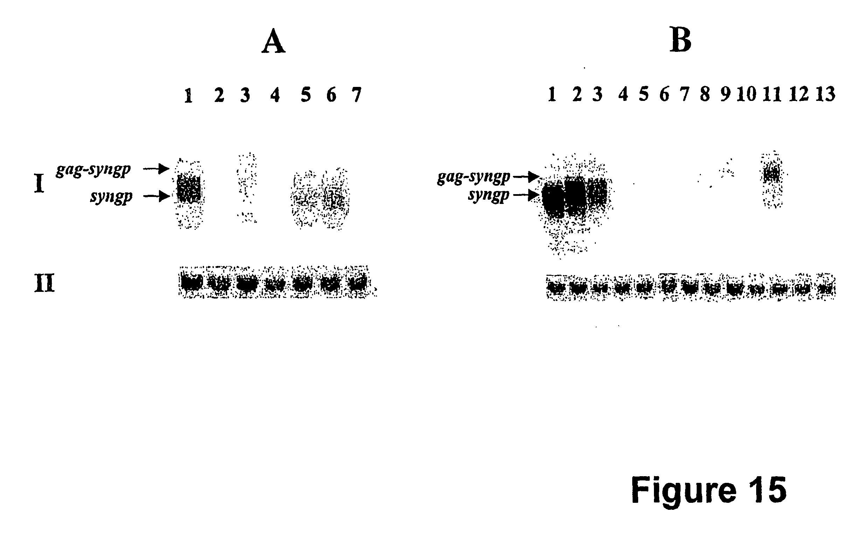

Figure 15 shows the effect on cytoplasmic RNA of insertion of HIV-1 *gag* upstream of the codon optimised gene. Cytoplasmic RNA was extracted 48 hours post transfection of 293T cells (5 µg of each pSYN plasmid was used and 1 µg of pCMV-Rev was co-transfected in some cases). The probe that was used was designed to be complementary to nt 1510-2290 of the codon optimised gene (I). A probe specific for human ubiquitin was used as an internal control (II).
Lanes: 1. pSYNGP , 2. pSYN9, 3. pSYN10, 4. pSYN10 + pCMV-Rev, 5. pSYN11, 6. pSYN11 + pCMV-Rev, 7. pCMV-Rev.
Lanes: 1. pSYNGP , 2. pSYNGP-RRE, 3. pSYNGP-RRE + pCMV-Rev, 4. pSYN12, 5. pSYN14, 6. pSYN14 + pCMV-Rev, 7. pSYN13, 8. pSYN15, 9. pSYN17, 10. pGP-RRE3, 11. pSYN6, 12. pSYN9, 13. pCMV-Rev.

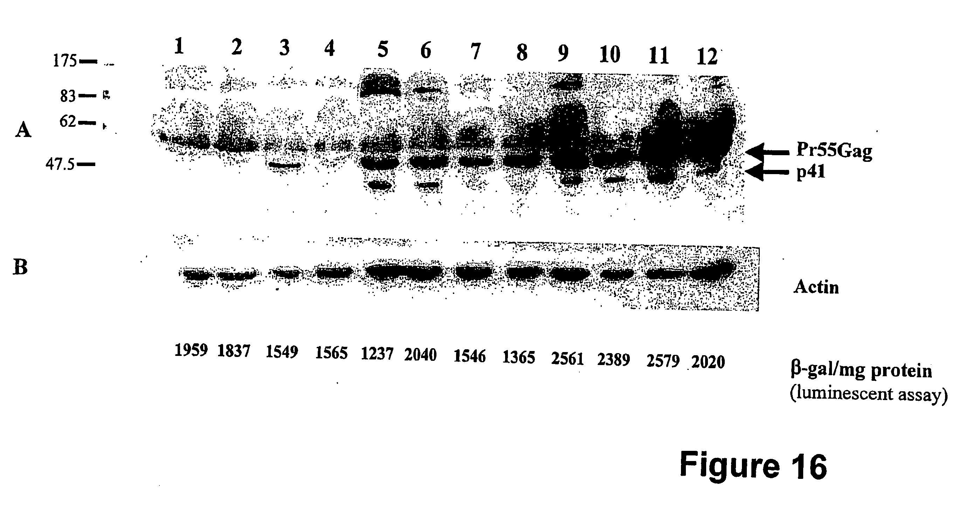

Figure 16 shows the effect of LMB on protein production. 293T cells were transfected with 1µg pCMV-Rev and 3µg of pGP-RRE3/pSYNGP/pSYNGP-RRE (+/- 1µg pCMV-Rev). Transfections were done in duplicate. 5 hours post transfection the medium was replaced with fresh medium in the first set and with fresh medium containing 7.5 nM LMB in the second. 20 hours later the cells were lysed and protein production was estimated by Western blot analysis. HIV-1 positive human serum was used to detect the gag-pol proteins (A) and an anti-actin antibody was used as an internal control (B). Lanes: 1. pGP-RRE3, 2. pGP-RRE3 + LMB, 3. pGP-RRE3 + pCMV-Rev, 4. pGP-RRE3 + pCMV-Rev + LMB, 5. pSYNGP, 6. pSYNGP + LMB, 7. pSYNGP + pCMV-Rev, 8. pSYNGP + pCMV-Rev + LMB, 9. pSYNGP-RRE, 10. pSYNGP-RRE + LMB, 11. pSYNGP-RRE + pCMV-Rev, 12. pSYNGP-RRE + pCMV-Rev + LMB.

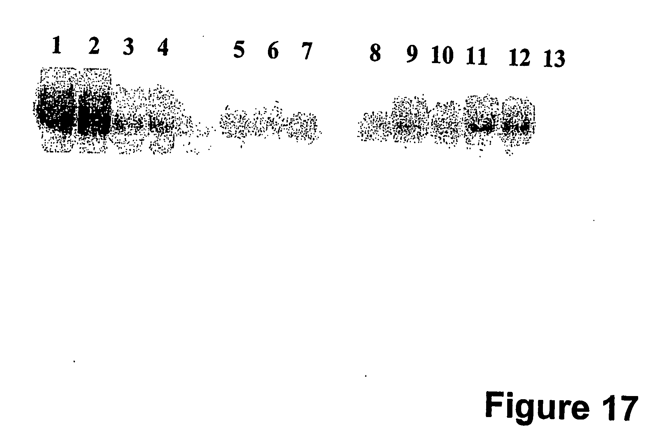

Figure 17 shows the cytoplasmic RNA levels of the vector genomes. 293T cells were transfected with 10 µg of each vector genome. Cytoplasmic RNA was extracted 48 hours post transfection. 20 µg of RNA were used from each sample for Northern blot analysis. The 700bp probe was designed to hybridise to all vector genome RNAs (see Materials and Methods). Lanes: 1. pH6nZ, 2. pH6nZ + pCMV-Rev, 3. pH6.1nZ, 4. pH6.1nZ + pCMV-Rev, 5. pHS1nZ, 6. pHS2nZ, 7. pHS3nZ, 8. pHS4nZ, 9. pHS5nZ, 10. pHS6nZ, 11. pHS7nZ, 12. pHS8nZ, 13. pCMV-Rev.

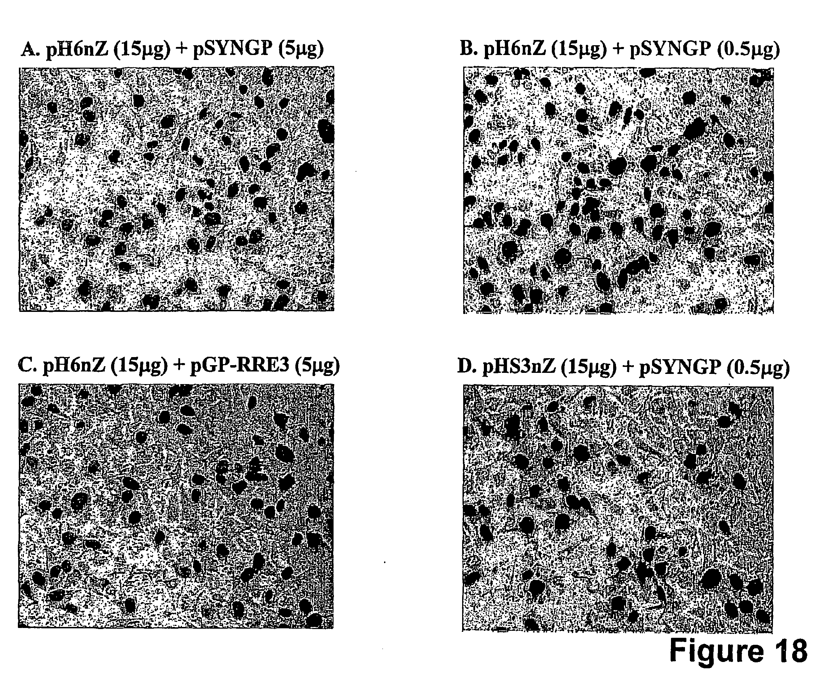

Figure 18 shows transduction efficiency at MOI 1. Viral stocks were generated by co-transfection of each *gag-pol* expression plasmid (5 or 0.5 µg), 15 µg pH6nZ or pHS3nZ (vector genome plasmid) and 5 µg pHCMVG (VSV envelope expression plasmid) on 293T cells. Virus was concentrated as previously described (45) and transduction efficiency was determined at m.o.i.'s 0.01-1 on HT1080 cells. There was a linear correlation of transduction efficiency and m.o.i. in all cases. An indicative picture at m.o.i. 1 is shown here. Transduction efficiency was >80% with either genome, either *gag-pol* and either high or low amounts of pSYNGP. Titres before concentration (I.U./ml): on 293T cells: A. 6.6x10⁵, B. 7.6x10⁵, C. 9.2x10⁵, D. 1.5x10⁵, on HT1080 cells: A. 6.0x10⁴, B. 9.9x10⁴, C. 8.0x10⁴, D. 2.9x10⁴. Titres after concentration (I.U./ml) on HT1080 cells: A. 6.0x10⁵, B. 2.0x10⁶, C. 1.4x10⁶, D. 2.0x10⁵.

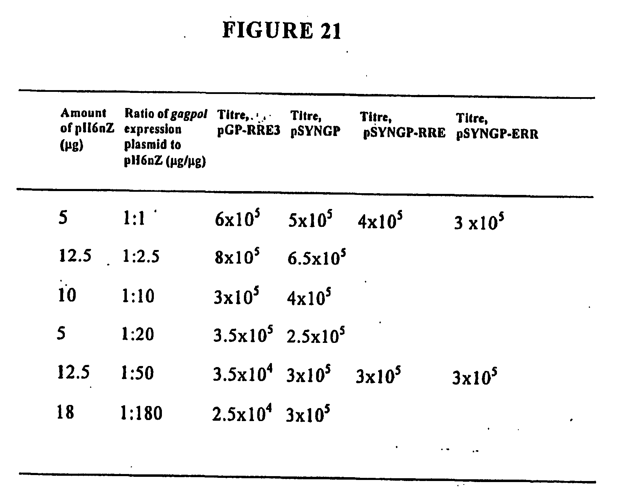

Figure 21 shows vector titers obtained with differed *gag-pol* constructs. Viral stocks were generated by co-transfection of each *gag-pol* expression plasmid, pH6nZ (vector genome plasmid) and pHCMVG (VSV envelope expression plasmid, 2.5µg for each transfection) on 293T cells. Titres (I.U./ml of virs stock) were measured on 293T cells by counting the number of blue colonies following X-Gal staining 48 hours after transduction. Experiments were performed at least twice and the variation between experiments was less than 15%.

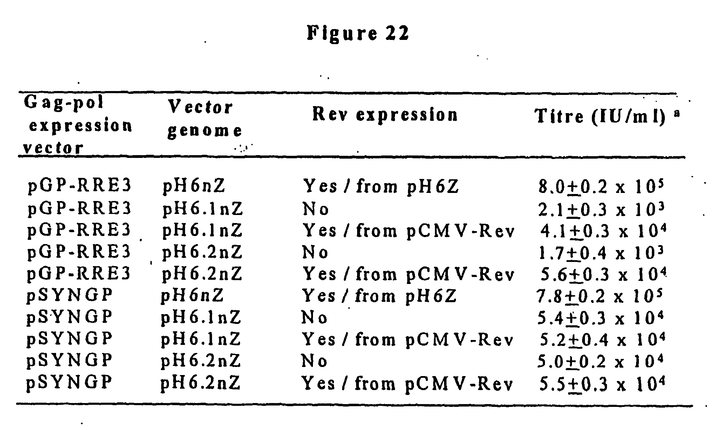

Figure 22 shows vector titres from the Rev/RRE (-) and (+) genomes: The retroviral vectors were generated as described in the Examples. Titres (I.U./ml of viral stock + SD) were determined in 293T cells.

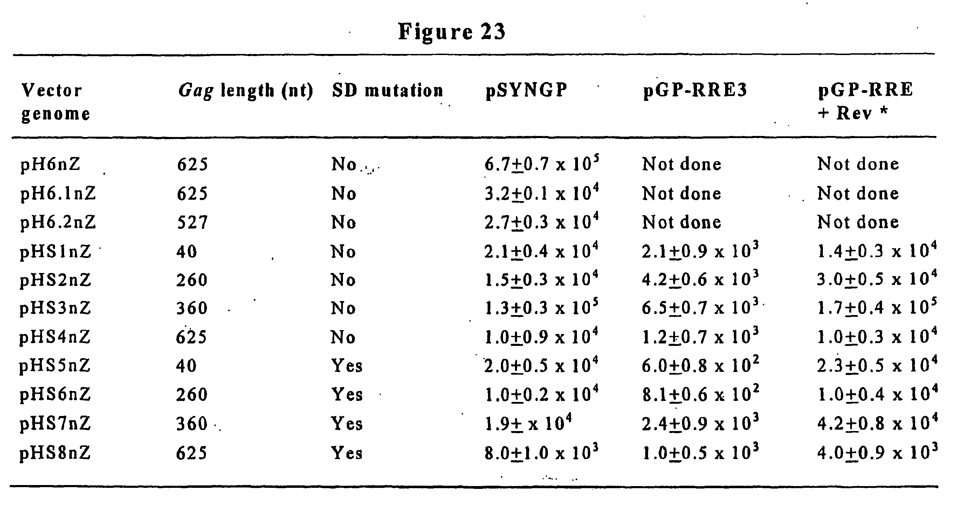

Figure 23 shows vector titres from the pHS series of vector genomes. The retroviral vector was generated as described in the Examples. Titres (I.U./ml of viral stock + SD) were determined in 293T cells. Rev is provided from pCMV-Rev. Note that pH6nZ expresses Rev and contains the RRE. None of the other genomes express Rev or contain the RRE. Expression from pSYNGP is Rev independent, whereas it is Rev dependent for pGP-RRE3.

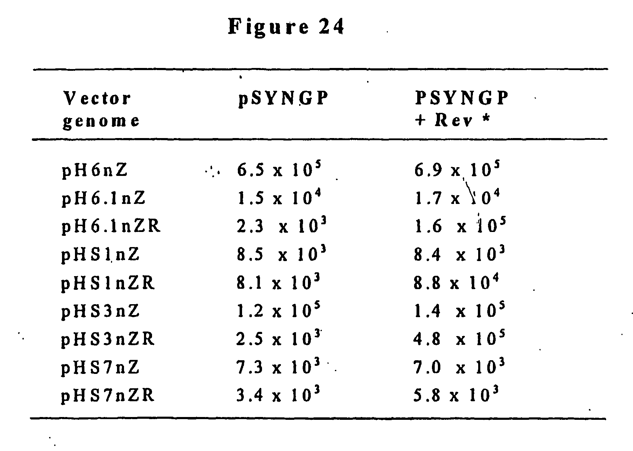

Figure 24 shows vector titres for the pHS series of vector genomes in the presence or absence of Rev/RRE. The retroviral vector was generated as described in the Examples. 5 µg of vector genome, 5 µg of pSYNGP and 2.5 µg of pHCMVG were used and titres (I.U./ml) were determined in 293T cells. Experiments were performed at least twice and the variation between experiments was less than 15%. Rev is provided from pCMV-Rev (1 µg). Note that pH6nZ expresses Rev and contains the RRE. None of the pHS genomes expresses Rev and only pHS1nZR, pHS3nZR, pHS7nZR and pH6.1nZR contain the RRE. gag-pol expression from pSYNGP is Rev independent.

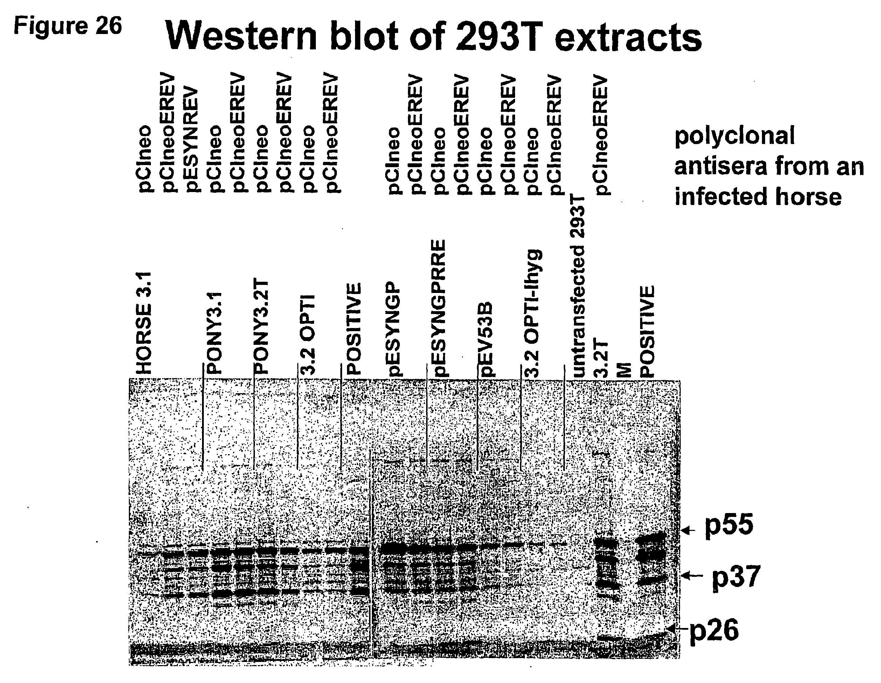

Figure 26 shows a Western blot of 293T extracts wherein 30:g of total cellular protein was separated by SDS/Page electrophoresis, transferred to nitro-cellulose and probed with anti EIAV antibodies. The secondary antibody was anti-Horse HRP (Sigma).

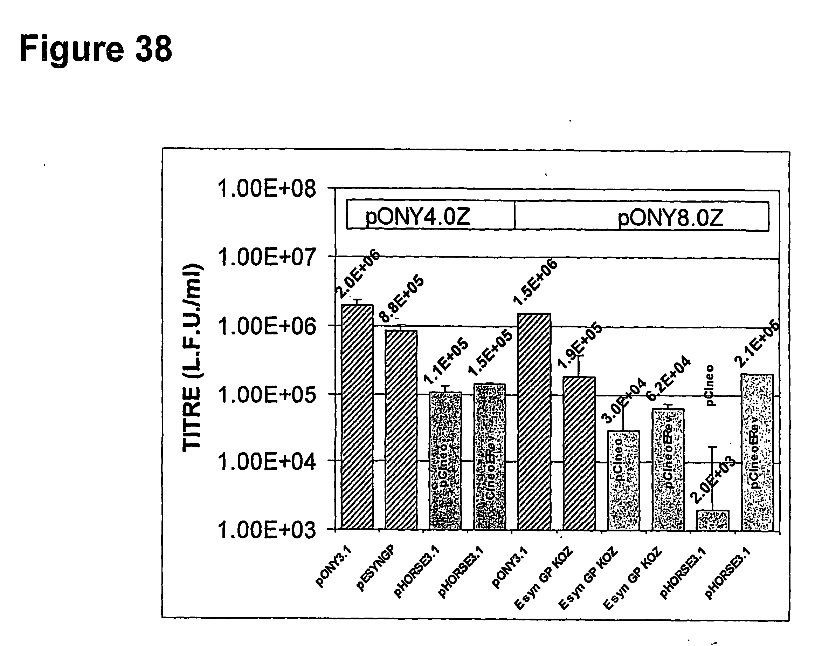

In Figure 38 the titres are shown in lacZ forming units (L.F.U.)/ml. The vectors used are indicated in boxes above the bars.
For ease of reference, we also set out the sequences listed in the accompanying
Sequence Listing:
SEQ ID NO:1 shows the sequence of the wild-type *gag-pol* sequence for the strain HXB2 (accession no. K03455);
SEQ ID NO:2 shows the sequence of pSYNGP;
SEQ ID NO:3 shows the sequence of the Envelope gene for HIV-1 MN (Genbank accession no. M17449);
SEQ ID NO:4 shows the sequence of SYNgp-160mn - codon optimised env sequence;
SEQ ID NO: 5 shows the sequence of pESYNGP;
SEQ ID NO:6 shows the sequence of LpESYNGP;
SEQ ID NO:7 shows the sequence of pESYNGPRRE;
SEQ ID NO:8 shows the sequence of LpESYNGPRRE;
SEQ ID NO:9 shows the sequence of pONY4.0Z;
SEQ ID NO:10 shows the sequence of pONY8.0Z;
SEQ ID NO:11 shows the sequence of pONY8.1Z;
SEQ ID NO:12 shows the sequence of pONY3. I;
SEQ ID NO:13 shows the sequence of pCIneoERev;
SEQ ID NO:14 shows the sequence of pESYNREV;
SEQ ID NO:15 shows the sequence of codon optimised HIV *gag-pol;*
SEQ ID NO:16 shows the sequence of codon optimised EIAV *gag-pol;*
SEQ ID NO:17 shows the sequence of pIRES1hygESYNGP;
SEQ ID NO:18 shows the sequence of pESDSYNGP; and
SEQ ID NO:19 shows the sequence of pONY8.3G FB29(-).

### Example 1 - HIV

### Cell lines

293T cells (33) and HeLa cells (34) were maintained in Dubecco's modified Eagle's medium containing 10% (v/v) fetal calf serum and supplemented with L-glutamine and antibiotics (penicillin-streptomycin). 293T cells were obtained from D. Baltimore (Rockefeller University).

### HIV-1 proviral clones

Proviral clones pWI3 (35) and pNL4-3 (36) were used.

### Construction of a Packaging System

In one of the present examples, a modified codon optimised HIV *env* sequence is used (SEQ I.D. No. 4). The corresponding *env* expression plasmid is designated pSYNgp160mn. The modified sequence contains extra motifs not used by (37). The extra sequences were taken from the HIV *env* sequence of strain MN and codon optimised. Any similar modification of the nucleic acid sequence would function similarly as long as it used codons corresponding to abundant tRNAs (38).

### Codon optimised HIV-1 gag-pol gene

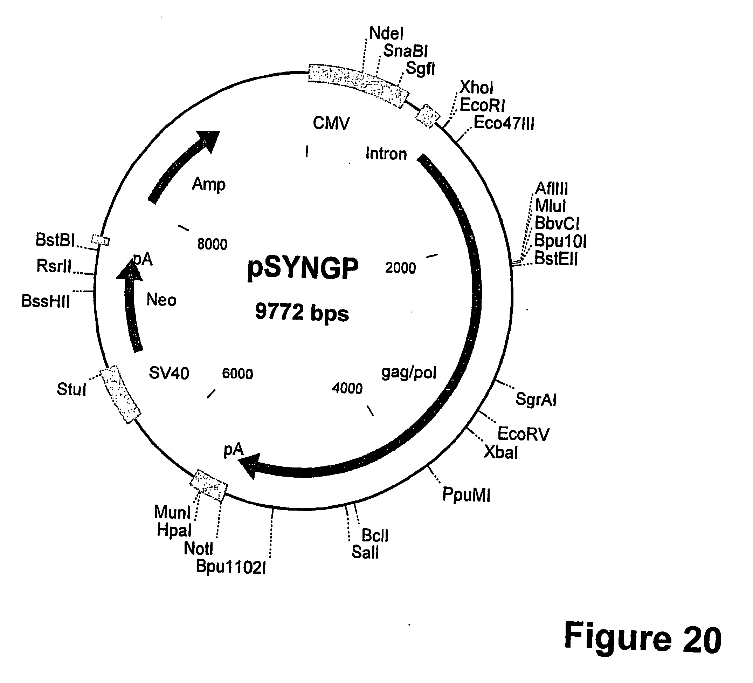

A codon optimised *gag-pol* gene, shown from nt 1108 to 5414 of SEQ ID NO: 2 was constructed by annealing a series of short overlapping oligonucleotides (approximately 30-40mers with 25% overlap, i.e. approximately 9 nucleotides). Oligonucleotides were purchased from R&D SYSTEMS (R&D Systems Europe Ltd, 4-10 The Quadrant, Barton Lane, Abingdon, OX14 3YS, UK). Codon optimisation was performed using the sequence of HXB-2 strain (AC: K03455) (39). The Kozak consensus sequence for optimal translation initiation (40) was also included. A fragment from base 1222 from the beginning of *gag* until the end of *gag* (1503) was not optimised in order to maintain the frameshift site and the overlap between the *gag* and *pol* reading frames. This was from clone pNL4-3. (When referring to base numbers within the *gag-pol* gene base 1 is the A of the *gag* ATG, which corresponds to base 790 from the beginning of the HXB2 sequence. When referring to sequences outside the *gag-pol* then the numbers refer to bases from the beginning of the HXB2 sequence, where base 1 corresponds to the beginning of the 5' LTR). Some deviations from optimisation were made in order to introduce convenient restriction sites. The final codon usage is shown in Figure 3b, which now resembles that of highly expressed human genes and is quite different from that of the wild type HIV-1 *gag-pol.* The gene was cloned into the mammalian expression vector pCIneo (Promega) in the *Eco*RI-*Not*I sites. The resulting plasmid was named pSYNGP (Figure 20, SEQ ID No 2). Sequencing of the gene in both strands verified the absence of any mistakes. A sequence comparison between the codon optimised and wild type HIV *gag-pol* sequence is shown in Figure 8.

### Rev/RRE constructs

The HIV-1 RRE sequence (bases 7769-8021 of the HXB2 sequence) was amplified by PCR from pWI3 proviral clone with primers bearing the *Not*I restriction site and was subsequently cloned into the *Not*I site of pSYNGP. The resulting plasmids were named pSYNGP-RRE (RRE in the correct orientation) and pSYNGP-ERR (RRE in the reverse orientation).

### Pseudotyped viral particles

In one form of the packaging system a synthetic *gag-pol* cassette is coexpressed with a heterologous envelope coding sequence. This could be for example VSV-G (44, 45), amphotropic MLV env (46, 47) or any other protein that would be incorporated into the HIV or EIAV particle (48). This includes molecules capable of targeting the vector to specific tissues.

### HIV-1 Vector genome constructs

pH6nZ is derived from pH4Z (49) by the addition of a single nucleotide to place an extra guanine residue that was missing from pH4Z at the 5' end of the vector genome transcript to optimise reverse transcription. In addition the gene coding for β-galactosidase (LacZ) was replaced by a gene encoding for a nuclear localising β-galactosidase. (We are grateful to Enca Martin-Rendon and Said Ismail for providing pH6nZ). In order to construct Rev(-) genome constructs the following modifications were made : a) A 1.8 kb *Pst*I *- Pst*I fragment was removed from pH6nZ, resulting in plasmid pH6.1nZ and b) an *Eco*NI (filled) - *Sph*I fragment was substituted with a *Spe*I (filled) - *Sph*I fragment from the same plasmid (pH6nZ), resulting in plasmid pH6.2nZ. In both cases sequences within *gag* (nt 1-625) were retained, as they have been shown to play a role in packaging (93). *Rev,* RRE and any other residual *env* sequences were removed. pH6.2nZ further contains the *env* splice acceptor, whereas pH6.1nZ does not.

A series of vectors encompassing further *gag* deletions plus or minus a mutant major splice donor (SD) (GT to CA mutation) were also derived from pH6Z. These were made by PCR with primers bearing a *Nar*I (5' primers) and an *Spe*I (3' primers) site. The PCR products were inserted into pH6Z at the *Nar*I - *Spe*I sites. The resulting vectors were named pHS1nZ (containing HIV-1 sequences up to *gag* 40), pHS2nZ (containing HIV-1 sequences up to *gag* 260), pHS3nZ (containing HIV-1 sequences up to *gag* 360), pHS4nZ (containing HIV-1 sequences up to *gag* 625), pHS5nZ (same as pHS1nZ but with a mutant SD), pHS6nZ (same as pHS2nZ but with a mutant SD), pHS7nZ (same as pHS3nZ but with a mutant SD) and pHS8nZ (same as pHS4nZ but with a mutant SD).

In addition, the RRE sequence (nt 7769-8021 of the HXB2 sequence) was inserted in the *Spe*I (filled) site of pH6.1nZ, pHS1nZ, pHS3nZ and pHS7nZ resulting in plasmids pH6.1nZR, pHS1nZR, pHS3nZR and pHS7nZR respectively.

Other modifications to the genome have been made including the generation of a SIN vector (by deletion of part of the 3' U3), the replacement of the LTRs with those from MLV or replacement of part of the 3'U3 with the MLV U3 region.

### Transient transfections, transductions and determination of viral titres

These were performed as previously described (49, 50). Briefly, 293T cells were seeded on 6cm dishes and 24 hours later they were transiently transfected by overnight calcium phosphate treatment. The medium was replaced 12 hours post-transfection and unless otherwise stated supernatants were harvested 48 hours post-transfection, filtered (through 0.22 or 0.45 µm filters) and titered by transduction of 293T cells. For this reason supernatant at appropriate dilutions of the original stock was added to 293T cells (plated onto 6 or 12 well plates 24 hours prior to transduction). 8 µg/ml Polybrene (Sigma) was added to each well and 48 hours post transduction viral titres were determined by X-gal staining.

### Luminescent β-gatactosidase (β-gal) assays

These were performed on total cell extracts using a luminescent β-gal reporter system (CLONTECH). Untransfected 293T cells were used as negative control and 293T cells transfected with pCMV-β-gal (CLONTECH) were used as positive control.

### RNA analysis

Total or cytoplasmic RNA was extracted from 293T cells by using the RNeasy mini kit (QUIAGEN) 36-48 hours post-transfection. 5-10 µg of RNA was subjected to Northern blot analysis as previously described (51). Correct fractionation was verified by staining of the agarose gel. A probe complementary to bases 1222-1503 of the *gag-pol* gene was amplified by PCR from HIV-1 pNL4-3 proviral clone and was used to detect both the codon optimised and wild type *gag-pol* mRNAs. A second probe, complementary to nt 1510-2290 of the codon optimised gene was also amplified by PCR from plasmid pSYNGP and was used to detect the codon optimised genes only. A 732 bp fragment complementary to all vector genomes used in this study was prepared by an *Spe*I-*Avr*II digestion of pH6nZ. A probe specific for ubiquitin (CLONTECH) was used to normalise the results. All probes were labelled by random labelling (STRATAGENE) with α-³²P dCTP (Amersham). The results were quantitated by using a Storm PhosphorImager (Molecular Dynamics) and shown in Figure 12. In the total cellular fractions the 47S rRNA precursor could be clearly seen, whereas it was absent from the cytoplasmic fractions. As expected (52), Rev stimulates the cytoplasmic accumulation of wild type *gag-pol* mRNA (lanes 1c and 2c). RNA levels were 10-20 fold higher for the codon optimised gene compared to the wild type one, both in total and cytoplasmic fractions (compare lanes 3t-2t, 3c-2c, 10c-8c). The RRE sequence did not significantly destabilise the codon optimised RNAs since RNA levels were similar for codon optimised RNAs whether or not they contained the RRE sequence (compare lanes 3 and 5). Rev did not markedly enhance cytoplasmic accumulation of the codon optimised *gag-pol* mRNAs, even when they contained the RRE sequence (differences in RNA levels were less than 2-fold, compare lanes 3-4 or 5-6).

It appeared from a comparison of Figures 10 and 12 that all of the increase in protein expression from *syngp* could be accounted for by the increase in RNA levels. In order to investigate whether this was due to saturating levels of RNA in the cell, we transfected 0.1, 1 and 10 µg of the wild type or codon optimised expression vectors into 293T cells and compared protein production. In all cases protein production was 10-fold higher for the codon optimised gene for the same amount of transfected DNA, while increase in protein levels was proportional to the amount of transfected DNA for each individual gene. It seems likely therefore that the enhanced expression of the codon optimised gene can be mainly attributed to the enhanced RNA levels present in the cytoplasm and not to increased translation.

### Protein analysis

Total cell lysates were prepared from 293T cells 48 hours post-transfection (unless otherwise stated) with an alkaline lysis buffer. For extraction of proteins from cell supernatants the supernatant was first passed through a 0.22µm filter and the vector particles were collected by centrifugation of 1 ml of supernatant at 21,000 g for 30 minutes. Pellets were washed with PBS and then re-suspended in a small volume (2-10 il) of lysis buffer. Equal protein amounts were separated on a SDS 10-12% (v/v) polyacrylamide gel. Proteins were transferred to nitrocellulose membranes which were probed sequentially with a 1:500 dilution of HIV-1 positive human serum (AIDS Reagent Project, ADP508, Panel E) and a 1:1000 dilution of horseradish peroxidase labelled anti-human IgG (Sigma, A0176). Proteins were visualised using the ECL or ECL-plus western blotting detection reagent (Amersham). To verify equal protein loading, membranes were stripped and re-probed with a 1:1000 dilution of anti-actin antibody (Sigma, A2066), followed by a 1:2000 dilution of horseradish peroxidase labelled anti-rabbit IgG (Vector Laboratories, PI-1000).

### Expression of gag-pol gene products and vector particle production

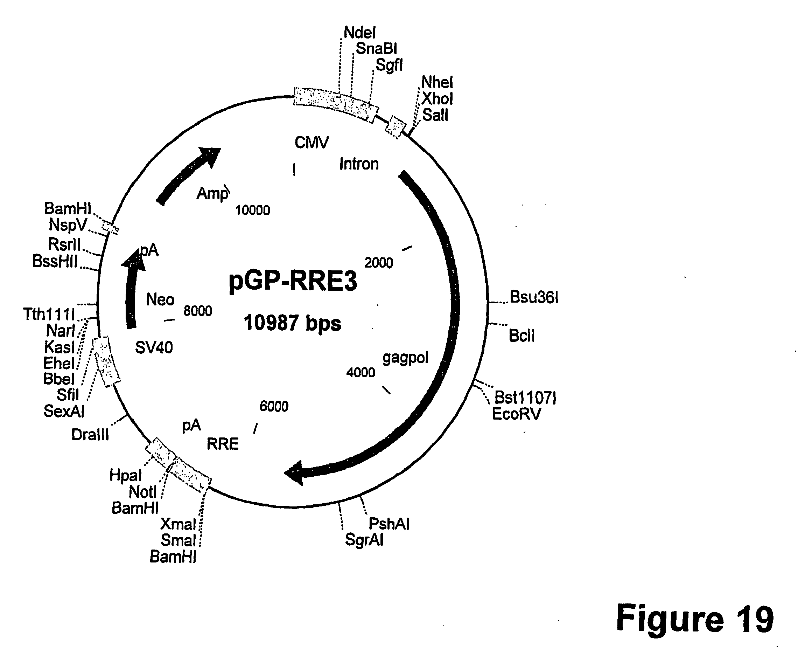

The wild type *gag-pol* (pGP-RRE3 - Figure 19) (49), and codon optimised expression vectors (pSYNGP, pSYNGP-RRE and pSYNGP-ERR) were transiently transfected into 293T cells. Transfections were performed in the presence or absence of a Rev expression vector, pCMV-Rev (53), in order to assess Rev-dependence for expression. Western blot analysis was performed on cell lysates and supernatants to assess protein production. The results are shown in Figure 10. As expected (54), expression of the wild type gene is observed only when Rev is provided *in trans* (lanes 2 and 3). In contrast, when the codon optimised *gag-pol* was used, there was high level expression in both the presence and absence of Rev (lanes 4 and 5), indicating that in this system there was no requirement for Rev. Protein levels were higher for the codon optimised gene than for the wild type *gag-pol* (compare lanes 4-9 with lane 3). The difference was more evident in the cell supernatants (approximately 10-fold higher protein levels for the codon optimised gene compared to the wild type one, quantitated by using a PhosphorImager) than in the cell lysates.

In previous studies where the RRE has been included in *gag-pol* expression vectors that had been engineered to remove INS sequences, inclusion of the RRE lead to a decrease in protein levels, that was restored by providing Rev *in trans* (55). In our hands, the presence of the RRE in the fully codon optimised *gag-pol* mRNA did not affect protein levels and provision of Rev *in trans* did not further enhance expression (lanes 6 and 7).

In order to compare translation rates between the wild type and codon optimised gene, protein production from the wild type and codon optimised expression vector was determined at several time intervals post transfection into 293T cells. Protein production and particle formation was determined by Western blot analysis and the results are shown in Figure 11. Protein production and particle formation was 10-fold higher for the codon optimised *gag-pol* at all time points.

To further determine whether this enhanced expression that was observed with the codon optimised gene was due to better translation or due to effects on the RNA, RNA analysis was carried out.

### The efficiency of vector production using the codon optimised gag-pol gene

To determine the effects of the codon optimised *gag-pol* on vector production, we used an HIV vector genome, pH6nZ and the VSV-G envelope expression plasmid pHCMVG (113), in combination with either pSYNGP, pSYNGP-RRE, pSYNGP-ERR or pGP-RRE3 as a source for the *gag-pol* in a plasmid ratio of 2 : 1 : 2 in a 3 plasmid co-transfection of 293T cells (49). Whole cell extracts and culture supernatants were evaluated by Western blot analysis for the presence of the *gag* and *gag-pol* gene products. Particle production was, as expected (Figure 10), 5-10 fold higher for the codon optimised genes when compared to the wild type.

To determine the effects of the codon optimised *gag-pol* gene on vector titres, several ratios of the vector components were used. The results are shown in Figure 21. Where the *gag-pol* was the limiting component in the system (as determined by the drop in titres observed with the wild type gene), titres were 10-fold higher for the codon optimised vectors. This is in agreement with the higher protein production observed for these vectors, but suggests that under normal conditions of vector production gag-pol is saturating and the codon optimisation gives no maximum yield advantage.

### The effect of HIV-1 gag INS sequences on the codon optimised gene is position dependent

It has previously been demonstrated that insertion of wild type HIV-1 *gag* sequences downstream of other RNAs, e.g. HIV-1 *tat* (56), HIV-1 *gag* (55) or CAT (57) can lead to a dramatic decrease in steady state mRNA levels, presumably as a result of the INS sequences. In other cases, e.g. for β-globin (58), it was shown that the effect was splice site dependent. Cellular AREs (AU-rich elements) that are found in the 3'UTR of labile mRNAs may confer mRNA destabilisation by inducing cytoplasmic deadenylation of the transcripts (59). To test whether HTV-1 *gag* INS sequences would destabilise the codon optimised RNA, the wild-type HIV-1 *gag* sequence, or parts of it (nt 1-625 or nt 625-1503), were amplified by PCR from the proviral clone pW13. All fragments were blunt ended and were inserted into pSYNGP or pSYNGP-RRE at either a blunted *Eco*R1 or *Not*I site (upstream or downstream of the codon optimised *gag-pol* gene repectively). As controls the wt HIV-1 *gag* in the reverse orientation (as INS sequences have been shown to act in an orientation dependent manner, (57) (pSYN7) and *lacZ,* excised from plasmid pCMV-βgal (CLONTECH) (in the correct orientation) (pSYN8) were also inserted in the same site. Contrary to our expectation, as shown in Figure 13, the wild type HIV-1 *gag* sequence did not appear to significantly affect RNA or protein levels of the codon optimised gene. We further constructed another series of plasmids (by PCR and from the same plasmids) where the wild type HIV-1 *gag* in the sense or reverse orientation, subfragments of *gag* (nt 1-625 or nt 625-1503), the wild type HIV-1 *gag* without the ATG or with a frameshift mutation 25 bases downstream of the ATG, or nt 72-1093 of LacZ (excised from plasmid pH6Z), or the first 1093 bases of *lacZ* with or without the ATG were inserted upstream of the codon optimised HIV-1 *gag-pol* gene in pSYNGP and/or pSYNGP-RRE (pSYN9-pSYN22, Figure 14). Northern blot analysis showed that insertion of the wild type HIV-1 *gag* gene upstream of the codon optimised HIV-1 *gag-pol* (pSYN9, pSYN10) lead to diminished RNA levels in the presence or absence of Rev/RRE (Figure 15A, lanes 1-4 and Figure 15B, lanes 1+12). The effect was not dependent on translation as insertion of a wild type HIV-1 *gag* lacking the ATG or with a frameshift mutation (pSYN12, pSYN13 and pSYN14) also diminished RNA levels (Figure 15B, lanes 1-7). Western blot analysis verified that there was no HIV-1 gag translation product for pSYN12-14. However, it is possible that, as the wt HIV-1 *gag* exhibits such an adverse codon usage, it may act as a non-translatable long 5' leader for *syngp,* and if this is the case, then the ATG mutation should not have any effects.

Insertion of smaller parts of the wild type HIV-1 *gag* gene (pSYN15 and pSYN17) also lead to a decrease in RNA levels (Figure 15B, lanes 1-3 and 8-9), but not to levels as low as when the whole *gag* sequence was used (lanes 1-3, 4-7 and 8-9 in Figure 15B). This indicates that the effect of INS sequences is dependent on their size. Insertion of the wild type HIV-1 *gag* in the reverse orientation (pSYN11) had no effect on RNA levels (Figure 15A, lanes 1 and 5-6). However a splicing event seemed to take place in that case, as indicated by the size of the RNA (equal to the size of the codon optimised *gag-pol* RNA) and by the translation product (gag-pol, in equal amounts compared to pSYNGP, as verified by Western blot analysis).

These data indicate therefore that wild type HIV-1 *gag* instability sequences act in a position and size dependent manner, probably irrespective of translation. It should also be noted that the RRE was unable to rescue the destabilised RNAs through interaction with Rev.

### Construction of an HIV-1 based vector system that lacks all the accessory proteins

Until now several HIV-1 based vector systems have been reported that lack all accessory proteins but Rev (49, 60). We wished to investigate whether the codon optimised gene would permit the construction of an HIV-1 based vector system that lacks all accessory proteins. We initially deleted *rev*/RRE and any residual *env* sequences, but kept the first 625 nucleotides of *gag*, as they have been shown to play a role in efficient packaging (61). Two vector genome constructs were made, pH6.1nZ (retaining only HIV sequences up to nt 625 of *gag*) and pH6.2nZ (same as pH6.1nZ, but also retaining the *env* splice acceptor). These were derived from a conventional HIV vector genome that contains RRE and expresses Rev (pH6nZ). Our 3-plasmid vector system now expressed only HIV-1 gag-pol and the VSV-G envelope proteins. Vector particle titres were determined as described in the previous section. A ratio of 2 : 2 : 1 of vector genome (pH6Z or pH6.1nZ or pH6.2nZ) : gag-pol expression vector (pGP-RRE3 or pSYNGP): pHCMV-G was used. Transfections were performed in the presence or absence of pCMV-Rev, as gag-pol expression was still Rev dependent for the wild type gene. The results are summarised in Figure 22 and indicate that an HIV vector could be produced in the total absence of Rev, but that maximum titres were compromised at 20-fold lower than could be achieved in the presence of Rev. As gag-pol expression should be the same for pSYNGP with pH6nZ or pH6.1nZ or pH6.2nZ (since it is Rev independent), as well as for pGP-RRE3 when Rev is provided *in trans,* we suspected that the vector genome retained a requirement for Rev and was therefore limiting the titres. To confirm this, Northern blot analysis was performed on cytoplasmic RNA prepared from cells transfected with pH6nZ or pH6.1nZ in the presence or absence of pCMV-Rev. As can be seen in Figure 17, lanes 1-4, the levels of cytoplasmic RNA derived from pH6nZ were 5-10 fold higher than those obtained with pH6.1nZ (compare lanes 1-2 to lanes 3-4). These data support the notion that RNA produced from the vector genome requires the Rev/RRE system to ensure high cytoplasmic levels. This may be due to inefficient nuclear export of the RNA, as INS sequences residing within *gag* were still present.

Further deletions in the *gag* sequences of the vector genome might therefore be necessary to restore titres. To date efficient packaging has been reported to require 360 (62) or 255 (63) nucleotides of *gag* in vectors that still retain *env* sequences, or about 40 nucleotides of *gag* in a particular combination of splice donor mutation, *gag* and *env* deletions (64, 63). In an attempt to remove the requirement for Rev/RRE in our vector genome without compromising efficient packaging we constructed a series of vectors derived from pH6nZ containing progressively larger deletions of HIV-1 sequences (only sequences upstream and within *gag* were retained) plus and minus a mutant major splice donor (SD) (GT to CA mutation). Vector particle titres were determined as before and the results are summarised in Figure 23. As can be seen, deletion of up to nt 360 in *gag* (vector pHS3nZ) resulted in an increase in titres (compared to pH6.1nZ or pH6.2nZ) and only a 5-fold decrease (titres were 1.3-1.7 x 10⁵) compared to pH6nZ. Further deletions resulted in titres lower than pHS3nZ and similar to pH6.1nZ. In addition, the SD mutation did not have a positive effect on vector titres and in the case of pHS3nZ it resulted in a 10-fold decrease in titres (compare titres for pHS3nZ and pHS7nZ in Figure 23). Northern blot analysis on cytoplasmic RNA (Figure 17, lanes 1 and 5-12) showed that RNA levels were indeed higher for pH6nZ, which could account for the maximum titres observed with this vector. RNA levels were equal for pHS1nZ (lane 5), pHS2nZ (lane 6) and pHS3nZ (lane 7) whereas titres were 5-8 fold higher for pHS3nZ. It is possible that further deletions (than that found in pHS3nZ) in *gag* might result in less efficient packaging (as for HIV-1 the packaging signal extends in *gag*) and therefore even though all 3 vectors produce similar amounts of RNA only pHS3nZ retains maximum packaging efficiency. It is also interesting to note that the SD mutation resulted in increased RNA levels in the cytoplasm (compare lanes 6 and 10, 7 and 11 or 8 and 12 in Figure 17) but equal or decreased titres (Figure 23). The GT dinucleotide that was mutated is in the stem of SL2 of the packaging signal (65). It has been reported that SL2 might not be very important for HIV-1 RNA encapsidation (65, 66), whereas SL3 is of great importance (67). Folding of the wild type and SD-mutant vector sequences with the RNAdraw software program revealed that the mutation alters significantly the secondary structure of the RNA and not only of SL2. It is likely therefore that although the SD mutation enhances cytoplasmic RNA levels it does not increase titres as it alters the secondary structure of the packaging signal.

To investigate whether the titre differences that were observed with the Rev minus vectors were indeed due to Rev dependence of the genomes, the RRE sequence (nt 7769 - 8021 of the HXB2 sequence) was inserted in the *Spe*I site (downstream of the *gag* sequence and just upstream of the internal CMV promoter) of pH6.1nZ, pHS1nZ, pHS3nZ and pHS7nZ, resulting in plasmids pH6.1nZR, pHS1nZR, pHS3nZR and pHS7nZR respectively. Vector particle titres were determined with pSYNGP and pHCMVG in the presence or absence of Rev (pCMV-Rev) as before and the results are summarised in Figure 24. In the absence of Rev titres were further compromised for pH6.1nZR (7-fold compared to pH6.1nZ), pHS3nZR (6-fold compared to pHS3nZ) and pHS7nZR (2.5-fold compared to pHS7nZ). This was expected, as the RRE also acts as an instability sequence (68) and so it would be expected to confer Rev-dependence. In the presence of Rev titres were restored to the maximum titres observed for pH6nZ in the case of pHS3nZR (5 x 10⁵) and pH6.1nZR (2 x 10⁵). Titres were not restored for pHS7nZR in the presence of Rev. This supports the hypothesis that the SD mutation in pHS7nZ affects the structure of the packaging signal and thus the packaging ability of this vector genome, as in this case Rev may be able to stimulate vector genome RNA levels, as for pHS3nZR and pH6.1nZR, but it can not affect the secondary structure of the packaging signal. For vector pHS1nZ inclusion of the RRE did not lead to a decrease in titres. This could be due to the fact that pHS1nZ contains only 40 nucleotides of *gag* sequences and therefore even with the RRE the size of instability sequences is not higher than for pHS2nZ that gives equal titres to pHS1nZ. Rev was able to partially restore titres for pHS1nZR (10-fold increase when compared to pHS1nZ and 8-fold lower than pH6nZ) but not fully as in the case of pHS3nZ. This is also in agreement with the hypothesis that 40 nucleotides of HIV-1 *gag* sequences might not be sufficient for efficient vector RNA packaging and this could account for the partial and not complete restoration in titres observed with pHS1nZR in the presence of Rev.

In addition, end-point titres were determined for pHS3nZ and pH6nZ with pSYNGP in HeLa and HT1080 human cell lines. In both cases titres followed the pattern observed in 293T cells, with titres being 2-3 fold lower for pHS3nZ than for pH6nZ (See Figure 10). Finally, transduction efficiency of vector produced with pHS3nZ or pH6nZ and different amounts of pSYNGP or pGP-RRE3 at different m.o.i.'s (and as high as 1) was determined in HT1080 cells. This experiment was performed as the high level gag-pol expression from pSYNGP may result in interference by genome-empty particles at high vector concentrations. As expected for VSVG pseudotyped retroviral particles (69) transduction efficiencies correlated with the m.o.i.'s, whether high or low amounts of pSYNGP were used and with pH6nZ or pHS3nZ. For m.o.i. 1 transduction efficiency was approximately 50-60% in all cases (Figure 18). The above data indicate that no interference due to genome-empty particles is observed in this experimental system.

### The codon optimised gag-pol gene does not use the exportin-1 nuclear export pathway

Rev mediates the export of unspliced and singly spliced HIV-1 mRNAs via the nuclear export receptor exportin-1 (CRM1) (70, 71, 72, 73, 74). Leptomycin B (LMB) has been shown to inhibit leucine-rich NES mediated nuclear export by disrupting the formation of the exportin-1/NES/RanGTP complex (75, 72). In particular, LMB inhibits nucleo-cytoplasmic translocation of Rev and Rev-dependent HIV mRNAs (76). To investigate whether exportin-1 mediates the export of the codon optimised *gag-pol* constructs, the effect of LMB on protein production was tested. Western blot analysis was performed on cell lysates from cells transfected with the *gag-pol* constructs (+/- pCMV-Rev) and treated or not with LMB (7.5 nM, for 20 hours, beginning treatment 5 hours post-transfection). To confirm that LMB had no global effects on transport, the expression of β-gal from the control plasmid pCMV-βGal was also measured. An actin internal control was used to account for protein variations between samples. The results are shown in Figure 16. As expected (76), the wild type *gag-pol* was not expressed in the presence of LMB (compare lanes 3 and 4), whereas LMB had no effect on protein production from the codon optimised *gag-pol*, irrespective of the presence of the RRE in the transcript and the provision of Rev *in trans* (compare lanes 5 and 6, 7 and 8, 9 and 10, 11 and 12, 5-6 and 11-12). The resistance of the expression of the codon-optimised *gag-pol* to inhibition by LMB indicates that the exportin-1 pathway is not used and therefore an alternative export pathway must be used. This offers a possible explanation for the Rev independent expression. The fact that the presence of a nonfunctional Rev/RRE interaction did not affect expression implies that the RRE does not necessarily act as an inhibitory (e.g. nuclear retention) signal *per se,* which is in agreement with previous observations (5, 58).

In conclusion, this is the first report of an HIV-1 based vector system, composed of pSYNGP, pHS3nZ and pHCMVG, where significant vector production can be achieved in the absence of all accessory proteins. These data indicate that in order to achieve maximum titres the HIV vector genome must be configured to retain efficient packaging and that this requires the retention of *gag* sequences and a splice donor. By reducing the *gag* sequence to 360 nt in pHS3nZ and combining this with pSYNGP it is possible to achieve titre of at least 10⁵ I.U./ml that is only 5-fold lower than the maximum levels achieved in the presence of Rev.

### Example 2 - EIAV

### Codon-optimised EIAV gag-pol expression cassettes

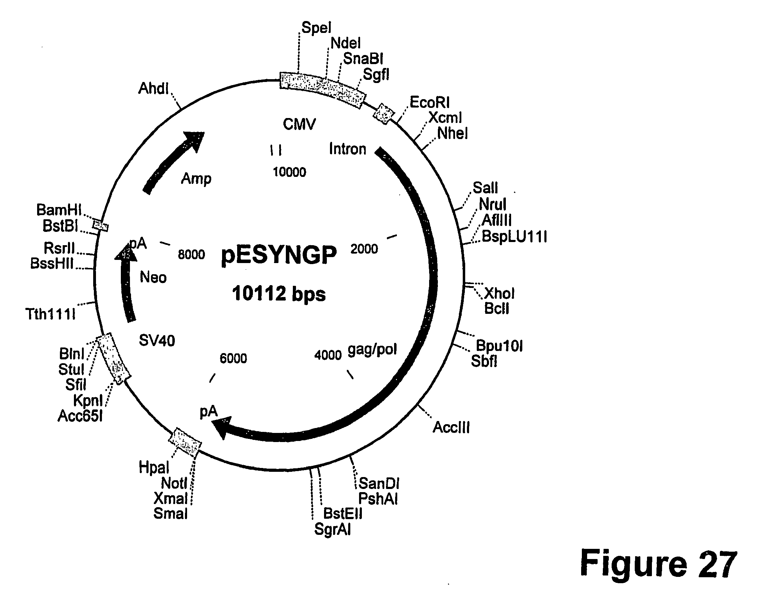

We also examined if the codon-optimisation process would alter the properties of the *gag-pol* gene of the non-primate lentivirus EIAV. The sequence is of the codon-optimised gene is shown from nt1103 to 5760 of SEQ ID NO:5 (Figure 9). The wild type and the codon-optimised sequences are denoted WT and CO, respectively. The codon usage was changed to that of highly expressed mammalian genes. pESYNGP (Figure 27 and SEQ ID NO:5) was made by transferring an *Xba*I-*Not*I fragment from a plasmid containing a codon-optimised EIAV gag/pol gene, synthesised by Operon Technologies Inc., Alameda, CA, into pCIneo (Promega). The gene was supplied in a proprietary plasmid backbone, GeneOp. The fragment transferred to pCIneo includes sequences flanking the codon-optimised EIAV gag/pol ORF: tctagaGAATTCGCCACC**ATG- EIAV gag/pol-TGA**ACCCGGGgcggccgc. The ATG start and TGA stop codons are shown in bold and the recognition sequences for *Xba*I and *Not*I sites in lower case.

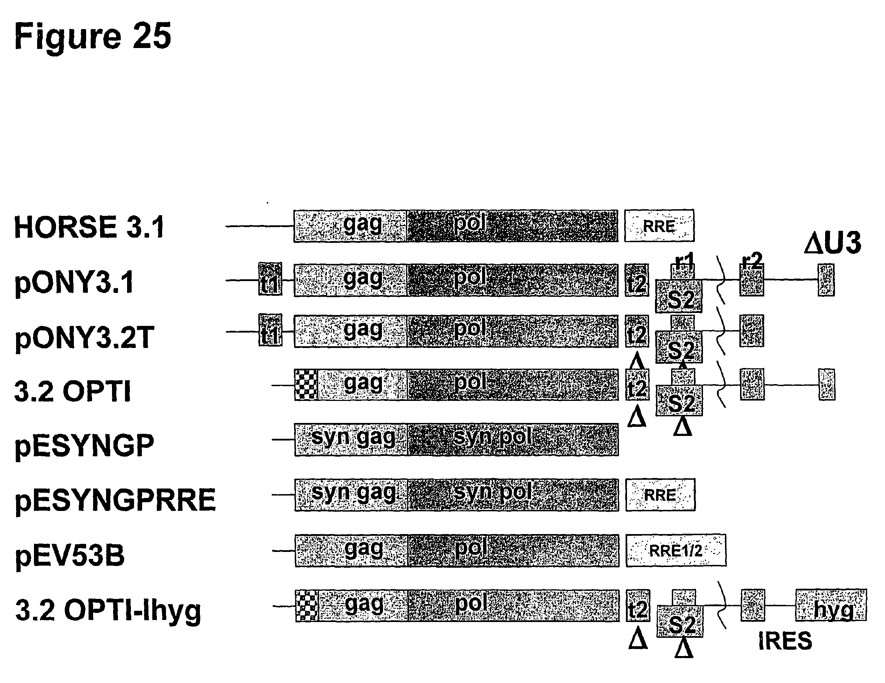

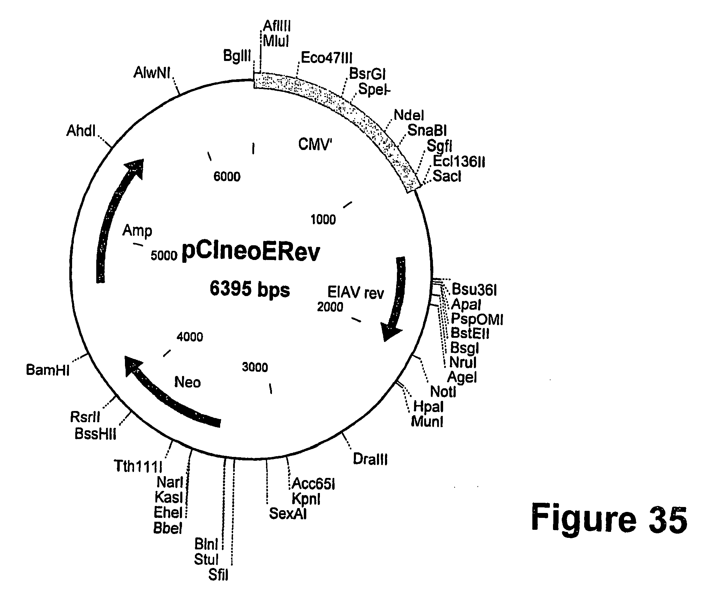

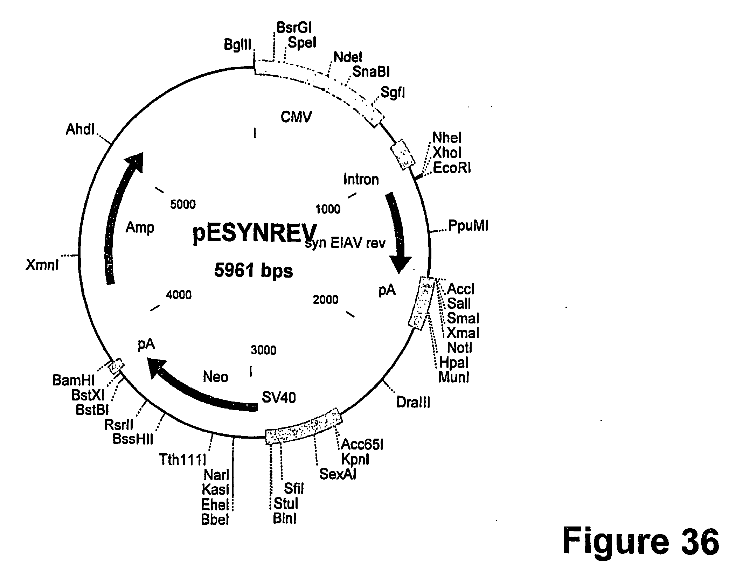

The expression of Gag/Pol from the codon-optimised gene was assessed with respect to that from various wild type EIAV gag/pol expression constructs by transient transfection of HEK 293T cells (Figure 25). Transfections were carried out using the calcium phosphate technique, using equal moles of each Gag/Pol expression plasmid together with a plasmid which expressed EIAV Rev either from the wild type sequence or from a codon-optimised version of the gene: pCIneoEREV (WO 99/32646) (Figure 35 and SEQ ID NO:13) or pESYNREV (Figure 36 and SEQ ID NO:14), respectively. pESYNREV is a pCIneo-based plasmid (Promega) which was made by introducing the *Eco*RI to *Sal*I fragment from a synthetic EIAV REV plasmid, made by Operon Technologies Alameda, CA. The plasmid backbone was the proprietary plasmid GeneOp in which was inserted a codon-optimised EIAV REV gene flanked by *Eco*RI and *Sal*I recognition sequences and a Kozak consensus sequence to drive efficient translation of the gene. The mass of DNA on each transfection was equalised by addition of pCIneo plasmid. In transfections in which a Rev expression plasmid was omitted, a similar mass of pCIneo (Promega) was used instead (lanes labelled pCIneo). Cytoplasmic extracts were prepared 48 hours post transfection and 15µg amounts of protein were fractionated by SDS-PAGE and then transferred to Hybond ECL. The Western blot was probed with a polyclonal antisera from an EIAV-infected horse and then with a secondary antibody, anti-horse horse-radish peroxidase conjugate. Development of the blot was carried out using the ECL kit (Amersham). Positive controls for the blotting and development procedure, and cytoplasmic extract from untransfected HEK 293T cells are as indicated. The positions of various EIAV proteins are indicated.

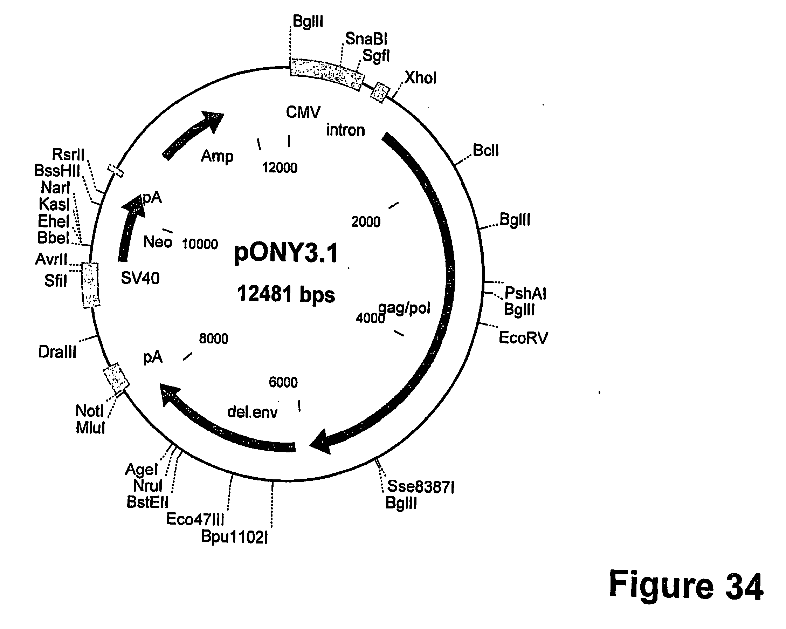

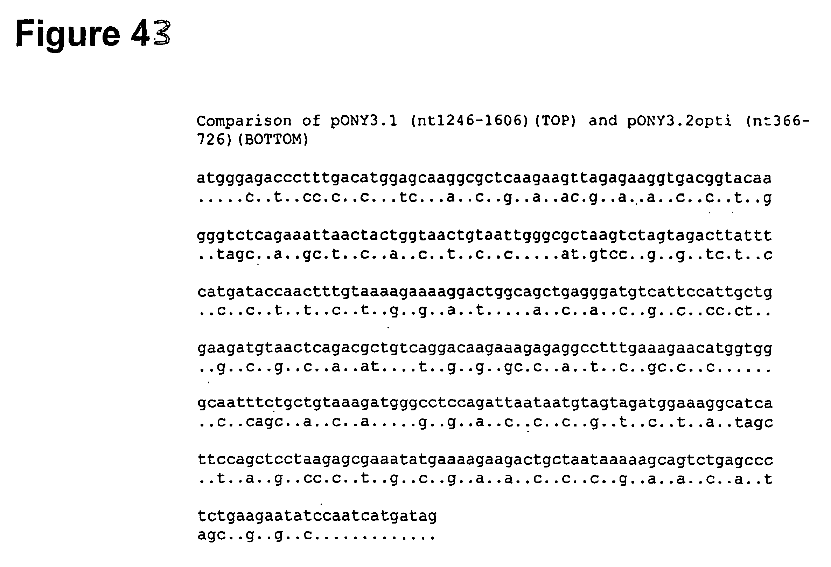

Expression from wild type *gag*/*pol* was achieved from various plasmids (see Figure 25). pONY3.2T is a derivative of pONY3.1 (WO99/32646)(Figure 34 and SEQ ID NO:12) in which mutations which ablate expression of Tat and S2 have been made. In addition, the EIAV sequence is truncated downstream of the second exon of *rev*. Specifically, expression of Tat is ablated by an 83nt deletion in exon 2 of tat which corresponds with respect to the wild type EIAV sequence, Acc. No. U01866, to deletion of nt 5234-5316 inclusive. S2 ORF expression is ablated by a 51nt deletion, corresponding to nt 5346-5396 of Acc. No. U01866. The EIAV sequence is deleted downstream of a position corresponding to nt 7815 of Acc. No. U01866. These alterations do not alter rev, hence expression of this gene is expressed as for pONY3.1. pONY3.2 OPTI is a derivative of pONY3.1 which has the same deletions for ablation of Tat and S2 expression as described above. In addition, the first 372nt of *gag* have been 'codon-optimised' for expression in human cells. The sequence of the wild type and codon-optimised sequences present in pONY3.2OPTI in this region are compared in Figure 43. Base differences between the sequences are indicated. The region which was codon-optimised represents the region of overlap between the vector and wild-type gag/pol expression constructs. Reduction of homology within this region would be expected to improve the safety profile of the vector system due to the reduced chances of recombination between the vector genome and the gag/pol transcripts. 3.2 OPTI-Ihyg is a derivative of 3.2 OPTI in which the *Sna*BI-*Not*I fragment of 3.2 OPTI is transferred to pIRESlhygro (Clontech) prepared for ligation by digestion with the same sites. The *gag*/*po*/ gene is thus placed upstream of the IRES hygromycin phosphotransferase. Of note is the fact that the resulting construct contains the intron from pCIneo, not from pIRES1hygro. pEV53B is a derivative of PEV53A (WO 98/51810) in which the EIAV-derived sequence upstream of the Gag initiation codon is reduced to include only the major splice donor and surrounding seqeunces: CAG/GTAAGATG, where the Gag initiation codon is shown in bold face.

The results (Figure 26) shown the Rev-dependence of Gag/Pol expression from pHORSE3.1 (WO 99/32646), which has an EIAV derived leader sequence starting just downstream of the primer binding site and an RRE placed downstream of *gag*/*pol* composed of the two EIAV sequences reported to have RRE activity. Expression was enhanced by the same amount when Rev expression was driven by wild type (pCIneoERev) (Figure 35) or codon-optimised (pESYNREV) (Figure 36) genes. This result confirms the functionality of the codon-optimised Rev expression plasmid.

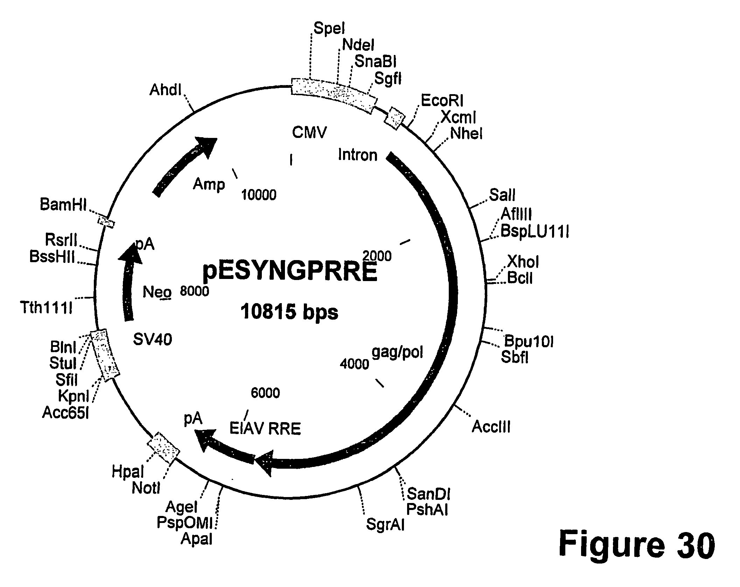

In contrast to expression of Gag/Pol from pONY3.1, expression from pESYNGP was not influenced by the presence of Rev, however it was slightly lower than from pONY3.1 or pON3.2T. Expression from pESYNGPRRE (Figure 30 and SEQ ID NO:7), in which the EIAV RRE sequence present in pHORSE3.1 is placed downstream of *gag*/*pol,* appeared slightly lower than from pESYNGP. the levels of expression from 3.2 OPTI and 3.2OPTI-Ihyg were significantly lower than from pESYNGP or pONY3.1, even in the presence of Rev. This result suggested that there may be determinants of Gag/Pol expression within the first 372nt of the *gag* and showed that 3.2 OPTI was unlikely to be useful as a basis for EIAV vector production. Furthermore it demonstrates that codon-optimisation of only certain regions of the whole gag/pol gene may not lead to high levels of Rev-independent expression.

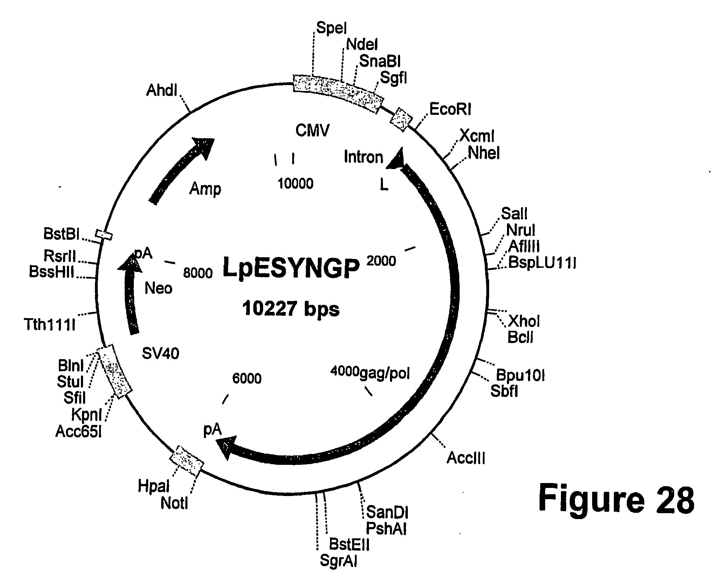

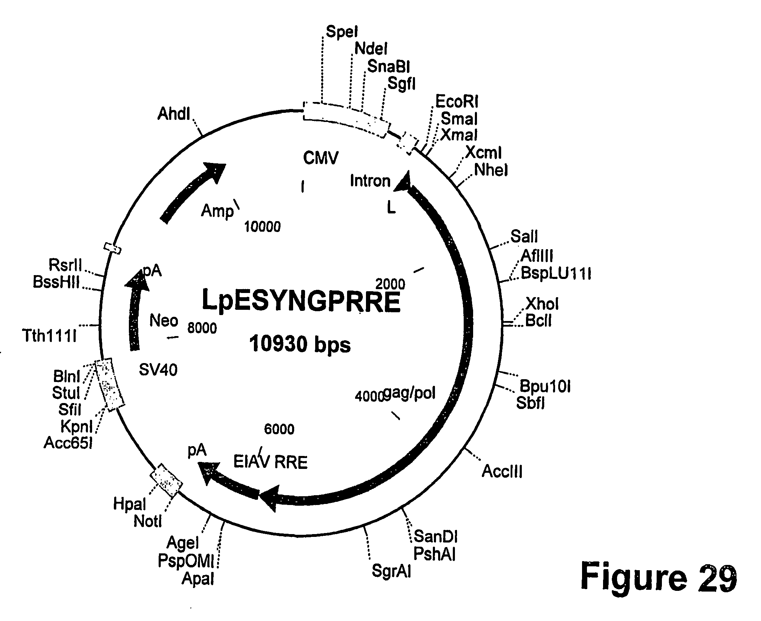

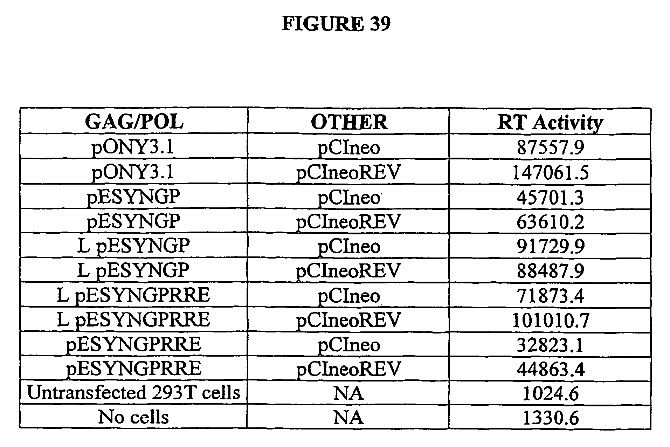

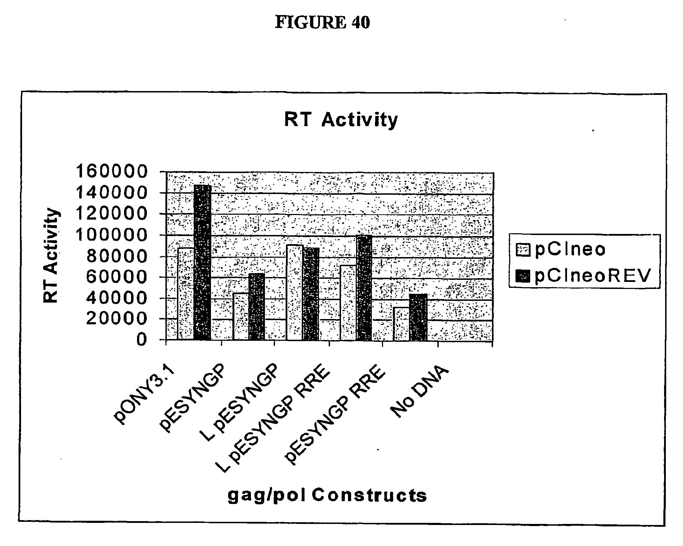

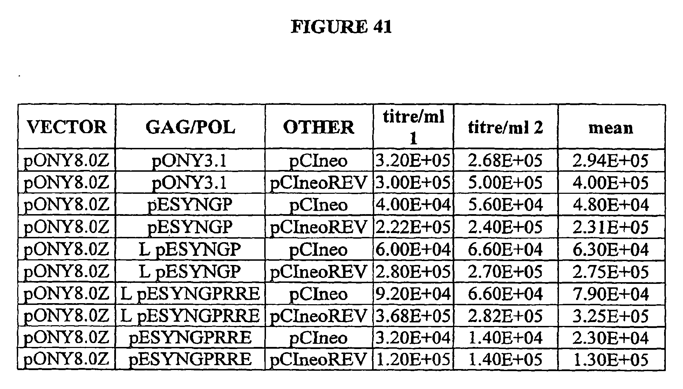

We have previously demonstrated (43) that the 5' leader (121bp upstream of the ATG start codon) and the RRE sequence (43) are important for high expression of the wild type EIAV *gag-pol.* Three constructs were made that contained either the leader sequence (LpESYNGP), the leader and RRE sequences (LpESYNGPRRE) or the RRE sequence (pESYNGPRRE). The sequences of these constructs are shown in SEQ ID NOS:6-8 and Figures 28-30. They were transfected into 293T cells in either the presence or absence of Rev expression plasmid. The cell supernatant was then measured for reverse transcriptase activity (RT), using a conventional RT assay, to evaluate which construct generated the highest amount of *gag-pol* mRNA. The results are shown in Figures 39 and 40. It is clear from these results that the 5' leader leads to an increase in RT activity. The ability of these Gag/Pol expression constructs to support formation of infectious vector particles was also tested by transient transfection of HEK 293 cells. The results of this analysis of show that all of the constructs could provide functional EIAV Gag/Pol, and show the Rev dependence of titre with the pONY8.0Z vector genome plasmid, which does not encode any EIAV proteins (Figure 41).

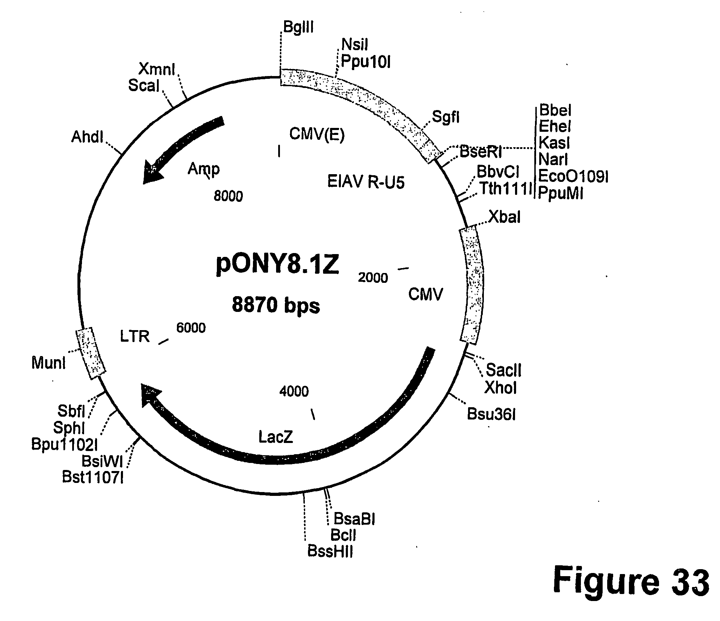

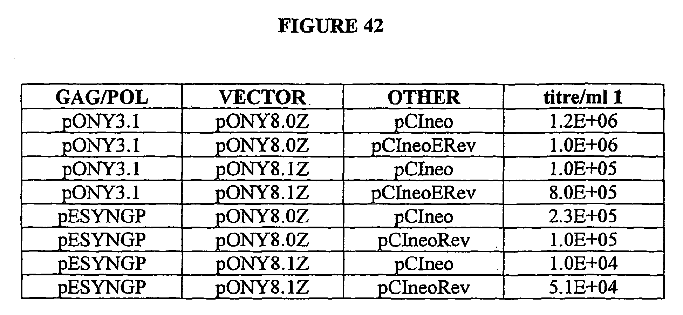

The ability of pESYNGP to act in concert with a minimal EIAV vector genome plasmid pONY8.1Z (Figure 33, SEQ ID NO:11) was evaluated (Figure 42). The result shows that the titres obtained with pESYNGP and pONY8.1Z are about 10-fold lower than from pONY3.1 and pONY8.1Z. This reduced titre reflects the lack of Rev protein in the system rather than a deficiency of Gag/Pol production which we have already shown is independent of Rev expression.

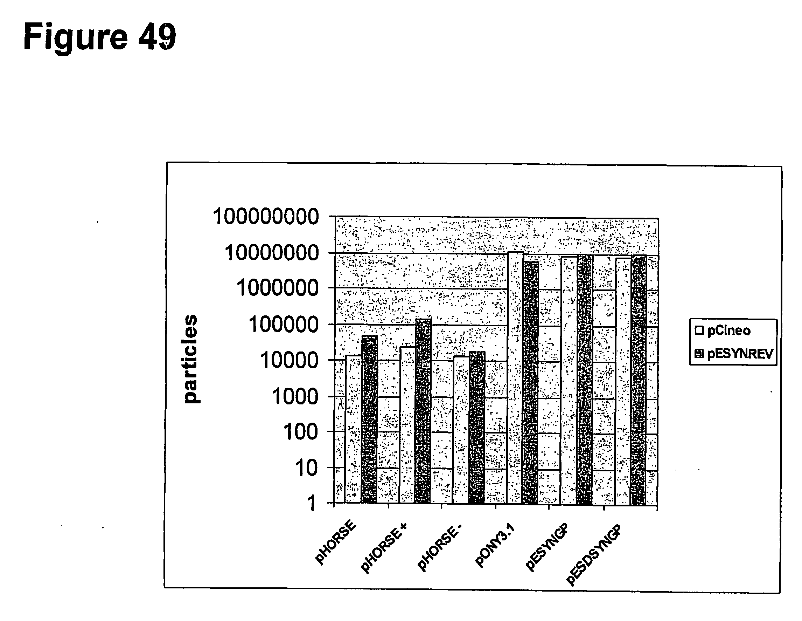

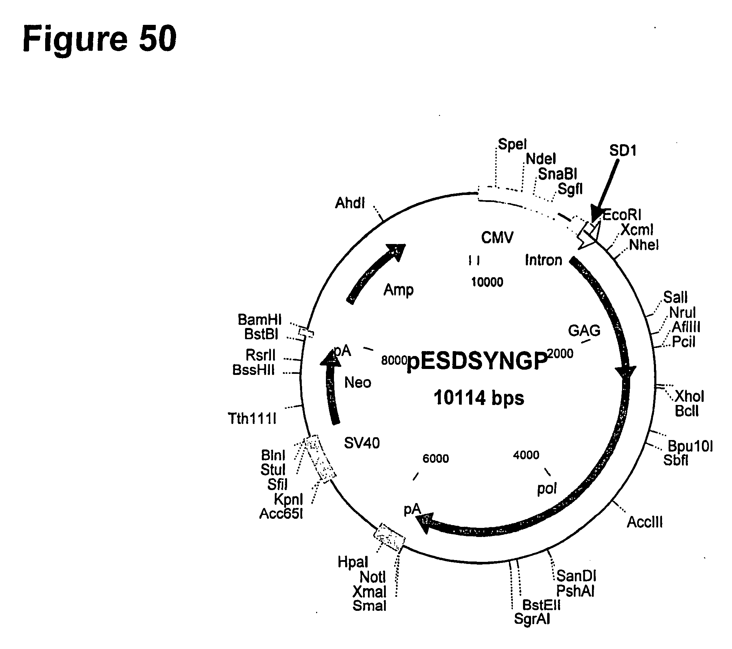

Expression of EIAV Gag/Pol was also tested from pESDSYNGP (Figure 50 and SEQ ID NO:18) in which the Kozak consensus sequence of Gag is replaced by the natural EIAV splice donor. pESDSYNGP was made from pESYNGP by exchange of the 306bp *Eco*RI-*Nhe*I fragment, which runs from just upstream of the start codon for gag/pol to approximately 300 base pairs inside the gag/pol ORF with a 308bp *Eco*RI-*Nhe*I fragment derived by digestion of a PCR product made using pESYNGP as template and using the following primers: SD FOR [GGCTAGAGAATTCCAGGTAAGATGGGCGATCCCCTCACCTGG] and SD REV [TTGGGTACTCCTCGCTAGGTTC]. This manipulation replaces the Kozak concensus sequence upstream of the ATG in pESYNGP with the splice donor found in EIAV. The sequence between the *Eco*RI site and the ATG of gag/pol is thus CAGGTAAG, exactly as found in the natural viral sequence. Therefore the mRNA is deleted with respect to sequences upstream but not downstream of the splice donor. The performance of pESDSYNGP was assessed relative to pESYNGP and other expression plasmids by measurement of reverse transcriptase activity in supernatants from transiently transfected HEK 293T cells using a Taqman-based version of the product enhanced reverse transcriptase (PERT) assay. In this method, reverse transcriptase associated with vector particles is released by mild detergent treatment and used to synthesize cDNA using MS2 bacteriophage RNA as template. MS2 RNA template and primer are present in excess hence the amount of cDNA is proportional to the amount of RT released from the particles. Therefore, the amount of cDNA synthesised is proportional to the number of particles. MS2 cDNA is then quantitated using Taqman technology. The assay is carried out on test samples in parallel with a vector stock of known titre and estimated particle content. The use of the standard allows creation of a 'standard curve' and allows the relative RT content of various samples to be calculated. The results of this analysis are shown in Figure 49. The results show that Gag/Pol expression is virtually identical from pESYNGP and pESDSYNGP. The results also indicate that expression is not significantly enhanced by Rev. The activity of the Rev expression plasmid is confirmed by the result obtained with pHORSE +, in which there is an RRE downstream of the wild type EIAV *gag*/*pol,* and that shows a 6-fold enhancement of expression in the presence of Rev. We also noted that the expression from pHORSE was enhanced 3-fold in the presence of Rev. Since this construct has no RRE it suggests that Rev may be having a non-specific enhancing effect on expression, possibly as a result of being expressed at high levels in this experimental system.

The ability of pESYNGP to participate in the formation of infectious viral vector particles, when co-transfected with plasmids for the vector genome and envelope was assessed by transient transfection of HEK 293T, as described previously (49, 50). Briefly, 293T cells were seeded on 6cm dishes (1.2 x 10⁶/dish) and 24 hours later they were transfected by the calcium phosphate procedure. The medium was replaced 12 hours post-transfection and supernatants were harvested 48 hours post-transfection, filtered (0.45 µm filters) and titered by transduction of D 17, canine osteosarcoma cells, in the presence of 8 µg/ml Polybrene (Sigma). Cells were seeded at 0.9 x 10⁵ /well in 12 well plates 24 hours prior to use in titration assays. Dilutions of supernatant were made in complete media (DMEM/10%FBS) and 0.5ml aliquots plated out onto the D17 cells. 4 hours after addition of the vector the media was supplemented with a further 1ml of media. Transduction was assessed by X-gal staining of cells 48 hours after addition of viral dilutions.

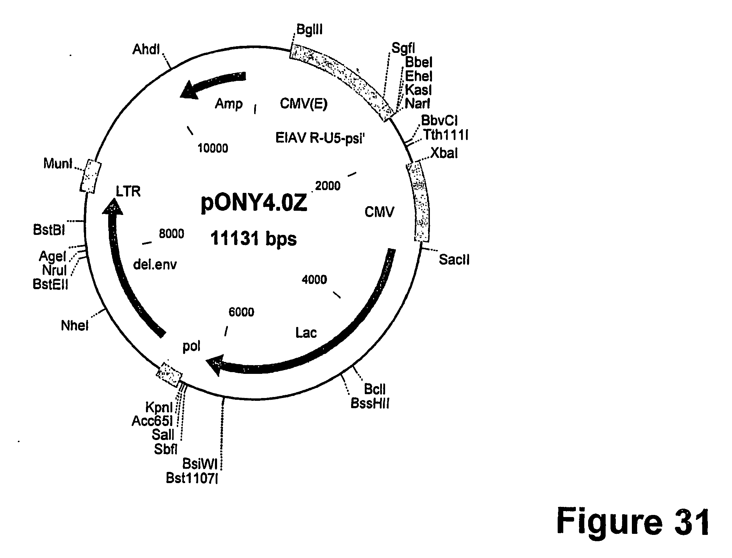

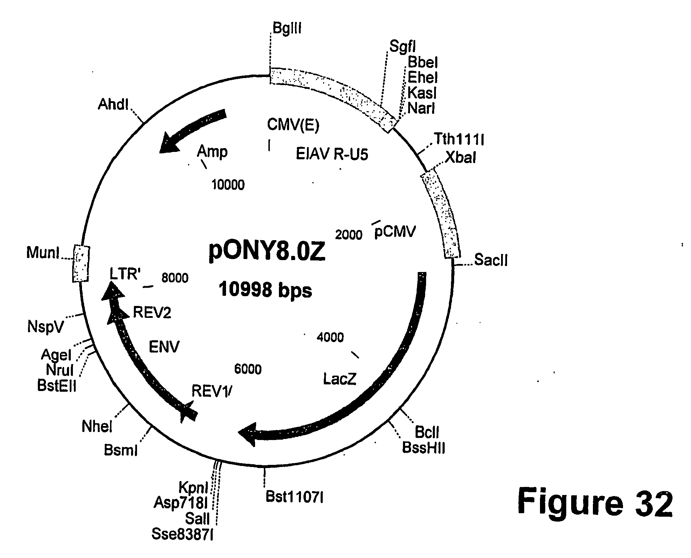

The vector genomes used for these experiments were pONY4.0Z (Figure 31 and SEQ ID NO:9) and pONY8.0Z (Figure 32 and SEQ ID NO:10).

pONY4.0Z (WO 99/32646) was derived from pONY2.11Z by replacement of the U3 region in the 5'LTR with the cytomegalovirus immediate early promoter (pCMV). This was carried out in such a way that the first base of the transcript derived from this CMV promoter corresponds to the first base of the R region. This manipulation results in the production of high levels of vector genome in transduced cells, particularly HEK 293T cells, and has been described previously (50). pONY4.0Z expresses all EIAV proteins except for envelope, expression of which is ablated by a deletion of 736nt between the *Hind*III sites present in *env*.

pONY8.0Z was derived from pONY4.0Z by introducing mutations which 1) prevented expression of TAT by an 83nt deletion in the exon 2 of tat) prevented S2 ORF expression by a 51nt deletion 3) prevented REV expression by deletion of a single base within exon 1 of rev and 4) prevented expression of the N-terminal portion of gag by insertion of T in ATG start codons, thereby changing the sequence to ATTG from ATG. With respect to the wild type EIAV sequence Acc. No. U01866 these correspond to deletion of nt 5234-5316 inclusive, nt 5346-5396 inclusive and nt 5538. The insertion of T residues was after nt 526 and 543.

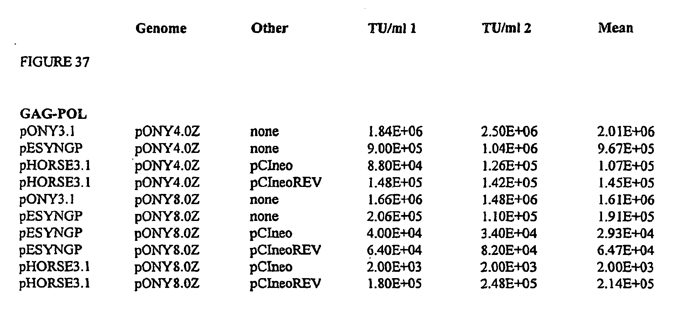

The results of this analysis are shown tabulated in Figure 37, and graphically in Figure 38. Transfections were carried out with only 3 plasmids (vector genome, gag/pol expression plasmid and VSV-G expression plasmid) - diagonal lined bars, or with four plasmids, which included the previous set of plasmids together with an additional plasmid encoding Rev or a similar plasmid not coding a functional protein - filled bars. The result show that high titres of vector can be achieved using pESYNGP to supply EIAV Gag/Pol. The highest titres were obtained using the Rev-expressing vector genome plasmid, pONY4.0Z, and they were only slightly lower than observed when Gag/Pol was supplied by pONY3.1. Lower titres were observed with pONY8.0Z vector genome plasmid with pESYNGP than with pONY3.1. This is due to the Rev expression requirement of pONY8.0Z. Rev is expressed by pONY3.1, but not pESYNGP. These results confirm the utility of the codon-optimised Gag/Pol expression plasmid.

### Use of the synthetic EIAV gag/pol gene in construction of cell lines which stably express EIAV gag/pol.

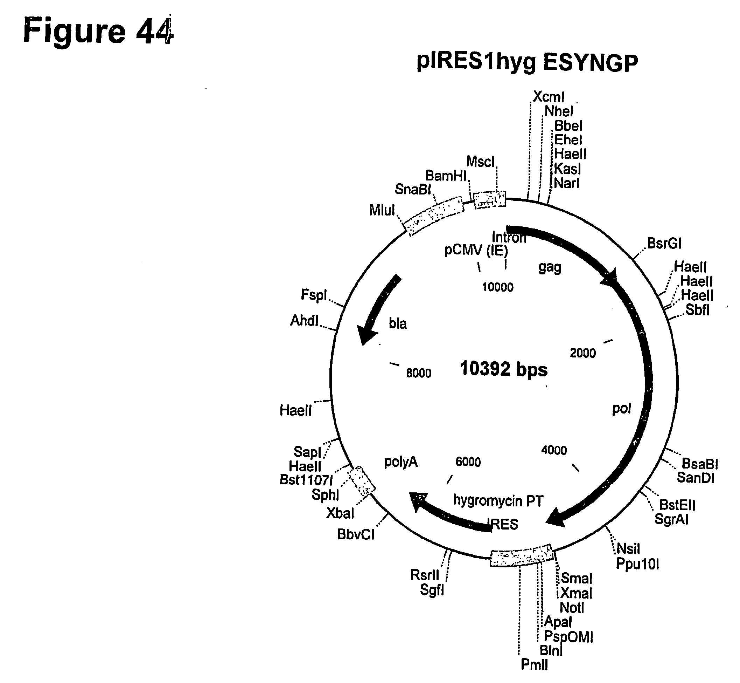

Cells lines which express high amounts of EIAV Gag/pol are required for the construction of packaging and producer cells for EIAV vectors. As a first step in their construction HEK 293 cells were stably transfected with pIRES1hyg ESYNGP (Figure 44 and SEQ ID NO:17), in which EIAV Gag/pol expression is driven by a CMV promoter, and is linked to an ORF for expression of hygromycin phosphotransferase by an EMCV IRES. pIRES1hyg ESYNGP was made as follows. The synthetic EIAV gag/pol gene and flanking sequences was transferred from pESYNGP into pIRES1hygro expression vector (Clontech). First, pESYNGP was digested with *Eco*RI*,* and the ends filled in by treatment with T4DNA polymerase and then digested with *Not*I, pIRES1hygro was prepared for ligation with this fragment by digestion with *Nsi*I*,* the ends trimmed flush by treatment with T4 DNA polymerase, then digested with *Not*I*.* Prior to transfection into HEK 293 cells pIRES1hyg ESYNGP was digested with *Ahd*I which linearises the plasmid.

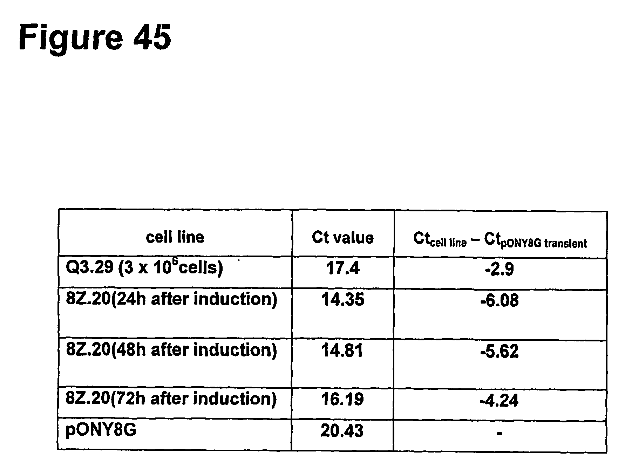

Clonal cell lines were derived by serial dilution and analysed for expression of Gag/Pol by a Taqman-based product enhanced reverse transcriptase (PERT) assay. Data for the cell line Q3.29, which expressed the highest level of Gag/Pol is shown. The analysis showed that the level of expression from the codon-optimised EIAV Gag/Pol cassette in Q3.29 was very similar to that seen for an EIAV producer line, 8Z.20, in which Gag/Pol is expressed from the pEV53B wild type expression cassette, that produced vector particles at titres of almost 10⁶ transducing units per ml. (Figure 45). Assuming exponential amplification during the assay, a difference of Ct value of 1.0 corresponds to a difference of 2-fold in concentration of the reverse transcriptase released from the particles. Therefore the difference in Gag/Pol expression between Q3.29 and 8Z.20 cells is approximately 2-8 fold. Furthermore the Ct values observed indicate that the level of expression of Gag/Pol is significantly higher than in samples of pONY8G vector particles with a titre of 2 x 10⁶ transducing units per ml on D17 cells, but made by transient transfection of HEK 293T cells. These data indicate that the codon-optimised EIAV Gag/Pol construct can be used in the construction of EIAV packaging and producer lines and confirms the previous result that expression is independent of Rev expression.

The Q3.29 cell line was then tested for its ability to support production of infectious vector particles when transfected with a vector genome plasmid, pONY8.0Z, and the VSV-G envelope expression plasmid, pRV67 and the EIAV REV expression plasmid, pESYNREV. In addition we also evaluated the performance of a plasmid pONY8.3G FB29 (-) which is modified form of the pONY8G vector genome plasmid. PONY8G is a standard EIAV vector genome used for comparison purposes. The modifications and construction of pONY8.3G FB29 (-) (SEQ ID NO:19) are described in PCT/GB00/03837 and briefly are 1) the introduction of loxP recognition sites upstream and downstream of the vector genome cassette 2) the placement of an expression cassette for codon-optimised REV, derived from pESYNREV, and driven by the FB29 U3 promoter downstream of the vector genome cassette and orientated so that the direction of transcription was towards the vector genome cassette. The REV expression cassette is located upstream of the 3' loxP site. Thus the pONY8.3G FB29 - plasmid carries expression cassettes for the vector genome RNA and for EIAV Rev.

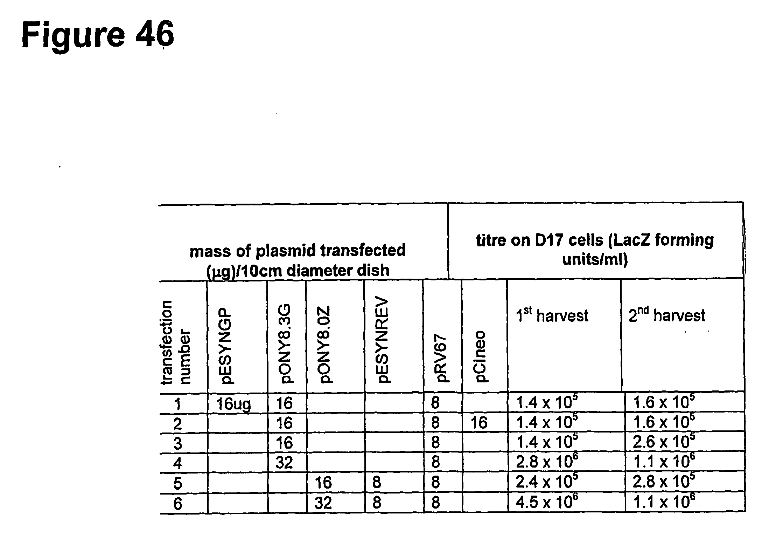

The titres were established by limiting dilution on D17 canine osteosarcoma cells and are shown in Figure 46

The titres obtained from transfections 2-6 were up to 4.5 x 10⁶ transducing units per ml indicating levels of Gag/Pol expression sufficient to support titres at least this high. The titres obtained were not higher when additional Gag/Pol was supplied (transfection 1) indicating that Gag/Pol expression was not the limitation on titre.

### Improved safety profile due to Gag/Pol expression from a codon-optimised expression construct

RCR formation takes place by recombination between different components of the vector system or by recombination of vector system components with nucleotide sequences present in the producer cells. Although recombination at the DNA level during construction of producer cell lines is possible (perhaps leading to insertional activation of endogenous retroelements or retroviruses) it is thought that recombination to produce RCR occurs mainly between RNA's undergoing reverse transcription, hence occurs within the mature vector particles. In consequence, recombination will be more likely to occur between RNA's which contain packaging signals, such as the vector genome and the *gag*/*pol* mRNA. Usually however the *gag*/*pol* transcript is modified so that it is deleted with respect to some or all defined packaging elements, thereby reducing the chances of its involvement in recombination.

The codon-optimisation process used to create the HIV and EIAV Gag/Pol expression plasmid, pSYNGP and pESYNGP, also results in disruption of sequences and structures that direct packaging as a result of introducing changes at approximately every 3^{rd} nucleotide position. We have obtained evidence for the lower level of incorporation of the codon-optimised RNA derived from pESYNGP into virions.

The packaging of mRNA's derived from a wild type *gag*/*pol* pEV53B expression cassette, and from the codon-optimised EIAV *gag*/*pol* expression cassette, pESYNGP, was compared. Medium was collected from a HEK 293 based cell-lines which were stably transfected with either pEV53B (cell line B-241), or with pESYNGP. Both cell lines produce vector particles which do not contain vector RNA and do not have envelopes. In some experiments, an EIAV vector genome plasmid (pECG3-CZW) was transfected into the cells to serve as an internal positive control for hybridisation and for the presence of particles capable of packaging RNA. pECG3-CZW is a derivative of pEC-LacZ (WO 98/51810) and was made from the latter by 1) reduction of *gag* sequences so that only the first 200nt of *gag,* rather than the first 577nt, was included and 2) inclusion of the woodchuck hepatitis virus post-transcriptional regulatory element (WHV PRE) (corresponding to nt 901-1800 of Acc. No. J04514) into the *Not*I site downstream of the LacZ reporter gene.

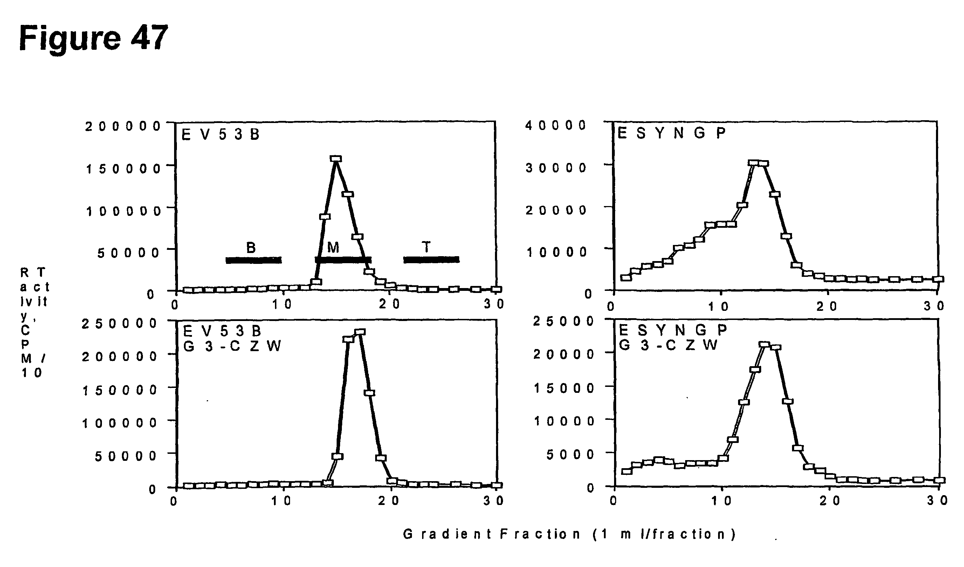

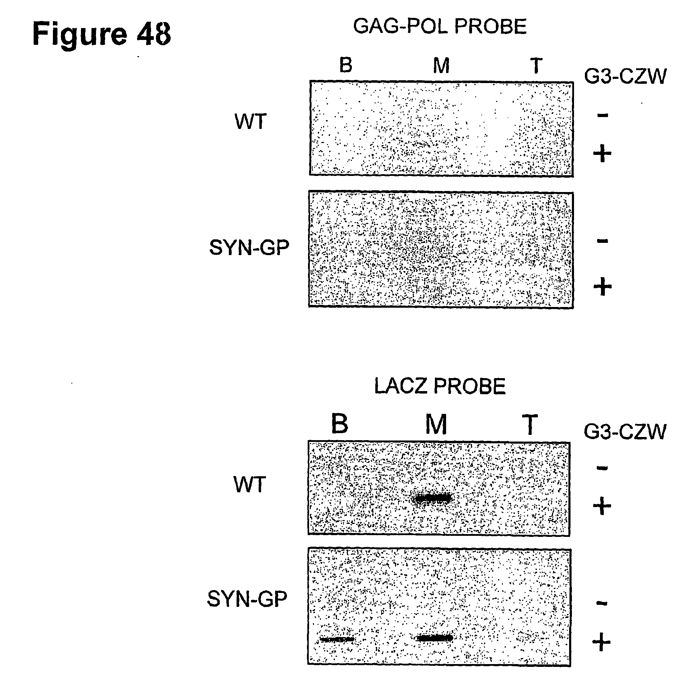

Viral particles derived from each of the cell lines were then partially purified from the medium by equilibrium density gradient centrifugation. To do this 10 ml of medium from producer cells, harvested at 24 hours after induction with sodium butyrate, was layered onto a 20-60% (w/w) sucrose gradient in TNE buffer (pH 7.4) and centrifuged for 24 hours at 25,000 rpm and 4° C in a SW28 rotor. Fractions were collected from the bottom and 10 µl of each fraction assayed for reverse transcriptase activity to locate viral particles. The results of this analysis are shown in (Figure 47) where the profile of reverse transcriptase activity is shown as a function of gradient fraction. In these figures, the top of the gradient is on the right. It should be noted that the levels of RT activity from the pESYNGP-expressing cell were significantly lower than from pEV53B expressing cells. To determine the RNA content of the purified virions, aliquots from the top, middle or bottom fractions were pooled (as indicated by the bars labeled T, M and B) and the RNA from each fraction was subjected to slot-blot hybridization analysis. Using a probe specific for a common region of wild type and synthetic *gag*/*pol,* encapsidation of RNA was easily detectable in the peak fractions (M) of virions synthesized from the wild type construct (pEV53B), but was not detected from virions synthesized from the synthetic Gag/Pol construct (pESYNGP)(Figure 48). The control for the presence of capsid capable of carrying out encapsidation was the EIAV G3-CZW vector genome which was readily detected in peak fractions from cells expressing either the wild type or synthetic gag/pol proteins. Even taking into account the different levels of expression from the wild type and synthetic Gag/Pol expression constructs this result indicates that the RNA from the codon-optimised *gag*/*pol* gene is packaged significantly less efficiently than the wild type gene and represents a significant improvement to the safety profile of the system. Of further note is that the RNA transcribed from pEV53B was packaged. This RNA is deleted with respect to sequences upstream of the splice donor sequence (CAG/GTAAG) and yet was still packaged. This points to the localisation of major packaging determinants within the gag coding region and is in contrast to the collected observations on the location of the packaging signal of HIV-1.

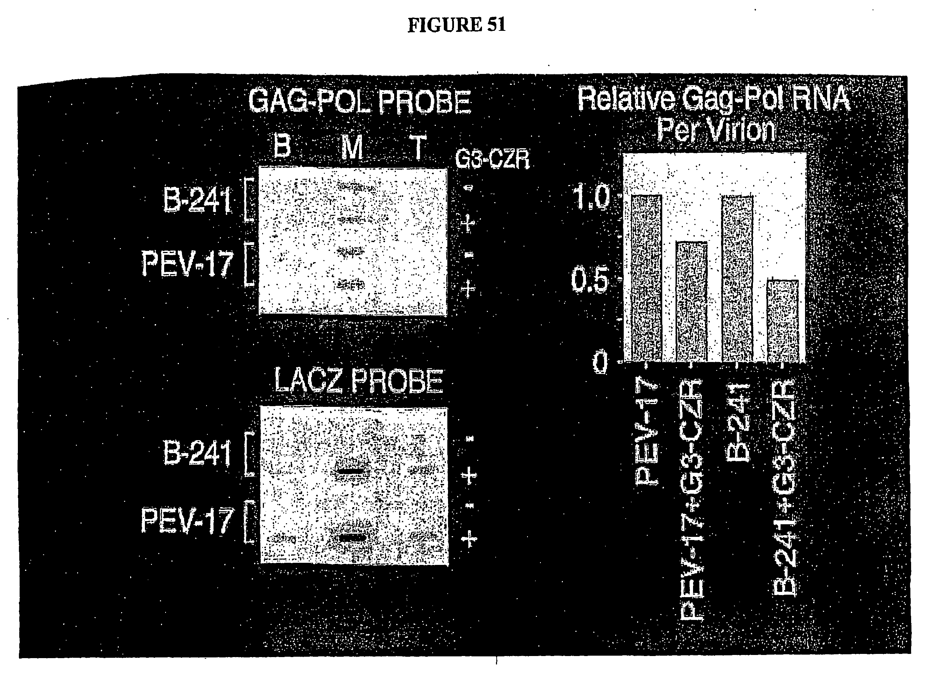

In additional experiments we have shown that the packaging of transcripts from pEV53B is only slightly lower than from pEV53A (Figure 51). This indicates further that major packaging sequences are located within the gag coding region. In these experiments cell line B-241 expressed pEV53B RNA and PEV-17 expressed pEV53A RNA. The EIAV vector genome used to confirm the presence of packaging competent vector particles was G3-CZR, which is the same as G3-CZW, described above, except for the replacement of the woodchuck post-transcriptional regulatory element with a sequence containing the EIAV RRE elements. Methodology was as described above.

### References

1. Miller, N., and J. Whelan. 1997. Hum Gene Ther. 8:803-15.
2. Lewis & Emerman. 1993. J. Virol. 68:510.
3. Naldini, L., U. Blomer, P. Gallay, D. Ory, R Mulligan, F. H. Gage, 1. M. Verma, and D. Trono. 1996. Science. 272:263-7.
4. Morgenstern & Land. 1990. Nucleic Acids Res. 18: 3587-3596.
5. Chang, D. D., and P. A. Sharp. 1989. Cell. 59:789-795.
6. Wang & Semenza. 1993. Proc Natl Acad Sci. 90:430.
7. Dachs et al. 1997. Nature Med. 5:515.
8. Firth et al. 1994. Proc Natl Acad Sci. 90: 6496-6500.
9. Madan et al. 1993. Proc Natl Acad Sci. 90:3928.
10. Semenza & Wang. 1992. Mol Cell Biol. 1992. 12: 5447-5454.
11. Takenaka et al. 1989. J Biol Chem. 264: 2363-2367.
12. Peshavaria & Day. 1991. Biochem J. 275: 427-433.
13. Inou et al. 1989. J Biol Chem. 264: 14954-14959.
14. Overell et al. 1988. Mol Cell Biol. 8: 1803-1808.
15. Attenello & Lee. 1984. Science. 226: 187-190.
16. Gazit et al. 1985. Cancer Res. 55: 1660-1663.
17. Yu et al. 1986. Proc Natl Acad Sci. 83: 3194-3198.
18. Dougherty & Temin. 1987. Proc Natl Acad Sci. 84: 1197-1201.
19. Hawley et al. 1987. Proc Natl Acad Sci. 84: 2406-2410.
20. Yee, J. K., A. Miyanohara, P. LaPorte, K. Bouic, J. C. Bums, and T. Friedmann. 1994. Proc. Natl. Acad. Sci. USA. 91:9564-8.
21. Jolley et al. 1983. Nucleic Acids Res. 11: 1855-1872.
22. Emerman & Tenim. 1984. Cell. 39: 449-467.
23. Herman & Coffin. 1987. Science. 236: 845-848.
24. Bender et al., 1987, J Virol 61: 1639-1646.
25. Pear et al., 1993, Proc Natl Acad Sci 90: 8392-8396.
26. Danos & Mulligan. 1998. Proc Natl Acad Sci. 85: 6460-6464.
27. Markowitz et al. 1988. Virology. 167: 400-406.
28. Cosset et al., 1995, J. Virol. 69: 7430-7436.
29. Mebatsion et al. 1997. Cell. 90: 841-847.
30. Marshall. 1998. Nature Biotechnology. 16: 129.
31. Nature Biotechnology. 1996. 14:556.
32. Wolff & Trubetskoy. 1998. Nature Biotechnology. 16: 421.
33. DuBridge, R B., P. Tang, H. C. Hsia, P.-M. Leong, J. H. Miller, and M. P. Calos. 1987. Mol. Cell. Biol. 7:379-387.
34. Gey, G. O., W. D. Coffman, and M. T. Kubicek. 1952. Cancer res. 12:264.
35. Kim, S. Y., R Byrn, J. Groopman, and D. Baltimore. 1989. J. Virol. 63:3708-3713.
36. Adachi, A., H. Gendelman, S. Koenig, T. Folks, R. Willey, A. Rabson, and M. Martin. 1986. J. Virol. 59:284-291.
37. Haas, J., E.-C. Park, and B. Seed. 1996. Current Biology. 6:315.
38. Zolotukhin, S., M. Potter, W. W. Hauswirth, J. Guy, and N. Muzyczka. 1996. A "humanized" green fluorescent protein cDNA adapted for high-level expression in mammalian cells. J Virol. 70:4646-54.
39. Fisher, A., E. Collalti, L. Ratner, R Gallo, and F. Wong-Staal. 1985. Nature. 316:262-265.
40. Kozak, M. 1992. [Review]. Annu. Rev. Cell Biol. 8:197-225.
41. Cassan, M., N. Delaunay, C. Vaquero, and J. P. Rousset. 1994. J. Virol. 68:1501-8.
42. Parkin, N. T., M. Chamorro, and H. E. Varmus. 1992. J. Virol. 66:5147-51. 68:3888-3895.
43. Mitrophanous K, Yoon S, Rohll J, Patil D, Wilkes F, Kim V, Kingsman S, Kingsman A, Mazarakis N, 1999. Gene Ther. 6 (11): 1808-18
44. Ory, D. S., B. A. Neugeboren, and R C. Mulligan. 1996. Proc Natl Acad Sci USA. 93:11400-6.
45. Zhu, Z. H., S. S. Chen, and A. S. Huang. 1990. J Acquir Immune Defic Syndr. 3:215-9.
46. Chesebro, B., K. Wehrly, and W. Maury. 1990. J Virol. 64:4553-7.
47. Spector, D. H., E. Wade, D. A. Wright, V. Koval, C. Clark, D. Jaquish, and S. A. Spector. 1990. J Virol. 64:2298-2308.
48. Valsesia Wittmann, S., A. Drynda, G. Deleage, M. Aumailley, J. M. Heard, O. Danos, G. Verdier, and F. L. Cosset. 1994. J Virol. 68:4609-19.
49. Kim, V. N., K. Mitrophanous, S. M. Kingsman, and K. A. J. 1998. J Virol 72: 811-816.
50. Soneoka, Y., P. M. Cannon, E. E. Ramsdale, J. C. Griffiths, G. Romano, S. M. Kingsman, and A. J. Kingsman. 1995. Nucleic Acids Res. 23:628-33.
51. Sagerstrom, C., and H. Sive. 1996. RNA blot analysis, p. 83-104. In P. Krieg (ed.), A laboratory guide to RNA: isolation, analysis and synthesis, vol. 1. Wiley-Liss Inc., New York.
52. Malim, M. H., J. Hauber, S. Y. Le, J. V. Maizel, and B. R. Cullen. 1989. Nature. 338:254-7.
53. Felber, B. K., M. Hadzopoulou Cladaras, C. Cladaras, T. Copeland, and G. N. Pavlakis. 1989. Proc. Natl. Acad. Sci. USA.. 86:1495-1499.
54. Hadzopoulou Cladaras, M., B. K. Felber, C. Cladaras, A. Athanassopoulos, A. Tse, and G. N. Pavlakis. 1989. J. Virol. 63:1265-74.
55. Schneider, R, M. Campbell, G. Nasioulas, B. K. Felber, and G. N. Pavlakis. 1997. J Virol. 71 :4892-903.
56. Schwartz, S., B. K. Felber, and G. N. Pavlakis. 1992. J. Virol. 66:150-159.
57. Maldarelli, F., M. A. Martin, and K. Strebel. 1991 J. Virol. 65:5732-5743.
58. Mikaelian, I., M. Krieg, M. Gait, and J. Karn. 1996. J. Mol. Biol. 257:246-264.
59. Xu, N., C.-Y. Chen, and A.-B. Shyu. 1997. Mol. Cell. Biol. 17:4611-4621.
60. Naldini, L. 1998. Curr. Opin. Biotechnol. 9:457-463.
61. Parolin, C., T. Dorfman, G. Palu, H. Gottlinger, and J. Sodroski. 1994J. Virol.
62. Dull, T., R Zufferey, M. Kelly, R Mandel, M. Nguyen, D. Trono, and L. Naldini. 1998. J. Virol. 72:8463-8471.
63. Cui, Y., T. Iwakama, and L.-J. Chang. 1999. J. Virol. 73:6171-6176.
64. Chang, L.-J., V. Urlacher, T. Iwakama, Y. Cui, and J. Zucali. 1999. Gene Ther. 6:715-728.
65. Harrison, G., G. Miele, E. Hunter, and A. Lever. 1998. J. Virol. 72:5886-5896.
66. McBride, M. S., and A. T. Panganiban. 1997. J. Virol. 71:2050-8.
67. Lever, A., H. Gottlinger, W. Haseltine, and J. Sodroski. 1989 J. Virol. 63:4085-7.
68. Brighty, D., and M. Rosenberg. 1994. Proc. Natl. Acad. Sci. USA. 91:8314-8318.
69. Arai, T., M. Takada, M. Ui, and H. Iba. 1999. Virology. 260:109-115.
70. Fornerod, M., M. Ohno, M. Yoshida, and I. W. Mattaj. 1997. Cell. 90:1051-1060.
71. Fridell, R A., H. P. Bogerd, and B. R. Cullen. 1996. Proc. Natl. Acad. Sci. USA. 93:4421-4.
72. Pollard, V., and M. Malim. 1998. Annu. Rev. Microbiol. 52:491-532.
73. Stade, K., C. S. Ford, C. Guthrie, and K. Weis. 1997. Cell. 90:1041-1050.
74. Ullman, K. S., M. Powers, A" and D. J. Forbes. 1997. Cell. 90:967-970.
75. Otero, G. C., M. E. Harris, J. E. Donello, and T. J. Hope. 1998. J. Virol. 72:7593-7597.
76. Wolff et al. 1997. Chem Biol. 4: 139-147.

### SEQUENCE LISTING PART OF THE DESCRIPTION

SEQ. ID. Into. I - Wild type gagpol sequence for strain HXB2 (accession no. K03455) 
ATGGGTGCGA GAGCGTCAGT ATTAAGCGGG GGAGAATTAG ATCGATGGGA AAAAATTCGG 60
TTAAGGCCAG GGGGAAAGAA AAAATATAAA TTAAAACATA TAGTATGGGC AAGCAGGGAG 120
CTAGAACGAT TCGCAGTTAA TCCTGGCCTG TTAGAAACAT CAGAAGGCTG TAGACAAATA 180
CTGGGACAGC TACAACCATC CCTTCAGACA GGATCAGAAG AACTTAGATC ATTATATAAT 240
ACAGTAGCAA CCCTCTATTG TGTGCATCAA AGGATAGAGA TAAAAGACAC CAAGGAAGCT 300
TTAGACAAGA TAGAGGAAGA GCAAAACAAA AGTAAGAAAA AAGCACAGCA AGCAGCAGCT 360
GACACAGGAC ACAGCAATCA GGTCAGCCAA AATTACCCTA TAGTGCAGAA CATCCAGGGG 420
CAAATGGTAC ATCAGGCCAT ATCACCTAGA ACTTTAAATG CATGGGTAAA AGTAGTAGAA 480
GAGAAGGCTT TCAGCCCAGA AGTGATACCC ATGTTTTCAG CATTATCAGA AGGAGCCACC 540
CCACAAGATT TAAACACCAT GCTAAACACA GTGGGGGGAC ATCAAGCAGC CATGCAAATG 600
TTAAAAGAGA CCATCAATGA GGAAGCTGCA GAATGGGATA GAGTGCATCC AGTGCATGCA 660
GGGCCTATTG CACCAGGCCA GATGAGAGAA CCAAGGGGAA GTGACATAGC AGGAACTACT 720
AGTACCCTTC AGGAACAAAT AGGATGGATG ACAAATAATC CACCTATCCC AGTAGGAGAA 780
ATTTATAAAA GATGGATAAT CCTGGGATTA AATAAAATAG TAAGAATGTA TAGCCCTACC 840
AGCATTCTGG ACATAAGACA AGGACCAAAG GAACCCTTTA GAGACTATGT AGACCGGTTC 900
TATAAAACTC TAAGAGCCGA GCAAGCTTCA CAGGAGGTAA AAAATTGGAT GACAGAAACC 960
TTGTTGGTCC AAAATGCGAA CCCAGATTGT AAGACTATTT TAAAAGCATT GGGACCAGCG 1020
GCTACACTAG AAGAAATGAT GACAGCATGT CAGGGAGTAG GAGGACCCGG CCATAAGGCA 1080
AGAGTTTTGG CTGAAGCAAT GAGCCAAGTA ACAAATTCAG CTACCATAAT GATGCAGAGA 1140
GGCAATTTTA GGAACCAAAG AAAGATTGTT AAGTGTTTCA ATTGTGGCAA AGAAGGGCAC 1200
ACAGCCAGAA ATTGCAGGGC CCCTAGGAAA AAGGGCTGTT GGAAATGTGG AAAGGAAGGA 1260
CACCAAATGA AAGATTGTAC TGAGAGACAG GCTAATTTTT TAGGGAAGAT CTGGCCTTCC 1320
TACAAGGGAA GGCCAGGGAA TTTTCTTCAG AGCAGACCAG AGCCAACAGC CCCACCAGAA 1380
GAGAGCTTCA GGTCTGGGGT AGAGACAACA ACTCCCCCTC AGAAGCAGGA GCCGATAGAC 1440
AAGGAACTGT ATCCTTTAAC TTCCCTCAGG TCACTCTTTG GCAACGACCC CTCGTCACAA 1500
TAAAGATAGG GGGGCAACTA AAGGAAGCTC TATTAGATAC AGGAGCAGAT GATACAGTAT 1560
TAGAAGAAAT GAGTTTGCCA GGAAGATGGA AACCAAAAAT GATAGGGGGA ATTGGAGGTT 1620
TTATCAAAGT AAGACAGTAT GATCAGATAC TCATAGAAAT CTGTGGACAT AAAGCTATAG 1680
GTACAGTATT AGTAGGACCT ACACCTGTCA ACATAATTGG AAGAAATCTG TTGACTCAGA 1740
TTGGTTGCAC TTTAAATTTT CCCATTAGCC CTATTGAGAC TGTACCAGTA AAATTAAAGC 1800
CAGGAATGGA TGGCCCAAAA GTTAAACAAT GGCCATTGAC AGAAGAAAAA ATAAAAGCAT 1860
TAGTAGAAAT TTGTACAGAG ATGGAAAAGG AAGGGAAAAT TTCAAAAATT GGGCCTGAAA 1920
ATCCATACAA TACTCCAGTA TTTGCCATAA AGAAAAAAGA CAGTACTAAA TGGAGAAAAT 1980
TAGTAGATTT CAGAGAACTT AATAAGAGAA CTCAAGACTT CTGGGAAGTT CAATTAGGAA 2040
TACCACATCC CGCAGGGTTA AAAAAGAAAA AATCAGTAAC AGTACTGGAT GTGGGTGATG 2100
CATATTTTTC AGTTCCCTTA GATGAAGACT TCAGGAAGTA TACTGCATTT ACCATACCTA 2160
GTATAAACAA TGAGACACCA GGGATTAGAT ATCAGTACAA TGTGCTTCCA CAGGGATGGA 2220
AAGGATCACC AGCAATATTC CAAAGTAGCA TGACAAAAAT CTTAGAGCCT TTTAGAAAAC 2280
AAAATCCAGA CATAGTTATC TATCAATACA TGGATGATTT GTATGTAGGA TCTGACTTAG 2340
AAATAGGGCA GCATAGAACA AAAATAGAGG AGCTGAGACA ACATCTGTTG AGGTGGGGAC 2400
TTACCACACC AGACAAAAAA CATCAGAAAG AACCTCCATT CCTTTGGATG GGTTATGAAC 2460
TCCATCCTGA TAAATGGACA GTACAGCCTA TAGTGCTGCC AGAAAAAGAC AGCTGGACTG 2520
TCAATGACAT ACAGAAGTTA GTGGGGAAAT TGAATTGGGC AAGTCAGATT TACCCAGGGA 2580
 
TTAAAGTAAG GCAATTATGT AAACTCCTTA GAGGAACCAA AGCACTAACA GAAGTAATAC 2640
CACTAACAGA AGAAGCAGAG CTAGAACTGG CAGAAAACAG AGAGATTCTA AAAGAACCAG 2700
TACATGGAGT GTATTATGAC CCATCAAAAG ACTTAATAGC AGAAATACAG AAGCAGGGGC 2760
AAGGCCAATG GACATATCAA ATTTATCAAG AGCCATTTAA AAATCTGAAA ACAGGAAAAT 2820
ATGCAAGAAT GAGGGGTGCC CACACTAATG ATGTAAAACA ATTAACAGAG GCAGTGCAAA 2880
AAATAACCAC AGAAAGCATA GTAATATGGG GAAAGACTCC TAAATTTAAA CTGCCCATAC 2940
AAAAGGAAAC ATGGGAAACA TGGTGGACAG AGTATTGGCA AGCCACCTGG ATTCCTGAGT 3000
GGGAGTTTGT TAATACCCCT CCCTTAGTGA AATTATGGTA CCAGTTAGAG AAAGAACCCA 3060
TAGTAGGAGC AGAAACCTTC TATGTAGATG GGGCAGCTAA CAGGGAGACT AAATTAGGAA 3120
AAGCAGGATA TGTTACTAAT AGAGGAAGAC AAAAAGTTGT CACCCTAACT GACACAACAA 3180
ATCAGAAGAC TGAGTTACAA GCAATTTATC TAGCTTTGCA GGATTCGGGA TTAGAAGTAA 3240
ACATAGTAAC AGACTCACAA TATGCATTAG GAATCATTCA AGCACAACCA GATCAAAGTG 3300
AATCAGAGTT AGTCAATCAA ATAATAGAGC AGTTAATAAA AAAGGAAAAG GTCTATCTGG 3360
CATGGGTACC AGCACACAAA GGAATTGGAG GAAATGAACA AGTAGATAAA TTAGTCAGTG 3420
CTGGAATCAG GAAAGTACTA TTTTTAGATG GAATAGATAA GGCCCAAGAT GAACATGAGA 3480
AATATCACAG TAATTGGAGA GCAATGGCTA GTGATTTTAA CCTGCCACCT GTAGTAGCAA 3540
AAGAAATAGT AGCCAGCTGT GATAAATGTC AGCTAAAAGG AGAAGCCATG CATGGACAAG 3600
TAGACTGTAG TCCAGGAATA TGGCAACTAG ATTGTACACA TTTAGAAGGA AAAGTTATCC 3660
TGGTAGCAGT TCATGTAGCC AGTGGATATA TAGAAGCAGA AGTTATTCCA GCAGAAACAG 3720
GGCAGGAAAC AGCATATTTT CTTTTAAAAT TAGCAGGAAG ATGGCCAGTA AAAACAATAC 3780
ATACTGACAA TGGCAGCAAT TTCACCGGTG CTACGGTTAG GGCCGCCTGT TGGTGGGCGG 3840
GAATCAAGCA GGAATTTGGA ATTCCCTACA ATCCCCAAAG TCAAGGAGTA GTAGAATCTA 3900
TGAATAAAGA ATTAAAGAAA ATTATAGGAC AGGTAAGAGA TCAGGCTGAA CATCTTAAGA 3960
CAGCAGTACA AATGGCAGTA TTCATCCACA ATTTTAAAAG AAAAGGGGGG ATTGGGGGGT 4020
ACAGTGCAGG GGAAAGAATA GTAGACATAA TAGCAACAGA CATACAAACT AAAGAATTAC 4080
AAAAACAAAT TACAAAAATT CAAAATTTTC GGGTTTATTA CAGGGACAGC AGAAATTCAC 4140
TTTGGAAAGG ACCAGCAAAG CTCCTCTGGA AAGGTGAAGG GGCAGTAGTA ATACAAGATA 4200
ATAGTGACAT AAAAGTAGTG CCAAGAAGAA AAGCAAAGAT CATTAGGGAT TATGGAAAAC 4260
AGATGGCAGG TGATGATTGT GTGGCAAGTA GACAGGATGA GGATTAG 4307
SEQ. ID. NO. 2 - pSYNGP 
TCAATATTGGCCATTAGCCATATTATTCATTGGTTATATAGCATAAATCAATATTGGCTA
TTGGCCATTGCATACGTTGTATCTATATCATAATATGTACATTTATATTGGCTCATGTCC
AATATGACCGCCATGTTGGCATTGATTATTGACTAGTTATTAATAGTAATCAATTACGGG
GTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCAT
AGTAACGCCAATAGGGACTTTCCATTGACGTCAATGGGTGGAGTATTTACGGTAAACTGC
CCACTTGGCAGTACATCAAGTGTATCATATGCCAAGTCCGCCCCCTATTGACGTCAATGA
CGGTAAATGGCCCGCCTGGCATTATGCCCAGTACATGACCTTACGGGACTTTCCTACTTG
GCAGTACATCTACGTATTAGTCATCGCTATTACCATGGTGATGCGGTTTTGGCAGTACAC
CAATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTG
CGATCGCCCGCCCCGTTGACGCAAATGGGCGGTAGGCGTGTACGGTGGGAGGTCTATATA
AGCAGAGCTCGTTTAGTGAACCGTCAGATCACTAGAAGCTTTATTGCGGTAGTTTATCAC
AGTTAAATTGCTAACGCAGTCAGTGCTTCTGACACAACAGTCTCGAACTTAAGCTGCAGT
GACTCTCTTAAGGTAGCCTTGCAGAAGTTGGTCGTGAGGCACTGGGCAGGTAAGTATCAA
GGTTACAAGACAGGTTTAAGGAGACCAATAGAAACTGGGCTTGTCGAGACAGAGAAGACT
CTTGCGTTTCTGATAGGCACCTATTGGTCTTACTGACATCCACTTTGCCTTTCTCTCCAC
AGGTGTCCACTCCCAGTTCAATTACAGCTCTTAAGGCTAGAGTACTTAATACGACTCACT
ATAGGCTAGCCTCGAGAATTCGCCACCATGGGCGCCCGCGCCAGCGTGCTGTCGGGCGGC
GAGCTGGACCGCTGGGAGAAGATCCGCCTGCGCCCCGGCGGCAAAAAGAAGTACAAGCTG
AAGCACATCGTGTGGGCCAGCCGCGAACTGGAGCGCTTCGCCGTGAACCCCGGCTCCTG
GAGACCAGCGAGGGGTGCCGCCAGATCCTCGGCCAACTGCAGCCCAGCCTGCAAACCGGC
AGCGAGGAGCTGCGCAGCCTGTACAACACCGTGGCCACGCTGTACTGCGTCCACCAGCGC
ATCGAAATCAAGGATACGAAAGAGGCCCTGGATAAAATCGAAGAGGAACAGAATAAGAGC
AAAAAGAAGGCCCAACAGGCCGCCGCGGACACCGGACACAGCAACCAGGTCAGCCAGAAC
TACCCCATCGTGCAGAACATCCAGGGGCAGATGGTGCACCAGGCCATCTCCCCCCGCACG
CTGAACGCCTGGGTGAAGGTGGTGGAAGAGAAGGCTTTTAGCCCGGAGGTGATACCCATG
TTCTCAGCCCTGTCAGAGGGAGCCACCCCCCAAGATCTGAACACCATGCTCAACACAGTG
GGGGGACACCAGGCCGCCATGCAGATGCTGAAGGAGACCATCAATGAGGAGGCTGCCGAA
TGGGATCGTGTGCATCCGGTGCACGCAGGGCCCATCGCACCGGGCCAGATGCGTGAGCCA
CGGGGCTCAGACATCGCCGGAACGACTAGTACCCTTCAGGAACAGATCGGCTGGATGACC
AACAACCCACCCATCCCGGTGGGAGAAATCTACAAACGCTGGATCATCCTGGGCCTGAAC
AAGATCGTGCGCATGTATAGCCCTACCAGCATCCTGGACATCCGCCAAGGCCCGAAGGAA
CCCTTTCGCGACTACGTGGACCGGTTCTACAAAACGCTCCGCGCCGAGCAGGCTAGCCAG
GAGGTGAAGAACTGGATGACCGAAACCCTGCTGGTCCAGAACGCGAACCCGGACTGCAAG
ACGATCCTGAAGGCCCTGGGCCCAGCGGCTACCCTAGAGGAAATGATGACCGCCTGTCAG
GGAGTGGGCGGACCCGGCCACAAGGCACGCGTCCTGGCTGAGGCCATGAGCCAGGTGACC
AACTCCGCTACCATCATGATGCAGCGCGGCAACTTTCGGAACCAACGCAAGATCGTCAAG
TGCTTCAACTGTGGCAAAGAAGGGCACACAGCCCGCAACTGCAGGGCCCCTAGGAAAAAG
GGCTGTTGGAAATGTGGAAAGGAAGGACACCAAATGAAAGATTGTACTGAGAGACAGGCT
AATTTTTTAGGGAAGATCTGGCCTTCCCACAAGGGAAGGCCAGGGAATTTTCTTCAGAGC
AGACCAGAGCCAACAGCCCCACCAGAAGAGAGCTTCAGGTTTGGGGAAGAGACAACAACT
CCCTCTCAGAAGCAGGAGCCGATAGACAAGGAACTGTATCCTTTAGCTTCCCTCAGATCA
CTCTTTGGCAGCGACCCCTCGTCACAATAAAGATAGGGGGGCAGCTCAAGGAGGCTCTCC
TGGACACCGGAGCAGACGACACCGTGCTGGAGGAGATGTCGTTGCCAGGCCGCTGGAAGC
CGAAGATGATCGGGGGAATCGGCGGTTTCATCAAGGTGCGCCAGTATGACCAGATCCTCA
TCGAAATCTGCGGCCACAAGGCTATCGGTACCGTGCTGGTGGGCCCCACACCCGTCAACA
TCATCGGACGCAACCTGTTGACGCAGATCGGTTGCACGCTGAACTTCCCCATTAGCCCTA
TCGAGACGGTACCGGTGAAGCTGAAGCCCGGGATGGACGGCCCGAAGGTCAAGCAATGGC
CATTGACAGAGGAGAAGATCAAGGCACTGGTGGAGATTTGCACAGAGATGGAAAAGGAAG
GGAAAATCTCCAAGATTGGGCCTGAGAACCCGTACAACACGCCGGTGTTCGCAATCAAGA
AGAAGGACTCGACGAAATGGCGCAAGCTGGTGGACTTCCGCGAGCTGAACAAGCGCACGC
AAGACTTCTGGGAGGTTCAGCTGGGCATCCCGCACCCCGCAGGGCTGAAGAAGAAGAAAT
CCGTGACCGTACTGGATGTGGGTGATGCCTACTTCTCCGTTCCCCTGGACGAAGACTTCA
GGAAGTACACTGCCTTCACAATCCCTTCGATCAACAACGAGACACCGGGGATTCGATATC
AGTACAACGTGCTGCCCCAGGGCTGGAAAGGCTCTCCCGCAATCTTCCAGAGTAGCATGA
CCAAAATCCTGGAGCCTTTCCGCAAACAGAACCCCGACATCGTCATCTATCAGTACATGG
ATGACTTGTACGTGGGCTCTGATCTAGAGATAGGGCAGCACCGCACCAAGATCGAGGAGC
TGCGCCAGCACCTGTTGAGGTGGGGACTGACCACACCCGACAAGAAGCACCAGAAGGAGC
 
CTCCCTTCCTCTGGATGGGTTACGAGCTGCACCCTGACAAATGGACCGTGCAGCCTATCG
TGCTGCCAGAGAAAGACAGCTGGACTGTCAACGACATACAGAAGCTGGTGGGAAGTTGA
ACTGGGCCAGTCAGATTTACCCAGGGATTAAGGTGAGGCAGCTGTGCAAACTCCTCCGCG
GAACCAAGGCACTCACAGAGGTGATCCCCCTAACCGAGGAGGCCGAGCTCGAACTGGCAG
AAAACCGAGAGATCCTAAAGGAGCCCGTGCACGGCGTGTACTATGACCCCTCCAAGGACC
TGATCGCCGAGATCCAGAAGCAGGGGCAAGGCCAGTGGACCTATCAGATTTACCAGGAGC
CCTTCAAGAACCTGAAGACCGGCAAGTACGCCCGGATGAGGGGTGCCCACACTAACGACG
TCAAGCAGCTGACCGAGGCCGTGCAGAAGATCACCACCGAAAGCATCGTGATCTGGGGAA
AGACTCCTAAGTTCAAGCTGCCCATCCAGAAGGAAACCTGGGAAACCTGGTGGACAGAGT
ATTGGCAGGCCACCTGGATTCCTGAGTGGGAGTTCGTCAACACCCCTCCCCTGGTGAAGC
TGTGGTACCAGCTGGAGAAGGAGCCCATAGTGGGCGCCGAAACCTTCTACGTGGATGGGG
CCGCTAACAGGGAGACTAAGCTGGGCAAAGCCGGATACGTCACTAACCGGGGCAGACAGA
AGGTTGTCACCCTCACTGACACCACCAACCAGAAGACTGAGCTGCAGGCCATTTACCTCG
CTTTGCAGGACTCGGGCCTGGAGGTGAACATCGTGACAGACTCTCAGTATGCCCTGGGCA
TCATTCAAGCCCAGCCAGACCAGAGTGAGTCCGAGCTGGTCAATCAGATCATCGAGCAGC
TGATCAAGAAGGAAAAGGTCTATCTGGCCTGGGTACCCGCCCACAAAGGCATTGGCGGCA
ATGAGCAGGTCGACAAGCTGGTCTCGGCTGGCATCAGGAAGGTGCTATTCCTGGATGGCA
TCGACAAGGCCCAGGACGAGCACGAGAAATACCACAGCAACTGGCGGGCCATGGCTAGCG
ACTTCAACCTGCCCCCTGTGGTGGCCAAAGAGATCGTGGCCAGCTGTGACAAGTGTCAGC
TCAAGGGCGAAGCCATGCATGGCCAGGTGGACTGTAGCCCCGGCATCTGGCAACTCGATT
GCACCCATCTGGAGGGCAAGGTTATCCTGGTAGCCGTCCATGTGGCCAGTGGCTACATCG
AGGCCGAGGTCATTCCCGCCGAAACAGGGCAGGAGACAGCCTACTTCCTCCTGAAGCTGG
CAGGCCGGTGGCCAGTGAAGACCATCCATACTGACAATGGCAGCAATTTCACCAGTGCTA
CGGTTAAGGCCGCCTGCTGGTGGGCGGGAATCAAGCAGGAGTTCGGGATCCCCTACAATC
CCCAGAGTCAGGGCGTCGTCGAGTCTATGAATAAGGAGTTAAAGAAGATTATCGGCCAGG
TCAGAGATCAGGCTGAGCATCTCAAGACCGCGGTCCAAATGGCGGTATTCATCCACAATT
TCAAGCGGAAGGGGGGGATTGGGGGGTACAGTGCGGGGGAGCGGATCGTGGACATCATCG
CGACCGACATCCAGACTAAGGAGCTGCAAAAGCAGATTACCAAGATTCAGAATTTCCGGG
TCTACTACAGGGACAGCAGAAATCCCCTCTGGAAAGGCCCAGCGAAGCTCCTCTGGAAGG
GTGAGGGGGCAGTAGTGATCCAGGATAATAGCGACATCAAGGTGGTGCCCAGAAGAAAGG
CGAAGATCATTAGGGATTATGGCAAACAGATGGCGGGTGATGATTGCGTGGCGAGCAGAC
AGGATGAGGATTAGGAATTGGGCTAGAGCGGCCGCTTCCCTTTAGTGAGGGTTAATGCTT
CGAGCAGACATGATAAGATACATTGATGAGTTTGGACAAACCACAACTAGAATGCAGTGA
AAAAAATGCTTTATTTGTGAAATTTGTGATGCTATTGCTTTATTTGTAACCATTATAAGC
TGCAATAAACAAGTTAACAACAACAATTGCATTCATTTTATGTTTCAGGTTCAGGGGGAG
ATGTGGGAGGTTTTTTAAAGCAAGTAAAACCTCTACAAATGTGGTAAAATCCGATAAGGA
TCGATCCGGGCTGGCGTAATAGCGAAGAGGCCCGCACCGATCGCCCTTCCCAACAGTTGC
GCAGCCTGAATGGCGAATGGACGCGCCCTGTAGCGGCGCATTAAGCGCGGCGGGTGTGGT
GGTTACGCGCAGCGTGACCGCTACACTTGCCAGCGCCCTAGCGCCCGCTCCTTTCGCTTT
CTTCCCTTCCTTTCTCGCCACGTTCGCCGGCTTTCCCCGTCAAGCTCTAAATCGGGGGCT
CCCTTTAGGGTTCCGATTTAGAGCTTTACGGCACCTCGACCGCAAAAAACTTGATTTGGG
TGATGGTTCACGTAGTGGGCCATCGCCCTGATAGACGGTTTTTCGCCCTTTGACGTTGGA
GTCCACGTTCTTTAATAGTGGACTCTTGTTCCAAACTGGAACAACACTCAACCCTATCTC
GGTCTATTCTTTTGATTTATAAGGGATTTTGCCGATTTCGGCCTATTGGTTAAAAAATGA
GCTGATTTAACAAATATTTAACGCGAATTTTAACAAAATATTAACGTTTACAATTTCGCC
TGATGCGGTATTTTCTCCTTACGCATCTGTGCGGTATTTCACACCGCATACGCGGATCTG
CGCAGCACCATGGCCTGAAATAACCTCTGAAAGAGGAACTTGGTTAGGTACCTTCTGAGG
CGGAAAGAACCAGCTGTGGAATGTGTGTCAGTTAGGGTGTGGAAAGTCCCCAGGCTCCCC
AGCAGGCAGAAGTATGCAAAGCATGCATCTCAATTAGTCAGCAACCAGGTGTGGAAAGTC
CCCAGGCTCCCCAGCAGGCAGAAGTATGCAAAGCATGCATCTCAATTAGTCAGCAACCAT
AGTCCCGCCCCTAACTCCGCCCATCCCGCCCCTAACTCCGCCCAGTTCCGCCCATTCTCC
GCCCCATGGCTGACTAATTTTTTTTATTTATGCAGAGGCCGAGGCCGCCTCGGCCTCTGA
GCTATTCCAGAAGTAGTGAGGAGGCTTTTTTGGAGGCCTAGGCTTTTGCAAAAAGCTTGA
TTCTTCTGACACAACAGTCTCGAACTTAAGGCTAGAGCCACCATGATTGAACAAGATGGA
TTGCACGCAGGTTCTCCGGCCGCTTGGGTGGAGAGGCTATTCGGCTATGACTGGGCACAA
CAGACAATCGGCTGCTCTGATGCCGCCGTGTTCCGGCTGTCAGCGCAGGGGCGCCCGGTT
CTTTTTGTCAAGACCGACCTGTCCGGTGCCCTGAATGAACTGCAGGACGAGGCAGCGCGG
CTATCGTGGCTGGCCACGACGGGCGTTCCTTGCGCAGCTGTGCTCGACGTTGTCACTGAA
GCGGGAAGGGACTGGCTGCTATTGGGCGAAGTGCCGGGGCAGGATCTCCTGTCATCTCAC
CTTGCTCCTGCCGAGAAAGTATCCATCATGGCTGATGCAATGCGGCGGCTGCATACGCTT
GATCCGGCTACCTGCCCATTCGACCACCAAGCGAAACATCGCATCGAGCGAGCACGTACT
 
CGGATGGAAGCCGGTCTTGTCGATCAGGATGATCTGGACGAAGAGCATCAGGGGCTCGCG
CCAGCCGAACTGTTCGCCAGGCTCAAGGCGCGCATGCCCGACGGCGAGGATCTCGTCGTG
ACCCATGGCGATGCCTGCTTGCCGAATATCATGGTGGAAAATGGCCGCTTTTCTGGATTC
ATCGACTGTGGCCGGCTGGGTGTGGCGGACCGCTATCAGGACATAGCGTTGGCTACCCGT
GATATTGCTGAAGAGCTTGGCGGCGAATGGGCTGACCGCTTCCTCGTGCTTTACGGTATC
GCCGCTCCCGATTCGCAGCGCATCGCCTTCTATCGCCTTCTTGACGAGTTCTTCTGAGCG
GGACTCTGGGGTTCGAAATGACCGACCAAGCGACGCCCAACCTGCCATCACGATGGCCGC
AATAAAATATCTTTATTTTCATTACATCTGTGTGTTGGTTTTTTGTGTGAATCGATAGCG
ATAAGGATCCGCGTATGGTGCACTCTCAGTACAATCTGCTCTGATGCCGCATAGTTAAGC
CAGCCCCGACACCCGCCAACACCCGCTGACGCGCCCTGACGGGCTTGTCTGCTCCCGGCA
TCCGCTTACAGACAAGCTGTGACCGTCTCCGGGAGCTGCATGTGTCAGAGGTTTTCACCG
TCATCACCGAAACGCGCGAGACGAAAGGGCCTCGTGATACGCCTATTTTTATAGGTTAAT
GTCATGATAATAATGGTTTCTTAGACGTCAGGTGGCACTTTTCGGGGAAATGTGCGCGGA
ACCCCTATTTGTTTATTTTTCTAAATACATTCAAATATGTATCCGCTCATGAGACAATAA
CCCTGATAAATGCTTCAATAATATTGAAAAAGGAAGAGTATGAGTATTCAACATTTCCGT
GTCGCCCTTATTCCCTTTTTTGCGGCATTTTGCCTTCCTGTTTTTGCTCACCCAGAAACG
CTGGTGAAAGTAAAAGATGCTGAAGATCAGTTGGGTGCACGAGTGGGTTACATCGAACTG
GATCTCAACAGCGGTAAGATCCTTGAGAGTTTTCGCCCCGAAGAACGTTTTCCAATGATG
AGCACTTTTAAAGTTCTGCTATGTGGCGCGGTATTATCCCGTATTGACGCCGGGCAAGAG
CAACTCGGTCGCCGCATACACTATTCTCAGAATGACTTGGTTGAGTACTCACCAGTCACA
GAAAAGCATCTTACGGATGGCATGACAGTAAGAGAATTATGCAGTGCTGCCATAACCATG
AGTGATAACACTGCGGCCAACTTACTTCTGACAACGATCGGAGGACCGAAGGAGCTAACC
GCTTTTTTGCACAACATGGGGGATCATGTAACTCGCCTTGATCGTTGGGAACCGGAGCTG
AATGAAGCCATACCAAACGACGAGCGTGACACCACGATGCCTGTAGCAATGGCAACAACG
TTGCGCAAACTATTAACTGGCGAACTACTTACTCTAGCTTCCCGGCAACAATTAATAGAC
TGGATGGAGGCGGATAAAGTTGCAGGACCACTTCTGCGCTCGGCCCTTCCGGCTGGCTGG
TTTATTGCTGATAAATCTGGAGCCGGTGAGCGTGGGTCTCGCGGTATCATTGCAGCACTG
GGGCCAGATGGTAAGCCCTCCCGTATCGTAGTTATCTACACGACGGGGAGTCAGGCAACT
ATGGATGAACGAAATAGACAGATCGCTGAGATAGGTGCCTCACTGATTAAGCATTGGTAA
CTGTCAGACCAAGTTTACTCATATATACTTTAGATTGATTTAAAACTTCATTTTTAATTT
AAAAGGATCTAGGTGAAGATCCTTTTTGATAATCTCATGACCAAAATCCCTTAACGTGAG
TTTTCGTTCCACTGAGCGTCAGACCCCGTAGAAAAGATCAAAGGATCTTCTTGAGATCCT
TTTTTTCTGCGCGTAATCTGCTGCTTGCAAACAAAAAAACCACCGCTACCAGCGGTGGTT
TGTTTGCCGGATCAAGAGCTACCAACTCTTTTTCCGAAGGTAACTGGCTTCAGCAGAGCG
CAGATACCAAATACTGTCCTTCTAGTGTAGCCGTAGTTAGGCCACCACTTCAAGAACTCT
GTAGCACCGCCTACATACCTCGCTCTGCTAATCCTGTTACCAGTGGCTGCTGCCAGTGGC
GATAAGTCGTGTCTTACCGGGTTGGACTCAAGACGATAGTTACCGGATAAGGCGCAGCGG
TCGGGCTGAACGGGGGGTTCGTGCACACAGCCCAGCTTGGAGCGAACGACCTACACCGAA
CTGAGATACCTACAGCGTGAGCTATGAGAAAGCGCCACGCTTCCCGAAGGGAGAAAGGCG
GACAGGTATCCGGTAAGCGGCAGGGTCGGAACAGGAGAGCGCACGAGGGAGCTTCCAGGG
GGAAACGCCTGGTATCTTTATAGTCCTGTCGGGTTTCGCCACCTCTGACTTGAGCGTCGA
TTTTTGTGATGCTCGTCAGGGGGGCGGAGCCTATGGAAAAACGCCAGCAACGCGGCCTTT
TTACGGTTCCTGGCCTTTTGCTGGCCTTTTGCTCACATGGCTCGACAGATCT
SEQ. ID. NO. 3 - Envelope Gene from HIV-1 MN (Genbank accession no. M17449) 
ATGAGAGTGA AGGGGATCAG GAGGAATTAT CAGCACTGGT GGGGATGGGG CACGATGCTC 60
CTTGGGTTAT TAATGATCTG TAGTGCTACA GAAAAATTGT GGGTCACAGT CTATTATGGG 120
GTACCTGTGT GGAAAGAAGC AACCACCACT CTATTTTGTG CATCAGATGC TAAAGCATAT 180
GATACAGAGG TACATAATGT TTGGGCCACA CAAGCCTGTG TACCCACAGA CCCCAACCCA 240
CAAGAAGTAG AATTGGTAAA TGTGACAGAA AATTTTAACA TGTGGAAAAA TAACATGGTA 300
GAACAGATGC ATGAGGATAT AATCAGTTTA TGGGATCAAA GCCTAAAGCC ATGTGTAAAA 360
TTAACCCCAC TCTGTGTTAC TTTAAATTGC ACTGATTTGA GGAATACTAC TAATACCAAT 420
AATAGTACTG CTAATAACAA TAGTAATAGC GAGGGAACAA TAAAGGGAGG AGAAATGAAA 480
AACTGCTCTT TCAATATCAC CACAAGCATA AGAGATAAGA TGCAGAAAGA ATATGCACTT 540
CTTTATAAAC TTGATATAGT ATCAATAGAT AATGATAGTA CCAGCTATAG GTTGATAAGT 600
TGTAATACCT CAGTCATTAC ACAAGCTTGT CCAAAGATAT CCTTTGAGCC AATTCCCATA 660
CACTATTGTG CCCCGGCTGG TTTTGCGATT CTAAAATGTA ACGATAAAAA GTTCAGTGGA 720
AAAGGATCAT GTAAAAATGT CAGCACAGTA CAATGTACAC ATGGAATTAG GCCAGTAGTA 780
TCAACTCAAC TGCTGTTAAA TGGCAGTCTA GCAGAAGAAG AGGTAGTAAT TAGATCTGAG 840
AATTTCACTG ATAATGCTAA AACCATCATA GTACATCTGA ATGAATCTGT ACAAATTAAT 900
TGTACAAGAC CCAACTACAA TAAAAGAAAA AGGATACATA TAGGACCAGG GAGAGCATTT 960
TATACAACAA AAAATATAAT AGGAACTATA AGACAAGCAC ATTGTAACAT TAGTAGAGCA 1020
AAATGGAATG ACACTTTAAG ACAGATAGTT AGCAAATTAA AAGAACAATT TAAGAATAAA 1080
ACAATAGTCT TTAATCAATC CTCAGGAGGG GACCCAGAAA TTGTAATGCA CAGTTTTAAT 1140
TGTGGAGGGG AATTTTTCTA CTGTAATACA TCACCACTGT TTAATAGTAC TTGGAATGGT 1200
AATAATACTT GGAATAATAC TACAGGGTCA AATAACAATA TCACACTTCA ATGCAAAATA 1260
AAACAAATTA TAAACATGTG GCAGGAAGTA GGAAAAGCAA TGTATGCCCC TCCCATTGAA 1320
GGACAAATTA GATGTTCATC AAATATTACA GGGCTACTAT TAACAAGAGA TGGTGGTAAG 1380
GACACGGACA CGAACGACAC CGAGATCTTC AGACCTGGAG GAGGAGATAT GAGGGACAAT 1440
TGGAGAAGTG AATTATATAA ATATAAAGTA GTAACAATTG AACCATTAGG AGTAGCACCC 1500
ACCAAGGCAA AGAGAAGAGT GGTGCAGAGA GAAAAAAGAG CAGCGATAGG AGCTCTGTTC 1560
CTTGGGTTCT TAGGAGCAGC AGGAAGCACT ATGGGCGCAG CGTCAGTGAC GCTGACGGTA 1620
CAGGCCAGAC TATTATTGTC TGGTATAGTG CAACAGCAGA ACAATTTGCT GAGGGCCATT 1680
GAGGCGCAAC AGCATATGTT GCAACTCACA GTCTGGGGCA TCAAGCAGCT CCAGGCAAGA 1740
GTCCTGGCTG TGGAAAGATA CCTAAAGGAT CAACAGCTCC TGGGGTTTTG GGGTTGCTCT 1800
GGAAAACTCA TTTGCACCAC TACTGTGCCT TGGAATGCTA GTTGGAGTAA TAAATCTCTG 1860
GATGATATTT GGAATAACAT GACCTGGATG CAGTGGGAAA GAGAAATTGA CAATTACACA 1920
AGCTTAATAT ACTCATTACT AGAAAAATCG CAAACCCAAC AAGAAAAGAA TGAACAAGAA 1980
TTATTGGAAT TGGATAAATG GGCAAGTTTG TGGAATTGGT TTGACATAAC AAATTGGCTG 2040
TGGTATATAA AAATATTCAT AATGATAGTA GGAGGCTTGG TAGGTTTAAG AATAGTTTTT 2100
 
GCTGTACTTT CTATAGTGAA TAGAGTTAGG CAGGGATACT CACCATTGTC GTTGCAGACC 2160
CGCCCCCCAG TTCCGAGGGG ACCCGACAGG CCCGAAGGAA TCGAAGAAGA AGGTGGAGAG 2220
AGAGACAGAG ACACATCCGG TCGATTAGTG CATGGATTCT TAGCAATTAT CTGGGTCGAC 2280
CTGCGGAGCC TGTTCCTCTT CAGCTACCAC CACAGAGACT TACTCTTGAT TGCAGCGAGG 2340
ATTGTGGAAC TTCTGGGACG CAGGGGGTGG GAAGTCCTCA AATATTGGTG GAATCTCCTA 2400
CAGTATTGGA GTCAGGAACT AAAGAGTAGT GCTGTTAGCT TGCTTAATGC CACAGCTATA 2460
GCAGTAGCTG AGGGGACAGA TAGGGTTATA GAAGTACTGC AAAGAGCTGG TAGAGCTATT 2520
CTCCACATAC CTACAAGAAT AAGACAGGGC TTGGAAAGGG CTTTGCTATA A 2571
SEQ. I.D. NO. 4 - SYNgp-160mn - codon optimised env sequence 
ATGAGGGTGA AGGGGATCCG CCGCAACTAC CAGCACTGGT GGGGCTGGGG CACGATGCTC 60
CTGGGGCTGC TGATGATCTG CAGCGCCACC GAGAAGCTGT GGGTGACCGT GTACTACGGC 120
GTGCCCGTGT GGAAGGAGGC CACCACCACC CTGTTCTGCG CCAGCGACGC CAAGGCGTAC 180
GACACCGAGG TGCACAACGT GTGGGCCACC CAGGCGTGCG TGCCCACCGA CCCCAACCCC 240
CAGGAGGTGG AGCTCGTGAA CGTGACCGAG AACTTCAACA TGTGGAAGAA CAACATGGTG 300
GAGCAGATGC ATGAGGACAT CATCAGCCTG TGGGACCAGA GCCTGAAGCC CTGCGTGAAG 360
CTGACCCCCC TGTGCGTGAC CCTGAACTGC ACCGACCTGA GGAACACCAC CAACACCAAC 420
AACAGCACCG CCAACAACAA CAGCAACAGC GAGGGCACCA TCAAGGGCGG CGAGATGAAG 480
AACTGCAGCT TCAACATCAC CACCAGCATC CGCGACAAGA TGCAGAAGGA GTACGCCCTG 540
CTGTACAAGC TGGATATCGT GAGCATCGAC AACGACAGCA CCAGCTACCG CCTGATCTCC 600
TGCAACACCA GCGTGATCAC CCAGGCCTGC CCCAAGATCA GCTTCGAGCC CATCCCCATC 660
CACTACTGCG CCCCCGCCGG CTTCGCCATC CTGAAGTGCA ACGACAAGAA GTTCAGCGGC 720
AAGGGCAGCT GCAAGAACGT GAGCACCGTG CAGTGCACCC ACGGCATCCG GCCGGTGGTG 780
AGCACCCAGC TCCTGCTGAA CGGCAGCCTG GCCGAGGAGG AGGTGGTGAT CCGCAGCGAG 840
AACTTCACCG ACAACGCCAA GACCATCATC GTGCACCTGA ATGAGAGCGT GCAGATCAAC 900
TGCACGCGTC CCAACTACAA CAAGCGCAAG CGCATCCACA TCGGCCCCGG GCGCGCCTTC 960
TACACCACCA AGAACATCAT CGGCACCATC CGCCAGGCCC ACTGCAACAT CTCTAGAGCC 1020
AAGTGGAACG ACACCCTGCG CCAGATCGTG AGCAAGCTGA AGGAGCAGTT CAAGAACAAG 1080
ACCATCGTGT TCAACCAGAG CAGCGGCGGC GACCCCGAGA TCGTGATGCA CAGCTTCAAC 1140
TGCGGCGGCG AATTCTTCTA CTGCAACACC AGCCCCCTGT TCAACAGCAC CTGGAACGGC 1200
AACAACACCT GGAACAACAC CACCGGCAGC AACAACAATA TTACCCTCCA GTGCAAGATC 1260
AAGCAGATCA TCAACATGTG GCAGGAGGTG GGCAAGGCCA TGTACGCCCC CCCCATCGAG 1320
GGCCAGATCC GGTGCAGCAG CAACATCACC GGTCTGCTGC TGACCCGCGA CGGCGGCAAG 1380
GACACCGACA CCAACGACAC CGAAATCTTC CGCCCCGGCG GCGGCGACAT GCGCGACAAC 1440
TGGAGATCTG AGCTGTACAA GTACAAGGTG GTGACGATCG AGCCCCTGGG CGTGGCCCCC 1500
ACCAAGGCCA AGCGCCGCGT GGTGCAGCGC GAGAAGCGGG CCGCCATCGG CGCCCTGTTC 1560
CTGGGCTTCC TGGGGGCGGC GGGCAGCACC ATGGGGGCCG CCAGCGTGAC CCTGACCGTG 1620
CAGGCCCGCC TGCTCCTGAG CGGCATCGTG CAGCAGCAGA ACAACCTCCT CCGCGCCATC 1680
GAGGCCCAGC AGCATATGCT CCAGCTCACC GTGTGGGGCA TCAAGCAGCT CCAGGCCCGC 1740
GTGCTGGCCG TGGAGCGCTA CCTGAAGGAC CAGCAGCTCC TGGGCTTCTG GGGCTGCTCC 1800
GGCAAGCTGA TCTGCACCAC CACGGTACCC TGGAACGCCT CCTGGAGCAA CAAGAGCCTG 1860
GACGACATCT GGAACAACAT GACCTGGATG CAGTGGGAGC GCGAGATCGA TAACTACACC 1920
AGCCTGATCT ACAGCCTGCT GGAGAAGAGC CAGACCCAGC AGGAGAAGAA CGAGCAGGAG 1980
CTGCTGGAGC TGGACAAGTG GGCGAGCCTG TGGAACTGGT TCGACATCAC CAACTGGCTG 2040
TGGTACATCA AAATCTTCAT CATGATTGTG GGCGGCCTGG TGGGCCTCCG CATCGTGTTC 2100
GCCGTGCTGA GCATCGTGAA CCGCGTGCGC CAGGGCTACA GCCCCCTGAG CCTCCAGACC 2160
 
CGGCCCCCCG TGCCGCGCGG GCCCGACCGC CCCGAGGGCA TCGAGGAGGA GGGCGGCGAG 2220
CGCGACCGCG ACACCAGCGG CAGGCTCGTG CACGGCTTCC TGGCGATCAT CTGGGTCGAC 2280
CTCCGCAGCC TGTTCCTGTT CAGCTACCAC CACCGCGACC TGCTGCTGAT CGCCGCCCGC 2340
ATCGTGGAAC TCCTAGGCCG CCGCGGCTGG GAGGTGCTGA AGTACTGGTG GAACCTCCTC 2400
CAGTATTGGA GCCAGGAGCT GAAGTCCAGC GCCGTGAGCC TGCTGAACGC CACCGCCATC 2460
GCCGTGGCCG AGGGCACCGA CCGCGTGATC GAGGTGCTCC AGAGGGCCGG GAGGGCGATC 2520
CTGCACATCC CCACCCGCAT CCGCCAGGGG CTCGAGAGGG CGCTGCTGTA A 2571
SEQ ID No. 5 - pESYNGP 
TCAATATTGGCCATTAGCCATATTATTCATTGGTTATATAGCATAAATCAATATTGGCTA
TTGGCCATTGCATACGTTGTATCTATATCATAATATGTACATTTATATTGGCTCATGTCC
AATATGACCGCCATGTTGGCATTGATTATTGACTAGTTATTAATAGTAATCAATTACGGG
GTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCAT
AGTAACGCCAATAGGGACTTTCCATTGACGTCAATGGGTGGAGTATTTACGGTAAACTGC
CCACTTGGCAGTACATCAAGTGTATCATATGCCAAGTCCGCCCCCTATTGACGTCAATGA
CGGTAAATGGCCCGCCTGGCATTATGCCCAGTACATGACCTTACGGGACTTTCCTACTTG
GCAGTACATCTACGTATTAGTCATCGCTATTACCATGGTGATGCGGTTTTGGCAGTACAC
CAATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTG
CGATCGCCCGCCCCGTTGACGCAAATGGGCGGTAGGCGTGTACGGTGGGAGGTCTATATA
AGCAGAGCTCGTTTAGTGAACCGTCAGATCACTAGAAGCTTTATTGCGGTAGTTTATCAC
AGTTAAATTGCTAACGCAGTCAGTGCTTCTGACACAACAGTCTCGAACTTAAGCTGCAGT
GACTCTCTTAAGGTAGCCTTGCAGAAGTTGGTCGTGAGGCACTGGGCAGGTAAGTATCAA
GGTTACAAGACAGGTTTAAGGAGACCAATAGAAACTGGGCTTGTCGAGACAGAGAAGACT
CTTGCGTTTCTGATAGGCACCTATTGGTCTTACTGACATCCACTTTGCCTTTCTCTCCAC
AGGTGTCCACTCCCAGTTCAATTACAGCTCTTAAGGCTAGAGTACTTAATACGACTCACT
ATAGGCTAGAGAATTCGCCACCATGGGCGATCCCCTCACCTGGTCCAAAGCCCTGAAGAA
ACTGGAAAAAGTCACCGTTCAGGGTAGCCAAAAGCTTACCACAGGCAATTGCAACTGGGC
ATTGTCCCTGGTGGATCTTTTCCACGACACTAATTTCGTTAAGGAGAAAGATTGGCAACT
CAGAGACGTGATCCCCCTCTTGGAGGACGTGACCCAAACATTGTCTGGGCAGGAGCGCGA
AGCTTTCGAGCGCACCTGGTGGGCCATCAGCGCAGTCAAAATGGGGCTGCAAATCAACAA
CGTGGTTGACGGTAAAGCTAGCTTTCAACTGCTCCGCGCTAAGTACGAGAAGAAAACCGC
CAACAAGAAACAATCCGAACCTAGCGAGGAGTACCCAATTATGATCGACGGCGCCGGCAA
TAGGAACTTCCGCCCACTGACTCCCAGGGGCTATACCACCTGGGTCAACACCATCCAGAC
AAACGGACTTTTGAACGAAGCCTCCCAGAACCTGTTCGGCATCCTGTCTGTGGACTGCAC
CTCCGAAGAAATGAATGCTTTTCTCGACGTGGTGCCAGGACAGGCTGGACAGAAACAGAT
CCTGCTCGATGCCATTGACAAGATCGCCGACGACTGGGATAATCGCCACCCCCTGCCAAA
CGCCCCTCTGGTGGCTCCCCCACAGGGGCCTATCCCTATGACCGCTAGGTTCATTAGGGG
ACTGGGGGTGCCCCGCGAACGCCAGATGGAGCCAGCATTTGACCAATTTAGGCAGACCTA
CAGACAGTGGATCATCGAAGCCATGAGCGAGGGGATTAAAGTCATGATCGGAAAGCCCAA
GGCACAGAACATCAGGCAGGGGGCCAAGGAACCATACCCTGAGTTTGTCGACAGGCTTCT
GTCCCAGATTAAATCCGAAGGCCACCCTCAGGAGATCTCCAAGTTCTTGACAGACACACT
GACTATCCAAAATGCAAATGAAGAGTGCAGAAACGCCATGAGGCACCTCAGACCTGAAGA
TACCCTGGAGGAGAAAATGTACGCATGTCGCGACATTGGCACTACCAAGCAAAAGATGAT
GCTGCTCGCCAAGGCTCTGCAAACCGGCCTGGCTGGTCCATTCAAAGGAGGAGCACTGAA
GGGAGGTCCATTGAAAGCTGCACAAACATGTTATAATTGTGGAAGCCAGGACATTTATC
TAGTCAATGTAGAGCACCTAAAGTCTGTTTTAAATGTAAACAGCCTGGACATTTCTCAAA
GCAATGCAGAAGTGTTCCAAAAAACGGAAGCAAGGGGCTCAAGGGAGGCCCCAGAAACA
AACTTTCCCGATACAACAGAAGAGTCAGCACAACAAATCTGTTGTACAAGAGACTCCTCA
GACTCAAAATCTGTACCCAGATCTGAGCGAAATAAAAAAGGAATACAATGTCAAGGAGAA
GGATCAAGTAGAGGATCTCAACCTGGACAGTTTGTGGAGTAACATACAATCTCGAGAAG
AGGCCCACTACCATCGTCCTGATCAATGACACCCCTCTTAATGTGCTGCTGGACACCGGA
GCCGACACCAGCGTTCTCACTACTGCTCACTATAACAGACTGAAATACAGAGGAAGGAAA
TACCAGGGCACAGGCATCATCGGCGTTGGAGGCAACGTCGAAACCTTTTCCACTCCTGTC
ACCATCAAAAAGAAGGGGAGACACATTAAAACCAGAATGCTGGTCGCCGACATCCCCGTC
ACCATCCTTGGCAGAGACATTCTCCAGGACCTGGGCGCTAAACTCGTGCTGGCACAACTG
TCTAAGGAAATCAAGTTCCGCAAGATCGAGCTGAAAGAGGGCACAATGGGTCCAAAAATC
CCCCAGTGGCCCCTGACCAAAGAGAAGCTTGAGGGCGCTAAGGAAATCGTGCAGCGCCTG
CTTTCTGAGGGCAAGATTAGCGAGGCCAGCGACAATAACCCTTACAACAGCCCCATCTTT
GTGATTAAGAAAAGGAGCGGCAAATGGAGACTCCTGCAGGACCTGAGGGAACTCAACAAG
ACCGTCCAGGTCGGAACTGAGATCTCTCGCGGACTGCCTCACCCCGGCGGCCTGATTAAA
TGCAAGCACATGACAGTCCTTGACATTGGAGACGCTTATTTACCATCCCCCTCGATCCT
GAATTTCGCCCCTATACTGCTTTTACCATCCCCAGCATCAATCACCAGGAGCCCGATAAA
CGCTATGTGTGGAAGTGCCTCCCCCAGGGATTTGTGCTTAGCCCCTACATTTACCAGAAG
 
ACACTTCAAGAGATCCTCCAACCTTTCCGCGAAAGATACCCAGAGGTTCAACTCTACCAA
TATATGGACGACCTGTTCATGGGGTCCAACGGGTCTAAGAAGCAGCACAAGGAACTCATC
ATCGAACTGAGGGCAATCCTCCTGGAGAAAGGCTTCGAGACACCCGACGACAAGCTGCAA
GAAGTTCCTCCATATAGCTGGCTGGGCTACCAGCTTTGCCCTGAAAACTGGAAAGTCCAG
AAGATGCAGTTGGATATGGTCAAGAACCCAACACTGAACGACGTCCAGAAGCTCATGGGC
AATATTACCTGGATGAGCTCCGGAATCCCTGGGCTTACCGTTAAGCACATTGCCGCAACT
ACAAAAGGATGCCTGGAGTTGAACCAGAAGGTCATTTGGACAGAGGAAGCTCAGAAGGAA
CTGGAGGAGAATAATGAAAAGATTAAGAATGCTCAAGGGCTCCAATACTACAATCCCGAA
GAAGAAATGTTGTGCGAGGTCGAAATCACTAAGAACTACGAAGCCACCTATGTCATCAAA
CAGTCCCAAGGCATCTTGTGGGCCGGAAAGAAAATCATGAAGGCCAACAAAGGCTGGTCC
ACCGTTAAAAATCTGATGCTCCTGCTCCAGCACGTCGCCACCGAGTCTATCACCCGCGTC
GGCAAGTGCCCCACCTTCAAAGTTCCCTTCACTAAGGAGCAGGTGATGTGGGAGATGCAA
AAAGGCTGGTACTACTCTTGGCTTCCCGAGATCGTCTACACCCACCAAGTGGTGCACGAC
GACTGGAGAATGAAGCTTGTCGAGGAGCCCACTAGCGGAATTACAATCTATACCGACGGC
GGAAAGCAAAACGGAGAGGGAATCGCTGCATACGTCACATCTAACGGCCGCACCAAGCAA
AAGAGGCTCGGCCCTGTCACTCACCAGGTGGCTGAGAGGATGGCTATCCAGATGGCCCTT
GAGGACACTAGAGACAAGCAGGTGAACATTGTGACTGACAGCTACTACTGCTGGAAAAAC
ATCACAGAGGGCCTTGGCCTGGAGGGACCCCAGTCTCCCTGGTGGCCTATCATCCAGAAT
ATCCGCGAAAAGGAAATTGTCTATTTCGCCTGGGTGCCTGGACACAAAGGAATTTACGGC
AACCAACTCGCCGATGAAGCCGCCAAAATTAAAGAGGAAATCATGCTTGCCTACCAGGGC
ACACAGATTAAGGAGAAGAGAGACGAGGACGCTGGCTTTGACCTGTGTGTGCCATACGAC
ATCATGATTCCCGTTAGCGACACAAAGATCATTCCAACCGATGTCAAGATCCAGGTGCCA
CCCAATTCATTTGGTTGGGTGACCGGAAAGTCCAGCATGGCTAAGCAGGGTCTTCTGATT
AACGGGGGAATCATTGATGAAGGATACACCGGCGAAATCCAGGTGATCTGCACAAATATC
GGCAAAAGCAATATTAAGCTTATCGAAGGGCAGAAGTTCGCTCAACTCATCATCCTCCAG
CACCACAGCAATTCAAGACAACCTTGGGACGAAAACAAGATTAGCCAGAGAGGTGACAAG
GGCTTCGGCAGCACAGGTGTGTTCTGGGTGGAGAACATCCAGGAAGCACAGGACGAGCAC
GAGAATTGGCACACCTCCCCTAAGATTTTGGCCCGCAATTACAAGATCCCACTGACTGTG
GCTAAGCAGATCACACAGGAATGCCCCCACTGCACCAAACAAGGTTCTGGCCCCGCCGGC
TGCGTGATGAGGTCCCCCAATCACTGGCAGGCAGATTGCACCCACCTCGACAACAAAATT
ATCCTGACCTTCGTGGAGAGCAATTCCGGCTACATCCACGCAACACTCCTCTCCAAGGAA
AATGCATTGTGCACCTCCCTCGCAATTCTGGAATGGGCCAGGCTGTTCTCTCCAAAATCC
CTGCACACCGACAACGGCACCAACTTTGTGGCTGAACCTGTGGTGAATCTGCTGAAGTTC
CTGAAAATCGCCCACACCACTGGCATTCCCTATCACCCTGAAAGCCAGGGCATTGTCGAG
AGGGCCAACAGAACTCTGAAAGAAAAGATCCAATCTCACAGAGACAATACACAGACATTG
GAGGCCGCACTTCAGCTCGCCCTTATCACCTGCAACAAAGGAAGAGAAAGCATGGGCGGC
CAGACCCCCTGGGAGGTCTTCATCACTAACCAGGCCCAGGTCATCCATGAAAAGCTGCTC
TTGCAGCAGGCCCAGTCCTCCAAAAAGTTCTGCTTTTATAAGATCCCCGGTGAGCACGAC
TGGAAAGGTCCTACAAGAGTTTTGTGGAAAGGAGACGGCGCAGTTGTGGTGAACGATGAG
GGCAAGGGGATCATCGCTGTGCCCCTGACACGCACCAAGCTTCTCATCAAGCCAAACTGA←
ACCCGGGGCGGCCGCTTCCCTTTAGTGAGGGTTAATGCTTCGAGCAGACATGATAAGATA
CATTGATGAGTTTGGACAAACCACAACTAGAATGCAGTGAAAAAAATGCTTTATTTGTGA
AATTTGTGATGCTATTGCTTTATTTGTAACCATTATAAGCTGCAATAAACAAGTTAACAA
CAACAATTGCATTCATTTTATGTTTCAGGTTCAGGGGGAGATGTGGGAGGTTTTTTAAAG
CAAGTAAAACCTCTACAAATGTGGTAAAATCCGATAAGGATCGATCCGGGCTGGCGTAAT
AGCGAAGAGGCCCGCACCGATCGCCCTTCCCAACAGTTGCGCAGCCTGAATGGCGAATGG
ACGCGCCCTGTAGCGGCGCATTAAGCGCGGCGGGTGTGGTGGTTACGCGCAGCGTGACCG
CTACACTTGCCAGCGCCCTAGCGCCCGCTCCTTTCGCTTTCTTCCCTTCCTTTCTCGCCA
CGTTCGCCGGCTTTCCCCGTCAAGCTCTAAATCGGGGGCTCCCTTTAGGGTTCCGATTTA
GAGCTTTACGGCACCTCGACCGCAAAAAACTTGATTTGGGTGATGGTTCACGTAGTGGGC
CATCGCCCTGATAGACGGTTTTTCGCCCTTTGACGTTGGAGTCCACGTTCTTTAATAGTG
GACTCTTGTTCCAAACTGGAACAACACTCAACCCTATCTCGGTCTATTCTTTTGATTTAT
AAGGGATTTTGCCGATTTCGGCCTATTGGTTAAAAAATGAGCTGATTTAACAAATATTTA
ACGCGAATTTTAACAAAATATTAACGTTTACAATTTCGCCTGATGCGGTATTTTCTCCTT
ACGCATCTGTGCGGTATTTCACACCGCATACGCGGATCTGCGCAGCACCATGGCCTGAAA
TAACCTCTGAAAGAGGAACTTGGTTAGGTACCTTCTGAGGCGGAAAGAACCAGCTGTGGA
ATGTGTGTCAGTTAGGGTGTGGAAAGTCCCCAGGCTCCCCAGCAGGCAGAAGTATGCAAA
GCATGCATCTCAATTAGTCAGCAACCAGGTGTGGAAAGTCCCCAGGCTCCCCAGCAGGCA
GAAGTATGCAAAGCATGCATCTCAATTAGTCAGCAACCATAGTCCCGCCCCTAACTCCGC
CCATCCCGCCCCTAACTCCGCCCAGTTCCGCCCATTCTCCGCCCCATGGCTGACTAATTT
TTTTTATTTATGCAGAGGCCGAGGCCGCCTCGGCCTCTGAGCTATTCCAGAAGTAGTGAG
 
GAGGCTTTTTTGGAGGCCTAGGCTTTTGCAAAAAGCTTGATTCTTCTGACACAACAGTCT
CGAACTTAAGGCTAGAGCCACCATGATTGAACAAGATGGATTGCACGCAGGTTCTCCGGC
CGCTTGGGTGGAGAGGCTATTCGGCTATGACTGGGCACAACAGACAATCGGCTGCTCTGA
TGCCGCCGTGTTCCGGCTGTCAGCGCAGGGGCGCCCGGTTCTTTTTGTCAAGACCGACCT
GTCCGGTGCCCTGAATGAACTGCAGGACGAGGCAGCGCGGCTATCGTGGCTGGCCACGAC
GGGCGTTCCTTGCGCAGCTGTGCTCGACGTTGTCACTGAAGCGGGAAGGGACTGGCTGCT
ATTGGGCGAAGTGCCGGGGCAGGATCTCCTGTCATCTCACCTTGCTCCTGCCGAGAAAGT
ATCCATCATGGCTGATGCAATGCGGCGGCTGCATACGCTTGATCCGGCTACCTGCCCATT
CGACCACCAAGCGAAACATCGCATCGAGCGAGCACGTACTCGGATGGAAGCCGGTCTTGT
CGATCAGGATGATCTGGACGAAGAGCATCAGGGGCTCGCGCCAGCCGAACTGTTCGCCAG
GCTCAAGGCGCGCATGCCCGACGGCGAGGATCTCGTCGTGACCCATGGCGATGCCTGCTT
GCCGAATATCATGGTGGAAAATGGCCGCTTTTCTGGATTCATCGACTGTGGCCGGCTGGG
TGTGGCGGACCGCTATCAGGACATAGCGTTGGCTACCCGTGATATTGCTGAAGAGCTTGG
CGGCGAATGGGCTGACCGCTTCCTCGTGCTTTACGGTATCGCCGCTCCCGATTCGCAGCG
CATCGCCTTCTATCGCCTTCTTGACGAGTTCTTCTGAGCGGGACTCTGGGGTTCGAAATG
ACCGACCAAGCGACGCCCAACCTGCCATCACGATGGCCGCAATAAAATATCTTTATTTTC
ATTACATCTGTGTGTTGGTTTTTTGTGTGAATCGATAGCGATAAGGATCCGCGTATGGTG
CACTCTCAGTACAATCTGCTCTGATGCCGCATAGTTAAGCCAGCCCCGACACCCGCCAAC
ACCCGCTGACGCGCCCTGACGGGCTTGTCTGCTCCCGGCATCCGCTTACAGACAAGCTGT
GACCGTCTCCGGGAGCTGCATGTGTCAGAGGTTTTCACCGTCATCACCGAAACGCGCGAG
ACGAAAGGGCCTCGTGATACGCCTATTTTTATAGGTTAATGTCATGATAATAATGGTTTC
TTAGACGTCAGGTGGCACTTTTCGGGGAAATGTGCGCGGAACCCCTATTTGTTTATTTTT
CTAAATACATTCAAATATGTATCCGCTCATGAGACAATAACCCTGATAAATGCTTCAATA
ATATTGAAAAAGGAAGAGTATGAGTATTCAACATTTCCGTGTCGCCCTTATTCCCTTTTT
TGCGGCATTTTGCCTTCCTGTTTTTGCTCACCCAGAAACGCTGGTGAAAGTAAAAGATGC
TGAAGATCAGTTGGGTGCACGAGTGGGTTACATCGAACTGGATCTCAACAGCGGTAAGAT
CCTTGAGAGTTTTCGCCCCGAAGAACGTTTTCCAATGATGAGCACTTTTAAAGTTCTGCT
ATGTGGCGCGGTATTATCCCGTATTGACGCCGGGCAAGAGCAACTCGGTCGCCGCATACA
CTATTCTCAGAATGACTTGGTTGAGTACTCACCAGTCACAGAAAAGCATCTTACGGATGG
CATGACAGTAAGAGAATTATGCAGTGCTGCCATAACCATGAGTGATAACACTGCGGCCAA
CTTACTTCTGACAACGATCGGAGGACCGAAGGAGCTAACCGCTTTTTTGCACAACATGGG
GGATCATGTAACTCGCCTTGATCGTTGGGAACCGGAGCTGAATGAAGCCATACCAAACGA
CGAGCGTGACACCACGATGCCTGTAGCAATGGCAACAACGTTGCGCAAACTATTAACTGG
CGAACTACTTACTCTAGCTTCCCGGCAACAATTAATAGACTGGATGGAGGCGGATAAAGT
TGCAGGACCACTTCTGCGCTCGGCCCTTCCGGCTGGCTGGTTTATTGCTGATAAATCTGG
AGCCGGTGAGCGTGGGTCTCGCGGTATCATTGCAGCACTGGGGCCAGATGGTAAGCCCTC
CCGTATCGTAGTTATCTACACGACGGGGAGTCAGGCAACTATGGATGAACGAAATAGACA
GATCGCTGAGATAGGTGCCTCACTGATTAAGCATTGGTAACTGTCAGACCAAGTTTACTC
ATATATACTTTAGATTGATTTAAAACTTCATTTTTAATTTAAAAGGATCTAGGTGAAGAT
CCTTTTTGATAATCTCATGACCAAAATCCCTTAACGTGAGTTTTCGTTCCACTGAGCGTC
AGACCCCGTAGAAAAGATCAAAGGATCTTCTTGAGATCCTTTTTTTCTGCGCGTAATCTG
CTGCTTGCAAACAAAAAAACCACCGCTACCAGCGGTGGTTTGTTTGCCGGATCAAGAGCT
ACCAACTCTTTTTCCGAAGGTAACTGGCTTCAGCAGAGCGCAGATACCAAATACTGTCCT
TCTAGTGTAGCCGTAGTTAGGCCACCACTTCAAGAACTCTGTAGCACCGCCTACATACCT
CGCTCTGCTAATCCTGTTACCAGTGGCTGCTGCCAGTGGCGATAAGTCGTGTCTTACCGG
GTTGGACTCAAGACGATAGTTACCGGATAAGGCGCAGCGGTCGGGCTGAACGGGGGGTTC
GTGCACACAGCCCAGCTTGGAGCGAACGACCTACACCGAACTGAGATACCTACAGCGTGA
GCTATGAGAAAGCGCCACGCTTCCCGAAGGGAGAAAGGCGGACAGGTATCCGGTAAGCGG
CAGGGTCGGAACAGGAGAGCGCACGAGGGAGCTTCCAGGGGGAAACGCCTGGTATCTTTA
TAGTCCTGTCGGGTTTCGCCACCTCTGACTTGAGCGTCGATTTTTGTGATGCTCGTCAGG
GGGGCGGAGCCTATGGAAAAACGCCAGCAACGCGGCCTTTTTACGGTTCCTGGCCTTTTG
CTGGCCTTTTGCTCACATGGCTCGACAGATCT
SEQ ID No. 6 - LpESYNGP 
TCAATATTGGCCATTAGCCATATTATTCATTGGTTATATAGCATAAATCAATATTGGCTA
TTGGCCATTGCATACGTTGTATCTATATCATAATATGTACATTTATATTGGCTCATGTCC
AATATGACCGCCATGTTGGCATTGATTATTGACTAGTTATTAATAGTAATCAATTACGGG
GTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCAT
AGTAACGCCAATAGGGACTTTCCATTGACGTCAATGGGTGGAGTATTTACGGTAAACTGC
CCACTTGGCAGTACATCAAGTGTATCATATGCCAAGTCCGCCCCCTATTGACGTCAATGA
CGGTAAATGGCCCGCCTGGCATTATGCCCAGTACATGACCTTACGGGACTTTCCTACTTG
GCAGTACATCTACGTATTAGTCATCGCTATTACCATGGTGATGCGGTTTTGGCAGTACAC
CAATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTG
CGATCGCCCGCCCCGTTGACGCAAATGGGCGGTAGGCGTGTACGGTGGGAGGTCTATATA
AGCAGAGCTCGTTTAGTGAACCGTCAGATCACTAGAAGCTTTATTGCGGTAGTTTATCAC
AGTTAAATTGCTAACGCAGTCAGTGCTTCTGACACAACAGTCTCGAACTTAAGCTGCAGT
GACTCTCTTAAGGTAGCCTTGCAGAAGTTGGTCGTGAGGCACTGGGCAGGTAAGTATCAA
GGTTACAAGACAGGTTTAAGGAGACCAATAGAAACTGGGCTTGTCGAGACAGAGAAGACT
CTTGCGTTTCTGATAGGCACCTATTGGTCTTACTGACATCCACTTTGCCTTTCTCTCCAC
AGGTGTCCACTCCCAGTTCAATTACAGCTCTTAAGGCTAGAGTACTTAATACGACTCACT
ATAGGCTAGAGAATTCGAGAGGGGCGCAGACCCTACCTGTTGAACCTGGCTGATCGTAGG
ATCCCCGGGACAGCAGAGGAGAACTTACAGAAGTCTTCTGGAGGTGTTCCTGGCCAGAAC
ACAGGAGGACAGGTAAGATGGCGATCCCCTCACCTGGTCCAAAGCCCTGAAGAAACTGG
AAAAAGTCACCGTTCAGGGTAGCCAAAAGCTTACCACAGGCAATTGCAACTGGGCATTGT
CCCTGGTGGATCTTTTCCACGACACTAATTTCGTTAAGGAGAAAGATTGGCAACTCAGAG
ACGTGATCCCCCTCTTGGAGGACGTGACCCAAACATTGTCTGGGCAGGAGCGCGAAGCTT
TCGAGCGCACCTGGTGGGCCATCAGCGCAGTCAAAATGGGGCTGCAAATCAACAACGTGG
TTGACGGTAAAGCTAGCTTTCAACTGCTCCGCGCTAAGTACGAGAAGAAAACCGCCAACA
AGAAACAATCCGAACCTAGCGAGGAGTACCCAATTATGATCGACGGCGCCGGCAATAGGA
ACTTCCGCCCACTGACTCCCAGGGGCTATACCACCTGGGTCAACACCATCCAGACAAACG
GACTTTTGAACGAAGCCTCCCAGAACCTGTTCGGCATCCTGTCTGTGGACTGCACCTCCG
AAGAAATGAATGCTTTTCTCGACGTGGTGCCAGGACAGGCTGGACAGAAACAGATCCTGC
TCGATGCCATTGACAAGATCGCCGACGACTGGGATAATCGCCACCCCCTGCCAAACGCCC
CTCTGGTGGCTCCCCCACAGGGGCCTATCCCTATGACCGCTAGGTTCATTAGGGGACTGG
GGGTGCCCCGCGAACGCCAGATGGAGCCAGCATTTGACCAATTTAGGCAGACCTACAGAC
AGTGGATCATCGAAGCCATGAGCGAGGGGATTAAAGTCATGATCGGAAAGCCCAAGGCAC
AGAACATCAGGCAGGGGGCCAAGGAACCATACCCTGAGTTTGTCGACAGGCTTCTGTCCC
AGATTAAATCCGAAGGCCACCCTCAGGAGATCTCCAAGTTCTTGACAGACACACTGACTA
TCCAAAATGCAAATGAAGAGTGCAGAAACGCCATGAGGCACCTCAGACCTGAAGATACCC
TGGAGGAGAAAATGTACGCATGTCGCGACATTGGCACTACCAAGCAAAAGATGATGCTGC
TCGCCAAGGCTCTGCAAACCGGCCTGGCTGGTCCATTCAAAGGAGGAGCACTGAAGGGAG
GTCCATTGAAAGCTGCACAAACATGTTATAATTGTGGGAAGCCAGGACATTTATCTAGTC
AATGTAGAGCACCTAAAGTCTGTTTTAAATGTAAACAGCCTGGACATTTCTCAAAGCAAT
GCAGAAGTGTTCCAAAAAACGGGAAGCAAGGGGCTCAAGGGAGGCCCCAGAAACAAACTT
TCCCGATACAACAGAAGAGTCAGCACAACAAATCTGTTGTACAAGAGACTCCTCAGACTC
AAAATCTGTACCCAGATCTGAGCGAAATAAAAAAGGAATACAATGTCAAGGAGAAGGATC
AAGTAGAGGATCTCAACCTGGACAGTTTGTGGAGTAACATACAATCTCGAGAAGAGGCC
CACTACCATCGTCCTGATCAATGACACCCCTCTTAATGTGCTGCTGGACACCGGAGCCGA
CACCAGCGTTCTCACTACTGCTCACTATAACAGACTGAAATACAGAGGAAGGAAATACCA
GGGCACAGGCATCATCGGCGTTGGAGGCAACGTCGAAACCTTTTCCACTCCTGTCACCAT
CAAAAAGAAGGGGAGACACATTAAAACCAGAATGCTGGTCGCCGACATCCCCGTCACCAT
CCTTGGCAGAGACATTCTCCAGGACCTGGGCGCTAAACTCGTGCTGGCACAACTGTCTAA
GGAAATCAAGTTCCGCAAGATCGAGCTGAAAGAGGGCACAATGGGTCCAAAAATCCCCCA
GTGGCCCCTGACCAAAGAGAAGCTTGAGGGCGCTAAGGAAATCGTGCAGCGCCTGCTTTC
TGAGGGCAAGATTAGCGAGGCCAGCGACAATAACCCTTACAACAGCCCCATCTTTGTGAT
TAAGAAAAGGAGCGGCAAATGGAGACTCCTGCAGGACCTGAGGGAACTCAACAAGACCGT
CCAGGTCGGAACTGAGATCTCTCGCGGACTGCCTCACCCCGGCGGCCTGATTAAATGCAA
GCACATGACAGTCCTTGACATTGGAGACGCTTATTTACCATCCCCCTCGATCCTGAATT
TCGCCCCTATACTGCTTTTACCATCCCCAGCATCAATCACCAGGAGCCCGATAAACGCTA
TGTGTGGAAGTGCCTCCCCCAGGGATTTGTGCTTAGCCCCTACATTTACCAGAAGACACT
TCAAGAGATCCTCCAACCTTTCCGCGAAAGATACCCAGAGGTTCAACTCTACCAATATAT
 
GGACGACCTGTTCATGGGGTCCAACGGGTCTAAGAAGCAGCACAAGGAACTCATCATCGA
ACTGAGGGCAATCCTCCTGGAGAAAGGCTTCGAGACACCCGACGACAAGCTGCAAGAAGT
TCCTCCATATAGCTGGCTGGGCTACCAGCTTTGCCCTGAAAACTGGAAAGTCCAGAAGAT
GCAGTTGGATATGGTCAAGAACCCAACACTGAACGACGTCCAGAAGCTCATGGGCAATAT
TACCTGGATGAGCTCCGGAATCCCTGGGCTTACCGTTAAGCACATTGCCGCAACTACAAA
AGGATGCCTGGAGTTGAACCAGAAGGTCATTTGGACAGAGGAAGCTCAGAAGGAACTGGA
GGAGAATAATGAAAAGATTAAGAATGCTCAAGGGCTCCAATACTACAATCCCGAAGAAGA
AATGTTGTGCGAGGTCGAAATCACTAAGAACTACGAAGCCACCTATGTCATCAAACAGTC
CCAAGGCATCTTGTGGGCCGGAAAGAAAATCATGAAGGCCAACAAGGCTGGTCCACCGT
TAAAAATCTGATGCTCCTGCTCCAGCACGTCGCCACCGAGTCTATCACCCGCGTCGGCAA
GTGCCCCACCTTCAAAGTTCCCTTCACTAAGGAGCAGGTGATGTGGAGATGCAAAAAGG
CTGGTACTACTCTTGGCTTCCCGAGATCGTCTACACCCACCAAGTGGTGCACGACGACTG
GAGAATGAAGCTTGTCGAGGAGCCCACTAGCGGAATTACAATCTATACCGACGGCGGAAA
GCAAAACGGAGAGGGAATCGCTGCATACGTCACATCTAACGGCCGCACCAAGCAAAAGAG
GCTCGGCCCTGTCACTCACCAGGTGGCTGAGAGGATGGCTATCCAGATGGCCCTTGAGGA
CACTAGAGACAAGCAGGTGAACATTGTGACTGACAGCTACTACTGCTGGAAAAACATCAC
AGAGGGCCTTGGCCTGGAGGGACCCCAGTCTCCCTGGTGGCCTATCATCCAGAATATCCG
CGAAAAGGAAATTGTCTATTTCGCCTGGGTGCCTGGACACAAAGGAATTTACGGCAACCA
ACTCGCCGATGAAGCCGCCAAAATTAAAGAGGAAATCATGCTTGCCTACCAGGGCACACA
GATTAAGGAGAAGAGAGACGAGGACGCTGGCTTTGACCTGTGTGTGCCATACGACATCAT
GATTCCCGTTAGCGACACAAAGATCATTCCAACCGATGTCAAGATCCAGGTGCCACCCAA
TTCATTTGGTTGGGTGACCGGAAAGTCCAGCATGGCTAAGCAGGGTCTTCTGATTAACGG
GGGAATCATTGATGAAGGATACACCGGCGAAATCCAGGTGATCTGCACAAATATCGGCAA
AAGCAATATTAAGCTTATCGAAGGGCAGAAGTTCGCTCAACTCATCATCCTCCAGCACCA
CAGCAATTCAAGACAACCTTGGGACGAAAACAAGATTAGCCAGAGAGGTGACAAGGGCTT
CGGCAGCACAGGTGTGTTCTGGGTGGAGAACATCCAGGAAGCACAGGACGAGCACGAGAA
TTGGCACACCTCCCCTAAGATTTTGGCCCGCAATTACAAGATCCCACTGACTGTGGCTAA
GCAGATCACACAGGAATGCCCCCACTGCACCAAACAAGGTTCTGGCCCCGCCGGCTGCGT
GATGAGGTCCCCCAATCACTGGCAGGCAGATTGCACCCACCTCGACAACAAAATTATCCT
GACCTTCGTGGAGAGCAATTCCGGCTACATCCACGCAACACTCCTCTCCAAGGAAAATGC
ATTGTGCACCTCCCTCGCAATTCTGGAATGGGCCAGGCTGTTCTCTCCAAAATCCCTGCA
CACCGACAACGGCACCAACTTTGTGGCTGAACCTGTGGTGAATCTGCTGAAGTTCCTGAA
AATCGCCCACACCACTGGCATTCCCTATCACCCTGAAAGCCAGGGCATTGTCGAGAGGGC
CAACAGAACTCTGAAAGAAAAGATCCAATCTCACAGAGACAATACACAGACATTGGAGGC
CGCACTTCAGCTCGCCCTTATCACCTGCAACAAAGGAAGAGAAAGCATGGGCGGCCAGAC
CCCCTGGGAGGTCTTCATCACTAACCAGGCCCAGGTCATCCATGAAAAGCTGCTCTTGCA
GCAGGCCCAGTCCTCCAAAAAGTTCTGCTTTTATAAGATCCCCGGTGAGCACGACTGGAA
AGGTCCTACAAGAGTTTTGTGGAAAGGAGACGGCGCAGTTGTGGTGAACGATGAGGGCAA
GGGGATCATCGCTGTGCCCCTGACACGCACCAAGCTTCTCATCAAGCCAAACTGAACCCG
GGCGGCCGCTTCCCTTTAGTGAGGGTTAATGCTTCGAGCAGACATGATAAGATACATTG
ATGAGTTTGGACAAACCACAACTAGAATGCAGTGAAAAAAATGCTTTATTTGTGAAATTT
GTGATGCTATTGCTTTATTTGTAACCATTATAAGCTGCAATAAACAAGTTAACAACAACA
ATTGCATTCATTTTATGTTTCAGGTTCAGGGGGAGATGTGGGAGGTTTTTTAAAGCAAGT
AAAACCTCTACAAATGTGGTAAAATCCGATAAGGATCGATCCGGGCTGGCGTAATAGCGA
AGAGGCCCGCACCGATCGCCCTTCCCAACAGTTGCGCAGCCTGAATGGCGAATGGACGCG
CCCTGTAGCGGCGCATTAAGCGCGGCGGGTGTGGTGGTTACGCGCAGCGTGACCGCTACA
CTTGCCAGCGCCCTAGCGCCCGCTCCTTTCGCTTTCTTCCCTTCCTTTCTCGCCACGTTC
GCCGGCTTTCCCCGTCAAGCTCTAAATCGGGGGCTCCCTTTAGGGTTCCGATTTAGAGCT
TTACGGCACCTCGACCGCAAAAAACTTGATTTGGGTGATGGTTCACGTAGTGGGCCATCG
CCCTGATAGACGGTTTTTCGCCCTTTGACGTTGGAGTCCACGTTCTTTAATAGTGGACTC
TTGTTCCAAACTGGAACAACACTCAACCCTATCTCGGTCTATTCTTTTGATTTATAAGGG
ATTTTGCCGATTTCGGCCTATTGGTTAAAAAATGAGCTGATTTAACAAATATTTAACGCG
AATTTTAACAAAATATTAACGTTTACAATTTCGCCTGATGCGGTATTTTCTCCTTACGCA
TCTGTGCGGTATTTCACACCGCATACGCGGATCTGCGCAGCACCATGGCCTGAAATAACC
TCTGAAAGAGGAACTTGGTTAGGTACCTTCTGAGGCGGAAAGAACCAGCTGTGGAATGTG
TGTCAGTTAGGGTGTGGAAAGTCCCCAGGCTCCCCAGCAGGCAGAAGTATGCAAAGCATG
CATCTCAATTAGTCAGCAACCAGGTGTGGAAAGTCCCCAGGCTCCCCAGCAGGCAGAAGT
ATGCAAAGCATGCATCTCAATTAGTCAGCAACCATAGTCCCGCCCCTAACTCCGCCCATC
CCGCCCCTAACTCCGCCCAGTTCCGCCCATTCTCCGCCCCATGGCTGACTAATTTTTTTT
ATTTATGCAGAGGCCGAGGCCGCCTCGGCCTCTGAGCTATTCCAGAAGTAGTGAGGAGGC
TTTTTTGGAGGCCTAGGCTTTTGCAAAAAGCTTGATTCTTCTGACACAACAGTCTCGAAC
 
TTAAGGCTAGAGCCACCATGATTGAACAAGATGGATTGCACGCAGGTTCTCCGGCCGCTT
GGGTGGAGAGGCTATTCGGCTATGACTGGGCACAACAGACAATCGGCTGCTCTGATGCCG
CCGTGTTCCGGCTGTCAGCGCAGGGGCGCCCGGTTCTTTTTGTCAAGACCGACCTGTCCG
GTGCCCTGAATGAACTGCAGGACGAGGCAGCGCGGCTATCGTGGCTGGCCACGACGGGCG
TTCCTTGCGCAGCTGTGCTCGACGTTGTCACTGAAGCGGGAAGGGACTGGCTGCTATTGG
GCGAAGTGCCGGGGCAGGATCTCCTGTCATCTCACCTTGCTCCTGCCGAGAAAGTATCCA
TCATGGCTGATGCAATGCGGCGGCTGCATACGCTTGATCCGGCTACCTGCCCATTCGACC
ACCAAGCGAAACATCGCATCGAGCGAGCACGTACTCGGATGGAAGCCGGTCTTGTCGATC
AGGATGATCTGGACGAAGAGCATCAGGGGCTCGCGCCAGCCGAACTGTTCGCCAGGCTCA
AGGCGCGCATGCCCGACGGCGAGGATCTCGTCGTGACCCATGGCGATGCCTGCTTGCCGA
ATATCATGGTGGAAAATGGCCGCTTTTCTGGATTCATCGACTGTGGCCGGCTGGGTGTGG
CGGACCGCTATCAGGACATAGCGTTGGCTACCCGTGATATTGCTGAAGAGCTTGGCGGCG
AATGGGCTGACCGCTTCCTCGTGCTTTACGGTATCGCCGCTCCCGATTCGCAGCGCATCG
CCTTCTATCGCCTTCTTGACGAGTTCTTCTGAGCGGGACTCTGGGGTTCGAAATGACCGA
CCAAGCGACGCCCAACCTGCCATCACGATGGCCGCAATAAAATATCTTTATTTTCATTAC
ATCTGTGTGTTGGTTTTTTGTGTGAATCGATAGCGATAAGGATCCGCGTATGGTGCACTC
TCAGTACAATCTGCTCTGATGCCGCATAGTTAAGCCAGCCCCGACACCCGCCAACACCCG
CTGACGCGCCCTGACGGGCTTGTCTGCTCCCGGCATCCGCTTACAGACAAGCTGTGACCG
TCTCCGGGAGCTGCATGTGTCAGAGGTTTTCACCGTCATCACCGAAACGCGCGAGACGAA
AGGGCCTCGTGATACGCCTATTTTTATAGGTTAATGTCATGATAATAATGGTTTCTTAGA
CGTCAGGTGGCACTTTTCGGGGAAATGTGCGCGGAACCCCTATTTGTTTATTTTTCTAAA
TACATTCAAATATGTATCCGCTCATGAGACAATAACCCTGATAAATGCTTCAATAATATT
GAAAAAGGAAGAGTATGAGTATTCAACATTTCCGTGTCGCCCTTATTCCCTTTTTTGCGG
CATTTTGCCTTCCTGTTTTTGCTCACCCAGAAACGCTGGTGAAAGTAAAAGATGCTGAAG
ATCAGTTGGGTGCACGAGTGGGTTACATCGAACTGGATCTCAACAGCGGTAAGATCCTTG
AGAGTTTTCGCCCCGAAGAACGTTTTCCAATGATGAGCACTTTTAAAGTTCTGCTATGTG
GCGCGGTATTATCCCGTATTGACGCCGGGCAAGAGCAACTCGGTCGCCGCATACACTATT
CTCAGAATGACTTGGTTGAGTACTCACCAGTCACAGAAAAGCATCTTACGGATGGCATGA
CAGTAAGAGAATTATGCAGTGCTGCCATAACCATGAGTGATAACACTGCGGCCAACTTAC
TTCTGACAACGATCGGAGGACCGAAGGAGCTAACCGCTTTTTTGCACAACATGGGGGATC
ATGTAACTCGCCTTGATCGTTGGGAACCGGAGCTGAATGAAGCCATACCAAACGACGAGC
GTGACACCACGATGCCTGTAGCAATGGCAACAACGTTGCGCAAACTATTAACTGGCGAAC
TACTTACTCTAGCTTCCCGGCAACAATTAATAGACTGGATGGAGGCGGATAAAGTTGCAG
GACCACTTCTGCGCTCGGCCCTTCCGGCTGGCTGGTTTATTGCTGATAAATCTGGAGCCG
GTGAGCGTGGGTCTCGCGGTATCATTGCAGCACTGGGGCCAGATGGTAAGCCCTCCCGTA
TCGTAGTTATCTACACGACGGGGAGTCAGGCAACTATGGATGAACGAAATAGACAGATCG
CTGAGATAGGTGCCTCACTGATTAAGCATTGGTAACTGTCAGACCAAGTTTACTCATATA
TACTTTAGATTGATTTAAAACTTCATTTTTAATTTAAAAGGATCTAGGTGAAGATCCTTT
TTGATAATCTCATGACCAAAATCCCTTAACGTGAGTTTTCGTTCCACTGAGCGTCAGACC
CCGTAGAAAAGATCAAAGGATCTTCTTGAGATCCTTTTTTTCTGCGCGTAATCTGCTGCT
TGCAAACAAAAAAACCACCGCTACCAGCGGTGGTTTGTTTGCCGGATCAAGAGCTACCAA
CTCTTTTTCCGAAGGTAACTGGCTTCAGCAGAGCGCAGATACCAAATACTGTCCTTCTAG
TGTAGCCGTAGTTAGGCCACCACTTCAAGAACTCTGTAGCACCGCCTACATACCTCGCTC
TGCTAATCCTGTTACCAGTGGCTGCTGCCAGTGGCGATAAGTCGTGTCTTACCGGGTTGG
ACTCAAGACGATAGTTACCGGATAAGGCGCAGCGGTCGGGCTGAACGGGGGGTTCGTGCA
CACAGCCCAGCTTGGAGCGAACGACCTACACCGAACTGAGATACCTACAGCGTGAGCTAT
GAGAAAGCGCCACGCTTCCCGAAGGGAGAAAGGCGGACAGGTATCCGGTAAGCGGCAGGG
TCGGAACAGGAGAGCGCACGAGGGAGCTTCCAGGGGGAAACGCCTGGTATCTTTATAGTC
CTGTCGGGTTTCGCCACCTCTGACTTGAGCGTCGATTTTTGTGATGCTCGTCAGGGGGGC
GGAGCCTATGGAAAAACGCCAGCAACGCGGCCTTTTTACGGTTCCTGGCCTTTTGCTGGC
CTTTTGCTCACATGGCTCGACAGATCT
SEQ ID No. 7 - pESYNGPRRE 
TCAATATTGGCCATTAGCCATATTATTCATTGGTTATATAGCATAAATCAATATTGGCTA
TTGGCCATTGCATACGTTGTATCTATATCATAATATGTACATTTATATTGGCTCATGTCC
AATATGACCGCCATGTTGGCATTGATTATTGACTAGTTATTAATAGTAATCAATTACGGG
GTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCAT
AGTAACGCCAATAGGGACTTTCCATTGACGTCAATGGGTGGAGTATTTACGGTAAACTGC
CCACTTGGCAGTACATCAAGTGTATCATATGCCAAGTCCGCCCCCTATTGACGTCAATGA
CGGTAAATGGCCCGCCTGGCATTATGCCCAGTACATGACCTTACGGGACTTTCCTACTTG
GCAGTACATCTACGTATTAGTCATCGCTATTACCATGGTGATGCGGTTTTGGCAGTACAC
CAATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTG
CGATCGCCCGCCCCGTTGACGCAAATGGGCGGTAGGCGTGTACGGTGGGAGGTCTATATA
AGCAGAGCTCGTTTAGTGAACCGTCAGATCACTAGAAGCTTTATTGCGGTAGTTTATCAC
AGTTAAATTGCTAACGCAGTCAGTGCTTCTGACACAACAGTCTCGAACTTAAGCTGCAGT
GACTCTCTTAAGGTAGCCTTGCAGAAGTTGGTCGTGAGGCACTGGGCAGGTAAGTATCAA
GGTTACAAGACAGGTTTAAGGAGACCAATAGAAACTGGGCTTGTCGAGACAGAGAAGACT
CTTGCGTTTCTGATAGGCACCTATTGGTCTTACTGACATCCACTTTGCCTTTCTCTCCAC
AGGTGTCCACTCCCAGTTCAATTACAGCTCTTAAGGCTAGAGTACTTAATACGACTCACT
ATAGGCTAGAGAATTCGCCACCATGGGCGATCCCCTCACCTGGTCCAAAGCCCTGAAGAA
ACTGGAAAAAGTCACCGTTCAGGGTAGCCAAAAGCTTACCACAGGCAATTGCAACTGGGC
ATTGTCCCTGGTGGATCTTTTCCACGACACTAATTTCGTTAAGGAGAAAGATTGGCAACT
CAGAGACGTGATCCCCCTCTTGGAGGACGTGACCCAAACATTGTCTGGGCAGGAGCGCGA
AGCTTTCGAGCGCACCTGGTGGGCCATCAGCGCAGTCAAAATGGGGCTGCAAATCAACAA
CGTGGTTGACGGTAAAGCTAGCTTTCAACTGCTCCGCGCTAAGTACGAGAAGAAAACCGC
CAACAAGAAACAATCCGAACCTAGCGAGGAGTACCCAATTATGATCGACGGCGCCGGCAA
TAGGAACTTCCGCCCACTGACTCCCAGGGGCTATACCACCTGGGTCAACACCATCCAGAC
AAACGGACTTTTGAACGAAGCCTCCCAGAACCTGTTCGGCATCCTGTCTGTGGACTGCAC
CTCCGAAGAAATGAATGCTTTTCTCGACGTGGTGCCAGGACAGGCTGGACAGAAACAGAT
CCTGCTCGATGCCATTGACAAGATCGCCGACGACTGGGATAATCGCCACCCCCTGCCAAA
CGCCCCTCTGGTGGCTCCCCCACAGGGGCCTATCCCTATGACCGCTAGGTTCATTAGGGG
ACTGGGGGTGCCCCGCGAACGCCAGATGGAGCCAGCATTTGACCAATTTAGGCAGACCTA
CAGACAGTGGATCATCGAAGCCATGAGCGAGGGGATTAAAGTCATGATCGGAAAGCCCAA
GGCACAGAACATCAGGCAGGGGGCCAAGGAACCATACCCTGAGTTTGTCGACAGGCTTCT
GTCCCAGATTAAATCCGAAGGCCACCCTCAGGAGATCTCCAAGTTCTTGACAGACACACT
GACTATCCAAAATGCAAATGAAGAGTGCAGAAACGCCATGAGGCACCTCAGACCTGAAGA
TACCCTGGAGGAGAAAATGTACGCATGTCGCGACATTGGCACTACCAAGCAAAAGATGAT
GCTGCTCGCCAAGGCTCTGCAAACCGGCCTGGCTGGTCCATTCAAAGGAGGAGCACTGAA
GGGAGGTCCATTGAAAGCTGCACAAACATGTTATAATTGTGGAAGCCAGGACATTTATC
TAGTCAATGTAGAGCACCTAAAGTCTGTTTTAAATGTAAACAGCCTGGACATTTCTCAAA
GCAATGCAGAAGTGTTCCAAAAAACGGAAGCAAGGGGCTCAAGGAGGCCCCAGAAACA
AACTTTCCCGATACAACAGAAGAGTCAGCACAACAAATCTGTTGTACAAGAGACTCCTCA
GACTCAAAATCTGTACCCAGATCTGAGCGAAATAAAAAAGGAATACAATGTCAAGGAGAA
GGATCAAGTAGAGGATCTCAACCTGGACAGTTTGTGGAGTAACATACAATCTCGAGAAG
AGGCCCACTACCATCGTCCTGATCAATGACACCCCTCTTAATGTGCTGCTGGACACCGGA
GCCGACACCAGCGTTCTCACTACTGCTCACTATAACAGACTGAAATACAGAGGAAGGAAA
TACCAGGGCACAGGCATCATCGGCGTTGGAGGCAACGTCGAAACCTTTTCCACTCCTGTC
ACCATCAAAAAGAAGGGGAGACACATTAAAACCAGAATGCTGGTCGCCGACATCCCCGTC
ACCATCCTTGGCAGAGACATTCTCCAGGACCTGGGCGCTAAACTCGTGCTGGCACAACTG
TCTAAGGAAATCAAGTTCCGCAAGATCGAGCTGAAAGAGGGCACAATGGGTCCAAAAATC
CCCCAGTGGCCCCTGACCAAAGAGAAGCTTGAGGGCGCTAAGGAAATCGTGCAGCGCCTG
CTTTCTGAGGGCAAGATTAGCGAGGCCAGCGACAATAACCCTTACAACAGCCCCATCTTT
GTGATTAAGAAAAGGAGCGGCAAATGGAGACTCCTGCAGGACCTGAGGGAACTCAACAAG
ACCGTCCAGGTCGGAACTGAGATCTCTCGCGGACTGCCTCACCCCGGCGGCCTGATTAAA
TGCAAGCACATGACAGTCCTTGACATTGGAGACGCTTATTTACCATCCCCCTCGATCCT
GAATTTCGCCCCTATACTGCTTTTACCATCCCCAGCATCAATCACCAGGAGCCCGATAAA
CGCTATGTGTGGAAGTGCCTCCCCCAGGGATTTGTGCTTAGCCCCTACATTTACCAGAAG
ACACTTCAAGAGATCCTCCAACCTTTCCGCGAAAGATACCCAGAGGTTCAACTCTACCAA
TATATGGACGACCTGTTCATGGGGTCCAACGGGTCTAAGAAGCAGCACAAGGAACTCATC
ATCGAACTGAGGGCAATCCTCCTGGAGAAAGGCTTCGAGACACCCGACGACAAGCTGCAA
 
GAAGTTCCTCCATATAGCTGGCTGGGCTACCAGCTTTGCCCTGAAAACTGGAAAGTCCAG
AAGATGCAGTTGGATATGGTCAAGAACCCAACACTGAACGACGTCCAGAAGCTCATGGGC
AATATTACCTGGATGAGCTCCGGAATCCCTGGGCTTACCGTTAAGCACATTGCCGCAACT
ACAAAAGGATGCCTGGAGTTGAACCAGAAGGTCATTTGGACAGAGGAAGCTCAGAAGGAA
CTGGAGGAGAATAATGAAAAGATTAAGAATGCTCAAGGGCTCCAATACTACAATCCCGAA
GAAGAAATGTTGTGCGAGGTCGAAATCACTAAGAACTACGAAGCCACCTATGTCATCAAA
CAGTCCCAAGGCATCTTGTGGGCCGGAAAGAAAATCATGAAGGCCAACAAAGGCTGGTCC
ACCGTTAAAAATCTGATGCTCCTGCTCCAGCACGTCGCCACCGAGTCTATCACCCGCGTC
GGCAAGTGCCCCACCTTCAAAGTTCCCTTCACTAAGGAGCAGGTGATGTGGAGATGCAA
AAAGGCTGGTACTACTCTTGGCTTCCCGAGATCGTCTACACCCACCAAGTGGTGCACGAC
GACTGGAGAATGAAGCTTGTCGAGGAGCCCACTAGCGGAATTACAATCTATACCGACGGC
GGAAAGCAAAACGGAGAGGGAATCGCTGCATACGTCACATCTAACGGCCGCACCAAGCAA
AAGAGGCTCGGCCCTGTCACTCACCAGGTGGCTGAGAGGATGGCTATCCAGATGGCCCTT
GAGGACACTAGAGACAAGCAGGTGAACATTGTGACTGACAGCTACTACTGCTGGAAAAAC
ATCACAGAGGGCCTTGGCCTGGAGGGACCCCAGTCTCCCTGGTGGCCTATCATCCAGAAT
ATCCGCGAAAAGGAAATTGTCTATTTCGCCTGGGTGCCTGGACACAAAGGAATTTACGGC
AACCAACTCGCCGATGAAGCCGCCAAAATTAAAGAGGAAATCATGCTTGCCTACCAGGGC
ACACAGATTAAGGAGAAGAGAGACGAGGACGCTGGCTTTGACCTGTGTGTGCCATACGAC
ATCATGATTCCCGTTAGCGACACAAAGATCATTCCAACCGATGTCAAGATCCAGGTGCCA
CCCAATTCATTTGGTTGGGTGACCGGAAAGTCCAGCATGGCTAAGCAGGGTCTTCTGATT
AACGGGGGAATCATTGATGAAGGATACACCGGCGAAATCCAGGTGATCTGCACAAATATC
GGCAAAAGCAATATTAAGCTTATCGAAGGGCAGAAGTTCGCTCAACTCATCATCCTCCAG
CACCACAGCAATTCAAGACAACCTTGGGACGAAAACAAGATTAGCCAGAGAGGTGACAAG
GGCTTCGGCAGCACAGGTGTGTTCTGGGTGGAGAACATCCAGGAAGCACAGGACGAGCAC
GAGAATTGGCACACCTCCCCTAAGATTTTGGCCCGCAATTACAAGATCCCACTGACTGTG
GCTAAGCAGATCACACAGGAATGCCCCCACTGCACCAAACAAGGTTCTGGCCCCGCCGGC
TGCGTGATGAGGTCCCCCAATCACTGGCAGGCAGATTGCACCCACCTCGACAACAAAATT
ATCCTGACCTTCGTGGAGAGCAATTCCGGCTACATCCACGCAACACTCCTCTCCAAGGAA
AATGCATTGTGCACCTCCCTCGCAATTCTGGAATGGGCCAGGCTGTTCTCTCCAAAATCC
CTGCACACCGACAACGGCACCAACTTTGTGGCTGAACCTGTGGTGAATCTGCTGAAGTTC
CTGAAAATCGCCCACACCACTGGCATTCCCTATCACCCTGAAAGCCAGGGCATTGTCGAG
AGGGCCAACAGAACTCTGAAAGAAAAGATCCAATCTCACAGAGACAATACACAGACATTG
GAGGCCGCACTTCAGCTCGCCCTTATCACCTGCAACAAAGGAAGAGAAAGCATGGGCGGC
CAGACCCCCTGGGAGGTCTTCATCACTAACCAGGCCCAGGTCATCCATGAAAAGCTGCTC
TTGCAGCAGGCCCAGTCCTCCAAAAAGTTCTGCTTTTATAAGATCCCCGGTGAGCACGAC
TGGAAAGGTCCTACAAGAGTTTTGTGGAAAGGAGACGGCGCAGTTGTGGTGAACGATGAG
GGCAAGGGGATCATCGCTGTGCCCCTGACACGCACCAAGCTTCTCATCAAGCCAAACTGA
ACCCGACGAATCCCAGGGGGAATCTCAACCCCTATTACCCAACAGTCAGAAAAATCTAAG
TGTGAGGAGAACACAATGTTTCAACCTTATTGTTATAATAATGACAGTAAGAACAGCATG
GCAGAATCGAAGGAAGCAAGAGACCAAGAAATGAACCTGAAAGAAGAATCTAAAGAAGAA
AAAAGAAGAAATGACTGGTGGAAAATAGGTATGTTTCTGTTATGCTTAGCCAGGGCCCTC
TGGAAGGTGACCAGTGGTGCAGGGTCCTCCGGCAGTCGTTACCTGAAGAAAAAATTCCAT
CACAAACATGCATCGCGAGAAGACACCTGGGACCAGGCCCAACACAACATACACCTAGCA
GGCGTGACCGGTGGATCAGGGGACAAATACTACAAGCAGAAGTACTCCAGGAACGACTGG
AATGGAGAATCAGAGGAGTACAACAGGCGGCCAAAGAGCTGGGTGAAGTCAATCGAGGCA
TTTGGAGAGAGCTATATTTCCGAGAAGACCAAAGGGGAGATTTCTCAGCCTGGGGCGGCT
ATCAACGAGCACAAGAACGGCTCTGGGGGGAACAATCCTCACCAAGGGTCCTTAGACCTG
GAGATTCGAAGCGAAGGAGGAAACATTTATGACTGTTGCATTAAAGCCCAAGAAGGAACT
CTCGCTATCCCTTGCTGTGGATTTCCCTTATGGCTATTTTGGGGGTCGGGGCGGCCGCTT
CCCTTTAGTGAGGGTTAATGCTTCGAGCAGACATGATAAGATACATTGATGAGTTTGGAC
AAACCACAACTAGAATGCAGTGAAAAAAATGCTTTATTTGTGAAATTTGTGATGCTATTG
CTTTATTTGTAACCATTATAAGCTGCAATAAACAAGTTAACAACAACAATTGCATTCATT
TTATGTTTCAGGTTCAGGGGGAGATGTGGGAGGTTTTTTAAAGCAAGTAAAACCTCTACA
AATGTGGTAAAATCCGATAAGGATCGATCCGGGCTGGCGTAATAGCGAAGAGGCCCGCAC
CGATCGCCCTTCCCAACAGTTGCGCAGCCTGAATGGCGAATGGACGCGCCCTGTAGCGGC
GCATTAAGCGCGGCGGGTGTGGTGGTTACGCGCAGCGTGACCGCTACACTTGCCAGCGCC
CTAGCGCCCGCTCCTTTCGCTTTCTTCCCTTCCTTTCTCGCCACGTTCGCCGGCTTTCCC
CGTCAAGCTCTAAATCGGGGGCTCCCTTTAGGGTTCCGATTTAGAGCTTTACGGCACCTC
GACCGCAAAAAACTTGATTTGGGTGATGGTTCACGTAGTGGGCCATCGCCCTGATAGACG
GTTTTTCGCCCTTTGACGTTGGAGTCCACGTTCTTTAATAGTGGACTCTTGTTCCAAACT
GGAACAACACTCAACCCTATCTCGGTCTATTCTTTTGATTTATAAGGGATTTTGCCGATT
 
TCGGCCTATTGGTTAAAAAATGAGCTGATTTAACAAATATTTAACGCGAATTTTAACAAA
ATATTAACGTTTACAATTTCGCCTGATGCGGTATTTTCTCCTTACGCATCTGTGCGGTAT
TTCACACCGCATACGCGGATCTGCGCAGCACCATGGCCTGAAATAACCTCTGAAAGAGGA
ACTTGGTTAGGTACCTTCTGAGGCGGAAAGAACCAGCTGTGGAATGTGTGTCAGTTAGGG
TGTGGAAAGTCCCCAGGCTCCCCAGCAGGCAGAAGTATGCAAAGCATGCATCTCAATTAG
TCAGCAACCAGGTGTGGAAAGTCCCCAGGCTCCCCAGCAGGCAGAAGTATGCAAAGCATG
CATCTCAATTAGTCAGCAACCATAGTCCCGCCCCTAACTCCGCCCATCCCGCCCCTAACT
CCGCCCAGTTCCGCCCATTCTCCGCCCCATGGCTGACTAATTTTTTTTATTTATGCAGAG
GCCGAGGCCGCCTCGGCCTCTGAGCTATTCCAGAAGTAGTGAGGAGGCTTTTTTGGAGGC
CTAGGCTTTTGCAAAAAGCTTGATTCTTCTGACACAACAGTCTCGAACTTAAGGCTAGAG
CCACCATGATTGAACAAGATGGATTGCACGCAGGTTCTCCGGCCGCTTGGGTGGAGAGGC
TATTCGGCTATGACTGGGCACAACAGACAATCGGCTGCTCTGATGCCGCCGTGTTCCGGC
TGTCAGCGCAGGGGCGCCCGGTTCTTTTTGTCAAGACCGACCTGTCCGGTGCCCTGAATG
AACTGCAGGACGAGGCAGCGCGGCTATCGTGGCTGGCCACGACGGGCGTTCCTTGCGCAG
CTGTGCTCGACGTTGTCACTGAAGCGGGAAGGGACTGGCTGCTATTGGGCGAAGTGCCGG
GGCAGGATCTCCTGTCATCTCACCTTGCTCCTGCCGAGAAAGTATCCATCATGGCTGATG
CAATGCGGCGGCTGCATACGCTTGATCCGGCTACCTGCCCATTCGACCACCAAGCGAAAC
ATCGCATCGAGCGAGCACGTACTCGGATGGAAGCCGGTCTTGTCGATCAGGATGATCTGG
ACGAAGAGCATCAGGGGCTCGCGCCAGCCGAACTGTTCGCCAGGCTCAAGGCGCGCATGC
CCGACGGCGAGGATCTCGTCGTGACCCATGGCGATGCCTGCTTGCCGAATATCATGGTGG
AAAATGGCCGCTTTTCTGGATTCATCGACTGTGGCCGGCTGGGTGTGGCGGACCGCTATC
AGGACATAGCGTTGGCTACCCGTGATATTGCTGAAGAGCTTGGCGGCGAATGGGCTGACC
GCTTCCTCGTGCTTTACGGTATCGCCGCTCCCGATTCGCAGCGCATCGCCTTCTATCGCC
TTCTTGACGAGTTCTTCTGAGCGGGACTCTGGGGTTCGAAATGACCGACCAAGCGACGCC
CAACCTGCCATCACGATGGCCGCAATAAAATATCTTTATTTTCATTACATCTGTGTGTTG
GTTTTTTGTGTGAATCGATAGCGATAAGGATCCGCGTATGGTGCACTCTCAGTACAATCT
GCTCTGATGCCGCATAGTTAAGCCAGCCCCGACACCCGCCAACACCCGCTGACGCGCCCT
GACGGGCTTGTCTGCTCCCGGCATCCGCTTACAGACAAGCTGTGACCGTCTCCGGGAGCT
GCATGTGTCAGAGGTTTTCACCGTCATCACCGAAACGCGCGAGACGAAAGGGCCTCGTGA
TACGCCTATTTTTATAGGTTAATGTCATGATAATAATGGTTTCTTAGACGTCAGGTGGCA
CTTTTCGGGGAAATGTGCGCGGAACCCCTATTTGTTTATTTTTCTAAATACATTCAAATA
TGTATCCGCTCATGAGACAATAACCCTGATAAATGCTTCAATAATATTGAAAAAGGAAGA
GTATGAGTATTCAACATTTCCGTGTCGCCCTTATTCCCTTTTTTGCGGCATTTTGCCTTC
CTGTTTTTGCTCACCCAGAAACGCTGGTGAAAGTAAAAGATGCTGAAGATCAGTTGGGTG
CACGAGTGGGTTACATCGAACTGGATCTCAACAGCGGTAAGATCCTTGAGAGTTTTCGCC
CCGAAGAACGTTTTCCAATGATGAGCACTTTTAAAGTTCTGCTATGTGGCGCGGTATTAT
CCCGTATTGACGCCGGGCAAGAGCAACTCGGTCGCCGCATACACTATTCTCAGAATGACT
TGGTTGAGTACTCACCAGTCACAGAAAAGCATCTTACGGATGGCATGACAGTAAGAGAAT
TATGCAGTGCTGCCATAACCATGAGTGATAACACTGCGGCCAACTTACTTCTGACAACGA
TCGGAGGACCGAAGGAGCTAACCGCTTTTTTGCACAACATGGGGGATCATGTAACTCGCC
TTGATCGTTGGGAACCGGAGCTGAATGAAGCCATACCAAACGACGAGCGTGACACCACGA
TGCCTGTAGCAATGGCAACAACGTTGCGCAAACTATTAACTGGCGAACTACTTACTCTAG
CTTCCCGGCAACAATTAATAGACTGGATGGAGGCGGATAAAGTTGCAGGACCACTTCTGC
GCTCGGCCCTTCCGGCTGGCTGGTTTATTGCTGATAAATCTGGAGCCGGTGAGCGTGGGT
CTCGCGGTATCATTGCAGCACTGGGGCCAGATGGTAAGCCCTCCCGTATCGTAGTTATCT
ACACGACGGGGAGTCAGGCAACTATGGATGAACGAAATAGACAGATCGCTGAGATAGGTG
CCTCACTGATTAAGCATTGGTAACTGTCAGACCAAGTTTACTCATATATACTTTAGATTG
ATTTAAAACTTCATTTTTAATTTAAAAGGATCTAGGTGAAGATCCTTTTTGATAATCTCA
TGACCAAAATCCCTTAACGTGAGTTTTCGTTCCACTGAGCGTCAGACCCCGTAGAAAAGA
TCAAAGGATCTTCTTGAGATCCTTTTTTTCTGCGCGTAATCTGCTGCTTGCAAACAAAAA
AACCACCGCTACCAGCGGTGGTTTGTTTGCCGGATCAAGAGCTACCAACTCTTTTTCCGA
AGGTAACTGGCTTCAGCAGAGCGCAGATACCAAATACTGTCCTTCTAGTGTAGCCGTAGT
TAGGCCACCACTTCAAGAACTCTGTAGCACCGCCTACATACCTCGCTCTGCTAATCCTGT
TACCAGTGGCTGCTGCCAGTGGCGATAAGTCGTGTCTTACCGGGTTGGACTCAAGACGAT
AGTTACCGGATAAGGCGCAGCGGTCGGGCTGAACGGGGGGTTCGTGCACACAGCCCAGCT
TGGAGCGAACGACCTACACCGAACTGAGATACCTACAGCGTGAGCTATGAGAAAGCGCCA
CGCTTCCCGAAGGGAGAAAGGCGGACAGGTATCCGGTAAGCGGCAGGGTCGGAACAGGAG
AGCGCACGAGGGAGCTTCCAGGGGGAAACGCCTGGTATCTTTATAGTCCTGTCGGGTTTC
GCCACCTCTGACTTGAGCGTCGATTTTTGTGATGCTCGTCAGGGGGGCGGAGCCTATGGA
AAAACGCCAGCAACGCGGCCTTTTTACGGTTCCTGGCCTTTTGCTGGCCTTTTGCTCACA
TGGCTCGACAGATCT
SEQ ID No. 8 - LpESYNGPRRE 
TCAATATTGGCCATTAGCCATATTATTCATTGGTTATATAGCATAAATCAATATTGGCTA
TTGGCCATTGCATACGTTGTATCTATATCATAATATGTACATTTATATTGGCTCATGTCC
AATATGACCGCCATGTTGGCATTGATTATTGACTAGTTATTAATAGTAATCAATTACGGG
GTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCAT
AGTAACGCCAATAGGGACTTTCCATTGACGTCAATGGGTGGAGTATTTACGGTAAACTGC
CCACTTGGCAGTACATCAAGTGTATCATATGCCAAGTCCGCCCCCTATTGACGTCAATGA
CGGTAAATGGCCCGCCTGGCATTATGCCCAGTACATGACCTTACGGGACTTTCCTACTTG
GCAGTACATCTACGTATTAGTCATCGCTATTACCATGGTGATGCGGTTTTGGCAGTACAC
CAATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTG
CGATCGCCCGCCCCGTTGACGCAAATGGGCGGTAGGCGTGTACGGTGGGAGGTCTATATA
AGCAGAGCTCGTTTAGTGAACCGTCAGATCACTAGAAGCTTTATTGCGGTAGTTTATCAC
AGTTAAATTGCTAACGCAGTCAGTGCTTCTGACACAACAGTCTCGAACTTAAGCTGCAGT
GACTCTCTTAAGGTAGCCTTGCAGAAGTTGGTCGTGAGGCACTGGGCAGGTAAGTATCAA
GGTTACAAGACAGGTTTAAGGAGACCAATAGAAACTGGGCTTGTCGAGACAGAGAAGACT
CTTGCGTTTCTGATAGGCACCTATTGGTCTTACTGACATCCACTTTGCCTTTCTCTCCAC
AGGTGTCCACTCCCAGTTCAATTACAGCTCTTAAGGCTAGAGTACTTAATACGACTCACT
ATAGGCTAGAGAATTCGAGAGGGGCGCAGACCCTACCTGTTGAACCTGGCTGATCGTAGG
ATCCCCGGGACAGCAGAGGAGAACTTACAGAAGTCTTCTGGAGGTGTTCCTGGCCAGAAC
ACAGGAGGACAGGTAAGATGGGCGATCCCCTCACCTGGTCCAAAGCCCTGAAGAAACTGG
AAAAAGTCACCGTTCAGGGTAGCCAAAAGCTTACCACAGGCAATTGCAACTGGGCATTGT
CCCTGGTGGATCTTTTCCACGACACTAATTTCGTTAAGGAGAAAGATTGGCAACTCAGAG
ACGTGATCCCCCTCTTGGAGGACGTGACCCAAACATTGTCTGGGCAGGAGCGCGAAGCTT
TCGAGCGCACCTGGTGGGCCATCAGCGCAGTCAAAATGGGGCTGCAAATCAACAACGTGG
TTGACGGTAAAGCTAGCTTTCAACTGCTCCGCGCTAAGTACGAGAAGAAAACCGCCAACA
AGAAACAATCCGAACCTAGCGAGGAGTACCCAATTATGATCGACGGCGCCGGCAATAGGA
ACTTCCGCCCACTGACTCCCAGGGGCTATACCACCTGGGTCAACACCATCCAGACAAACG
GACTTTTGAACGAAGCCTCCCAGAACCTGTTCGGCATCCTGTCTGTGGACTGCACCTCCG
AAGAAATGAATGCTTTTCTCGACGTGGTGCCAGGACAGGCTGGACAGAAACAGATCCTGC
TCGATGCCATTGACAAGATCGCCGACGACTGGGATAATCGCCACCCCCTGCCAAACGCCC
CTCTGGTGGCTCCCCCACAGGGGCCTATCCCTATGACCGCTAGGTTCATTAGGGGACTGG
GGGTGCCCCGCGAACGCCAGATGGAGCCAGCATTTGACCAATTTAGGCAGACCTACAGAC
AGTGGATCATCGAAGCCATGAGCGAGGGGATTAAAGTCATGATCGGAAAGCCCAAGGCAC
AGAACATCAGGCAGGGGGCCAAGGAACCATACCCTGAGTTTGTCGACAGGCTTCTGTCCC
AGATTAAATCCGAAGGCCACCCTCAGGAGATCTCCAAGTTCTTGACAGACACACTGACTA
TCCAAAATGCAAATGAAGAGTGCAGAAACGCCATGAGGCACCTCAGACCTGAAGATACCC
TGGAGGAGAAAATGTACGCATGTCGCGACATTGGCACTACCAAGCAAAAGATGATGCTGC
TCGCCAAGGCTCTGCAAACCGGCCTGGCTGGTCCATTCAAAGGAGGAGCACTGAAGGGAG
GTCCATTGAAAGCTGCACAAACATGTTATAATTGTGGGAAGCCAGGACATTTATCTAGTC
AATGTAGAGCACCTAAAGTCTGTTTTAAATGTAAACAGCCTGGACATTTCTCAAAGCAAT
GCAGAAGTGTTCCAAAAAACGGGAAGCAAGGGGCTCAAGGGAGGCCCCAGAAACAAACTT
TCCCGATACAACAGAAGAGTCAGCACAACAAATCTGTTGTACAAGAGACTCCTCAGACTC
AAAATCTGTACCCAGATCTGAGCGAAATAAAAAAGGAATACAATGTCAAGGAGAAGGATC
AAGTAGAGGATCTCAACCTGGACAGTTTGTGGAGTAACATACAATCTCGAGAAGAGGCC
CACTACCATCGTCCTGATCAATGACACCCCTCTTAATGTGCTGCTGGACACCGGAGCCGA
CACCAGCGTTCTCACTACTGCTCACTATAACAGACTGAAATACAGAGGAAGGAAATACCA
GGGCACAGGCATCATCGGCGTTGGAGGCAACGTCGAAACCTTTTCCACTCCTGTCACCAT
CAAAAAGAAGGGGAGACACATTAAAACCAGAATGCTGGTCGCCGACATCCCCGTCACCAT
CCTTGGCAGAGACATTCTCCAGGACCTGGGCGCTAAACTCGTGCTGGCACAACTGTCTAA
GGAAATCAAGTTCCGCAAGATCGAGCTGAAAGAGGGCACAATGGGTCCAAAAATCCCCCA
GTGGCCCCTGACCAAAGAGAAGCTTGAGGGCGCTAAGGAAATCGTGCAGCGCCTGCTTTC
TGAGGGCAAGATTAGCGAGGCCAGCGACAATAACCCTTACAACAGCCCCATCTTTGTGAT
TAAGAAAAGGAGCGGCAAATGGAGACTCCTGCAGGACCTGAGGGAACTCAACAAGACCGT
CCAGGTCGGAACTGAGATCTCTCGCGGACTGCCTCACCCCGGCGGCCTGATTAAATGCAA
GCACATGACAGTCCTTGACATTGGAGACGCTTATTTTACCATCCCCCTCGATCCTGAATT
TCGCCCCTATACTGCTTTTACCATCCCCAGCATCAATCACCAGGAGCCCGATAAACGCTA
TGTGTGGAAGTGCCTCCCCCAGGGATTTGTGCTTAGCCCCTACATTTACCAGAAGACACT
TCAAGAGATCCTCCAACCTTTCCGCGAAAGATACCCAGAGGTTCAACTCTACCAATATAT
 
GGACGACCTGTTCATGGGGTCCAACGGGTCTAAGAAGCAGCACAAGGAACTCATCATCGA
ACTGAGGGCAATCCTCCTGGAGAAAGGCTTCGAGACACCCGACGACAAGCTGCAAGAAGT
TCCTCCATATAGCTGGCTGGGCTACCAGCTTTGCCCTGAAAACTGGAAAGTCCAGAAGAT
GCAGTTGGATATGGTCAAGAACCCAACACTGAACGACGTCCAGAAGCTCATGGGCAATAT
TACCTGGATGAGCTCCGGAATCCCTGGGCTTACCGTTAAGCACATTGCCGCAACTACAAA
AGGATGCCTGGAGTTGAACCAGAAGGTCATTTGGACAGAGGAAGCTCAGAAGGAACTGGA
GGAGAATAATGAAAAGATTAAGAATGCTCAAGGGCTCCAATACTACAATCCCGAAGAAGA
AATGTTGTGCGAGGTCGAAATCACTAAGAACTACGAAGCCACCTATGTCATCAAACAGTC
CCAAGGCATCTTGTGGGCCGGAAAGAAAATCATGAAGGCCAACAAAGGCTGGTCCACCGT
TAAAAATCTGATGCTCCTGCTCCAGCACGTCGCCACCGAGTCTATCACCCGCGTCGGCAA
GTGCCCCACCTTCAAAGTTCCCTTCACTAAGGAGCAGGTGATGTGGGAGATGCAAAAAGG
CTGGTACTACTCTTGGCTTCCCGAGATCGTCTACACCCACCAAGTGGTGCACGACGACTG
GAGAATGAAGCTTGTCGAGGAGCCCACTAGCGGAATTACAATCTATACCGACGGCGGAAA
GCAAAACGGAGAGGGAATCGCTGCATACGTCACATCTAACGGCCGCACCAAGCAAAAGAG
GCTCGGCCCTGTCACTCACCAGGTGGCTGAGAGGATGGCTATCCAGATGGCCCTTGAGGA
CACTAGAGACAAGCAGGTGAACATTGTGACTGACAGCTACTACTGCTGGAAAAACATCAC
AGAGGGCCTTGGCCTGGAGGGACCCCAGTCTCCCTGGTGGCCTATCATCCAGAATATCCG
CGAAAAGGAAATTGTCTATTTCGCCTGGGTGCCTGGACACAAAGGAATTTACGGCAACCA
ACTCGCCGATGAAGCCGCCAAAATTAAAGAGGAAATCATGCTTGCCTACCAGGGCACACA
GATTAAGGAGAAGAGAGACGAGGACGCTGGCTTTGACCTGTGTGTGCCATACGACATCAT
GATTCCCGTTAGCGACACAAAGATCATTCCAACCGATGTCAAGATCCAGGTGCCACCCAA
TTCATTTGGTTGGGTGACCGGAAAGTCCAGCATGGCTAAGCAGGGTCTTCTGATTAACGG
GGGAATCATTGATGAAGGATACACCGGCGAAATCCAGGTGATCTGCACAAATATCGGCAA
AAGCAATATTAAGCTTATCGAAGGGCAGAAGTTCGCTCAACTCATCATCCTCCAGCACCA
CAGCAATTCAAGACAACCTTGGGACGAAAACAAGATTAGCCAGAGAGGTGACAAGGGCTT
CGGCAGCACAGGTGTGTTCTGGGTGGAGAACATCCAGGAAGCACAGGACGAGCACGAGAA
TTGGCACACCTCCCCTAAGATTTTGGCCCGCAATTACAAGATCCCACTGACTGTGGCTAA
GCAGATCACACAGGAATGCCCCCACTGCACCAAACAAGGTTCTGGCCCCGCCGGCTGCGT
GATGAGGTCCCCCAATCACTGGCAGGCAGATTGCACCCACCTCGACAACAAAATTATCCT
GACCTTCGTGGAGAGCAATTCCGGCTACATCCACGCAACACTCCTCTCCAAGGAAAATGC
ATTGTGCACCTCCCTCGCAATTCTGGAATGGGCCAGGCTGTTCTCTCCAAAATCCCTGCA
CACCGACAACGGCACCAACTTTGTGGCTGAACCTGTGGTGAATCTGCTGAAGTTCCTGAA
AATCGCCCACACCACTGGCATTCCCTATCACCCTGAAAGCCAGGGCATTGTCGAGAGGGC
CAACAGAACTCTGAAAGAAAAGATCCAATCTCACAGAGACAATACACAGACATTGGAGGC
CGCACTTCAGCTCGCCCTTATCACCTGCAACAAAGGAAGAGAAAGCATGGGCGGCCAGAC
CCCCTGGGAGGTCTTCATCACTAACCAGGCCCAGGTCATCCATGAAAAGCTGCTCTTGCA
GCAGGCCCAGTCCTCCAAAAAGTTCTGCTTTTATAAGATCCCCGGTGAGCACGACTGGAA
AGGTCCTACAAGAGTTTTGTGGAAAGGAGACGGCGCAGTTGTGGTGAACGATGAGGGCAA
GGGGATCATCGCTGTGCCCCTGACACGCACCAAGCTTCTCATCAAGCCAAACTGAACCCG
ACGAATCCCAGGGGGAATCTCAACCCCTATTACCCAACAGTCAGAAAAATCTAAGTGTGA
GGAGAACACAATGTTTCAACCTTATTGTTATAATAATGACAGTAAGAACAGCATGGCAGA
ATCGAAGGAAGCAAGAGACCAAGAAATGAACCTGAAAGAAGAATCTAAAGAAGAAAAAAG
AAGAAATGACTGGTGGAAAATAGGTATGTTTCTGTTATGCTTAGCCAGGGCCCTCTGGAA
GGTGACCAGTGGTGCAGGGTCCTCCGGCAGTCGTTACCTGAAGAAAAAATTCCATCACAA
ACATGCATCGCGAGAAGACACCTGGGACCAGGCCCAACACAACATACACCTAGCAGGCGT
GACCGGTGGATCAGGGGACAAATACTACAAGCAGAAGTACTCCAGGAACGACTGGAATGG
AGAATCAGAGGAGTACAACAGGCGGCCAAAGAGCTGGGTGAAGTCAATCGAGGCATTTGG
AGAGAGCTATATTTCCGAGAAGACCAAAGGGGAGATTTCTCAGCCTGGGGCGGCTATCAA
CGAGCACAAGAACGGCTCTGGGGGGAACAATCCTCACCAAGGGTCCTTAGACCTGGAGAT
TCGAAGCGAAGGAGGAAACATTTATGACTGTTGCATTAAAGCCCAAGAAGGAACTCTCGC
TATCCCTTGCTGTGGATTTCCCTTATGGCTATTTTGGGGGTCGGGGCGGCCGCTTCCCTT
TAGTGAGGGTTAATGCTTCGAGCAGACATGATAAGATACATTGATGAGTTTGGACAAACC
ACAACTAGAATGCAGTGAAAAAAATGCTTTATTTGTGAAATTTGTGATGCTATTGCTTTA
TTTGTAACCATTATAAGCTGCAATAAACAAGTTAACAACAACAATTGCATTCATTTTATG
TTTCAGGTTCAGGGGGAGATGTGGGAGGTTTTTTAAAGCAAGTAAAACCTCTACAAATGT
GGTAAAATCCGATAAGGATCGATCCGGGCTGGCGTAATAGCGAAGAGGCCCGCACCGATC
GCCCTTCCCAACAGTTGCGCAGCCTGAATGGCGAATGGACGCGCCCTGTAGCGGCGCATT
AAGCGCGGCGGGTGTGGTGGTTACGCGCAGCGTGACCGCTACACTTGCCAGCGCCCTAGC
GCCCGCTCCTTTCGCTTTCTTCCCTTCCTTTCTCGCCACGTTCGCCGGCTTTCCCCGTCA
AGCTCTAAATCGGGGGCTCCCTTTAGGGTTCCGATTTAGAGCTTTACGGCACCTCGACCG
CAAAAAACTTGATTTGGGTGATGGTTCACGTAGTGGGCCATCGCCCTGATAGACGGTTTT
 
TCGCCCTTTGACGTTGGAGTCCACGTTCTTTAATAGTGGACTCTTGTTCCAAACTGGAAC
AACACTCAACCCTATCTCGGTCTATTCTTTTGATTTATAAGGGATTTTGCCGATTTCGGC
CTATTGGTTAAAAAATGAGCTGATTTAACAAATATTTAACGCGAATTTTAACAAAATATT
AACGTTTACAATTTCGCCTGATGCGGTATTTTCTCCTTACGCATCTGTGCGGTATTTCAC
ACCGCATACGCGGATCTGCGCAGCACCATGGCCTGAAATAACCTCTGAAAGAGGAACTTG
GTTAGGTACCTTCTGAGGCGGAAAGAACCAGCTGTGGAATGTGTGTCAGTTAGGGTGTGG
AAAGTCCCCAGGCTCCCCAGCAGGCAGAAGTATGCAAAGCATGCATCTCAATTAGTCAGC
AACCAGGTGTGGAAAGTCCCCAGGCTCCCCAGCAGGCAGAAGTATGCAAAGCATGCATCT
CAATTAGTCAGCAACCATAGTCCCGCCCCTAACTCCGCCCATCCCGCCCCTAACTCCGCC
CAGTTCCGCCCATTCTCCGCCCCATGGCTGACTAATTTTTTTTATTTATGCAGAGGCCGA
GGCCGCCTCGGCCTCTGAGCTATTCCAGAAGTAGTGAGGAGGCTTTTTTGGAGGCCTAGG
CTTTTGCAAAAAGCTTGATTCTTCTGACACAACAGTCTCGAACTTAAGGCTAGAGCCACC
ATGATTGAACAAGATGGATTGCACGCAGGTTCTCCGGCCGCTTGGGTGGAGAGGCTATTC
GGCTATGACTGGGCACAACAGACAATCGGCTGCTCTGATGCCGCCGTGTTCCGGCTGTCA
GCGCAGGGGCGCCCGGTTCTTTTTGTCAAGACCGACCTGTCCGGTGCCCTGAATGAACTG
CAGGACGAGGCAGCGCGGCTATCGTGGCTGGCCACGACGGGCGTTCCTTGCGCAGCTGTG
CTCGACGTTGTCACTGAAGCGGGAAGGGACTGGCTGCTATTGGGCGAAGTGCCGGGGCAG
GATCTCCTGTCATCTCACCTTGCTCCTGCCGAGAAAGTATCCATCATGGCTGATGCAATG
CGGCGGCTGCATACGCTTGATCCGGCTACCTGCCCATTCGACCACCAAGCGAAACATCGC
ATCGAGCGAGCACGTACTCGGATGGAAGCCGGTCTTGTCGATCAGGATGATCTGGACGAA
GAGCATCAGGGGCTCGCGCCAGCCGAACTGTTCGCCAGGCTCAAGGCGCGCATGCCCGAC
GGCGAGGATCTCGTCGTGACCCATGGCGATGCCTGCTTGCCGAATATCATGGTGGAAAAT
GGCCGCTTTTCTGGATTCATCGACTGTGGCCGGCTGGGTGTGGCGGACCGCTATCAGGAC
ATAGCGTTGGCTACCCGTGATATTGCTGAAGAGCTTGGCGGCGAATGGGCTGACCGCTTC
CTCGTGCTTTACGGTATCGCCGCTCCCGATTCGCAGCGCATCGCCTTCTATCGCCTTCTT
GACGAGTTCTTCTGAGCGGGACTCTGGGGTTCGAAATGACCGACCAAGCGACGCCCAACC
TGCCATCACGATGGCCGCAATAAAATATCTTTATTTTCATTACATCTGTGTGTTGGTTTT
TTGTGTGAATCGATAGCGATAAGGATCCGCGTATGGTGCACTCTCAGTACAATCTGCTCT
GATGCCGCATAGTTAAGCCAGCCCCGACACCCGCCAACACCCGCTGACGCGCCCTGACGG
GCTTGTCTGCTCCCGGCATCCGCTTACAGACAAGCTGTGACCGTCTCCGGGAGCTGCATG
TGTCAGAGGTTTTCACCGTCATCACCGAAACGCGCGAGACGAAAGGGCCTCGTGATACGC
CTATTTTTATAGGTTAATGTCATGATAATAATGGTTTCTTAGACGTCAGGTGGCACTTTT
CGGGGAAATGTGCGCGGAACCCCTATTTGTTTATTTTTCTAAATACATTCAAATATGTAT
CCGCTCATGAGACAATAACCCTGATAAATGCTTCAATAATATTGAAAAAGGAAGAGTATG
AGTATTCAACATTTCCGTGTCGCCCTTATTCCCTTTTTTGCGGCATTTTGCCTTCCTGTT
TTTGCTCACCCAGAAACGCTGGTGAAAGTAAAAGATGCTGAAGATCAGTTGGGTGCACGA
GTGGGTTACATCGAACTGGATCTCAACAGCGGTAAGATCCTTGAGAGTTTTCGCCCCGAA
GAACGTTTTCCAATGATGAGCACTTTTAAAGTTCTGCTATGTGGCGCGGTATTATCCCGT
ATTGACGCCGGGCAAGAGCAACTCGGTCGCCGCATACACTATTCTCAGAATGACTTGGTT
GAGTACTCACCAGTCACAGAAAAGCATCTTACGGATGGCATGACAGTAAGAGAATTATGC
AGTGCTGCCATAACCATGAGTGATAACACTGCGGCCAACTTACTTCTGACAACGATCGGA
GGACCGAAGGAGCTAACCGCTTTTTTGCACAACATGGGGGATCATGTAACTCGCCTTGAT
CGTTGGGAACCGGAGCTGAATGAAGCCATACCAAACGACGAGCGTGACACCACGATGCCT
GTAGCAATGGCAACAACGTTGCGCAAACTATTAACTGGCGAACTACTTACTCTAGCTTCC
CGGCAACAATTAATAGACTGGATGGAGGCGGATAAAGTTGCAGGACCACTTCTGCGCTCG
GCCCTTCCGGCTGGCTGGTTTATTGCTGATAAATCTGGAGCCGGTGAGCGTGGGTCTCGC
GGTATCATTGCAGCACTGGGGCCAGATGGTAAGCCCTCCCGTATCGTAGTTATCTACACG
ACGGGGAGTCAGGCAACTATGGATGAACGAAATAGACAGATCGCTGAGATAGGTGCCTCA
CTGATTAAGCATTGGTAACTGTCAGACCAAGTTTACTCATATATACTTTAGATTGATTTA
AAACTTCATTTTTAATTTAAAAGGATCTAGGTGAAGATCCTTTTTGATAATCTCATGACC
AAAATCCCTTAACGTGAGTTTTCGTTCCACTGAGCGTCAGACCCCGTAGAAAAGATCAAA
GGATCTTCTTGAGATCCTTTTTTTCTGCGCGTAATCTGCTGCTTGCAAACAAAAAAACCA
CCGCTACCAGCGGTGGTTTGTTTGCCGGATCAAGAGCTACCAACTCTTTTTCCGAAGGTA
ACTGGCTTCAGCAGAGCGCAGATACCAAATACTGTCCTTCTAGTGTAGCCGTAGTTAGGC
CACCACTTCAAGAACTCTGTAGCACCGCCTACATACCTCGCTCTGCTAATCCTGTTACCA
GTGGCTGCTGCCAGTGGCGATAAGTCGTGTCTTACCGGGTTGGACTCAAGACGATAGTTA
CCGGATAAGGCGCAGCGGTCGGGCTGAACGGGGGGTTCGTGCACACAGCCCAGCTTGGAG
CGAACGACCTACACCGAACTGAGATACCTACAGCGTGAGCTATGAGAAAGCGCCACGCTT
CCCGAAGGGAGAAAGGCGGACAGGTATCCGGTAAGCGGCAGGGTCGGAACAGGAGAGCGC
ACGAGGGAGCTTCCAGGGGGAAACGCCTGGTATCTTTATAGTCCTGTCGGGTTTCGCCAC
CTCTGACTTGAGCGTCGATTTTTGTGATGCTCGTCAGGGGGGCGGAGCCTATGGAAAAAC
 
GCCAGCAACGCGGCCTTTTTACGGTTCCTGGCCTTTTGCTGGCCTTTTGCTCACATGGCT
CGACAGATCT
SEQ ID No. 9 - pONY4 .0Z 
CTAAATTGTAAGCGTTAATATTTTGTTAAAATTCGCGTTAAATTTTTGTTAAATCAGCTC
ATTTTTTAACCAATAGGCCGAAATCGGCAAAATCCCTTATAAATCAAAAGAATAGACCGA
GATAGGGTTGAGTGTTGTTCCAGTTTGGAACAAGAGTCCACTATTAAAGAACGTGGACTC
CAACGTCAAAGGGCGAAAAACCGTCTATCAGGGCGATGGCCCACTACGTGAACCATCACC
CTAATCAAGTTTTTTGGGGTCGAGGTGCCGTAAAGCACTAAATCGGAACCCTAAAGGGAG
CCCCCGATTTAGAGCTTGACGGGGAAAGCCAACCTGGCTTATCGAAATTAATACGACTCA
CTATAGGGAGACCGGCAGATCTTGAATAATAAAATGTGTGTTTGTCCGAAATACGCGTTT
TGAGATTTCTGTCGCCGACTAAATTCATGTCGCGCGATAGTGGTGTTTATCGCCGATAGA
GATGGCGATATTGGAAAAATTGATATTTGAAAATATGGCATATTGAAAATGTCGCCGATG
TGAGTTTCTGTGTAACTGATATCGCCATTTTTCCAAAAGTGATTTTTGGGCATACGCGAT
ATCTGGCGATAGCGCTTATATCGTTTACGGGGGATGGCGATAGACGACTTTGGTGACTTG
GGCGATTCTGTGTGTCGCAAATATCGCAGTTTCGATATAGGTGACAGACGATATGAGGCT
ATATCGCCGATAGAGGCGACATCAAGCTGGCACATGGCCAATGCATATCGATCTATACAT
TGAATCAATATTGGCCATTAGCCATATTATTCATTGGTTATATAGCATAAATCAATATTG
GCTATTGGCCATTGCATACGTTGTATCCATATCGTAATATGTACATTTATATTGGCTCAT
GTCCAACATTACCGCCATGTTGACATTGATTATTGACTAGTTATTAATAGTAATCAATTA
CGGGGTCATTAGTTCATAGCCCATATATGGAGTTCCGCGTTACATAACTTACGGTAAATG
GCCCGCCTGGCTGACCGCCCAACGACCCCCGCCCATTGACGTCAATAATGACGTATGTTC
CCATAGTAACGCCAATAGGGACTTTCCATTGACGTCAATGGGTGGAGTATTTACGGTAAA
CTGCCCACTTGGCAGTACATCAAGTGTATCATATGCCAAGTCCGCCCCCTATTGACGTCA
ATGACGGTAAATGGCCCGCCTGGCATTATGCCCAGTACATGACCTTACGGGACTTTCCTA
CTTGGCAGTACATCTACGTATTAGTCATCGCTATTACCATGGTGATGCGGTTTTGGCAGT
ACACCAATGGGCGTGGATAGCGGTTTGACTCACGGGGATTTCCAAGTCTCCACCCCATTG
ACGTCAATGGGAGTTTGTTTTGGCACCAAAATCAACGGGACTTTCCAAAATGTCGTAACA
ACTGCGATCGCCCGCCCCGTTGACGCAAATGGGCGGTAGGCGTGTACGGTGGGAGGTCTA
TATAAGCAGAGCTCGTTTAGTGAACCGGCACTCAGATTCTGCGGTCTGAGTCCCTTCTC
TGCTGGGCTGAAAAGGCCTTTGTAATAAATATAATTCTCTACTCAGTCCCTGTCTCTAGT
TTGTCTGTTCGAGATCCTACAGTTGGCGCCCGAACAGGGACCTGAGAGGGGCGCAGACCC
TACCTGTTGAACCTGGCTGATCGTAGGATCCCCGGGACAGCAGAGGAGAACTTACAGAAG
TCTTCTGGAGGTGTTCCTGGCCAGAACACAGGAGGACAGGTAAGATGGGAGACCCTTTGA
CATGGAGCAAGGCGCTCAAGAAGTTAGAGAAGGTGACGGTACAAGGGTCTCAGAAATTAA
CTACTGGTAACTGTAATTGGGCGCTAAGTCTAGTAGACTTATTTCATGATACCAACTTTG
TAAAAGAAAAGGACTGGCAGCTGAGGGATGTCATTCCATTGCTGGAAGATGTAACTCAGA
CGCTGTCAGGACAAGAAAGAGAGGCCTTTGAAAGAACATGGTGGGCAATTTCTGCTGTAA
AGATGGGCCTCCAGATTAATAATGTAGTAGATGGAAAGGCATCATTCCAGCTCCTAAGAG
CGAAATATGAAAAGAAGACTGCTAATAAAAAGCAGTCTGAGCCCTCTGAAGAATATCTCT
AGAACTAGTGGATCCCCCGGGCTGCAGGAGTGGGGAGGCACGATGGCCGCTTTGGTCGAG
GCGGATCCGGCCATTAGCCATATTATTCATTGGTTATATAGCATAAATCAATATTGGCTA
TTGGCCATTGCATACGTTGTATCCATATCATAATATGTACATTTATATTGGCTCATGTCC
AACATTACCGCCATGTTGACATTGATTATTGACTAGTTATTAATAGTAATCAATTACGGG
GTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCAT
AGTAACGCCAATAGGGACTTTCCATTGACGTCAATGGGTGGAGTATTTACGGTAAACTGC
CCACTTGGCAGTACATCAAGTGTATCATATGCCAAGTACGCCCCCTATTGACGTCAATGA
CGGTAAATGGCCCGCCTGGCATTATGCCCAGTACATGACCTTATGGGACTTTCCTACTTG
GCAGTACATCTACGTATTAGTCATCGCTATTACCATGGTGATGCGGTTTTGGCAGTACAT
CAATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTC
CGCCCCATTGACGCAAATGGGCGGTAGGCATGTACGGTGGGAGGTCTATATAAGCAGAGC
TCGTTTAGTGAACCGTCAGATCGCCTGGAGACGCCATCCACGCTGTTTTGACCTCCATAG
AAGACACCGGGACCGATCCAGCCTCCGCGGCCCCAAGCTTCAGCTGCTCGAGGATCTGCG
GATCCGGGGAATTCCCCAGTCTCAGGATCCACCATGGGGGATCCCGTCGTTTTACAACGT
CGTGACTGGGAAAACCCTGGCGTTACCCAACTTAATCGCCTTGCAGCACATCCCCCTTTC
GCCAGCTGGCGTAATAGCGAAGAGGCCCGCACCGATCGCCCTTCCCAACAGTTGCGCAGC
CTGAATGGCGAATGGCGCTTTGCCTGGTTTCCGGCACCAGAAGCGGTGCCGGAAAGCTGG
CTGGAGTGCGATCTTCCTGAGGCCGATACTGTCGTCGTCCCCTCAAACTGGCAGATGCAC
GGTTACGATGCGCCCATCTACACCAACGTAACCTATCCCATTACGGTCAATCCGCCGTTT
GTTCCCACGGAGAATCCGACGGGTTGTTACTCGCTCACATTTAATGTTGATGAAAGCTGG
CTACAGGAAGGCCAGACGCGAATTATTTTTGATGGCGTTAACTCGGCGTTTCATCTGTGG
 
TGCAACGGGCGCTGGGTCGGTTACGGCCAGGACAGTCGTTTGCCGTCTGAATTTGACCTG
AGCGCATTTTTACGCGCCGGAGAAAACCGCCTCGCGGTGATGGTGCTGCGTTGGAGTGAC
GGCAGTTATCTGGAAGATCAGGATATGTGGCGGATGAGCGGCATTTTCCGTGACGTCTCG
TTGCTGCATAAACCGACTACACAAATCAGCGATTTCCATGTTGCCACTCGCTTTAATGAT
GATTTCAGCCGCGCTGTACTGGAGGCTGAAGTTCAGATGTGCGGCGAGTTGCGTGACTAC
CTACGGGTAACAGTTTCTTTATGGCAGGGTGAAACGCAGGTCGCCAGCGGCACCGCGCCT
TTCGGCGGTGAAATTATCGATGAGCGTGGTGGTTATGCCGATCGCGTCACACTACGTCTG
AACGTCGAAAACCCGAAACTGTGGAGCGCCGAAATCCCGAATCTCTATCGTGCGGTGGTT
GAACTGCACACCGCCGACGGCACGCTGATTGAAGCAGAAGCCTGCGATGTCGGTTTCCGC
GAGGTGCGGATTGAAAATGGTCTGCTGCTGCTGAACGGCAAGCCGTTGCTGATTCGAGGC
GTTAACCGTCACGAGCATCATCCTCTGCATGGTCAGGTCATGGATGAGCAGACGATGGTG
CAGGATATCCTGCTGATGAAGCAGAACAACTTTAACGCCGTGCGCTGTTCGCATTATCCG
AACCATCCGCTGTGGTACACGCTGTGCGACCGCTACGGCCTGTATGTGGTGGATGAAGCC
AATATTGAAACCCACGGCATGGTGCCAATGAATCGTCTGACCGATGATCCGCGCTGGCTA
CCGGCGATGAGCGAACGCGTAACGCGAATGGTGCAGCGCGATCGTAATCACCCGAGTGTG
ATCATCTGGTCGCTGGGGAATGAATCAGGCCACGGCGCTAATCACGACGCGCTGTATCGC
TGGATCAAATCTGTCGATCCTTCCCGCCCGGTGCAGTATGAAGGCGGCGGAGCCGACACC
ACGGCCACCGATATTATTTGCCCGATGTACGCGCGCGTGGATGAAGACCAGCCCTTCCCG
GCTGTGCCGAAATGGTCCATCAAAAAATGGCTTTCGCTACCTGGAGAGACGCGCCCGCTG
ATCCTTTGCGAATACGCCCACGCGATGGGTAACAGTCTTGGCGGTTTCGCTAAATACTGG
CAGGCGTTTCGTCAGTATCCCCGTTTACAGGGCGGCTTCGTCTGGGACTGGGTGGATCAG
TCGCTGATTAAATATGATGAAAACGGCAACCCGTGGTCGGCTTACGGCGGTGATTTTGGC
GATACGCCGAACGATCGCCAGTTCTGTATGAACGGTCTGGTCTTTGCCGACCGCACGCCG
CATCCAGCGCTGACGGAAGCAAAACACCAGCAGCAGTTTTTCCAGTTCCGTTTATCCGGG
CAAACCATCGAAGTGACCAGCGAATACCTGTTCCGTCATAGCGATAACGAGCTCCTGCAC
TGGATGGTGGCGCTGGATGGTAAGCCGCTGGCAAGCGGTGAAGTGCCTCTGGATGTCGCT
CCACAAGGTAAACAGTTGATTGAACTGCCTGAACTACCGCAGCCGGAGAGCGCCGGGCAA
CTCTGGCTCACAGTACGCGTAGTGCAACCGAACGCGACCGCATGGTCAGAAGCCGGGCAC
ATCAGCGCCTGGCAGCAGTGGCGTCTGGCGGAAAACCTCAGTGTGACGCTCCCCGCCGCG
TCCCACGCCATCCCGCATCTGACCACCAGCGAAATGGATTTTTGCATCGAGCTGGGTAAT
AAGCGTTGGCAATTTAACCGCCAGTCAGGCTTTCTTTCACAGATGTGGATTGGCGATAAA
AAACAACTGCTGACGCCGCTGCGCGATCAGTTCACCCGTGCACCGCTGGATAACGACATT
GGCGTAAGTGAAGCGACCCGCATTGACCCTAACGCCTGGGTCGAACGCTGGAAGGCGGCG
GGCCATTACCAGGCCGAAGCAGCGTTGTTGCAGTGCACGGCAGATACACTTGCTGATGCG
GTGCTGATTACGACCGCTCACGCGTGGCAGCATCAGGGGAAAACCTTATTTATCAGCCGG
AAAACCTACCGGATTGATGGTAGTGGTCAAATGGCGATTACCGTTGATGTTGAAGTGGCG
AGCGATACACCGCATCCGGCGCGGATTGGCCTGAACTGCCAGCTGGCGCAGGTAGCAGAG
CGGGTAAACTGGCTCGGATTAGGGCCGCAAGAAAACTATCCCGACCGCCTTACTGCCGCC
TGTTTTGACCGCTGGGATCTGCCATTGTCAGACATGTATACCCCGTACGTCTTCCCGAGC
GAAAACGGTCTGCGCTGCGGGACGCGCGAATTGAATTATGGCCCACACCAGTGGCGCGGC
GACTTCCAGTTCAACATCAGCCGCTACAGTCAACAGCAACTGATGGAAACCAGCCATCGC
CATCTGCTGCACGCGGAAGAAGGCACATGGCTGAATATCGACGGTTTCCATATGGGGATT
GGTGGCGACGACTCCTGGAGCCCGTCAGTATCGGCGGAATTCCAGCTGAGCGCCGGTCGC
TACCATTACCAGTTGGTCTGGTGTCAAAAATAATAATAACCGGGCAGGGGGGATCCGCAG
ATCCGGCTGTGGAATGTGTGTCAGTTAGGGTGTGGAAAGTCCCCAGGCTCCCCAGCAGGC
AGAAGTATGCAAAGCATGCCTGCAGGAATTCGATATCAAGCTTATCGATACCGTCGACCT
CGAGGGGGGGCCCGGTACCCAGCTTTTGTTCCCTTTAGTGAGGGTTAATTGCGCGGGAAG
TATTTATCACTAATCAAGCACAAGTAATACATGAGAAACTTTTACTACAGCAAGCACAAT
CCTCCAAAAAATTTTGTTTTTACAAAATCCCTGGTGAACATGATTGGAAGGGACCTACTA
GGGTGCTGTGGAAGGGTGATGGTGCAGTAGTAGTTAATGATGAAGGAAAGGGAATAATTG
CTGTACCATTAACCAGGACTAAGTTACTAATAAAACCAAATTGAGTATTGTTGCAGGAAG
CAAGACCCAACTACCATTGTCAGCTGTGTTTCCTGAGGTCTCTAGGAATTGATTACCTCG
ATGCTTCATTAAGGAAGAAGAATAAACAAAGACTGAAGGCAATCCAACAAGGAAGACAAC
CTCAATATTTGTTATAAGGTTTGATATATGGGAGTATTTGGTAAAGGGGTAACATGGTCA
GCATCGCATTCTATGGGGGAATCCCAGGGGGAATCTCAACCCCTATTACCCAACAGTCAG
AAAAATCTAAGTGTGAGGAGAACACAATGTTTCAACCTTATTGTTATAATAATGACAGTA
AGAACAGCATGGCAGAATCGAAGGAAGCAAGAGACCAAGAAATGAACCTGAAAGAAGAAT
CTAAAGAAGAAAAAAGAAGAAATGACTGGTGGAAAATAGGTATGTTTCTGTTATGCTTAG
CAGGAACTACTGGAGGAATACTTTGGTGGTATGAAGGACTCCCACAGCAACATTATATAG
GGTTGGTGGCGATAGGGGGAAGATTAAACGGATCTGGCCAATCAAATGCTATAGAATGCT
GGGGTTCCTTCCCGGGGTGTAGACCATTTCAAAATTACTTCAGTTATGAGACCAATAGAA
 
GCATGCATATGGATAATAATACTGCTACATTATTAGAAGCTTTAACCAATATAACTGCTC
TATAAATAACAAAACAGAATTAGAAACATGGAAGTTAGTAAAGACTTCTGGCATAACTCC
TTTACCTATTTCTTCTGAAGCTAACACTGGACTAATTAGACATAAGAGAGATTTTGGTAT
AAGTGCAATAGTGGCAGCTATTGTAGCCGCTACTGCTATTGCTGCTAGCGCTACTATGTC
TTATGTTGCTCTAACTGAGGTTAACAAAATAATGGAAGTACAAAATCATACTTTTGAGGT
AGAAAATAGTACTCTAAATGGTATGGATTTAATAGAACGACAAATAAAGATATTATATGC
TATGATTCTTCAAACACATGCAGATGTTCAACTGTTAAAGGAAAGACAACAGGTAGAGGA
GACATTTAATTTAATTGGATGTATAGAAAGAACACATGTATTTTGTCATACTGGTCATCC
CTGGAATATGTCATGGGGACATTTAAATGAGTCAACACAATGGGATGACTGGGTAAGCAA
AATGGAAGATTTAAATCAAGAGATACTAACTACACTTCATGGAGCCAGGAACAATTTGGC
ACAATCCATGATAACATTCAATACACCAGATAGTATAGCTCAATTTGGAAAAGACCTTTG
GAGTCATATTGGAAATTGGATTCCTGGATTGGGAGCTTCCATTATAAAATATATAGTGAT
GTTTTTGCTTATTTATTTGTTACTAACCTCTTCGCCTAAGATCCTCAGGGCCCTCTGGAA
GGTGACCAGTGGTGCAGGGTCCTCCGGCAGTCGTTACCTGAAGAAAAAATTCCATCACAA
ACATGCATCGCGAGAAGACACCTGGGACCAGGCCCAACACAACATACACCTAGCAGGCGT
GACCGGTGGATCAGGGGACAAATACTACAAGCAGAAGTACTCCAGGAACGACTGGAATGG
AGAATCAGAGGAGTACAACAGGCGGCCAAAGAGCTGGGTGAAGTCAATCGAGGCATTTGG
AGAGAGCTATATTTCCGAGAAGACCAAAGGGGAGATTTCTCAGCCTGGGGCGGCTATCAA
CGAGCACAAGAACGGCTCTGGGGGGAACAATCCTCACCAAGGGTCCTTAGACCTGGAGAT
TCGAAGCGAAGGAGGAAACATTTATGACTGTTGCATTAAAGCCCAAGAAGGAACTCTCGC
TATCCCTTGCTGTGGATTTCCCTTATGGCTATTTTGGGGACTAGTAATTATAGTAGGACG
CATAGCAGGCTATGGATTACGTGGACTCGCTGTTATAATAAGGATTTGTATTAGAGGCTT
AAATTTGATATTTGAAATAATCAGAAAAATGCTTGATTATATTGGAAGAGCTTTAAATCC
TGGCACATCTCATGTATCAATGCCTCAGTATGTTTAGAAAAACAAGGGGGGAACTGTGGG
GTTTTTATGAGGGGTTTTATAAATGATTATAAGAGTAAAAAGAAAGTTGCTGATGCTCTC
ATAACCTTGTATAACCCAAAGGACTAGCTCATGTTGCTAGGCAACTAAACCGCAATAACC
GCATTTGTGACGCGAGTTCCCCATTGGTGACGCGTTAACTTCCTGTTTTTACAGTATATA
AGTGCTTGTATTCTGACAATTGGGCACTCAGATTCTGCGGTCTGAGTCCCTTCTCTGCTG
GGCTGAAAAGGCCTTTGTAATAAATATAATTCTCTACTCAGTCCCTGTCTCTAGTTTGTC
TGTTCGAGATCCTACAGAGCTCATGCCTTGGCGTAATCATGGTCATAGCTGTTTCCTGTG
TGAAATTGTTATCCGCTCACAATTCCACACAACATACGAGCCGGAAGCATAAAGTGTAAA
GCCTGGGGTGCCTAATGAGTGAGCTAACTCACATTAATTGCGTTGCGCTCACTGCCCGCT
TTCCAGTCGGGAAACCTGTCGTGCCAGCTGCATTAATGAATCGGCCAACGCGCGGGGAGA
GGCGGTTTGCGTATTGGGCGCTCTTCCGCTTCCTCGCTCACTGACTCGCTGCGCTCGGTC
GTTCGGCTGCGGCGAGCGGTATCAGCTCACTCAAAGGCGGTAATACGGTTATCCACAGAA
TCAGGGGATAACGCAGGAAAGAACATGTGAGCAAAAGGCCAGCAAAAGGCCAGGAACCGT
AAAAAGGCCGCGTTGCTGGCGTTTTTCCATAGGCTCCGCCCCCCTGACGAGCATCACAAA
AATCGACGCTCAAGTCAGAGGTGGCGAAACCCGACAGGACTATAAAGATACCAGGCGTTT
CCCCCTGGAAGCTCCCTCGTGCGCTCTCCTGTTCCGACCCTGCCGCTTACCGGATACCTG
TCCGCCTTTCTCCCTTCGGGAAGCGTGGCGCTTTCTCATAGCTCACGCTGTAGGTATCTC
AGTTCGGTGTAGGTCGTTCGCTCCAAGCTGGGCTGTGTGCACGAACCCCCCGTTCAGCCC
GACCGCTGCGCCTTATCCGGTAACTATCGTCTTGAGTCCAACCCGGTAAGACACGACTTA
TCGCCACTGGCAGCAGCCACTGGTAACAGGATTAGCAGAGCGAGGTATGTAGGCGGTGCT
ACAGAGTTCTTGAAGTGGTGGCCTAACTACGGCTACACTAGAAGGACAGTATTTGGTATC
TGCGCTCTGCTGAAGCCAGTTACCTTCGGAAAAAGAGTTGGTAGCTCTTGATCCGGCAAA
CAAACCACCGCTGGTAGCGGTGGTTTTTTTGTTTGCAAGCAGCAGATTACGCGCAGAAAA
AAAGGATCTCAAGAAGATCCTTTGATCTTTTCTACGGGGTCTGACGCTCAGTGGAACGAA
AACTCACGTTAAGGGATTTTGGTCATGAGATTATCAAAAAGGATCTTCACCTAGATCCTT
TTAAATTAAAAATGAAGTTTTAAATCAATCTAAAGTATATATGAGTAAACTTGGTCTGAC
AGTTACCAATGCTTAATCAGTGAGGCACCTATCTCAGCGATCTGTCTATTTCGTTCATCC
ATAGTTGCCTGACTCCCCGTCGTGTAGATAACTACGATACGGGAGGGCTTACCATCTGGC
CCCAGTGCTGCAATGATACCGCGAGACCCACGCTCACCGGCTCCAGATTTATCAGCAATA
AACCAGCCAGCCGGAAGGGCCGAGCGCAGAAGTGGTCCTGCAACTTTATCCGCCTCCATC
CAGTCTATTAATTGTTGCCGGGAAGCTAGAGTAAGTAGTTCGCCAGTTAATAGTTTGCGC
AACGTTGTTGCCATTGCTACAGGCATCGTGGTGTCACGCTCGTCGTTTGGTATGGCTTCA
TTCAGCTCCGGTTCCCAACGATCAAGGCGAGTTACATGATCCCCCATGTTGTGCAAAAAA
GCGGTTAGCTCCTTCGGTCCTCCGATCGTTGTCAGAAGTAAGTTGGCCGCAGTGTTATCA
CTCATGGTTATGGCAGCACTGCATAATTCTCTTACTGTCATGCCATCCGTAAGATGCTTT
TCTGTGACTGGTGAGTACTCAACCAAGTCATTCTGAGAATAGTGTATGCGGCGACCGAGT
TGCTCTTGCCCGGCGTCAATACGGGATAATACCGCGCCACATAGCAGAACTTTAAAAGTG
CTCATCATTGGAAAACGTTCTTCGGGGCGAAAACTCTCAAGGATCTTACCGCTGTTGAGA
 
TCCAGTTCGATGTAACCCACTCGTGCACCCAACTGATCTTCAGCATCTTTTACTTTCACC
AGCGTTTCTGGGTGAGCAAAAACAGGAAGGCAAAATGCCGCAAAAAAGGGAATAAGGGCG
ACACGGAAATGTTGAATACTCATACTCTTCCTTTTTCAATATTATTGAAGCATTTATCAG
GGTTATTGTCTCATGAGCGGATACATATTTGAATGTATTTAGAAAAATAAACAAATAGGG
GTTCCGCGCACATTTCCCCGAAAAGTGCCAC
SEQ ID No. 10 - pONY8.0Z 
AGATCTTGAATAATAAAATGTGTGTTTGTCCGAAATACGCGTTTTGAGATTTCTGTCGCC
GACTAAATTCATGTCGCGCGATAGTGGTGTTTATCGCCGATAGAGATGGCGATATTGGAA
AAATTGATATTTGAAAATATGGCATATTGAAAATGTCGCCGATGTGAGTTTCTGTGTAAC
TGATATCGCCATTTTTCCAAAAGTGATTTTTGGGCATACGCGATATCTGGCGATAGCGCT
TATATCGTTTACGGGGGATGGCGATAGACGACTTTGGTGACTTGGGCGATTCTGTGTGTC
GCAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCA
TACGTTGTATCCATATCGTAATATGTACATTTATATTGGCTCATGTCCAACATTACCGCC
ATGTTGACATTGATTATTGACTAGTTATTAATAGTAATCAATTACGGGGTCATTAGTTCA
TAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAGT
ACATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTGG
ATAGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTGCGATCGCCCGCC
CCGTTGACGCAAATGGGCGGTAGGCGTGTACGGTGGGAGGTCTATATAAGCAGAGCTCGT
TTAGTGAACCGGGCACTCAGATTCTGCGGTCTGAGTCCCTTCTCTGCTGGGCTGAAAAGG
CCTTTGTAATAAATATAATTCTCTACTCAGTCCCTGTCTCTAGTTTGTCTGTTCGAGATC
CTACAGTTGGCGCCCGAACAGGGACCTGAGAGGGGCGCAGACCCTACCTGTTGAACCTGG
CTGATCGTAGGATCCCCGGGACAGCAGAGGAGAACTTACAGAAGTCTTCTGGAGGTGTTC
CTGGCCAGAACACAGGAGGACAGGTAAGATTGGGAGACCCTTTGACATTGGAGCAAGGCG
CTCAAGAAGTTAGAGAAGGTGACGGTACAAGGGTCTCAGAAATTAACTACTGGTAACTGT
AATTGGGCGCTAAGTCTAGTAGACTTATTTCATGATACCAACTTTGTAAAAGAAAAGGAC
TGGCAGCTGAGGGATGTCATTCCATTGCTGGAAGATGTAACTCAGACGCTGTCAGGACAA
GAAAGAGAGGCCTTTGAAAGAACATGGTGGGCAATTTCTGCTGTAAAGATGGGCCTCCAG
ATTAATAATGTAGTAGATGGAAAGGCATCATTCCAGCTCCTAAGAGCGAAATATGAAAAG
AAGACTGCTAATAAAAAGCAGTCTGAGCCCTCTGAAGAATATCTCTAGAACTAGTGGATC
CCCCGGGCTGCAGGAGTGGGGAGGCACGATGGCCGCTTTGGTCGAGGCGGATCCGGCCAT
TAGCCATATTATTCATTGGTTATATAGCATAAATCAATATTGGCTATTGGCCATTGCATA
CGTTGTATCCATATCATAATATGTACATTTATATTGGCTCATGTCCAACATTACCGCCAT
GTTGACATTGATTATTGACTAGTTATTAATAGTAATCAATTACGGGGTCATTAGTTCATA
GCCCATATATGGAGTTCCGCGTTACATAACTTACGGTAAATGGCCCGCCTGGCTGACCGC
CCAACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAGTAC
ATCAAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTACG
TATTAGTCATCGCTATTACCATGGTGATGCGGTTTTGGCAGTACATCAATGGGCGTGGAT
AGCGGTTTGACTCACGGGGATTTCCAAGTCTCCACCCCATTGACGTCAATGGGAGTTTGT
TTTGGCACCAAAATCAACGGGACTTTCCAAAATGTCGTAACAACTCCGCCCCATTGACGC
AAATGGGCGGTAGGCATGTACGGTGGGAGGTCTATATAAGCAGAGCTCGTTTAGTGAACC
GTCAGATCGCCTGGAGACGCCATCCACGCTGTTTTGACCTCCATAGAAGACACCGGGACC
GATCCAGCCTCCGCGGCCCCAAGCTTCAGCTGCTCGAGGATCTGCGGATCCGGGGAATTC
CCCAGTCTCAGGATCCACCATGGGGGATCCCGTCGTTTTACAACGTCGTGACTGGGAAAA
CCCTGGCGTTACCCAACTTAATCGCCTTGCAGCACATCCCCCTTTCGCCAGCTGGCGTAA
TAGCGAAGAGGCCCGCACCGATCGCCCTTCCCAACAGTTGCGCAGCCTGAATGGCGAATG
GCGCTTTGCCTGGTTTCCGGCACCAGAAGCGGTGCCGGAAAGCTGGCTGGAGTGCGATCT
TCCTGAGGCCGATACTGTCGTCGTCCCCTCAAACTGGCAGATGCACGGTTACGATGCGCC
CATCTACACCAACGTAACCTATCCCATTACGGTCAATCCGCCGTTTGTTCCCACGGAGAA
TCCGACGGGTTGTTACTCGCTCACATTTAATGTTGATGAAAGCTGGCTACAGGAAGGCCA
GACGCGAATTATTTTTGATGGCGTTAACTCGGCGTTTCATCTGTGGTGCAACGGGCGCTG
GGTCGGTTACGGCCAGGACAGTCGTTTGCCGTCTGAATTTGACCTGAGCGCATTTTTACG
CGCCGGAGAAAACCGCCTCGCGGTGATGGTGCTGCGTTGGAGTGACGGCAGTTATCTGGA
AGATCAGGATATGTGGCGGATGAGCGGCATTTTCCGTGACGTCTCGTTGCTGCATAAACC
GACTACACAAATCAGCGATTTCCATGTTGCCACTCGCTTTAATGATGATTTCAGCCGCGC
TGTACTGGAGGCTGAAGTTCAGATGTGCGGCGAGTTGCGTGACTACCTACGGGTAACAGT
TTCTTTATGGCAGGGTGAAACGCAGGTCGCCAGCGGCACCGCGCCTTTCGGCGGTGAAAT
 
TATCGATGAGCGTGGTGGTTATGCCGATCGCGTCACACTACGTCTGAACGTCGAAAACCC
GAAACTGTGGAGCGCCGAAATCCCGAATCTCTATCGTGCGGTGGTTGAACTGCACACCGC
CGACGGCACGCTGATTGAAGCAGAAGCCTGCGATGTCGGTTTCCGCGAGGTGCGGATTGA
AAATGGTCTGCTGCTGCTGAACGGCAAGCCGTTGCTGATTCGAGGCGTTAACCGTCACGA
GCATCATCCTCTGCATGGTCAGGTCATGGATGAGCAGACGATGGTGCAGGATATCCTGCT
GATGAAGCAGAACAACTTTAACGCCGTGCGCTGTTCGCATTATCCGAACCATCCGCTGTG
GTACACGCTGTGCGACCGCTACGGCCTGTATGTGGTGGATGAAGCCAATATTGAAACCCA
CGGCATGGTGCCAATGAATCGTCTGACCGATGATCCGCGCTGGCTACCGGCGATGAGCGA
ACGCGTAACGCGAATGGTGCAGCGCGATCGTAATCACCCGAGTGTGATCATCTGGTCGCT
GGGGAATGAATCAGGCCACGGCGCTAATCACGACGCGCTGTATCGCTGGATCAAATCTGT
CGATCCTTCCCGCCCGGTGCAGTATGAAGGCGGCGGAGCCGACACCACGGCCACCGATAT
TATTTGCCCGATGTACGCGCGCGTGGATGAAGACCAGCCCTTCCCGGCTGTGCCGAAATG
GTCCATCAAAAAATGGCTTTCGCTACCTGGAGAGACGCGCCCGCTGATCCTTTGCGAATA
CGCCCACGCGATGGGTAACAGTCTTGGCGGTTTCGCTAAATACTGGCAGGCGTTTCGTCA
GTATCCCCGTTTACAGGGCGGCTTCGTCTGGGACTGGGTGGATCAGTCGCTGATTAAATA
TGATGAAAACGGCAACCCGTGGTCGGCTTACGGCGGTGATTTTGGCGATACGCCGAACGA
TCGCCAGTTCTGTATGAACGGTCTGGTCTTTGCCGACCGCACGCCGCATCCAGCGCTGAC
GGAAGCAAAACACCAGCAGCAGTTTTTCCAGTTCCGTTTATCCGGGCAAACCATCGAAGT
GACCAGCGAATACCTGTTCCGTCATAGCGATAACGAGCTCCTGCACTGGATGGTGGCGCT
GGATGGTAAGCCGCTGGCAAGCGGTGAAGTGCCTCTGGATGTCGCTCCACAAGGTAAACA
GTTGATTGAACTGCCTGAACTACCGCAGCCGGAGAGCGCCGGGCAACTCTGGCTCACAGT
ACGCGTAGTGCAACCGAACGCGACCGCATGGTCAGAAGCCGGGCACATCAGCGCCTGGCA
GCAGTGGCGTCTGGCGGAAAACCTCAGTGTGACGCTCCCCGCCGCGTCCCACGCCATCCC
GCATCTGACCACCAGCGAAATGGATTTTTGCATCGAGCTGGGTAATAAGCGTTGGCAATT
TAACCGCCAGTCAGGCTTTCTTTCACAGATGTGGATTGGCGATAAAAAACAACTGCTGAC
GCCGCTGCGCGATCAGTTCACCCGTGCACCGCTGGATAACGACATTGGCGTAAGTGAAGC
GACCCGCATTGACCCTAACGCCTGGGTCGAACGCTGGAAGGCGGCGGGCCATTACCAGGC
CGAAGCAGCGTTGTTGCAGTGCACGGCAGATACACTTGCTGATGCGGTGCTGATTACGAC
CGCTCACGCGTGGCAGCATCAGGGGAAAACCTTATTTATCAGCCGGAAAACCTACCGGAT
TGATGGTAGTGGTCAAATGGCGATTACCGTTGATGTTGAAGTGGCGAGCGATACACCGCA
TCCGGCGCGGATTGGCCTGAACTGCCAGCTGGCGCAGGTAGCAGAGCGGGTAAACTGGCT
CGGATTAGGGCCGCAAGAAAACTATCCCGACCGCCTTACTGCCGCCTGTTTTGACCGCTG
GGATCTGCCATTGTCAGACATGTATACCCCGTACGTCTTCCCGAGCGAAAACGGTCTGCG
CTGCGGGACGCGCGAATTGAATTATGGCCCACACCAGTGGCGCGGCGACTTCCAGTTCAA
CATCAGCCGCTACAGTCAACAGCAACTGATGGAAACCAGCCATCGCCATCTGCTGCACGC
GGAAGAAGGCACATGGCTGAATATCGACGGTTTCCATATGGGGATTGGTGGCGACGACTC
CTGGAGCCCGTCAGTATCGGCGGAATTCCAGCTGAGCGCCGGTCGCTACCATTACCAGTT
GGTCTGGTGTCAAAAATAATAATAACCGGGCAGGGGGGATCCGCAGATCCGGCTGTGGAA
TGTGTGTCAGTTAGGGTGTGGAAAGTCCCCAGGCTCCCCAGCAGGCAGAAGTATGCAAAG
CATGCCTGCAGGAATTCGATATCAAGCTTATCGATACCGTCGACCTCGAGGGGGGGCCCG
GTACCCAGCTTTTGTTCCCTTTAGTGAGGGTTAATTGCGCGGAAGTATTTATCACTAAT
CAAGCACAAGTAATACATGAGAAACTTTTACTACAGCAAGCACAATCCTCCAAAAAATTT
TGTTTTTACAAAATCCCTGGTGAACATGATTGGAAGGGACCTACTAGGGTGCTGTGGAAG
GGTGATGGTGCAGTAGTAGTTAATGATGAAGGAAAGGGAATAATTGCTGTACCATTAACC
AGGACTAAGTTACTAATAAAACCAAATTGAGTATTGTTGCAGGAAGCAAGACCCAACTAC
CATTGTCAGCTGTGTTTCCTGACCTCAATATTTGTTATAAGGTTTGATATGAATCCCAGG
GGGAATCTCAACCCCTATTACCCAACAGTCAGAAAAATCTAAGTGTGAGGAGAACACAAT
GTTTCAACCTTATTGTTATAATAATGACAGTAAGAACAGCATGGCAGAATCGAAGGAAGC
AAGAGACCAAGAATGAACCTGAAAGAAGAATCTAAAGAAGAAAAAGAAGAAATGACTGG
TGGAAAATAGGTATGTTTCTGTTATGCTTAGCAGGAACTACTGGAGGAATACTTTGGTGG
TATGAAGGACTCCCACAGCAACATTATATAGGGTTGGTGGCGATAGGGGGAAGATTAAAC
GGATCTGGCCAATCAAATGCTATAGAATGCTGGGGTTCCTTCCCGGGGTGTAGACCATTT
CAAAATTACTTCAGTTATGAGACCAATAGAAGCATGCATATGGATAATAATACTGCTACA
TTATTAGAAGCTTTAACCAATATAACTGCTCTATAAATAACAAAACAGAATTAGAAACAT
GGAAGTTAGTAAAGACTTCTGGCATAACTCCTTTACCTATTTCTTCTGAAGCTAACACTG
GACTAATTAGACATAAGAGAGATTTTGGTATAAGTGCAATAGTGGCAGCTATTGTAGCCG
CTACTGCTATTGCTGCTAGCGCTACTATGTCTTATGTTGCTCTAACTGAGGTTAACAAAA
TAATGGAAGTACAAAATCATACTTTTGAGGTAGAAAATAGTACTCTAAATGGTATGGATT
TAATAGAACGACAAATAAAGATATTATATGCTATGATTCTTCAAACACATGCAGATGTTC
AACTGTTAAAGGAAAGACAACAGGTAGAGGAGACATTTAATTTAATTGGATGTATAGAAA
GAACACATGTATTTTGTCATACTGGTCATCCCTGGAATATGTCATGGGGACATTTAAATG
 
AGTCAACACAATGGGATGACTGGGTAAGCAAAATGGAAGATTTAAATCAAGAGATACTAA
CTACACTTCATGGAGCCAGGAACAATTTGGCACAATCCATGATAACATTCAATACACCAG
ATAGTATAGCTCAATTTGGAAAAGACCTTTGGAGTCATATTGGAAATTGGATTCCTGGAT
TGGGAGCTTCCATTATAAAATATATAGTGATGTTTTGCTTATTTATTTGTTACTAACCT
CTTCGCCTAAGATCCTCAGGGCCCTCTGGAAGGTGACCAGTGGTGCAGGGTCCTCCGGCA
GTCGTTACCTGAAGAAAAAATTCCATCACAAACATGCATCGCGAGAAGACACCTGGGACC
AGGCCCAACACAACATACACCTAGCAGGCGTGACCGGTGGATCAGGGGACAAATACTACA
AGCAGAAGTACTCCAGGAACGACTGGAATGGAGAATCAGAGGAGTACAACAGGCGGCCAA
AGAGCTGGGTGAAGTCAATCGAGGCATTTGGAGAGAGCTATATTTCCGAGAAGACCAAAG
GGGAGATTTCTCAGCCTGGGGCGGCTATCAACGAGCACAAGAACGGCTCTGGGGGGAACA
ATCCTCACCAAGGGTCCTTAGACCTGGAGATTCGAAGCGAAGGAGGAAACATTTATGACT
GTTGCATTAAAGCCCAAGAAGGAACTCTCGCTATCCCTTGCTGTGGATTTCCCTTATGGC
TATTTTGGGGACTAGTAATTATAGTAGGACGCATAGCAGGCTATGGATTACGTGGACTCG
CTGTTATAATAAGGATTTGTATTAGAGGCTTAAATTTGATATTTGAAATAATCAGAAAAA
TGCTTGATTATATTGGAAGAGCTTTAAATCCTGGCACATCTCATGTATCAATGCCTCAGT
ATGTTTAGAAAAACAAGGGGGGAACTGTGGGGTTTTTATGAGGGGTTTTATAAATGATTA
TAAGAGTAAAAAGAAAGTTGCTGATGCTCTCATAACCTTGTATAACCCAAAGGACTAGCT
CATGTTGCTAGGCAACTAAACCGCAATAACCGCATTTGTGACGCGAGTTCCCCATTGGTG
ACGCGTTAACTTCCTGTTTTTACAGTATATAAGTGCTTGTATTCTGACAATTGGGCACTC
AGATTCTGCGGTCTGAGTCCCTTCTCTGCTGGGCTGAAAAGGCCTTTGTAATAAATATAA
TTCTCTACTCAGTCCCTGTCTCTAGTTTGTCTGTTCGAGATCCTACAGAGCTCATGCCTT
GGCGTAATCATGGTCATAGCTGTTTCCTGTGTGAAATTGTTATCCGCTCACAATTCCACA
CAACATACGAGCCGGAAGCATAAAGTGTAAAGCCTGGGGTGCCTAATGAGTGAGCTAACT
CACATTAATTGCGTTGCGCTCACTGCCCGCTTTCCAGTCGGGAAACCTGTCGTGCCAGCT
GCATTAATGAATCGGCCAACGCGCGGGGAGAGGCGGTTTGCGTATTGGGCGCTCTTCCGC
TTCCTCGCTCACTGACTCGCTGCGCTCGGTCGTTCGGCTGCGGCGAGCGGTATCAGCTCA
CTCAAAGGCGGTAATACGGTTATCCACAGAATCAGGGGATAACGCAGGAAAGAACATGTG
AGCAAAAGGCCAGCAAAAGGCCAGGAACCGTAAAAAGGCCGCGTTGCTGGCGTTTTTCCA
TAGGCTCCGCCCCCCTGACGAGCATCACAAAAATCGACGCTCAAGTCAGAGGTGGCGAAA
CCCGACAGGACTATAAAGATACCAGGCGTTTCCCCCTGGAAGCTCCCTCGTGCGCTCTCC
TGTTCCGACCCTGCCGCTTACCGGATACCTGTCCGCCTTTCTCCCTTCGGGAAGCGTGGC
GCTTTCTCATAGCTCACGCTGTAGGTATCTCAGTTCGGTGTAGGTCGTTCGCTCCAAGCT
GGGCTGTGTGCACGAACCCCCCGTTCAGCCCGACCGCTGCGCCTTATCCGGTAACTATCG
TCTTGAGTCCAACCCGGTAAGACACGACTTATCGCCACTGGCAGCAGCCACTGGTAACAG
GATTAGCAGAGCGAGGTATGTAGGCGGTGCTACAGAGTTCTTGAAGTGGTGGCCTAACTA
CGGCTACACTAGAAGGACAGTATTTGGTATCTGCGCTCTGCTGAAGCCAGTTACCTTCGG
AAAAAGAGTTGGTAGCTCTTGATCCGGCAAACAAACCACCGCTGGTAGCGGTGGTTTTTT
TGTTTGCAAGCAGCAGATTACGCGCAGAAAAAAAGGATCTCAAGAAGATCCTTTGATCTT
TTCTACGGGGTCTGACGCTCAGTGGAACGAAAACTCACGTTAAGGGATTTTGGTCATGAG
ATTATCAAAAAGGATCTTCACCTAGATCCTTTTAAATTAAAAATGAAGTTTTAAATCAAT
CTAAAGTATATATGAGTAAACTTGGTCTGACAGTTACCAATGCTTAATCAGTGAGGCACC
TATCTCAGCGATCTGTCTATTTCGTTCATCCATAGTTGCCTGACTCCCCGTCGTGTAGAT
AACTACGATACGGGAGGGCTTACCATCTGGCCCCAGTGCTGCAATGATACCGCGAGACCC
ACGCTCACCGGCTCCAGATTTATCAGCAATAAACCAGCCAGCCGGAAGGGCCGAGCGCAG
AAGTGGTCCTGCAACTTTATCCGCCTCCATCCAGTCTATTAATTGTTGCCGGGAAGCTAG
AGTAAGTAGTTCGCCAGTTAATAGTTTGCGCAACGTTGTTGCCATTGCTACAGGCATCGT
GGTGTCACGCTCGTCGTTTGGTATGGCTTCATTCAGCTCCGGTTCCCAACGATCAAGGCG
AGTTACATGATCCCCCATGTTGTGCAAAAAAGCGGTTAGCTCCTTCGGTCCTCCGATCGT
TGTCAGAAGTAAGTTGGCCGCAGTGTTATCACTCATGGTTATGGCAGCACTGCATAATTC
TCTTACTGTCATGCCATCCGTAAGATGCTTTTCTGTGACTGGTGAGTACTCAACCAAGTC
ATTCTGAGAATAGTGTATGCGGCGACCGAGTTGCTCTTGCCCGGCGTCAATACGGGATAA
TACCGCGCCACATAGCAGAACTTTAAAAGTGCTCATCATTGGAAAACGTTCTTCGGGGCG
AAAACTCTCAAGGATCTTACCGCTGTTGAGATCCAGTTCGATGTAACCCACTCGTGCACC
CAACTGATCTTCAGCATCTTTTACTTTCACCAGCGTTTCTGGGTGAGCAAAAACAGGAAG
GCAAAATGCCGCAAAAAAGGGAATAAGGGCGACACGGAAATGTTGAATACTCATACTCTT
CCTTTTTCAATATTATTGAAGCATTTATCAGGGTTATTGTCTCATGAGCGGATACATATT
TGAATGTATTTAGAAAAATAAACAAATAGGGGTTCCGCGCACATTTCCCCGAAAAGTGCC
ACCTAAATTGTAAGCGTTAATATTTTGTTAAAATTCGCGTTAAATTTTTGTTAAATCAGC
TCATTTTTTAACCAATAGGCCGAAATCGGCAAAATCCCTTATAAATCAAAAGAATAGACC
GAGATAGGGTTGAGTGTTGTTCCAGTTTGGAACAAGAGTCCACTATTAAAGAACGTGGAC
TCCAACGTCAAAGGGCGAAAAACCGTCTATCAGGGCGATGGCCCACTACGTGAACCATCA
 
CCCTAATCAAGTTTTTTGGGGTCGAGGTGCCGTAAAGCACTAAATCGGAACCCTAAAGGG
AGCCCCCGATTTAGAGCTTGACGGGGAAAGCCAACCTGGCTTATCGAAATTAATACGACT
CACTATAGGGAGACCGGC
SEQ ID No. 11 - pONY8.1Z 
AGATCTTGAATAATAAAATGTGTGTTTGTCCGAAATACGCGTTTTGAGATTTCTGTCGCC
GACTAAATTCATGTCGCGCGATAGTGGTGTTTATCGCCGATAGAGATGGCGATATTGGAA
AAATTGATATTTGAAAATATGGCATATTGAAAATGTCGCCGATGTGAGTTTCTGTGTAAC
TGATATCGCCATTTTTCCAAAAGTGATTTTTGGGCATACGCGATATCTGGCGATAGCGCT
TATATCGTTTACGGGGGATGGCGATAGACGACTTTGGTGACTTGGGCGATTCTGTGTGTC
GCAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCA
TACGTTGTATCCATATCGTAATATGTACATTTATATTGGCTCATGTCCAACATTACCGCC
ATGTTGACATTGATTATTGACTAGTTATTAATAGTAATCAATTACGGGGTCATTAGTTCA
TAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAGT
ACATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTGG
ATAGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTGCGATCGCCCGCC
CCGTTGACGCAAATGGGCGGTAGGCGTGTACGGTGGGAGGTCTATATAAGCAGAGCTCGT
TTAGTGAACCGGGCACTCAGATTCTGCGGTCTGAGTCCCTTCTCTGCTGGGCTGAAAAGG
CCTTTGTAATAAATATAATTCTCTACTCAGTCCCTGTCTCTAGTTTGTCTGTTCGAGATC
CTACAGTTGGCGCCCGAACAGGGACCTGAGAGGGGCGCAGACCCTACCTGTTGAACCTGG
CTGATCGTAGGATCCCCGGGACAGCAGAGGAGAACTTACAGAAGTCTTCTGGAGGTGTTC
CTGGCCAGAACACAGGAGGACAGGTAAGATTGGGAGACCCTTTGACATTGGAGCAAGGCG
CTCAAGAAGTTAGAGAAGGTGACGGTACAAGGGTCTCAGAAATTAACTACTGGTAACTGT
AATTGGGCGCTAAGTCTAGTAGACTTATTTCATGATACCAACTTTGTAAAAGAAAAGGAC
TGGCAGCTGAGGGATGTCATTCCATTGCTGGAAGATGTAACTCAGACGCTGTCAGGACAA
GAAAGAGAGGCCTTTGAAAGAACATGGTGGCAATTTCTGCTGTAAAGATGGCCTCCAG
ATTAATAATGTAGTAGATGGAAAGGCATCATTCCAGCTCCTAAGAGCGAAATATGAAAAG
AAGACTGCTAATAAAAAGCAGTCTGAGCCCTCTGAAGAATATCTCTAGAACTAGTGGATC
CCCCGGGCTGCAGGAGTGGGGAGGCACGATGGCCGCTTTGGTCGAGGCGGATCCGGCCAT
TAGCCATATTATTCATTGGTTATATAGCATAAATCAATATTGGCTATTGGCCATTGCATA
CGTTGTATCCATATCATAATATGTACATTTATATTGGCTCATGTCCAACATTACCGCCAT
GTTGACATTGATTATTGACTAGTTATTAATAGTAATCAATTACGGGGTCATTAGTTCATA
GCCCATATATGGAGTTCCGCGTTACATAACTTACGGTAAATGGCCCGCCTGGCTGACCGC
CCAACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAGTAC
ATCAAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTACG
TATTAGTCATCGCTATTACCATGGTGATGCGGTTTTGGCAGTACATCAATGGGCGTGGAT
AGCGGTTTGACTCACGGGGATTTCCAAGTCTCCACCCCATTGACGTCAATGGGAGTTTGT
TTTGGCACCAAAATCAACGGGACTTTCCAAAATGTCGTAACAACTCCGCCCCATTGACGC
AAATGGGCGGTAGGCATGTACGGTGGGAGGTCTATATAAGCAGAGCTCGTTTAGTGAACC
GTCAGATCGCCTGGAGACGCCATCCACGCTGTTTTGACCTCCATAGAAGACACCGGGACC
GATCCAGCCTCCGCGGCCCCAAGCTTCAGCTGCTCGAGGATCTGCGGATCCGGGGAATTC
CCCAGTCTCAGGATCCACCATGGGGGATCCCGTCGTTTTACAACGTCGTGACTGGGAAAA
CCCTGGCGTTACCCAACTTAATCGCCTTGCAGCACATCCCCCTTTCGCCAGCTGGCGTAA
TAGCGAAGAGGCCCGCACCGATCGCCCTTCCCAACAGTTGCGCAGCCTGAATGGCGAATG
GCGCTTTGCCTGGTTTCCGGCACCAGAAGCGGTGCCGGAAAGCTGGCTGGAGTGCGATCT
TCCTGAGGCCGATACTGTCGTCGTCCCCTCAAACTGGCAGATGCACGGTTACGATGCGCC
CATCTACACCAACGTAACCTATCCCATTACGGTCAATCCGCCGTTTGTTCCCACGGAGAA
TCCGACGGGTTGTTACTCGCTCACATTTAATGTTGATGAAAGCTGGCTACAGGAAGGCCA
GACGCGAATTATTTTTGATGGCGTTAACTCGGCGTTTCATCTGTGGTGCAACGGGCGCTG
GGTCGGTTACGGCCAGGACAGTCGTTTGCCGTCTGAATTTGACCTGAGCGCATTTTTACG
CGCCGGAGAAAACCGCCTCGCGGTGATGGTGCTGCGTTGGAGTGACGGCAGTTATCTGGA
AGATCAGGATATGTGGCGGATGAGCGGCATTTTCCGTGACGTCTCGTTGCTGCATAAACC
GACTACACAAATCAGCGATTTCCATGTTGCCACTCGCTTTAATGATGATTTCAGCCGCGC
TGTACTGGAGGCTGAAGTTCAGATGTGCGGCGAGTTGCGTGACTACCTACGGGTAACAGT
TTCTTTATGGCAGGGTGAAACGCAGGTCGCCAGCGGCACCGCGCCTTTCGGCGGTGAAAT
 
TATCGATGAGCGTGGTGGTTATGCCGATCGCGTCACACTACGTCTGAACGTCGAAAACCC
GAAACTGTGGAGCGCCGAAATCCCGAATCTCTATCGTGCGGTGGTTGAACTGCACACCGC
CGACGGCACGCTGATTGAAGCAGAAGCCTGCGATGTCGGTTTCCGCGAGGTGCGGATTGA
AAATGGTCTGCTGCTGCTGAACGGCAAGCCGTTGCTGATTCGAGGCGTTAACCGTCACGA
GCATCATCCTCTGCATGGTCAGGTCATGGATGAGCAGACGATGGTGCAGGATATCCTGCT
GATGAAGCAGAACAACTTTAACGCCGTGCGCTGTTCGCATTATCCGAACCATCCGCTGTG
GTACACGCTGTGCGACCGCTACGGCCTGTATGTGGTGGATGAAGCCAATATTGAAACCCA
CGGCATGGTGCCAATGAATCGTCTGACCGATGATCCGCGCTGGCTACCGGCGATGAGCGA
ACGCGTAACGCGAATGGTGCAGCGCGATCGTAATCACCCGAGTGTGATCATCTGGTCGCT
GGGGAATGAATCAGGCCACGGCGCTAATCACGACGCGCTGTATCGCTGGATCAAATCTGT
CGATCCTTCCCGCCCGGTGCAGTATGAAGGCGGCGGAGCCGACACCACGGCCACCGATAT
TATTTGCCCGATGTACGCGCGCGTGGATGAAGACCAGCCCTTCCCGGCTGTGCCGAAATG
GTCCATCAAAAAATGGCTTTCGCTACCTGGAGAGACGCGCCCGCTGATCCTTTGCGAATA
CGCCCACGCGATGGGTAACAGTCTTGGCGGTTTCGCTAAATACTGGCAGGCGTTTCGTCA
GTATCCCCGTTTACAGGGCGGCTTCGTCTGGGACTGGGTGGATCAGTCGCTGATTAAATA
TGATGAAAACGGCAACCCGTGGTCGGCTTACGGCGGTGATTTTGGCGATACGCCGAACGA
TCGCCAGTTCTGTATGAACGGTCTGGTCTTTGCCGACCGCACGCCGCATCCAGCGCTGAC
GGAAGCAAAACACCAGCAGCAGTTTTTCCAGTTCCGTTTATCCGGGCAAACCATCGAAGT
GACCAGCGAATACCTGTTCCGTCATAGCGATAACGAGCTCCTGCACTGGATGGTGGCGCT
GGATGGTAAGCCGCTGGCAAGCGGTGAAGTGCCTCTGGATGTCGCTCCACAAGGTAAACA
GTTGATTGAACTGCCTGAACTACCGCAGCCGGAGAGCGCCGGGCAACTCTGGCTCACAGT
ACGCGTAGTGCAACCGAACGCGACCGCATGGTCAGAAGCCGGGCACATCAGCGCCTGGCA
GCAGTGGCGTCTGGCGGAAAACCTCAGTGTGACGCTCCCCGCCGCGTCCCACGCCATCCC
GCATCTGACCACCAGCGAAATGGATTTTTGCATCGAGCTGGGTAATAAGCGTTGGCAATT
TAACCGCCAGTCAGGCTTTCTTTCACAGATGTGGATTGGCGATAAAAAACAACTGCTGAC
GCCGCTGCGCGATCAGTTCACCCGTGCACCGCTGGATAACGACATTGGCGTAAGTGAAGC
GACCCGCATTGACCCTAACGCCTGGGTCGAACGCTGGAAGGCGGCGGGCCATTACCAGGC
CGAAGCAGCGTTGTTGCAGTGCACGGCAGATACACTTGCTGATGCGGTGCTGATTACGAC
CGCTCACGCGTGGCAGCATCAGGGGAAAACCTTATTTATCAGCCGGAAAACCTACCGGAT
TGATGGTAGTGGTCAAATGGCGATTACCGTTGATGTTGAAGTGGCGAGCGATACACCGCA
TCCGGCGCGGATTGGCCTGAACTGCCAGCTGGCGCAGGTAGCAGAGCGGGTAAACTGGCT
CGGATTAGGGCCGCAAGAAAACTATCCCGACCGCCTTACTGCCGCCTGTTTTGACCGCTG
GGATCTGCCATTGTCAGACATGTATACCCCGTACGTCTTCCCGAGCGAAAACGGTCTGCG
CTGCGGGACGCGCGAATTGAATTATGGCCCACACCAGTGGCGCGGCGACTTCCAGTTCAA
CATCAGCCGCTACAGTCAACAGCAACTGATGGAAACCAGCCATCGCCATCTGCTGCACGC
GGAAGAAGGCACATGGCTGAATATCGACGGTTTCCATATGGGGATTGGTGGCGACGACTC
CTGGAGCCCGTCAGTATCGGCGGAATTCCAGCTGAGCGCCGGTCGCTACCATTACCAGTT
GGTCTGGTGTCAAAAATAATAATAACCGGGCAGGGGGGATCCGCAGATCCGGCTGTGGAA
TGTGTGTCAGTTAGGGTGTGGAAAGTCCCCAGGCTCCCCAGCAGGCAGAAGTATGCAAAG
CATGCCTGCAGGAATTCGATATCAAGCTTATCGATACCGTCGAATTGGAAGAGCTTTAAA
TCCTGGCACATCTCATGTATCAATGCCTCAGTATGTTTAGAAAAACAAGGGGGGAACTGT
GGGGTTTTTATGAGGGGTTTTATAAATGATTATAAGAGTAAAAAGAAAGTTGCTGATGCT
CTCATAACCTTGTATAACCCAAAGGACTAGCTCATGTTGCTAGGCAACTAAACCGCAATA
ACCGCATTTGTGACGCGAGTTCCCCATTGGTGACGCGTTAACTTCCTGTTTTTACAGTAT
ATAAGTGCTTGTATTCTGACAATTGGGCACTCAGATTCTGCGGTCTGAGTCCCTTCTCTG
CTGGGCTGAAAAGGCCTTTGTAATAAATATAATTCTCTACTCAGTCCCTGTCTCTAGTTT
GTCTGTTCGAGATCCTACAGAGCTCATGCCTTGGCGTAATCATGGTCATAGCTGTTTCCT
GTGTGAAATTGTTATCCGCTCACAATTCCACACAACATACGAGCCGGAAGCATAAAGTGT
AAAGCCTGGGGTGCCTAATGAGTGAGCTAACTCACATTAATTGCGTTGCGCTCACTGCCC
GCTTTCCAGTCGGGAAACCTGTCGTGCCAGCTGCATTAATGAATCGGCCAACGCGCGGGG
AGAGGCGGTTTGCGTATTGGGCGCTCTTCCGCTTCCTCGCTCACTGACTCGCTGCGCTCG
GTCGTTCGGCTGCGGCGAGCGGTATCAGCTCACTCAAAGGCGGTAATACGGTTATCCACA
GAATCAGGGGATAACGCAGGAAAGAACATGTGAGCAAAAGGCCAGCAAAAGGCCAGGAAC
CGTAAAAAGGCCGCGTTGCTGGCGTTTTTCCATAGGCTCCGCCCCCCTGACGAGCATCAC
AAAAATCGACGCTCAAGTCAGAGGTGGCGAAACCCGACAGGACTATAAAGATACCAGGCG
TTTCCCCCTGGAAGCTCCCTCGTGCGCTCTCCTGTTCCGACCCTGCCGCTTACCGGATAC
CTGTCCGCCTTTCTCCCTTCGGGAAGCGTGGCGCTTTCTCATAGCTCACGCTGTAGGTAT
CTCAGTTCGGTGTAGGTCGTTCGCTCCAAGCTGGGCTGTGTGCACGAACCCCCCGTTCAG
CCCGACCGCTGCGCCTTATCCGGTAACTATCGTCTTGAGTCCAACCCGGTAAGACACGAC
TTATCGCCACTGGCAGCAGCCACTGGTAACAGGATTAGCAGAGCGAGGTATGTAGGCGGT
GCTACAGAGTTCTTGAAGTGGTGGCCTAACTACGGCTACACTAGAAGGACAGTATTTGGT
 
ATCTGCGCTCTGCTGAAGCCAGTTACCTTCGGAAAAAGAGTTGGTAGCTCTTGATCCGGC
AAACAAACCACCGCTGGTAGCGGTGGTTTTTTTGTTTGCAAGCAGCAGATTACGCGCAGA
AAAAAAGGATCTCAAGAAGATCCTTTGATCTTTTCTACGGGGTCTGACGCTCAGTGGAAC
GAAAACTCACGTTAAGGGATTTTGGTCATGAGATTATCAAAAAGGATCTTCACCTAGATC
CTTTTAAATTAAAAATGAAGTTTTAAATCAATCTAAAGTATATATGAGTAAACTTGGTCT
GACAGTTACCAATGCTTAATCAGTGAGGCACCTATCTCAGCGATCTGTCTATTTCGTTCA
TCCATAGTTGCCTGACTCCCCGTCGTGTAGATAACTACGATACGGGAGGGCTTACCATCT
GGCCCCAGTGCTGCAATGATACCGCGAGACCCACGCTCACCGGCTCCAGATTTATCAGCA
ATAAACCAGCCAGCCGGAAGGGCCGAGCGCAGAAGTGGTCCTGCAACTTTATCCGCCTCC
ATCCAGTCTATTAATTGTTGCCGGGAAGCTAGAGTAAGTAGTTCGCCAGTTAATAGTTTG
CGCAACGTTGTTGCCATTGCTACAGGCATCGTGGTGTCACGCTCGTCGTTTGGTATGGCT
TCATTCAGCTCCGGTTCCCAACGATCAAGGCGAGTTACATGATCCCCCATGTTGTGCAAA
AAAGCGGTTAGCTCCTTCGGTCCTCCGATCGTTGTCAGAAGTAAGTTGGCCGCAGTGTTA
TCACTCATGGTTATGGCAGCACTGCATAATTCTCTTACTGTCATGCCATCCGTAAGATGC
TTTTCTGTGACTGGTGAGTACTCAACCAAGTCATTCTGAGAATAGTGTATGCGGCGACCG
AGTTGCTCTTGCCCGGCGTCAATACGGGATAATACCGCGCCACATAGCAGAACTTTAAAA
GTGCTCATCATTGGAAAACGTTCTTCGGGGCGAAAACTCTCAAGGATCTTACCGCTGTTG
AGATCCAGTTCGATGTAACCCACTCGTGCACCCAACTGATCTTCAGCATCTTTTACTTTC
ACCAGCGTTTCTGGGTGAGCAAAAACAGGAAGGCAAAATGCCGCAAAAAAGGGAATAAGG
GCGACACGGAAATGTTGAATACTCATACTCTTCCTTTTTCAATATTATTGAAGCATTTAT
CAGGGTTATTGTCTCATGAGCGGATACATATTTGAATGTATTTAGAAAAATAAACAAATA
GGGGTTCCGCGCACATTTCCCCGAAAAGTGCCACCTAAATTGTAAGCGTTAATATTTTGT
TAAAATTCGCGTTAAATTTTTGTTAAATCAGCTCATTTTTTAACCAATAGGCCGAAATCG
GCAAAATCCCTTATAAATCAAAAGAATAGACCGAGATAGGGTTGAGTGTTGTTCCAGTTT
GGAACAAGAGTCCACTATTAAAGAACGTGGACTCCAACGTCAAAGGGCGAAAAACCGTCT
ATCAGGGCGATGGCCCACTACGTGAACCATCACCCTAATCAAGTTTTTTGGGGTCGAGGT
GCCGTAAAGCACTAAATCGGAACCCTAAAGGGAGCCCCCGATTTAGAGCTTGACGGGGAA
AGCCAACCTGGCTTATCGAAATTAATACGACTCACTATAGGGAGACCGGC
SEQ ID No. 12 - pONY3.1 
AGATCTTCAATATTGGCCATTAGCCATATTATTCATTGGTTATATAGCATAAATCAATAT
TGGCTATTGGCCATTGCATACGTTGTATCTATATCATAATATGTACATTTATATTGGCTC
ATGTCCAATATGACCGCCATGTTGGCATTGATTATTGACTAGTTATTAATAGTAATCAAT
TACGGGGTCATTAGTTCATAGCCCATATATGGAGTTCCGCGTTACATAACTTACGGTAAA
TGGCCCGCCTGGCTGACCGCCCAACGACCCCCGCCCATTGACGTCAATAATGACGTATGT
TCCCATAGTAACGCCAATAGGGACTTTCCATTGACGTCAATGGGTGGAGTATTTACGGTA
AACTGCCCACTTGGCAGTACATCAAGTGTATCATATGCCAAGTCCGCCCCCTATTGACGT
CAATGACGGTAAATGGCCCGCCTGGCATTATGCCCAGTACATGACCTTACGGGACTTTCC
TACTTGGCAGTACATCTACGTATTAGTCATCGCTATTACCATGGTGATGCGGTTTTGGCA
GTACACCAATGGGCGTGGATAGCGGTTTGACTCACGGGGATTTCCAAGTCTCCACCCCAT
TGACGTCAATGGGAGTTTGTTTTGGCACCAAAATCAACGGGACTTTCCAAAATGTCGTAA
CAACTGCGATCGCCCGCCCCGTTGACGCAAATGGGCGGTAGGCGTGTACGGTGGGAGGTC
TATATAAGCAGAGCTCGTTTAGTGAACCGTCAGATCACTAGAAGCTTTATTGCGGTAGTT
TATCACAGTTAAATTGCTAACGCAGTCAGTGCTTCTGACACAACAGTCTCGAACTTAAGC
TGCAGTGACTCTCTTAAGGTAGCCTTGCAGAAGTTGGTCGTGAGGCACTGGGCAGGTAAG
TATCAAGGTTACAAGACAGGTTTAAGGAGACCAATAGAAACTGGGCTTGTCGAGACAGAG
AAGACTCTTGCGTTTCTGATAGGCACCTATTGGTCTTACTGACATCCACTTTGCCTTTCT
CTCCACAGGTGTCCACTCCCAGTTCAATTACAGCTCTTAAGGCTAGAGTACTTAATACGA
CTCACTATAGGCTAGCCTCGAGGTCGACGGTATCGCCCGAACAGGGACCTGAGAGGGGCG
CAGACCCTACCTGTTGAACCTGGCTGATCGTAGGATCCCCGGGACAGCAGAGGAGAACTT
ACAGAAGTCTTCTGGAGGTGTTCCTGGCCAGAACACAGGAGGACAGGTAAGATGGGAGAC
CCTTTGACATGGAGCAAGGCGCTCAAGAAGTTAGAGAAGGTGACGGTACAAGGGTCTCAG
AAATTAACTACTGGTAACTGTAATTGGGCGCTAAGTCTAGTAGACTTATTTCATGATACC
AACTTTGTAAAAGAAAAGGACTGGCAGCTGAGGGATGTCATTCCATTGCTGGAAGATGTA
ACTCAGACGCTGTCAGGACAAGAAAGAGAGGCCTTTGAAAGAACATGGTGGGCAATTTCT
GCTGTAAAGATGGGCCTCCAGATTAATAATGTAGTAGATGGAAAGGCATCATTCCAGCTC
CTAAGAGCGAAATATGAAAAGAAGACTGCTAATAAAAAGCAGTCTGAGCCCTCTGAAGAA
TATCCAATCATGATAGATGGGGCTGGAAACAGAAATTTTAGACCTCTAACACCTAGAGGA
TATACTACTTGGGTGAATACCATACAGACAAATGGTCTATTAAATGAAGCTAGTCAAAAC
TTATTTGGGATATTATCAGTAGACTGTACTTCTGAAGAAATGAATGCATTTTTGGATGTG
GTACCTGGCCAGGCAGGACAAAAGCAGATATTACTTGATGCAATTGATAAGATAGCAGAT
GATTGGGATAATAGACATCCATTACCGAATGCTCCACTGGTGGCACCACCACAAGGGCCT
ATTCCCATGACAGCAAGGTTTATTAGAGGTTTAGGAGTACCTAGAGAAAGACAGATGGAG
CCTGCTTTTGATCAGTTTAGGCAGACATATAGACAATGGATAATAGAAGCCATGTCAGAA
GGCATCAAAGTGATGATTGGAAAACCTAAAGCTCAAAATATTAGGCAAGGAGCTAAGGAA
CCTTACCCAGAATTTGTAGACAGACTATTATCCCAAATAAAAAGTGAGGGACATCCACAA
GAGATTTCAAAATTCTTGACTGATACACTGACTATTCAGAACGCAAATGAGGAATGTAGA
AATGCTATGAGACATTTAAGACCAGAGGATACATTAGAAGAGAAAATGTATGCTTGCAGA
GACATTGGAACTACAAAACAAAAGATGATGTTATTGGCAAAAGCACTTCAGACTGGTCTT
GCGGGCCCATTTAAAGGTGGAGCCTTGAAAGGAGGGCCACTAAAGGCAGCACAAACATGT
TATAACTGTGGGAAGCCAGGACATTTATCTAGTCAATGTAGAGCACCTAAAGTCTGTTTT
AAATGTAAACAGCCTGGACATTTCTCAAAGCAATGCAGAAGTGTTCCAAAAAACGGGAAG
CAAGGGGCTCAAGGGAGGCCCCAGAAACAAACTTTCCCGATACAACAGAAGAGTCAGCAC
AACAAATCTGTTGTACAAGAGACTCCTCAGACTCAAAATCTGTACCCAGATCTGAGCGAA
ATAAAAAAGGAATACAATGTCAAGGAGAAGGATCAAGTAGAGGATCTCAACCTGGACAGT
TTGTGGGAGTAACATATAATCTAGAGAAAAGGCCTACTACAATAGTATTAATTAATGATA
CTCCCTTAAATGTACTGTTAGACACAGGAGCAGATACTTCAGTGTTGACTACTGCACATT
ATAATAGGTTAAAATATAGAGGGAGAAAATATCAAGGGACGGAATAATAGGAGTGGGAG
GAAATGTGGAAACATTTTCTACGCCTGTGACTATAAAGAAAAAGGGTAGACACATTAAGA
CAAGAATGCTAGTGGCAGATATTCCAGTGACTATTTTGGGACGAGATATTCTTCAGGACT
TAGGTGCAAAATTGGTTTTGGCACAGCTCTCCAAGGAAATAAAATTTAGAAAAATAGAGT
TAAAAGAGGGCACAATGGGGCCAAAAATTCCTCAATGGCCACTCACTAAGGAGAAACTAG
AAGGGGCCAAAGAGATAGTCCAAAGACTATTGTCAGAGGGAAAAATATCAGAAGCTAGTG
ACAATAATCCTTATAATTCACCCATATTTGTAATAAAAAAGAGGTCTGGCAAATGGAGGT
TATTACAAGATCTGAGAGAATTAAACAAAACAGTACAAGTAGGAACGGAAATATCCAGAG
GATTGCCTCACCCGGGAGGATTAATTAAATGTAAACACATGACTGTATTAGATATTGGAG
ATGCATATTTCACTATACCCTTAGATCCAGAGTTTAGACCATATACAGCTTTCACTATTC
CCTCCATTAATCATCAAGAACCAGATAAAAGATATGTGTGGAAATGTTTACCACAAGGAT
TCGTGTTGAGCCCATATATATATCAGAAAACATTACAGGAAATTTTACAACCTTTTAGGG
 
AAAGATATCCTGAAGTACAATTGTATCAATATATGGATGATTTGTTCATGGAAGTAATG
GTTCTAAAAAACAACACAAAGAGTTAATCATAGAATTAAGGGCGATCTTACTGGAAAAGG
GTTTTGAGACACCAGATGATAAATTACAAGAAGTGCCACCTTATAGCTGGCTAGGTTATC
AACTTTGTCCTGAAAATTGGAAAGTACAAAAAATGCAATTAGACATGGTAAAGAATCCAA
CCCTTAATGATGTGCAAAAATTAATGGGGAATATAACATGGATGAGCTCAGGGATCCCAG
GGTTGACAGTAAAACACATTGCAGCTACTACTAAGGGATGTTTAGAGTTGAATCAAAAAG
TAATTTGGACGGAAGAGGCACAAAAAGAGTTAGAAGAAAATAATGAGAAGATTAAAAATG
CTCAAGGGTTACAATATTATAATCCAGAAGAAGAAATGTTATGTGAGGTTGAAATTACAA
AAAATTATGAGGCAACTTATGTTATAAAACAATCACAAGGAATCCTATGGGCAGGTAAAA
AGATTATGAAGGCTAATAAGGGATGGTCAACAGTAAAAAATTTAATGTTATTGTTGCAAC
ATGTGGCAACAGAAAGTATTACTAGAGTAGGAAAATGTCCAACGTTTAAGGTACCATTTA
CCAAAGAGCAAGTAATGTGGGAAATGCAAAAAGGATGGTATTATTCTTGGCTCCCAGAAA
TAGTATATACACATCAAGTAGTTCATGATGATTGGAGAATGAAATTGGTAGAAGAACCTA
CATCAGGAATAACAATATACACTGATGGGGGAAAACAAAATGGAGAAGGAATAGCAGCTT
ATGTGACCAGTAATGGGAGAACTAAACAGAAAAGGTTAGGACCTGTCACTCATCAAGTTG
CTGAAAGAATGGCAATACAAATGGCATTAGAGGATACCAGAGATAAACAAGTAAATATAG
TAACTGATAGTTATTATTGTTGGAAAAATATTACAGAAGGATTAGGTTTAGAAGGACCAC
AAAGTCCTTGGTGGCCTATAATACAAAATATACGAGAAAAAGAGATAGTTTATTTTGCTT
GGGTACCTGGTCACAAAGGGATATATGGTAATCAATTGGCAGATGAAGCCGCAAAAATAA
AAGAAGAAATCATGCTAGCATACCAAGGCACACAAATTAAAGAGAAAAGAGATGAAGATG
CAGGGTTTGACTTATGTGTTCCTTATGACATCATGATACCTGTATCTGACACAAAAATCA
TACCCACAGATGTAAAAATTCAAGTTCCTCCTAATAGCTTTGGATGGGTCACTGGAAAT
CATCAATGGCAAAACAGGGGTTATTAATTAATGGAGGAATAATTGATGAAGGATATACAG
GAGAAATACAAGTGATATGTACTAATATTGGAAAAAGTAATATTAAATTAATAGAGGGAC
AAAAATTTGCACAATTAATTATACTACAGCATCACTCAAATTCCAGACAGCCTTGGGATG
AAAATAAAATATCTCAGAGAGGGGATAAAGGATTTGGAAGTACAGGAGTATTCTGGGTAG
AAAATATTCAGGAAGCACAAGATGAACATGAGAATTGGCATACATCACCAAAGATATTGG
CAAGAAATTATAAGATACCATTGACTGTAGCAAAACAGATAACTCAAGAATGTCCTCATT
GCACTAAGCAAGGATCAGGACCTGCAGGTTGTGTCATGAGATCTCCTAATCATTGGCAGG
CAGATTGCACACATTTGGACAATAAGATAATATTGACTTTTGTAGAGTCAAATTCAGGAT
ACATACATGCTACATTATTGTCAAAAGAAAATGCATTATGTACTTCATTGGCTATTTTAG
AATGGGCAAGATTGTTTTCACCAAAGTCCTTACACACAGATAACGGCACTAATTTTGTGG
CAGAACCAGTTGTAAATTTGTTGAAGTTCCTAAAGATAGCACATACCACAGGAATACCAT
ATCATCCAGAAAGTCAGGGTATTGTAGAAAGGGCAAATAGGACCTTGAAAGAGAAGATTC
AAAGTCATAGAGACAACACTCAAACACTGGAGGCAGCTTTACAACTTGCTCTCATTACTT
GTAACAAAGGGAGGGAAAGTATGGAGGACAGACACCATGGGAAGTATTTATCACTAATC
AAGCACAAGTAATACATGAGAAACTTTTACTACAGCAAGCACAATCCTCCAAAAAATTTT
GTTTTTACAAAATCCCTGGTGAACATGATTGGAAGGGACCTACTAGGGTGCTGTGGAAGG
GTGATGGTGCAGTAGTAGTTAATGATGAAGGAAAGGGAATAATTGCTGTACCATTAACCA
GGACTAAGTTACTAATAAAACCAAATTGAGTATTGTTGCAGGAAGCAAGACCCAACTACC
ATTGTCAGCTGTGTTTCCTGAGGTCTCTAGGAATTGATTACCTCGATGCTTCATTAAGGA
AGAAGAATAAACAAAGACTGAAGGCAATCCAACAAGGAAGACAACCTCAATATTTGTTAT
AAGGTTTGATATATGGGAGTATTTGGTAAAGGGGTAACATGGTCAGCATCGCATTCTATG
GGGGAATCCCAGGGGGAATCTCAACCCCTATTACCCAACAGTCAGAAAAATCTAAGTGTG
AGGAGAACACAATGTTTCAACCTTATTGTTATAATAATGACAGTAAGAACAGCATGGCAG
AATCGAAGGAAGCAAGAGACCAAGAAATGAACCTGAAAGAAGAATCTAAAGAAGAAAAAA
GAAGAAATGACTGGTGGAAAATAGGTATGTTTCTGTTATGCTTAGCAGGAACTACTGGAG
GAATACTTTGGTGGTATGAAGGACTCCCACAGCAACATTATATAGGGTTGGTGGCGATAG
GGGGAAGATTAAACGGATCTGGCCAATCAAATGCTATAGAATGCTGGGGTTCCTTCCCGG
GGTGTAGACCATTTCAAAATTACTTCAGTTATGAGACCAATAGAAGCATGCATATGGATA
ATAATACTGCTACATTATTAGAAGCTTTAACCAATATAACTGCTCTATAAATAACAAAAC
AGAATTAGAAACATGGAAGTTAGTAAAGACTTCTGGCATAACTCCTTTACCTATTTCTTC
TGAAGCTAACACTGGACTAATTAGACATAAGAGAGATTTTGGTATAAGTGCAATAGTGGC
AGCTATTGTAGCCGCTACTGCTATTGCTGCTAGCGCTACTATGTCTTATGTTGCTCTAAC
TGAGGTTAACAAAATAATGGAAGTACAAAATCATACTTTTGAGGTAGAAAATAGTACTCT
AAATGGTATGGATTTAATAGAACGACAAATAAAGATATTATATGCTATGATTCTTCAAAC
ACATGCAGATGTTCAACTGTTAAAGGAAAGACAACAGGTAGAGGAGACATTTAATTTAAT
TGGATGTATAGAAAGAACACATGTATTTTGTCATACTGGTCATCCCTGGAATATGTCATG
GGGACATTTAAATGAGTCAACACAATGGGATGACTGGGTAAGCAAAATGGAAGATTTAAA
TCAAGAGATACTAACTACACTTCATGGAGCCAGGAACAATTTGGCACAATCCATGATAAC
ATTCAATACACCAGATAGTATAGCTCAATTTGGAAAAGACCTTTGGAGTCATATTGGAAA
 
TTGGATTCCTGGATTGGAGCTTCCATTATAAAATATATAGTGATGTTTTTGCTTATTTA
TTTGTTACTAACCTCTTCGCCTAAGATCCTCAGGGCCCTCTGGAAGGTGACCAGTGGTGC
AGGGTCCTCCGGCAGTCGTTACCTGAAGAAAAAATTCCATCACAAACATGCATCGCGAGA
AGACACCTGGGACCAGGCCCAACACAACATACACCTAGCAGGCGTGACCGGTGGATCAGG
GGACAAATACTACAAGCAGAAGTACTCCAGGAACGACTGGAATGGAGAATCAGAGGAGTA
CAACAGGCGGCCAAAGAGCTGGGTGAAGTCAATCGAGGCATTTGGAGAGAGCTATATTTC
CGAGAAGACCAAAGGGGAGATTTCTCAGCCTGGGGCGGCTATCAACGAGCACAAGAACGG
CTCTGGGGGGAACAATCCTCACCAAGGGTCCTTAGACCTGGAGATTCGAAGCGAAGGAGG
AAACATTTATGACTGTTGCATTAAAGCCCAAGAAGGAACTCTCGCTATCCCTTGCTGTGG
ATTTCCCTTATGGCTATTTTGGGACTAGTAATTATAGTAGGACGCATAGCAGGCTATGG
ATTACGTGGACTCGCTGTTATAATAAGGATTTGTATTAGAGGCTTAAATTTGATATTTGA
AATAATCAGAAAAATGCTTGATTATATTGGAAGAGCTTTAAATCCTGGCACATCTCATGT
ATCAATGCCTCAGTATGTTTAGAAAAACAAGGGGGGAACTGTGGGGTTTTTATGAGGGGT
TTTATAAATGATTATAAGAGTAAAAAGAAAGTTGCTGATGCTCTCATAACCTTGTATAAC
CCAAAGGACTAGCTCATGTTGCTAGGCAACTAAACCGCAATAACCGCATTTGTGACGCGA
GTTCCCCATTGGTGACGCGTGGTACCTCTAGAGTCGACCCGGGCGGCCGCTTCCCTTTAG
TGAGGGTTAATGCTTCGAGCAGACATGATAAGATACATTGATGAGTTTGGACAAACCACA
ACTAGAATGCAGTGAAAAAAATGCTTTATTTGTGAAATTTGTGATGCTATTGCTTTATTT
GTAACCATTATAAGCTGCAATAAACAAGTTAACAACAACAATTGCATTCATTTTATGTTT
CAGGTTCAGGGGGAGATGTGGGAGGTTTTTTAAAGCAAGTAAAACCTCTACAAATGTGGT
AAAATCCGATAAGGATCGATCCGGGCTGGCGTAATAGCGAAGAGGCCCGCACCGATCGCC
CTTCCCAACAGTTGCGCAGCCTGAATGGCGAATGGACGCGCCCTGTAGCGGCGCATTAAG
CGCGGCGGGTGTGGTGGTTACGCGCAGCGTGACCGCTACACTTGCCAGCGCCCTAGCGCC
CGCTCCTTTCGCTTTCTTCCCTTCCTTTCTCGCCACGTTCGCCGGCTTTCCCCGTCAAGC
TCTAAATCGGGGGCTCCCTTTAGGGTTCCGATTTAGAGCTTTACGGCACCTCGACCGCAA
AAAACTTGATTTGGGTGATGGTTCACGTAGTGGGCCATCGCCCTGATAGACGGTTTTTCG
CCCTTTGACGTTGGAGTCCACGTTCTTTAATAGTGGACTCTTGTTCCAAACTGGAACAAC
ACTCAACCCTATCTCGGTCTATTCTTTTGATTTATAAGGGATTTTGCCGATTTCGGCCTA
TTGGTTAAAAAATGAGCTGATTTAACAAATATTTAACGCGAATTTTAACAAAATATTAAC
GTTTACAATTTCGCCTGATGCGGTATTTTCTCCTTACGCATCTGTGCGGTATTTCACACC
GCATACGCGGATCTGCGCAGCACCATGGCCTGAAATAACCTCTGAAAGAGGAACTTGGTT
AGGTACCTTCTGAGGCGGAAAGAACCAGCTGTGGAATGTGTGTCAGTTAGGGTGTGGAAA
GTCCCCAGGCTCCCCAGCAGGCAGAAGTATGCAAAGCATGCATCTCAATTAGTCAGCAAC
CAGGTGTGGAAAGTCCCCAGGCTCCCCAGCAGGCAGAAGTATGCAAAGCATGCATCTCAA
TTAGTCAGCAACCATAGTCCCGCCCCTAACTCCGCCCATCCCGCCCCTAACTCCGCCCAG
TTCCGCCCATTCTCCGCCCCATGGCTGACTAATTTTTTTTATTTATGCAGAGGCCGAGGC
CGCCTCGGCCTCTGAGCTATTCCAGAAGTAGTGAGGAGGCTTTTTTGGAGGCCTAGGCTT
TTGCAAAAAGCTTGATTCTTCTGACACAACAGTCTCGAACTTAAGGCTAGAGCCACCATG
ATTGAACAAGATGGATTGCACGCAGGTTCTCCGGCCGCTTGGGTGGAGAGGCTATTCGGC
TATGACTGGGCACAACAGACAATCGGCTGCTCTGATGCCGCCGTGTTCCGGCTGTCAGCG
CAGGGGCGCCCGGTTCTTTTTGTCAAGACCGACCTGTCCGGTGCCCTGAATGAACTGCAG
GACGAGGCAGCGCGGCTATCGTGGCTGGCCACGACGGGCGTTCCTTGCGCAGCTGTGCTC
GACGTTGTCACTGAAGCGGGAAGGGACTGGCTGCTATTGGGCGAAGTGCCGGGGCAGGAT
CTCCTGTCATCTCACCTTGCTCCTGCCGAGAAAGTATCCATCATGGCTGATGCAATGCGG
CGGCTGCATACGCTTGATCCGGCTACCTGCCCATTCGACCACCAAGCGAAACATCGCATC
GAGCGAGCACGTACTCGGATGGAAGCCGGTCTTGTCGATCAGGATGATCTGGACGAAGAG
CATCAGGGGCTCGCGCCAGCCGAACTGTTCGCCAGGCTCAAGGCGCGCATGCCCGACGGC
GAGGATCTCGTCGTGACCCATGGCGATGCCTGCTTGCCGAATATCATGGTGGAAAATGGC
CGCTTTTCTGGATTCATCGACTGTGGCCGGCTGGGTGTGGCGGACCGCTATCAGGACATA
GCGTTGGCTACCCGTGATATTGCTGAAGAGCTTGGCGGCGAATGGGCTGACCGCTTCCTC
GTGCTTTACGGTATCGCCGCTCCCGATTCGCAGCGCATCGCCTTCTATCGCCTTCTTGAC
GAGTTCTTCTGAGCGGGACTCTGGGGTTCGAAATGACCGACCAAGCGACGCCCAACCTGC
CATCACGATGGCCGCAATAAAATATCTTTATTTTCATTACATCTGTGTGTTGGTTTTTTG
TGTGAATCGATAGCGATAAGGATCCGCGTATGGTGCACTCTCAGTACAATCTGCTCTGAT
GCCGCATAGTTAAGCCAGCCCCGACACCCGCCAACACCCGCTGACGCGCCCTGACGGGCT
TGTCTGCTCCCGGCATCCGCTTACAGACAAGCTGTGACCGTCTCCGGGAGCTGCATGTGT
CAGAGGTTTTCACCGTCATCACCGAAACGCGCGAGACGAAAGGGCCTCGTGATACGCCTA
TTTTTATAGGTTAATGTCATGATAATAATGGTTTCTTAGACGTCAGGTGGCACTTTTCGG
GGAAATGTGCGCGGAACCCCTATTTGTTTATTTTTCTAAATACATTCAAATATGTATCCG
CTCATGAGACAATAACCCTGATAAATGCTTCAATAATATTGAAAAAGGAAGAGTATGAGT
ATTCAACATTTCCGTGTCGCCCTTATTCCCTTTTTTGCGGCATTTTGCCTTCCTGTTTTT
 
GCTCACCCAGAAACGCTGGTGAAAGTAAAAGATGCTGAAGATCAGTTGGGTGCACGAGTG
GGTTACATCGAACTGGATCTCAACAGCGGTAAGATCCTTGAGAGTTTTCGCCCCGAAGAA
CGTTTTCCAATGATGAGCACTTTTAAAGTTCTGCTATGTGGCGCGGTATTATCCCGTATT
GACGCCGGGCAAGAGCAACTCGGTCGCCGCATACACTATTCTCAGAATGACTTGGTTGAG
TACTCACCAGTCACAGAAAAGCATCTTACGGATGGCATGACAGTAAGAGAATTATGCAGT
GCTGCCATAACCATGAGTGATAACACTGCGGCCAACTTACTTCTGACAACGATCGGAGGA
CCGAAGGAGCTAACCGCTTTTTTGCACAACATGGGGGATCATGTAACTCGCCTTGATCGT
TGGGAACCGGAGCTGAATGAAGCCATACCAAACGACGAGCGTGACACCACGATGCCTGTA
GCAATGGCAACAACGTTGCGCAAACTATTAACTGGCGAACTACTTACTCTAGCTTCCCGG
CAACAATTAATAGACTGGATGGAGGCGGATAAAGTTGCAGGACCACTTCTGCGCTCGGCC
CTTCCGGCTGGCTGGTTTATTGCTGATAAATCTGGAGCCGGTGAGCGTGGGTCTCGCGGT
ATCATTGCAGCACTGGGGCCAGATGGTAAGCCCTCCCGTATCGTAGTTATCTACACGACG
GGGAGTCAGGCAACTATGGATGAACGAAATAGACAGATCGCTGAGATAGGTGCCTCACTG
ATTAAGCATTGGTAACTGTCAGACCAAGTTTACTCATATATACTTTAGATTGATTTAAAA
CTTCATTTTTAATTTAAAAGGATCTAGGTGAAGATCCTTTTTGATAATCTCATGACCAAA
ATCCCTTAACGTGAGTTTTCGTTCCACTGAGCGTCAGACCCCGTAGAAAAGATCAAAGGA
TCTTCTTGAGATCCTTTTTTTCTGCGCGTAATCTGCTGCTTGCAAACAAAAAAACCACCG
CTACCAGCGGTGGTTTGTTTGCCGGATCAAGAGCTACCAACTCTTTTTCCGAAGGTAACT
GGCTTCAGCAGAGCGCAGATACCAAATACTGTCCTTCTAGTGTAGCCGTAGTTAGGCCAC
CACTTCAAGAACTCTGTAGCACCGCCTACATACCTCGCTCTGCTAATCCTGTTACCAGTG
GCTGCTGCCAGTGGCGATAAGTCGTGTCTTACCGGGTTGGACTCAAGACGATAGTTACCG
GATAAGGCGCAGCGGTCGGGCTGAACGGGGGGTTCGTGCACACAGCCCAGCTTGGAGCGA
ACGACCTACACCGAACTGAGATACCTACAGCGTGAGCTATGAGAAAGCGCCACGCTTCCC
GAAGGGAGAAAGGCGGACAGGTATCCGGTAAGCGGCAGGGTCGGAACAGGAGAGCGCACG
AGGGAGCTTCCAGGGGGAAACGCCTGGTATCTTTATAGTCCTGTCGGGTTTCGCCACCTC
TGACTTGAGCGTCGATTTTTGTGATGCTCGTCAGGGGGGCGGAGCCTATGGAAAAACGCC
AGCAACGCGGCCTTTTTACGGTTCCTGGCCTTTTGCTGGCCTTTTGCTCACATGGCTCGA
C
SEQ ID No. 13- pCIneoERev 
TGAATAATAAAATGTGTGTTTGTCCGAAATACGCGTTTTGAGATTTCTGTCGCCGACTAA
ATTCATGTCGCGCGATAGTGGTGTTTATCGCCGATAGAGATGGCGATATTGGAAAAATTG
ATATTTGAAAATATGGCATATTGAAAATGTCGCCGATGTGAGTTTCTGTGTAACTGATAT
CGCCATTTTTCCAAAAGTGATTTTTGGGCATACGCGATATCTGGCGATAGCGCTTATATC
GTTTACGGGGGATGGCGATAGACGACTTTGGTGACTTGGGCGATTCTGTGTGTCGCAAAT
ATCGCAGTTTCGATATAGGTGACAGACGATATGAGGCTATATCGCCGATAGAGGCGACAT
CAAGCTGGCACATGGCCAATGCATATCGATCTATACATTGAATCAATATTGGCCATTAGC
CATATTATTCATTGGTTATATAGCATAAATCAATATTGGCTATTGGCCATTGCATACGTT
GTATCCATATCGTAATATGTACATTTATATTGGCTCATGTCCAACATTACCGCCATGTTG
ACATTGATTATTGACTAGTTATTAATAGTAATCAATTACGGGGTCATTAGTTCATAGCCC
ATATATGGAGTTCCGCGTTACATAACTTACGGTAAATGGCCCGCCTGGCTGACCGCCCAA
CGACCCCCGCCCATTGACGTCAATAATGACGTATGTTCCCATAGTAACGCCAATAGGGAC
TTTCCATTGACGTCAATGGGTGGAGTATTTACGGTAAACTGCCCACTTGGCAGTACATCA
AGTGTATCATATGCCAAGTCCGCCCCCTATTGACGTCAATGACGGTAAATGGCCCGCCTG
GCATTATGCCCAGTACATGACCTTACGGGACTTTCCTACTTGGCAGTACATCTACGTATT
AGTCATCGCTATTACCATGGTGATGCGGTTTTGGCAGTACACCAATGGGCGTGGATAGCG
GTTTGACTCACGGGGATTTCCAAGTCTCCACCCCATTGACGTCAATGGGAGTTTGTTTTG
GCACCAAAATCAACGGGACTTTCCAAAATGTCGTAACAACTGCGATCGCCCGCCCCGTTG
ACGCAAATGGGCGGTAGGCGTGTACGGTGGGAGGTCTATATAAGCAGAGCTCGTTTAGTG
AACCGTCAGATCACTAGAAGCTTTATTGCGGTAGTTTATCACAGTTAAATTGCTAACGCA
GTCAGTGCTTCTGACACAACAGTCTCGAACTTAAGCTGCAGTGACTCTCTTAAGGTAGCC
TTGCAGAAGTTGGTCGTGAGGCACTGGGCAGGTAAGTATCAAGGTTACAAGACAGGTTTA
AGGAGACCAATAGAAACTGGGCTTGTCGAGACAGAGAAGACTCTTGCGTTTCTGATAGGC
ACCTATTGGTCTTACTGACATCCACTTTGCCTTTCTCTCCACAGGTGTCCACTCCCAGTT
CAATTACAGCTCTTAAGGCTAGAGTACTTAATACGACTCACTATAGGCTAGTAACGGCCG
CCAGTGTGCTGGAATTCGGCTTATGGCAGAATCGAAGGAAGCAAGAGACCAAGAAATGAA
CCTGAAAGAAGAATCTAAAGAAGAAAAAAGAAGAAATGACTGGTGGAAAATAGATCCTCA
GGGCCCTCTGGAAGGTGACCAGTGGTGCAGGGTCCTCCGGCAGTCGTTACCTGAAGAAAA
AATTCCATCACAAACATGCATCGCGAGAAGACACCTGGGACCAGGCCCAACACAACATAC
ACCTAGCAGGCGTGACCGGTGGATCAGGGGACAAATACTACAAGCAGAAGTACTCCAGGA
ACGACTGGAATGGAGAATCAGAGGAGTACAACAGGCGGCCAAAGAGCTGGGTGAAGTCAA
TCGAGGCATTTGGAGAGAGCTATATTTCCGAGAAGACCAAAGGGGAGATTTCTCAGCCTG
GGGCGGCTATCAACGAGCACAAGAACGGCTCTGGGGGGAACAATCCTCACCAAGGGTCCT
TAGACCTGGAGATTCGAAGCGAAGGAGGAAACATTTATGAAGCCGAATTCTGCAGATATC
CATCACACTGGCGGCCGCTTCCCTTTAGTGAGGGTTAATGCTTCGAGCAGACATGATAAG
ATACATTGATGAGTTTGGACAAACCACAACTAGAATGCAGTGAAAAAAATGCTTTATTTG
TGAAATTTGTGATGCTATTGCTTTATTTGTAACCATTATAAGCTGCAATAAACAAGTTAA
CAACAACAATTGCATTCATTTTATGTTTCAGGTTCAGGGGGAGATGTGGGAGGTTTTTTA
AAGCAAGTAAAACCTCTACAAATGTGGTAAAATCCGATAAGGATCGATCCGGGCTGGCGT
AATAGCGAAGAGGCCCGCACCGATCGCCCTTCCCAACAGTTGCGCAGCCTGAATGGCGAA
TGGACGCGCCCTGTAGCGGCGCATTAAGCGCGGCGGGTGTGGTGGTTACGCGCAGCGTGA
CCGCTACACTTGCCAGCGCCCTAGCGCCCGCTCCTTTCGCTTTCTTCCCTTCCTTTCTCG
CCACGTTCGCCGGCTTTCCCCGTCAAGCTCTAAATCGGGGGCTCCCTTTAGGGTTCCGAT
TTAGAGCTTTACGGCACCTCGACCGCAAAAAACTTGATTTGGGTGATGGTTCACGTAGTG
GGCCATCGCCCTGATAGACGGTTTTTCGCCCTTTGACGTTGGAGTCCACGTTCTTTAATA
GTGGACTCTTGTTCCAAACTGGAACAACACTCAACCCTATCTCGGTCTATTCTTTTGATT
TATAAGGGATTTTGCCGATTTCGGCCTATTGGTTAAAAAATGAGCTGATTTAACAAATAT
TTAACGCGAATTTTAACAAAATATTAACGTTTACAATTTCGCCTGATGCGGTATTTTCTC
CTTACGCATCTGTGCGGTATTTCACACCGCATACGCGGATCTGCGCAGCACCATGGCCTG
AAATAACCTCTGAAAGAGGAACTTGGTTAGGTACCTTCTGAGGCGGAAAGAACCAGCTGT
GGAATGTGTGTCAGTTAGGGTGTGGAAAGTCCCCAGGCTCCCCAGCAGGCAGAAGTATGC
AAAGCATGCATCTCAATTAGTCAGCAACCAGGTGTGGAAAGTCCCCAGGCTCCCCAGCAG
GCAGAAGTATGCAAAGCATGCATCTCAATTAGTCAGCAACCATAGTCCCGCCCCTAACTC
CGCCCATCCCGCCCCTAACTCCGCCCAGTTCCGCCCATTCTCCGCCCCATGGCTGACTAA
TTTTTTTTATTTATGCAGAGGCCGAGGCCGCCTCGGCCTCTGAGCTATTCCAGAAGTAGT
GAGGAGGCTTTTTTGGAGGCCTAGGCTTTTGCAAAAAGCTTGATTCTTCTGACACAACAG
TCTCGAACTTAAGGCTAGAGCCACCATGATTGAACAAGATGGATTGCACGCAGGTTCTCC
GGCCGCTTGGGTGGAGAGGCTATTCGGCTATGACTGGGCACAACAGACAATCGGCTGCTC
TGATGCCGCCGTGTTCCGGCTGTCAGCGCAGGGGCGCCCGGTTCTTTTTGTCAAGACCGA
 
CCTGTCCGGTGCCCTGAATGAACTGCAGGACGAGGCAGCGCGGCTATCGTGGCTGGCCAC
GACGGGCGTTCCTTGCGCAGCTGTGCTCGACGTTGTCACTGAAGCGGGAAGGGACTGGCT
GCTATTGGGCGAAGTGCCGGGGCAGGATCTCCTGTCATCTCACCTTGCTCCTGCCGAGAA
AGTATCCATCATGGCTGATGCAATGCGGCGGCTGCATACGCTTGATCCGGCTACCTGCCC
ATTCGACCACCAAGCGAAACATCGCATCGAGCGAGCACGTACTCGGATGGAAGCCGGTCT
TGTCGATCAGGATGATCTGGACGAAGAGCATCAGGGGCTCGCGCCAGCCGAACTGTTCGC
CAGGCTCAAGGCGCGCATGCCCGAGGGCGAGGATCTCGTCGTGACCCATGGCGATGCCTG
CTTGCCGAATATCATGGTGGAAAATGGCCGCTTTTCTGGATTCATCGACTGTGGCCGGCT
GGGTGTGGCGGACCGCTATCAGGACATAGCGTTGGCTACCCGTGATATTGCTGAAGAGCT
TGGCGGCGAATGGGCTGACCGCTTCCTCGTGCTTTACGGTATCGCCGCTCCCGATTCGCA
GCGCATCGCCTTCTATCGCCTTCTTGACGAGTTCTTCTGAGCGGGACTCTGGGGTTCGAA
ATGACCGACCAAGCGACGCCCAACCTGCCATCACGATGGCCGCAATAAAATATCTTTATT
TTCATTACATCTGTGTGTTGGTTTTTTGTGTGAATCGATAGCGATAAGGATCCGCGTATG
GTGCACTCTCAGTACAATCTGCTCTGATGCCGCATAGTTAAGCCAGCCCCGACACCCGCC
AACACCCGCTGACGCGCCCTGACGGGCTTGTCTGCTCCCGGCATCCGCTTACAGACAAGC
TGTGACCGTCTCCGGGAGCTGCATGTGTCAGAGGTTTTCACCGTCATCACCGAAACGCGC
GAGACGAAAGGGCCTCGTGATACGCCTATTTTTATAGGTTAATGTCATGATAATAATGGT
TTCTTAGACGTCAGGTGGCACTTTTCGGGGAAATGTGCGCGGAACCCCTATTTGTTTATT
TTTCTAAATACATTCAAATATGTATCCGCTCATGAGACAATAACCCTGATAAATGCTTCA
ATAATATTGAAAAAGGAAGAGTATGAGTATTCAACATTTCCGTGTCGCCCTTATTCCCTT
TTTTGCGGCATTTTGCCTTCCTGTTTTTGCTCACCCAGAAACGCTGGTGAAAGTAAAAGA
TGCTGAAGATCAGTTGGGTGCACGAGTGGGTTACATCGAACTGGATCTCAACAGCGGTAA
GATCCTTGAGAGTTTTCGCCCCGAAGAACGTTTTCCAATGATGAGCACTTTTAAAGTTCT
GCTATGTGGCGCGGTATTATCCCGTATTGACGCCGGGCAAGAGCAACTCGGTCGCCGCAT
ACACTATTCTCAGAATGACTTGGTTGAGTACTCACCAGTCACAGAAAAGCATCTTACGGA
TGGCATGACAGTAAGAGAATTATGCAGTGCTGCCATAACCATGAGTGATAACACTGCGGC
CAACTTACTTCTGACAACGATCGGAGGACCGAAGGAGCTAACCGCTTTTTTGCACAACAT
GGGGGATCATGTAACTCGCCTTGATCGTTGGGAACCGGAGCTGAATGAAGCCATACCAAA
CGACGAGCGTGACACCACGATGCCTGTAGCAATGGCAACAACGTTGCGCAAACTATTAAC
TGGCGAACTACTTACTCTAGCTTCCCGGCAACAATTAATAGACTGGATGGAGGCGGATAA
AGTTGCAGGACCACTTCTGCGCTCGGCCCTTCCGGCTGGCTGGTTTATTGCTGATAAATC
TGGAGCCGGTGAGCGTGGGTCTCGCGGTATCATTGCAGCACTGGGGCCAGATGGTAAGCC
CTCCCGTATCGTAGTTATCTACACGACGGGGAGTCAGGCAACTATGGATGAACGAAATAG
ACAGATCGCTGAGATAGGTGCCTCACTGATTAAGCATTGGTAACTGTCAGACCAAGTTTA
CTCATATATACTTTAGATTGATTTAAAACTTCATTTTTAATTTAAAAGGATCTAGGTGAA
GATCCTTTTTGATAATCTCATGACCAAAATCCCTTAACGTGAGTTTTCGTTCCACTGAGC
GTCAGACCCCGTAGAAAAGATCAAAGGATCTTCTTGAGATCCTTTTTTTCTGCGCGTAAT
CTGCTGCTTGCAAACAAAAAAACCACCGCTACCAGCGGTGGTTTGTTTGCCGGATCAAGA
GCTACCAACTCTTTTTCCGAAGGTAACTGGCTTCAGCAGAGCGCAGATACCAAATACTGT
CCTTCTAGTGTAGCCGTAGTTAGGCCACCACTTCAAGAACTCTGTAGCACCGCCTACATA
CCTCGCTCTGCTAATCCTGTTACCAGTGGCTGCTGCCAGTGGCGATAAGTCGTGTCTTAC
CGGGTTGGACTCAAGACGATAGTTACCGGATAAGGCGCAGCGGTCGGGCTGAACGGGGGG
TTCGTGCACACAGCCCAGCTTGGAGCGAACGACCTACACCGAACTGAGATACCTACAGCG
TGAGCTATGAGAAAGCGCCACGCTTCCCGAAGGGAGAAAGGCGGACAGGTATCCGGTAAG
CGGCAGGGTCGGAACAGGAGAGCGCACGAGGGAGCTTCCAGGGGGAAACGCCTGGTATCT
TTATAGTCCTGTCGGGTTTCGCCACCTCTGACTTGAGCGTCGATTTTTGTGATGCTCGTC
AGGGGGGCGGAGCCTATGGAAAAACGCCAGCAACGCGGCCTTTTTACGGTTCCTGGCCTT
TTGCTGGCCTTTTGCTCACATGGCTCGACAGATCT
SEQ ID No. 14- pESYNREV 
TCAATATTGGCCATTAGCCATATTATTCATTGGTTATATAGCATAAATCAATATTGGCTA
TTGGCCATTGCATACGTTGTATCTATATCATAATATGTACATTTATATTGGCTCATGTCC
AATATGACCGCCATGTTGGCATTGATTATTGACTAGTTATTAATAGTAATCAATTACGGG
GTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCAT
AGTAACGCCAATAGGGACTTTCCATTGACGTCAATGGGTGGAGTATTTACGGTAAACTGC
CCACTTGGCAGTACATCAAGTGTATCATATGCCAAGTCCGCCCCCTATTGACGTCAATGA
CGGTAAATGGCCCGCCTGGCATTATGCCCAGTACATGACCTTACGGGACTTTCCTACTTG
GCAGTACATCTACGTATTAGTCATCGCTATTACCATGGTGATGCGGTTTTGGCAGTACAC
CAATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTG
CGATCGCCCGCCCCGTTGACGCAAATGGGCGGTAGGCGTGTACGGTGGGAGGTCTATATA
AGCAGAGCTCGTTTAGTGAACCGTCAGATCACTAGAAGCTTTATTGCGGTAGTTTATCAC
AGTTAAATTGCTAACGCAGTCAGTGCTTCTGACACAACAGTCTCGAACTTAAGCTGCAGT
GACTCTCTTAAGGTAGCCTTGCAGAAGTTGGTCGTGAGGCACTGGGCAGGTAAGTATCAA
GGTTACAAGACAGGTTTAAGGAGACCAATAGAAACTGGGCTTGTCGAGACAGAGAAGACT
CTTGCGTTTCTGATAGGCACCTATTGGTCTTACTGACATCCACTTTGCCTTTCTCTCCAC
AGGTGTCCACTCCCAGTTCAATTACAGCTCTTAAGGCTAGAGTACTTAATACGACTCACT
ATAGGCTAGCCTCGAGAATTCGCCACCATGGCTGAGAGCAAGGAGGCCAGGGATCAAGAG
ATGAACCTCAAGGAAGAGAGCAAAGAGGAGAAGCGCCGCAACGACTGGTGGAAGATCGAC
CCACAAGGCCCCCTGGAGGGGGACCAGTGGTGCCGCGTGCTGAGACAGTCCCTGCCCGAG
GAGAAGATTCCTAGCCAGACCTGCATCGCCAGAAGACACCTCGGCCCCGGTCCCACCCAG
CACACACCCTCCAGAAGGGATAGGTGGATTAGGGGCCAGATTTTGCAAGCCGAGGTCCTC
CAAGAAAGGCTGGAATGGAGAATTAGGGGCGTGCAACAAGCCGCTAAAGAGCTGGGAGAG
GTGAATCGCGGCATCTGGAGGGAGCTCTACTTCCGCGAGGACCAGAGGGGCGATTTCTCC
GCATGGGGAGGCTACCAGAGGGCACAAGAAAGGCTGTGGGGCGAGCAGAGCAGCCCCCGC
GTCTTGAGGCCCGGAGACTCCAAAAGACGCCGCAAACACCTGTGAAGTCGACCCGGGCGG
CCGCTTCCCTTTAGTGAGGGTTAATGCTTCGAGCAGACATGATAAGATACATTGATGAGT
TTGGACAAACCACAACTAGAATGCAGTGAAAAAAATGCTTTATTTGTGAAATTTGTGATG
CTATTGCTTTATTTGTAACCATTATAAGCTGCAATAAACAAGTTAACAACAACAATTGCA
TTCATTTTATGTTTCAGGTTCAGGGGGAGATGTGGGAGGTTTTTTAAAGCAAGTAAAACC
TCTACAAATGTGGTAAAATCCGATAAGGATCGATCCGGGCTGGCGTAATAGCGAAGAGGC
CCGCACCGATCGCCCTTCCCAACAGTTGCGCAGCCTGAATGGCGAATGGACGCGCCCTGT
AGCGGCGCATTAAGCGCGGCGGGTGTGGTGGTTACGCGCAGCGTGACCGCTACACTTGCC
AGCGCCCTAGCGCCCGCTCCTTTCGCTTTCTTCCCTTCCTTTCTCGCCACGTTCGCCGGC
TTTCCCCGTCAAGCTCTAAATCGGGGGCTCCCTTTAGGGTTCCGATTTAGAGCTTTACGG
CACCTCGACCGCAAAAAACTTGATTTGGGTGATGGTTCACGTAGTGGGCCATCGCCCTGA
TAGACGGTTTTTCGCCCTTTGACGTTGGAGTCCACGTTCTTTAATAGTGGACTCTTGTTC
CAAACTGGAACAACACTCAACCCTATCTCGGTCTATTCTTTTGATTTATAAGGGATTTTG
CCGATTTCGGCCTATTGGTTAAAAAATGAGCTGATTTAACAAATATTTAACGCGAATTTT
AACAAAATATTAACGTTTACAATTTCGCCTGATGCGGTATTTTCTCCTTACGCATCTGTG
CGGTATTTCACACCGCATACGCGGATCTGCGCAGCACCATGGCCTGAAATAACCTCTGAA
AGAGGAACTTGGTTAGGTACCTTCTGAGGCGGAAAGAACCAGCTGTGGAATGTGTGTCAG
TTAGGGTGTGGAAAGTCCCCAGGCTCCCCAGCAGGCAGAAGTATGCAAAGCATGCATCTC
AATTAGTCAGCAACCAGGTGTGGAAAGTCCCCAGGCTCCCCAGCAGGCAGAAGTATGCAA
AGCATGCATCTCAATTAGTCAGCAACCATAGTCCCGCCCCTAACTCCGCCCATCCCGCCC
CTAACTCCGCCCAGTTCCGCCCATTCTCCGCCCCATGGCTGACTAATTTTTTTTATTTAT
GCAGAGGCCGAGGCCGCCTCGGCCTCTGAGCTATTCCAGAAGTAGTGAGGAGGCTTTTTT
GGAGGCCTAGGCTTTTGCAAAAAGCTTGATTCTTCTGACACAACAGTCTCGAACTTAAGG
CTAGAGCCACCATGATTGAACAAGATGGATTGCACGCAGGTTCTCCGGCCGCTTGGGTGG
AGAGGCTATTCGGCTATGACTGGGCACAACAGACAATCGGCTGCTCTGATGCCGCCGTGT
TCCGGCTGTCAGCGCAGGGGCGCCCGGTTCTTTTTGTCAAGACCGACCTGTCCGGTGCCC
TGAATGAACTGCAGGACGAGGCAGCGCGGCTATCGTGGCTGGCCACGACGGGCGTTCCTT
GCGCAGCTGTGCTCGACGTTGTCACTGAAGCGGGAAGGGACTGGCTGCTATTGGGCGAAG
TGCCGGGGCAGGATCTCCTGTCATCTCACCTTGCTCCTGCCGAGAAAGTATCCATCATGG
CTGATGCAATGCGGCGGCTGCATACGCTTGATCCGGCTACCTGCCCATTCGACCACCAAG
CGAAACATCGCATCGAGCGAGCACGTACTCGGATGGAAGCCGGTCTTGTCGATCAGGATG
ATCTGGACGAAGAGCATCAGGGGCTCGCGCCAGCCGAACTGTTCGCCAGGCTCAAGGCGC
GCATGCCCGACGGCGAGGATCTCGTCGTGACCCATGGCGATGCCTGCTTGCCGAATATCA
 
TGGTGGAAAATGGCCGCTTTTCTGGATTCATCGACTGTGGCCGGCTGGGTGTGGCGGACC
GCTATCAGGACATAGCGTTGGCTACCCGTGATATTGCTGAAGAGCTTGGCGGCGAATGGG
CTGACCGCTTCCTCGTGCTTTACGGTATCGCCGCTCCCGATTCGCAGCGCATCGCCTTCT
ATCGCCTTCTTGACGAGTTCTTCTGAGCGGGACTCTGGGGTTCGAAATGACCGACCAAGC
GACGCCCAACCTGCCATCACGATGGCCGCAATAAAATATCTTTATTTTCATTACATCTGT
GTGTTGGTTTTTTGTGTGAATCGATAGCGATAAGGATCCGCGTATGGTGCACTCTCAGTA
CAATCTGCTCTGATGCCGCATAGTTAAGCCAGCCCCGACACCCGCCAACACCCGCTGACG
CGCCCTGACGGGCTTGTCTGCTCCCGGCATCCGCTTACAGACAAGCTGTGACCGTCTCCG
GGAGCTGCATGTGTCAGAGGTTTTCACCGTCATCACCGAAACGCGCGAGACGAAAGGGCC
TCGTGATACGCCTATTTTTATAGGTTAATGTCATGATAATAATGGTTTCTTAGACGTCAG
GTGGCACTTTTCGGGGAAATGTG[?][?]CGGAACCCCTATTTGTTTATTTTTCTAAATACATT
CAAATATGTATCCGCTCATGAGA[?][?]ATAACCCTGATAAATGCTTCAATAATATTGAAAAA
GGAAGAGTATGAGTATTCAACATTTCCGTGTCGCCCTTATTCCCTTTTTTGCGGCATTTT
GCCTTCCTGTTTTTGCTCACCCAGAAACGCTGGTGAAAGTAAAAGATGCTGAAGATCAGT
TGGGTGCACGAGTGGGTTACATCGAACTGGATCTCAACAGCGGTAAGATCCTTGAGAGTT
TTCGCCCCGAAGAACGTTTTCCAATGATGAGCACTTTTAAAGTTCTGCTATGTGGCGCGG
TATTATCCCGTATTGACGCCGGGCAAGAGCAACTCGGTCGCCGCATACACTATTCTCAGA
ATGACTTGGTTGAGTACTCACCAGTCACAGAAAAGCATCTTACGGATGGCATGACAGTAA
GAGAATTATGCAGTGCTGCCATAACCATGAGTGATAACACTGCGGCCAACTTACTTCTGA
CAACGATCGGAGGACCGAAGGAGCTAACCGCTTTTTTGCACAACATGGGGGATCATGTAA
CTCGCCTTGATCGTTGGGAACCGGAGCTGAATGAAGCCATACCAAACGACGAGCGTGACA
CCACGATGCCTGTAGCAATGGCAACAACGTTGCGCAAACTATTAACTGGCGAACTACTTA
CTCTAGCTTCCCGGCAACAATTAATAGACTGGATGGAGGCGGATAAAGTTGCAGGACCAC
TTCTGCGCTCGGCCCTTCCGGCTGGCTGGTTTATTGCTGATAAATCTGGAGCCGGTGAGC
GTGGGTCTCGCGGTATCATTGCAGCACTGGGGCCAGATGGTAAGCCCTCCCGTATCGTAG
TTATCTACACGACGGGGAGTCAGGCAACTATGGATGAACGAAATAGACAGATCGCTGAGA
TAGGTGCCTCACTGATTAAGCATTGGTAACTGTCAGACCAAGTTTACTCATATATACTTT
AGATTGATTTAAAACTTCATTTTTAATTTAAAAGGATCTAGGTGAAGATCCTTTTTGATA
ATCTCATGACCAAAATCCCTTAACGTGAGTTTTCGTTCCACTGAGCGTCAGACCCCGTAG
AAAAGATCAAAGGATCTTCTTGAGATCCTTTTTTTCTGCGCGTAATCTGCTGCTTGCAAA
CAAAAAAACCACCGCTACCAGCGGTGGTTTGTTTGCCGGATCAAGAGCTACCAACTCTTT
TTCCGAAGGTAACTGGCTTCAGCAGAGCGCAGATACCAAATACTGTCCTTCTAGTGTAGC
CGTAGTTAGGCCACCACTTCAAGAACTCTGTAGCACCGCCTACATACCTCGCTCTGCTAA
TCCTGTTACCAGTGGCTGCTGCCAGTGGCGATAAGTCGTGTCTTACCGGGTTGGACTCAA
GACGATAGTTACCGGATAAGGCGCAGCGGTCGGGCTGAACGGGGGGTTCGTGCACACAGC
CCAGCTTGGAGCGAACGACCTACACCGAACTGAGATACCTACAGCGTGAGCTATGAGAAA
GCGCCACGCTTCCCGAAGGGAGAAAGGCGGACAGGTATCCGGTAAGCGGCAGGGTCGGAA
CAGGAGAGCGCACGAGGGAGCTTCCAGGGGGAAACGCCTGGTATCTTTATAGTCCTGTCG
GGTTTCGCCACCTCTGACTTGAGCGTCGATTTTTGTGATGCTCGTCAGGGGGGCGGAGCC
TATGGAAAAACGCCAGCAACGCGGCCTTTTTACGGTTCCTGGCCTTTTGCTGGCCTTTTG
CTCACATGGCTCGACAGATCT
Seq ID No: 15 codon optimised HIV *gag-pol* 
ATGGGCGCCCGCGCCAGCGTGCTGTCGGGCGGCGAGCTGGACCGCTGGGAGAAGATCCGC
CTGCGCCCCGGCGGCAAAAAGAAGTACAAGCTGAAGCACATCGTGTGGGCCAGCCGCGAA
CTGGAGCGCTTCGCCGTGAACCCCGGCTCCTGGAGACCAGCGAGGGGTGCCGCCAGATC
CTCGGCCAACTGCAGCCCAGCCTGCAAACCGGCAGCGAGGAGCTGCGCAGCCTGTACAAC
ACCGTGGCCACGCTGTACTGCGTCCACCAGCGCATCGAAATCAAGGATACGAAAGAGGCC
CTGGATAAAATCGAAGAGGAACAGAATAAGAGCAAAAAGAAGGCCCAACAGGCCGCCGCG
GACACCGGACACAGCAACCAGGTCAGCCAGAACTACCCCATCGTGCAGAACATCCAGGGG
CAGATGGTGCACCAGGCCATCTCCCCCCGCACGCTGAACGCCTGGGTGAAGGTGGTGGAA
GAGAAGGCTTTTAGCCCGGAGGTGATACCCATGTTCTCAGCCCTGTCAGAGGGAGCCACC
CCCCAAGATCTGAACACCATGCTCAACACAGTGGGGGGACACCAGGCCGCCATGCAGATG
CTGAAGGAGACCATCAATGAGGAGGCTGCCGAATGGGATCGTGTGCATCCGGTGCACGCA
GGGCCCATCGCACCGGGCCAGATGCGTGAGCCACGGGGCTCAGACATCGCCGGAACGACT
AGTACCCTTCAGGAACAGATCGGCTGGATGACCAACAACCCACCCATCCCGGTGGGAGAA
ATCTACAAACGCTGGATCATCCTGGGCCTGAACAAGATCGTGCGCATGTATAGCCCTACC
AGCATCCTGGACATCCGCCAAGGCCCGAAGGAACCCTTTCGCGACTACGTGGACCGGTTC
TACAAAACGCTCCGCGCCGAGCAGGCTAGCCAGGAGGTGAAGAACTGGATGACCGAAACC
CTGCTGGTCCAGAACGCGAACCCGGACTGCAAGACGATCCTGAAGGCCCTGGGCCCAGCG
GCTACCCTAGAGGAAATGATGACCGCCTGTCAGGGAGTGGGCGGACCCGGCCACAAGGCA
CGCGTCCTGGCTGAGGCCATGAGCCAGGTGACCAACTCCGCTACCATCATGATGCAGCGC
GGCAACTTTCGGAACCAACGCAAGATCGTCAAGTGCTTCAACTGTGGCAAAGAAGGGCAC
ACAGCCCGCAACTGCAGGGCCCCTAGGAAAAAGGGCTGTTGGAAATGTGGAAAGGAAGGA
CACCAAATGAAAGATTGTACTGAGAGACAGGCTAATTTTTTAGGGAAGATCTGGCCTTCC
CACAAGGGAAGGCCAGGGAATTTTCTTCAGAGCAGACCAGAGCCAACAGCCCCACCAGAA
GAGAGCTTCAGGTTTGGGGAAGAGACAACAACTCCCTCTCAGAAGCAGGAGCCGATAGAC
AAGGAACTGTATCCTTTAGCTTCCCTCAGATCACTCTTTGGCAGCGACCCCTCGTCACAA
TAAAGATAGGGGGGCAGCTCAAGGAGGCTCTCCTGGACACCGGAGCAGACGACACCGTGC
TGGAGGAGATGTCGTTGCCAGGCCGCTGGAAGCCGAAGATGATCGGGGGAATCGGCGGTT
TCATCAAGGTGCGCCAGTATGACCAGATCCTCATCGAAATCTGCGGCCACAAGGCTATCG
GTACCGTGCTGGTGGGCCCCACACCCGTCAACATCATCGGACGCAACCTGTTGACGCAGA
TCGGTTGCACGCTGAACTTCCCCATTAGCCCTATCGAGACGGTACCGGTGAAGCTGAAGC
CCGGGATGGACGGCCCGAAGGTCAAGCAATGGCCATTGACAGAGGAGAAGATCAAGGCAC
TGGTGGAGATTTGCACAGAGATGGAAAAGGAAGGAAAAATCTCCAAGATTGGCCTGAGA
ACCCGTACAACACGCCGGTGTTCGCAATCAAGAAGAAGGACTCGACGAAATGGCGCAAGC
TGGTGGACTTCCGCGAGCTGAACAAGCGCACGCAAGACTTCTGGGAGGTTCAGCTGGGCA
TCCCGCACCCCGCAGGGCTGAAGAAGAAGAAATCCGTGACCGTACTGGATGTGGGTGATG
CCTACTTCTCCGTTCCCCTGGACGAAGACTTCAGGAAGTACACTGCCTTCACAATCCCTT
CGATCAACAACGAGACACCGGGGATTCGATATCAGTACAACGTGCTGCCCCAGGGCTGGA
AAGGCTCTCCCGCAATCTTCCAGAGTAGCATGACCAAAATCCTGGAGCCTTTCCGCAAAC
AGAACCCCGACATCGTCATCTATCAGTACATGGATGACTTGTACGTGGGCTCTGATCTAG
AGATAGGGCAGCACCGCACCAAGATCGAGGAGCTGCGCCAGCACCTGTTGAGGTGGGGAC
TGACCACACCCGACAAGAAGCACCAGAAGGAGCCTCCCTTCCTCTGGATGGGTTACGAGC
TGCACCCTGACAAATGGACCGTGCAGCCTATCGTGCTGCCAGAGAAAGACAGCTGGACTG
TCAACGACATACAGAAGCTGGTGGGGAAGTTGAACTGGGCCAGTCAGATTTACCCAGGGA
TTAAGGTGAGGCAGCTGTGCAAACTCCTCCGCGGAACCAAGGCACTCACAGAGGTGATCC
CCCTAACCGAGGAGGCCGAGCTCGAACTGGCAGAAAACCGAGAGATCCTAAAGGAGCCCG
TGCACGGCGTGTACTATGACCCCTCCAAGGACCTGATCGCCGAGATCCAGAAGCAGGGGC
AAGGCCAGTGGACCTATCAGATTTACCAGGAGCCCTTCAAGAACCTGAAGACCGGCAAGT
ACGCCCGGATGAGGGGTGCCCACACTAACGACGTCAAGCAGCTGACCGAGGCCGTGCAGA
AGATCACCACCGAAAGCATCGTGATCTGGGGAAAGACTCCTAAGTTCAAGCTGCCCATCC
AGAAGGAAACCTGGGAAACCTGGTGGACAGAGTATTGGCAGGCCACCTGGATTCCTGAGT
GGGAGTTCGTCAACACCCCTCCCCTGGTGAAGCTGTGGTACCAGCTGGAGAAGGAGCCCA
TAGTGGGCGCCGAAACCTTCTACGTGGATGGGGCCGCTAACAGGGAGACTAAGCTGGGCA
AAGCCGGATACGTCACTAACCGGGGCAGACAGAAGGTTGTCACCCTCACTGACACCACCA
ACCAGAAGACTGAGCTGCAGGCCATTTACCTCGCTTTGCAGGACTCGGGCCTGGAGGTGA
ACATCGTGACAGACTCTCAGTATGCCCTGGGCATCATTCAAGCCCAGCCAGACCAGAGTG
AGTCCGAGCTGGTCAATCAGATCATCGAGCAGCTGATCAAGAAGGAAAAGGTCTATCTGG
CCTGGGTACCCGCCCACAAAGGCATTGGCGGCAATGAGCAGGTCGACAAGCTGGTCTCGG
CTGGCATCAGGAAGGTGCTATTCCTGGATGGCATCGACAAGGCCCAGGACGAGCACGAGA
AATACCACAGCAACTGGCGGGCCATGGCTAGCGACTTCAACCTGCCCCCTGTGGTGGCCA
AAGAGATCGTGGCCAGCTGTGACAAGTGTCAGCTCAAGGGCGAAGCCATGCATGGCCAGG
TGGACTGTAGCCCCGGCATCTGGCAACTCGATTGCACCCATCTGGAGGGCAAGGTTATCC
TGGTAGCCGTCCATGTGGCCAGTGGCTACATCGAGGCCGAGGTCATTCCCGCCGAAACAG
GGCAGGAGACAGCCTACTTCCTCCTGAAGCTGGCAGGCCGGTGGCCAGTGAAGACCATCC
ATACTGACAATGGCAGCAATTTCACCAGTGCTACGGTTAAGGCCGCCTGCTGGTGGGCGG
GAATCAAGCAGGAGTTCGGGATCCCCTACAATCCCCAGAGTCAGGGCGTCGTCGAGTCTA
TGAATAAGGAGTTAAAGAAGATTATCGGCCAGGTCAGAGATCAGGCTGAGCATCTCAAGA
CCGCGGTCCAAATGGCGGTATTCATCCACAATTTCAAGCGGAAGGGGGGGATTGGGGGGT
ACAGTGCGGGGGAGCGGATCGTGGACATCATCGCGACCGACATCCAGACTAAGGAGCTGC
AAAAGCAGATTACCAAGATTCAGAATTTCCGGGTCTACTACAGGGACAGCAGAAATCCCC
TCTGGAAAGGCCCAGCGAAGCTCCTCTGGAAGGGTGAGGGGGCAGTAGTGATCCAGGATA
ATAGCGACATCAAGGTGGTGCCCAGAAGAAAGGCGAAGATCATTAGGGATTATGGCAAAC
AGATGGCGGGTGATGATTGCGTGGCGAGCAGACAGGATGAGGATTAG
Seq ID No: 16 codon optimised EIAV *gag-pol* 
ATGGGCGATCCCCTCACCTGGTCCAAAGCCCTGAAGAAACTGGAAAAAGTCACCGTTCAG
GGTAGCCAAAAGCTTACCACAGGCAATTGCAACTGGGCATTGTCCCTGGTGGATCTTTTC
CACGACACTAATTTCGTTAAGGAGAAAGATTGGCAACTCAGAGACGTGATCCCCCTCTTG
GAGGACGTGACCCAAACATTGTCTGGGCAGGAGCGCGAAGCTTTCGAGCGCACCTGGTGG
GCCATCAGCGCAGTCAAAATGGGGCTGCAAATCAACAACGTGGTTGACGGTAAAGCTAGC
TTTCAACTGCTCCGCGCTAAGTACGAGAAGAAAACCGCCAACAAGAAACAATCCGAACCT
AGCGAGGAGTACCCAATTATGATCGACGGCGCCGGCAATAGGAACTTCCGCCCACTGACT
CCCAGGGGCTATACCACCTGGGTCAACACCATCCAGACAAACGGACTTTTGAACGAAGCC
TCCCAGAACCTGTTCGGCATCCTGTCTGTGGACTGCACCTCCGAAGAAATGAATGCTTTT
CTCGACGTGGTGCCAGGACAGGCTGGACAGAAACAGATCCTGCTCGATGCCATTGACAAG
ATCGCCGACGACTGGGATAATCGCCACCCCCTGCCAAACGCCCCTCTGGTGGCTCCCCCA
CAGGGGCCTATCCCTATGACCGCTAGGTTCATTAGGGGACTGGGGGTGCCCCGCGAACGC
CAGATGGAGCCAGCATTTGACCAATTTAGGCAGACCTACAGACAGTGGATCATCGAAGCC
ATGAGCGAGGGGATTAAAGTCATGATCGGAAAGCCCAAGGCACAGAACATCAGGCAGGGG
GCCAAGGAACCATACCCTGAGTTTGTCGACAGGCTTCTGTCCCAGATTAAATCCGAAGGC
CACCCTCAGGAGATCTCCAAGTTCTTGACAGACACACTGACTATCCAAAATGCAAATGAA
GAGTGCAGAAACGCCATGAGGCACCTCAGACCTGAAGATACCCTGGAGGAGAAAATGTAC
GCATGTCGCGACATTGGCACTACCAAGCAAAAGATGATGCTGCTCGCCAAGGCTCTGCAA
ACCGGCCTGGCTGGTCCATTCAAAGGAGGAGCACTGAAGGGAGGTCCATTGAAAGCTGCA
CAAACATGTTATAATTGTGGGAAGCCAGGACATTTATCTAGTCAATGTAGAGCACCTAAA
GTCTGTTTTAAATGTAAACAGCCTGGACATTTCTCAAAGCAATGCAGAAGTGTTCCAAAA
AACGGGAAGCAAGGGGCTCAAGGGAGGCCCCAGAAACAAACTTTCCCGATACAACAGAAG
AGTCAGCACAACAAATCTGTTGTACAAGAGACTCCTCAGACTCAAAATCTGTACCCAGAT
CTGAGCGAAATAAAAAAGGAATACAATGTCAAGGAGAAGGATCAAGTAGAGGATCTCAAC
CTGGACAGTTTGTGGGAGTAACATACAATCTCGAGAAGAGGCCCACTACCATCGTCCTGA
TCAATGACACCCCTCTTAATGTGCTGCTGGACACCGGAGCCGACACCAGCGTTCTCACTA
CTGCTCACTATAACAGACTGAAATACAGAGGAAGGAAATACCAGGGCACAGGCATCATCG
GCGTTGGAGGCAACGTCGAAACCTTTTCCACTCCTGTCACCATCAAAAAGAAGGGGAGAC
ACATTAAAACCAGAATGCTGGTCGCCGACATCCCCGTCACCATCCTTGGCAGAGACATTC
TCCAGGACCTGGGCGCTAAACTCGTGCTGGCACAACTGTCTAAGGAAATCAAGTTCCGCA
AGATCGAGCTGAAAGAGGGCACAATGGGTCCAAAAATCCCCCAGTGGCCCCTGACCAAAG
AGAAGCTTGAGGGCGCTAAGGAAATCGTGCAGCGCCTGCTTTCTGAGGGCAAGATTAGCG
AGGCCAGCGACAATAACCCTTACAACAGCCCCATCTTTGTGATTAAGAAAAGGAGCGGCA
AATGGAGACTCCTGCAGGACCTGAGGGAACTCAACAAGACCGTCCAGGTCGGAACTGAGA
TCTCTCGCGGACTGCCTCACCCCGGCGGCCTGATTAAATGCAAGCACATGACAGTCCTTG
ACATTGGAGACGCTTATTTTACCATCCCCCTCGATCCTGAATTTCGCCCCTATACTGCTT
TTACCATCCCCAGCATCAATCACCAGGAGCCCGATAAACGCTATGTGTGGAAGTGCCTCC
CCCAGGGATTTGTGCTTAGCCCCTACATTTACCAGAAGACACTTCAAGAGATCCTCCAAC
CTTTCCGCGAAAGATACCCAGAGGTTCAACTCTACCAATATATGGACGACCTGTTCATGG
GGTCCAACGGGTCTAAGAAGCAGCACAAGGAACTCATCATCGAACTGAGGGCAATCCTCC
TGGAGAAAGGCTTCGAGACACCCGACGACAAGCTGCAAGAAGTTCCTCCATATAGCTGGC
TGGGCTACCAGCTTTGCCCTGAAAACTGGAAAGTCCAGAAGATGCAGTTGGATATGGTCA
AGAACCCAACACTGAACGACGTCCAGAAGCTCATGGGCAATATTACCTGGATGAGCTCCG
GAATCCCTGGGCTTACCGTTAAGCACATTGCCGCAACTACAAAAGGATGCCTGGAGTTGA
ACCAGAAGGTCATTTGGACAGAGGAAGCTCAGAAGGAACTGGAGGAGAATAATGAAAAGA
TTAAGAATGCTCAAGGGCTCCAATACTACAATCCCGAAGAAGAAATGTTGTGCGAGGTCG
AAATCACTAAGAACTACGAAGCCACCTATGTCATCAAACAGTCCCAAGGCATCTTGTGGG
CCGGAAAGAAAATCATGAAGGCCAACAAAGGCTGGTCCACCGTTAAAAATCTGATGCTCC
TGCTCCAGCACGTCGCCACCGAGTCTATCACCCGCGTCGGCAAGTGCCCCACCTTCAAAG
TTCCCTTCACTAAGGAGCAGGTGATGTGGGAGATGCAAAAAGGCTGGTACTACTCTTGGC
TTCCCGAGATCGTCTACACCCACCAAGTGGTGCACGACGACTGGAGAATGAAGCTTGTCG
AGGAGCCCACTAGCGGAATTACAATCTATACCGACGGCGGAAAGCAAAACGGAGAGGGAA
TCGCTGCATACGTCACATCTAACGGCCGCACCAAGCAAAAGAGGCTCGGCCCTGTCACTC
ACCAGGTGGCTGAGAGGATGGCTATCCAGATGGCCCTTGAGGACACTAGAGACAAGCAGG
TGAACATTGTGACTGACAGCTACTACTGCTGGAAAAACATCACAGAGGGCCTTGGCCTGG
AGGGACCCCAGTCTCCCTGGTGGCCTATCATCCAGAATATCCGCGAAAAGGAAATTGTCT
ATTTCGCCTGGGTGCCTGGACACAAAGGAATTTACGGCAACCAACTCGCCGATGAAGCCG
CCAAAATTAAAGAGGAAATCATGCTTGCCTACCAGGGCACACAGATTAAGGAGAAGAGAG
ACGAGGACGCTGGCTTTGACCTGTGTGTGCCATACGACATCATGATTCCCGTTAGCGACA
CAAAGATCATTCCAACCGATGTCAAGATCCAGGTGCCACCCAATTCATTTGGTTGGGTGA
CCGGAAAGTCCAGCATGGCTAAGCAGGGTCTTCTGATTAACGGGGGAATCATTGATGAAG
GATACACCGGCGAAATCCAGGTGATCTGCACAAATATCGGCAAAAGCAATATTAAGCTTA
TCGAAGGGCAGAAGTTCGCTCAACTCATCATCCTCCAGCACCACAGCAATTCAAGACAAC
CTTGGGACGAAAACAAGATTAGCCAGAGAGGTGACAAGGGCTTCGGCAGCACAGGTGTGT
TCTGGGTGGAGAACATCCAGGAAGCACAGGACGAGCACGAGAATTGGCACACCTCCCCTA
AGATTTTGGCCCGCAATTACAAGATCCCACTGACTGTGGCTAAGCAGATCACACAGGAAT
GCCCCCACTGCACCAAACAAGGTTCTGGCCCCGCCGGCTGCGTGATGAGGTCCCCCAATC
ACTGGCAGGCAGATTGCACCCACCTCGACAACAAAATTATCCTGACCTTCGTGGAGAGCA
ATTCCGGCTACATCCACGCAACACTCCTCTCCAAGGAAAATGCATTGTGCACCTCCCTCG
 
CAATTCTGGAATGGGCCAGGCTGTTCTCTCCAAAATCCCTGCACACCGACAACGGCACCA
ACTTTGTGGCTGAACCTGTGGTGAATCTGCTGAAGTTCCTGAAAATCGCCCACACCACTG
GCATTCCCTATCACCCTGAAAGCCAGGGCATTGTCGAGAGGGCCAACAGAACTCTGAAAG
AAAAGATCCAATCTCACAGAGACAATACACAGACATTGGAGGCCGCACTTCAGCTCGCCC
TTATCACCTGCAACAAAGGAAGAGAAAGCATGGGCGGCCAGACCCCCTGGGAGGTCTTCA
TCACTAACCAGGCCCAGGTCATCCATGAAAAGCTGCTCTTGCAGCAGGCCCAGTCCTCCA
AAAAGTTCTGCTTTTATAAGATCCCCGGTGAGCACGACTGGAAAGGTCCTACAAGAGTTT
TGTGGAAAGGAGACGGCGCAGTTGTGGTGAACGATGAGGGCAAGGGGATCATCGCTGTGC
CCCTGACACGCACCAAGCTTCTCATCAAGCCAAACTGA
SEQ ID NO:17
   pIRES1hyg ESYNGP 
AATTCGCCACCATGGGCGATCCCCTCACCTGGTCCAAAGCCCTGAAGAAACTGGAAAAAG
TCACCGTTCAGGGTAGCCAAAAGCTTACCACAGGCAATTGCAACTGGGCATTGTCCCTGG
TGGATCTTTTCCACGACACTAATTTCGTTAAGGAGAAAGATTGGCAACTCAGAGACGTGA
TCCCCCTCTTGGAGGACGTGACCCAAACATTGTCTGGGCAGGAGCGCGAAGCTTTCGAGC
GCACCTGGTGGGCCATCAGCGCAGTCAAAATGGGGCTGCAAATCAACAACGTGGTTGACG
GTAAAGCTAGCTTTCAACTGCTCCGCGCTAAGTACGAGAAGAAAACCGCCAACAAGAAAC
AATCCGAACCTAGCGAGGAGTACCCAATTATGATCGACGGCGCCGGCAATAGGAACTTCC
GCCCACTGACTCCCAGGGGCTATACCACCTGGGTCAACACCATCCAGACAAACGGACTTT
TGAACGAAGCCTCCCAGAACCTGTTCGGCATCCTGTCTGTGGACTGCACCTCCGAAGAAA
TGAATGCTTTTCTCGACGTGGTGCCAGGACAGGCTGGACAGAAACAGATCCTGCTCGATG
CCATTGACAAGATCGCCGACGACTGGGATAATCGCCACCCCCTGCCAAACGCCCCTCTGG
TGGCTCCCCCACAGGGGCCTATCCCTATGACCGCTAGGTTCATTAGGGGACTGGGGGTGC
CCCGCGAACGCCAGATGGAGCCAGCATTTGACCAATTTAGGCAGACCTACAGACAGTGGA
TCATCGAAGCCATGAGCGAGGGGATTAAAGTCATGATCGGAAAGCCCAAGGCACAGAACA
TCAGGCAGGGGGCCAAGGAACCATACCCTGAGTTTGTCGACAGGCTTCTGTCCCAGATTA
AATCCGAAGGCCACCCTCAGGAGATCTCCAAGTTCTTGACAGACACACTGACTATCCAAA
ATGCAAATGAAGAGTGCAGAAACGCCATGAGGCACCTCAGACCTGAAGATACCCTGGAGG
AGAAAATGTACGCATGTCGCGACATTGGCACTACCAAGCAAAAGATGATGCTGCTCGCCA
AGGCTCTGCAAACCGGCCTGGCTGGTCCATTCAAAGGAGGAGCACTGAAGGGAGGTCCAT
TGAAAGCTGCACAAACATGTTATAATTGTGGAAGCCAGGACATTTATCTAGTCAATGTA
GAGCACCTAAAGTCTGTTTTAAATGTAAACAGCCTGGACATTTCTCAAAGCAATGCAGAA
GTGTTCCAAAAAACGGAAGCAAGGGGCTCAAGGAGGCCCCAGAAACAAACTTTCCCGA
TACAACAGAAGAGTCAGCACAACAAATCTGTTGTACAAGAGACTCCTCAGACTCAAAATC
TGTACCCAGATCTGAGCGAAATAAAAAAGGAATACAATGTCAAGGAGAAGGATCAAGTAG
AGGATCTCAACCTGGACAGTTTGTGGAGTAACATACAATCTCGAGAAGAGGCCCACTAC
CATCGTCCTGATCAATGACACCCCTCTTAATGTGCTGCTGGACACCGGAGCCGACACCAG
CGTTCTCACTACTGCTCACTATAACAGACTGAAATACAGAGGAAGGAAATACCAGGGCAC
AGGCATCATCGGCGTTGGAGGCAACGTCGAAACCTTTTCCACTCCTGTCACCATCAAAAA
GAAGGGGAGACACATTAAAACCAGAATGCTGGTCGCCGACATCCCCGTCACCATCCTTGG
CAGAGACATTCTCCAGGACCTGGGCGCTAAACTCGTGCTGGCACAACTGTCTAAGGAAAT
CAAGTTCCGCAAGATCGAGCTGAAAGAGGGCACAATGGGTCCAAAAATCCCCCAGTGGCC
CCTGACCAAAGAGAAGCTTGAGGGCGCTAAGGAAATCGTGCAGCGCCTGCTTTCTGAGGG
CAAGATTAGCGAGGCCAGCGACAATAACCCTTACAACAGCCCCATCTTTGTGATTAAGAA
AAGGAGCGGCAAATGGAGACTCCTGCAGGACCTGAGGGAACTCAACAAGACCGTCCAGGT
CGGAACTGAGATCTCTCGCGGACTGCCTCACCCCGGCGGCCTGATTAAATGCAAGCACAT
GACAGTCCTTGACATTGGAGACGCTTATTTTACCATCCCCCTCGATCCTGAATTTCGCCC
CTATACTGCTTTTACCATCCCCAGCATCAATCACCAGGAGCCCGATAAACGCTATGTGTG
GAAGTGCCTCCCCCAGGGATTTGTGCTTAGCCCCTACATTTACCAGAAGACACTTCAAGA
GATCCTCCAACCTTTCCGCGAAAGATACCCAGAGGTTCAACTCTACCAATATATGGACGA
CCTGTTCATGGGGTCCAACGGTCTAAGAAGCAGCACAAGGAACTCATCATCGAACTGAG
GGCAATCCTCCTGGAGAAAGGCTTCGAGACACCCGACGACAAGCTGCAAGAAGTTCCTCC
ATATAGCTGGCTGGGCTACCAGCTTTGCCCTGAAAACTGGAAAGTCCAGAAGATGCAGTT
GGATATGGTCAAGAACCCAACACTGAACGACGTCCAGAAGCTCATGGGCAATATTACCTG
GATGAGCTCCGGAATCCCTGGGCTTACCGTTAAGCACATTGCCGCAACTACAAAAGGATG
CCTGGAGTTGAACCAGAAGGTCATTTGGACAGAGGAAGCTCAGAAGGAACTGGAGGAGAA
TAATGAAAAGATTAAGAATGCTCAAGGGCTCCAATACTACAATCCCGAAGAAGAAATGTT
GTGCGAGGTCGAAATCACTAAGAACTACGAAGCCACCTATGTCATCAAACAGTCCCAAGG
CATCTTGTGGGCCGGAAAGAAAATCATGAAGGCCAACAAAGGCTGGTCCACCGTTAAAAA
TCTGATGCTCCTGCTCCAGCACGTCGCCACCGAGTCTATCACCCGCGTCGGCAAGTGCCC
CACCTTCAAAGTTCCCTTCACTAAGGAGCAGGTGATGTGGGAGATGCAAAAAGGCTGGTA
CTACTCTTGGCTTCCCGAGATCGTCTACACCCACCAAGTGGTGCACGACGACTGGAGAAT
GAAGCTTGTCGAGGAGCCCACTAGCGGAATTACAATCTATACCGACGGCGGAAAGCAAAA
CGGAGAGGGAATCGCTGCATACGTCACATCTAACGGCCGCACCAAGCAAAAGAGGCTCGG
CCCTGTCACTCACCAGGTGGCTGAGAGGATGGCTATCCAGATGGCCCTTGAGGACACTAG
 
AGACAAGCAGGTGAACATTGTGACTGACAGCTACTACTGCTGGAAAAACATCACAGAGGG
CCTTGGCCTGGAGGGACCCCAGTCTCCCTGGTGGCCTATCATCCAGAATATCCGCGAAAA
GGAAATTGTCTATTTCGCCTGGGTGCCTGGACACAAAGGAATTTACGGCAACCAACTCGC
CGATGAAGCCGCCAAAATTAAAGAGGAAATCATGCTTGCCTACCAGGGCACACAGATTAA
GGAGAAGAGAGACGAGGACGCTGGCTTTGACCTGTGTGTGCCATACGACATCATGATTCC
CGTTAGCGACACAAAGATCATTCCAACCGATGTCAAGATCCAGGTGCCACCCAATTCATT
TGGTTGGGTGACCGGAAAGTCCAGCATGGCTAAGCAGGGTCTTCTGATTAACGGGGGAAT
CATTGATGAAGGATACACCGGCGAAATCCAGGTGATCTGCACAAATATCGGCAAAAGCAA
TATTAAGCTTATCGAAGGGCAGAAGTTCGCTCAACTCATCATCCTCCAGCACCACAGCAA
TTCAAGACAACCTTGGGACGAAAACAAGATTAGCCAGAGAGGTGACAAGGGCTTCGGCAG
CACAGGTGTGTTCTGGGTGGAGAACATCCAGGAAGCACAGGACGAGCACGAGAATTGGCA
CACCTCCCCTAAGATTTTGGCCCGCAATTACAAGATCCCACTGACTGTGGCTAAGCAGAT
CACACAGGAATGCCCCCACTGCACCAAACAAGGTTCTGGCCCCGCCGGCTGCGTGATGAG
GTCCCCCAATCACTGGCAGGCAGATTGCACCCACCTCGACAACAAAATTATCCTGACCTT
CGTGGAGAGCAATTCCGGCTACATCCACGCAACACTCCTCTCCAAGGAAAATGCATTGTG
CACCTCCCTCGCAATTCTGGAATGGGCCAGGCTGTTCTCTCCAAAATCCCTGCACACCGA
CAACGGCACCAACTTTGTGGCTGAACCTGTGGTGAATCTGCTGAAGTTCCTGAAAATCGC
CCACACCACTGGCATTCCCTATCACCCTGAAAGCCAGGGCATTGTCGAGAGGGCCAACAG
AACTCTGAAAGAAAAGATCCAATCTCACAGAGACAATACACAGACATTGGAGGCCGCACT
TCAGCTCGCCCTTATCACCTGCAACAAAGGAAGAGAAAGCATGGGCGGCCAGACCCCCTG
GGAGGTCTTCATCACTAACCAGGCCCAGGTCATCCATGAAAAGCTGCTCTTGCAGCAGGC
CCAGTCCTCCAAAAAGTTCTGCTTTTATAAGATCCCCGGTGAGCACGACTGGAAAGGTCC
TACAAGAGTTTTGTGGAAAGGAGACGGCGCAGTTGTGGTGAACGATGAGGGCAAGGGGAT
CATCGCTGTGCCCCTGACACGCACCAAGCTTCTCATCAAGCCAAACTGAACCCGGGGCGG
CCGCACTAGAGGAATTCGCCCCTCTCCCTCCCCCCCCCCTAACGTTACTGGCCGAAGCCG
CTTGGAATAAGGCCGGTGTGTGTTTGTCTATATGTGATTTTCCACCATATTGCCGTCTTT
TGGCAATGTGAGGGCCCGGAAACCTGGCCCTGTCTTCTTGACGAGCATTCCTAGGGGTCT
TTCCCCTCTCGCCAAAGGAATGCAAGGTCTGTTGAATGTCGTGAAGGAAGCAGTTCCTCT
GGAAGCTTCTTGAAGACAAACAACGTCTGTAGCGACCCTTTGCAGGCAGCGGAACCCCCC
ACCTGGCGACAGGTGCCTCTGCGGCCAAAAGCCACGTGTATAAGATACACCTGCAAAGGC
GGCACAACCCCAGTGCCACGTTGTGAGTTGGATAGTTGTGGAAAGAGTCAAATGGCTCTC
CTCAAGCGTAGTCAACAAGGGGCTGAAGGATGCCCAGAAGGTACCCCATTGTATGGGAAT
CTGATCTGGGGCCTCGGTGCACATGCTTTACATGTGTTTAGTCGAGGTTAAAAAAGCTCT
AGGCCCCCCGAACCACGGGGACGTGGTTTTCCTTTGAAAAACACGATGATAAGCTTGCCA
CAACCCCGTACCAAAGATGGATAGATCCGGAAAGCCTGAACTCACCGCGACGTCTGTCGA
GAAGTTTCTGATCGAAAAGTTCGACAGCGTCTCCGACCTGATGCAGCTCTCGGAGGGCGA
AGAATCTCGTGCTTTCAGCTTCGATGTAGGAGGGCGTGGATATGTCCTGCGGGTAAATAG
CTGCGCCGATGGTTTCTACAAAGATCGTTATGTTTATCGGCACTTTGCATCGGCCGCGCT
CCCGATTCCGGAAGTGCTTGACATTGGGGAATTCAGCGAGAGCCTGACCTATTGCATCTC
CCGCCGTGCACAGGGTGTCACGTTGCAAGACCTGCCTGAAACCGAACTGCCCGCTGTTCT
GCAGCCGGTCGCGGAGGCCATGGATGCGATCGCTGCGGCCGATCTTAGCCAGACGAGCGG
GTTCGGCCCATTCGGACCGCAAGGAATCGGTCAATACACTACATGGCGTGATTTCATATG
CGCGATTGCTGATCCCCATGTGTATCACTGGCAAACTGTGATGGACGACACCGTCAGTGC
GTCCGTCGCGCAGGCTCTCGATGAGCTGATGCTTTGGGCCGAGGACTGCCCCGAAGTCCG
GCACCTCGTGCACGCGGATTTCGGCTCCAACAATGTCCTGACGGACAATGGCCGCATAAC
AGCGGTCATTGACTGGAGCGAGGCGATGTTCGGGGATTCCCAATACGAGGTCGCCAACAT
CTTCTTCTGGAGGCCGTGGTTGGCTTGTATGGAGCAGCAGACGCGCTACTTCGAGCGGAG
GCATCCGGAGCTTGCAGGATCGCCGCGGCTCCGGGCGTATATGCTCCGCATTGGTCTTGA
CCAACTCTATCAGAGCTTGGTTGACGGCAATTTCGATGATGCAGCTTGGGCGCAGGGTCG
ATGCGACGCAATCGTCCGATCCGGAGCCGGGACTGTCGGGCGTACACAAATCGCCCGCAG
AAGCGCGGCCGTCTGGACCGATGGCTGTGTAGAAGTACTCGCCGATAGTGGAAACCGACG
CCCCAGCACTCGTCCGAGGGCAAAGGAATAGAGTAGATGCCGACCGAACAAGAGCTGATT
TCGAGAACGCCTCAGCCAGCAACTCGCGCGAGCCTAGCAAGGCAAATGCGAGAGAACGGC
CTTACGCTTGGTGGCACAGTTCTCGTCCACAGTTCGCTAAGCTCGCTCGGCTGGGTCGCG
GGAGGGCCGGTCGCAGTGATTCAGGCCCTTCTGGATTGTGTTGGTCCCCAGGGCACGATT
GTCATGCCCACGCACTCGGGTGATCTGACTGATCCCGCAGATTGGAGATCGCCGCCCGTG
CCTGCCGATTGGGTGCAGATCTAGAGCTCGCTGATCAGCCTCGACTGTGCCTCTAGTTGC
CAGCCATCTGTTGTTTGCCCCTCCCCCGTGCCTTCCTTGACCCTGGAAGGTGCCACTCCC
ACTGTCCTTTCCTAATAAAATGAGGAAATTGCATCGCATTGTCTGAGTAGGTGTCATTCT
ATTCTGGGGGGTGGGGTGGGGCAGGACAGCAAGGGGGAGGATTGGGAAGACAATAGCAGG
CATGCTGGGGATGCGGTGGGCTCTATGGCTTCTGAGGCGGAAAGAACCAGCTGGGGCTCG
 
AGTGCATTCTAGTTGTGGTTTGTCCAAACTCATCAATGTATCTTATCATGTCTGTATACC
GTCGACCTCTAGCTAGAGCTTGGCGTAATCATGGTCATAGCTGTTTCCTGTGTGAAATTG
TTATCCGCTCACAATTCCACACAACATACGAGCCGGAAGCATAAAGTGTAAAGCCTGGGG
TGCCTAATGAGTGAGCTAACTCACATTAATTGCGTTGCGCTCACTGCCCGCTTTCCAGTC
GGGAAACCTGTCGTGCCAGCTGCATTAATGAATCGGCCAACGCGCGGGGAGAGGCGGTTT
GCGTATTGGGCGCTCTTCCGCTTCCTCGCTCACTGACTCGCTGCGCTCGGTCGTTCGGCT
GCGGCGAGCGGTATCAGCTCACTCAAAGGCGGTAATACGGTTATCCACAGAATCAGGGGA
TAACGCAGGAAAGAACATGTGAGCAAAAGGCCAGCAAAAGGCCAGGAACCGTAAAAAGGC
CGCGTTGCTGGCGTTTTTCCATAGGCTCCGCCCCCCTGACGAGCATCACAAAAATCGACG
CTCAAGTCAGAGGTGGCGAAACCCGACAGGACTATAAAGATACCAGGCGTTTCCCCCTGG
AAGCTCCCTCGTGCGCTCTCCTGTTCCGACCCTGCCGCTTACCGGATACCTGTCCGCCTT
TCTCCCTTCGGGAAGCGTGGCGCTTTCTCAATGCTCACGCTGTAGGTATCTCAGTTCGGT
GTAGGTCGTTCGCTCCAAGCTGGGCTGTGTGCACGAACCCCCCGTTCAGCCCGACCGCTG
CGCCTTATCCGGTAACTATCGTCTTGAGTCCAACCCGGTAAGACACGACTTATCGCCACT
GGCAGCAGCCACTGGTAACAGGATTAGCAGAGCGAGGTATGTAGGCGGTGCTACAGAGTT
CTTGAAGTGGTGGCCTAACTACGGCTACACTAGAAGGACAGTATTTGGTATCTGCGCTCT
GCTGAAGCCAGTTACCTTCGGAAAAAGAGTTGGTAGCTCTTGATCCGGCAAACAAACCAC
CGCTGGTAGCGGTGGTTTTTTTGTTTGCAAGCAGCAGATTACGCGCAGAAAAAAAGGATC
TCAAGAAGATCCTTTGATCTTTTCTACGGGGTCTGACGCTCAGTGGAACGAAAACTCACG
TTAAGGGATTTTGGTCATGAGATTATCAAAAAGGATCTTCACCTAGATCCTTTTAAATTA
AAAATGAAGTTTTAAATCAATCTAAAGTATATATGAGTAAACTTGGTCTGACAGTTACCA
ATGCTTAATCAGTGAGGCACCTATCTCAGCGATCTGTCTATTTCGTTCATCCATAGTTGC
CTGACTCCCCGTCGTGTAGATAACTACGATACGGGAGGGCTTACCATCTGGCCCCAGTGC
TGCAATGATACCGCGAGACCCACGCTCACCGGCTCCAGATTTATCAGCAATAAACCAGCC
AGCCGGAAGGGCCGAGCGCAGAAGTGGTCCTGCAACTTTATCCGCCTCCATCCAGTCTAT
TAATTGTTGCCGGGAAGCTAGAGTAAGTAGTTCGCCAGTTAATAGTTTGCGCAACGTTGT
TGCCATTGCTACAGGCATCGTGGTGTCACGCTCGTCGTTTGGTATGGCTTCATTCAGCTC
CGGTTCCCAACGATCAAGGCGAGTTACATGATCCCCCATGTTGTGCAAAAAAGCGGTTAG
CTCCTTCGGTCCTCCGATCGTTGTCAGAAGTAAGTTGGCCGCAGTGTTATCACTCATGGT
TATGGCAGCACTGCATAATTCTCTTACTGTCATGCCATCCGTAAGATGCTTTTCTGTGAC
TGGTGAGTACTCAACCAAGTCATTCTGAGAATAGTGTATGCGGCGACCGAGTTGCTCTTG
CCCGGCGTCAATACGGGATAATACCGCGCCACATAGCAGAACTTTAAAAGTGCTCATCAT
TGGAAAACGTTCTTCGGGGCGAAAACTCTCAAGGATCTTACCGCTGTTGAGATCCAGTTC
GATGTAACCCACTCGTGCACCCAACTGATCTTCAGCATCTTTTACTTTCACCAGCGTTTC
TGGGTGAGCAAAAACAGGAAGGCAAAATGCCGCAAAAAAGGGAATAAGGGCGACACGGAA
ATGTTGAATACTCATACTCTTCCTTTTTCAATATTATTGAAGCATTTATCAGGGTTATTG
TCTCATGAGCGGATACATATTTGAATGTATTTAGAAAAATAAACAAATAGGGGTTCCGCG
CACATTTCCCCGAAAAGTGCCACCTGACGTCGACGGATCGGGAGATCTCCCGATCCCCTA
TGGTCGACTCTCAGTACAATCTGCTCTGATGCCGCATAGTTAAGCCAGTATCTGCTCCCT
GCTTGTGTGTTGGAGGTCGCTGAGTAGTGCGCGAGCAAAATTTAAGCTACAACAAGGCAA
GGCTTGACCGACAATTGCATGAAGAATCTGCTTAGGGTTAGGCGTTTTGCGCTGCTTCGC
GATGTACGGGCCAGATATACGCGTTGACATTGATTATTGACTAGTTATTAATAGTAATCA
ATTACGGGGTCATTAGTTCATAGCCCATATATGGAGTTCCGCGTTACATAACTTACGGTA
AATGGCCCGCCTGGCTGACCGCCCAACGACCCCCGCCCATTGACGTCAATAATGACGTAT
GTTCCCATAGTAACGCCAATAGGGACTTTCCATTGACGTCAATGGGTGGACTATTTACGG
TAAACTGCCCACTTGGCAGTACATCAAGTGTATCATATGCCAAGTACGCCCCCTATTGAC
GTCAATGACGGTAAATGGCCCGCCTGGCATTATGCCCAGTACATGACCTTATGGGACTTT
CCTACTTGGCAGTACATCTACGTATTAGTCATCGCTATTACCATGGTGATGCGGTTTTGG
CAGTACATCAATGGGCGTGGATAGCGGTTTGACTCACGGGGATTTCCAAGTCTCCACCCC
ATTGACGTCAATGGGAGTTTGTTTTGGCACCAAAATCAACGGGACTTTCCAAAATGTCGT
AACAACTCCGCCCCATTGACGCAAATGGGCGGTAGGCGTGTACGGTGGGAGGTCTATATA
AGCAGAGCTCTCTGGCTAACTAGAGAACCCACTGCTTACTGGCTTATCGAAATTAATACG
ACTCACTATAGGGAGACCCAAGCTTGGTACCGAGCTCGGATCCACTAGTAACGGCCGCCA
GTGTGCTGGAATTAATTCGCTGTCTGCGAGGGCCAGCTGTTGGGGTGAGTACTCCCTCTC
AAAAGCGGGCATGACTTCTGCGCTAAGATTGTCAGTTTCCAAAAACGAGGAGGATTTGAT
ATTCACCTGGCCCGCGGTGATGCCTTTGAGGGTGGCCGCGTCCATCTGGTCAGAAAAGAC
AATCTTTTTGTTGTCAAGCTTGAGGTGTGGCAGGCTTGAGATCTGGCCATACACTTGAGT
GACAATGACATCCACTTTGCCTTTCTCTCCACAGGTGTCCACTCCCAGGTCCAACTGCAG
GTCGATCGAGCA
SEQ ID NO:18
   pESDSYNGP 
TCAATATTGGCCATTAGCCATATTATTCATTGGTTATATAGCATAAATCAATATTGGCTA
TTGGCCATTGCATACGTTGTATCTATATCATAATATGTACATTTATATTGGCTCATGTCC
AATATGACCGCCATGTTGGCATTGATTATTGACTAGTTATTAATAGTAATCAATTACGGG
GTCATTAGTTCATAGCCCATATATGGAGTTCCGCGTTACATAACTTACGGTAAATGGCCC
GCCTGGCTGACCGCCCAACGACCCCCGCCCATTGACGTCAATAATGACGTATGTTCCCAT
AGTAACGCCAATAGGGACTTTCCATTGACGTCAATGGGTGGAGTATTTACGGTAAACTGC
CCACTTGGCAGTACATCAAGTGTATCATATGCCAAGTCCGCCCCCTATTGACGTCAATGA
CGGTAAATGGCCCGCCTGGCATTATGCCCAGTACATGACCTTACGGGACTTTCCTACTTG
GCAGTACATCTACGTATTAGTCATCGCTATTACCATGGTGATGCGGTTTTGGCAGTACAC
CAATGGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACAACTG
CGATCGCCCGCCCCGTTGACGCAAATGGGCGGTAGGCGTGTACGGTGGGAGGTCTATATA
AGCAGAGCTCGTTTAGTGAACCGTCAGATCACTAGAAGCTTTATTGCGGTAGTTTATCAC
AGTTAAATTGCTAACGCAGTCAGTGCTTCTGACACAACAGTCTCGAACTTAAGCTGCAGT
GACTCTCTTAAGGTAGCCTTGCAGAAGTTGGTCGTGAGGCACTGGGCAGGTAAGTATCAA
GGTTACAAGACAGGTTTAAGGAGACCAATAGAAACTGGGCTTGTCGAGACAGAGAAGACT
CTTGCGTTTCTGATAGGCACCTATTGGTCTTACTGACATCCACTTTGCCTTTCTCTCCAC
AGGTGTCCACTCCCAGTTCAATTACAGCTCTTAAGGCTAGAGTACTTAATACGACTCACT
ATAGGCTAGAGAATTCCAGGTAAGATGGGCGATCCCCTCACCTGGTCCAAAGCCCTGAAG
AAACTGGAAAAAGTCACCGTTCAGGGTAGCCAAAAGCTTACCACAGGCAATTGCAACTGG
GCATTGTCCCTGGTGGATCTTTTCCACGACACTAATTTCGTTAAGGAGAAAGATTGGCAA
CTCAGAGACGTGATCCCCCTCTTGGAGGACGTGACCCAAACATTGTCTGGCAGGAGCGC
GAAGCTTTCGAGCGCACCTGGTGGGCCATCAGCGCAGTCAAAATGGGGCTGCAAATCAAC
AACGTGGTTGACGGTAAAGCTAGCTTTCAACTGCTCCGCGCTAAGTACGAGAAGAAAACC
GCCAACAAGAAACAATCCGAACCTAGCGAGGAGTACCCAATTATGATCGACGGCGCCGGC
AATAGGAACTTCCGCCCACTGACTCCCAGGGGCTATACCACCTGGGTCAACACCATCCAG
ACAAACGGACTTTTGAACGAAGCCTCCCAGAACCTGTTCGGCATCCTGTCTGTGGACTGC
ACCTCCGAAGAAATGAATGCTTTTCTCGACGTGGTGCCAGGACAGGCTGGACAGAAACAG
ATCCTGCTCGATGCCATTGACAAGATCGCCGACGACTGGGATAATCGCCACCCCCTGCCA
AACGCCCCTCTGGTGGCTCCCCCACAGGGGCCTATCCCTATGACCGCTAGGTTCATTAGG
GGACTGGGGGTGCCCCGCGAACGCCAGATGGAGCCAGCATTTGACCAATTTAGGCAGACC
TACAGACAGTGGATCATCGAAGCCATGAGCGAGGGGATTAAAGTCATGATCGGAAAGCCC
AAGGCACAGAACATCAGGCAGGGGGCCAAGGAACCATACCCTGAGTTTGTCGACAGGCTT
CTGTCCCAGATTAAATCCGAAGGCCACCCTCAGGAGATCTCCAAGTTCTTGACAGACACA
CTGACTATCCAAAATGCAAATGAAGAGTGCAGAAACGCCATGAGGCACCTCAGACCTGAA
GATACCCTGGAGGAGAAAATGTACGCATGTCGCGACATTGGCACTACCAAGCAAAAGATG
ATGCTGCTCGCCAAGGCTCTGCAAACCGGCCTGGCTGGTCCATTCAAAGGAGGAGCACTG
AAGGGAGGTCCATTGAAAGCTGCACAAACATGTTATAATTGTGGGAAGCCAGGACATTTA
TCTAGTCAATGTAGAGCACCTAAAGTCTGTTTTAAATGTAAACAGCCTGGACATTTCTCA
AAGCAATGCAGAAGTGTTCCAAAAAACGGGAAGCAAGGGGCTCAAGGGAGGCCCCAGAAA
CAAACTTTCCCGATACAACAGAAGAGTCAGCACAACAAATCTGTTGTACAAGAGACTCCT
CAGACTCAAAATCTGTACCCAGATCTGAGCGAAATAAAAAAGGAATACAATGTCAAGGAG
AAGGATCAAGTAGAGGATCTCAACCTGGACAGTTTGTGGAGTAACATACAATCTCGAGA
AGAGGCCCACTACCATCGTCCTGATCAATGACACCCCTCTTAATGTGCTGCTGGACACCG
GAGCCGACACCAGCGTTCTCACTACTGCTCACTATAACAGACTGAAATACAGAGGAAGGA
AATACCAGGGCACAGGCATCATCGGCGTTGGAGGCAACGTCGAAACCTTTTCCACTCCTG
TCACCATCAAAAAGAAGGGGAGACACATTAAAACCAGAATGCTGGTCGCCGACATCCCCG
TCACCATCCTTGGCAGAGACATTCTCCAGGACCTGGGCGCTAAACTCGTGCTGGCACAAC
TGTCTAAGGAAATCAAGTTCCGCAAGATCGAGCTGAAAGAGGGCACAATGGGTCCAAAAA
TCCCCCAGTGGCCCCTGACCAAAGAGAAGCTTGAGGGCGCTAAGGAAATCGTGCAGCGCC
TGCTTTCTGAGGGCAAGATTAGCGAGGCCAGCGACAATAACCCTTACAACAGCCCCATCT
TTGTGATTAAGAAAAGGAGCGGCAAATGGAGACTCCTGCAGGACCTGAGGGAACTCAACA
AGACCGTCCAGGTCGGAACTGAGATCTCTCGCGGACTGCCTCACCCCGGCGGCCTGATTA
AATGCAAGCACATGACAGTCCTTGACATTGGAGACGCTTATTTACCATCCCCCTCGATC
 
CTGAATTTCGCCCCTATACTGCTTTTACCATCCCCAGCATCAATCACCAGGAGCCCGATA
AACGCTATGTGTGGAAGTGCCTCCCCCAGGGATTTGTGCTTAGCCCCTACATTTACCAGA
AGACACTTCAAGAGATCCTCCAACCTTTCCGCGAAAGATACCCAGAGGTTCAACTCTACC
AATATATGGACGACCTGTTCATGGGGTCCAACGGTCTAAGAAGCAGCACAAGGAACTCA
TCATCGAACTGAGGGCAATCCTCCTGGAGAAAGGCTTCGAGACACCCGACGACAAGCTGC
AAGAAGTTCCTCCATATAGCTGGCTGGGCTACCAGCTTTGCCCTGAAAACTGGAAAGTCC
AGAAGATGCAGTTGGATATGGTCAAGAACCCAACACTGAACGACGTCCAGAAGCTCATGG
GCAATATTACCTGGATGAGCTCCGGAATCCCTGGGCTTACCGTTAAGCACATTGCCGCAA
CTACAAAAGGATGCCTGGAGTTGAACCAGAAGGTCATTTGGACAGAGGAAGCTCAGAAGG
AACTGGAGGAGAATAATGAAAAGATTAAGAATGCTCAAGGGCTCCAATACTACAATCCCG
AAGAAGAAATGTTGTGCGAGGTCGAAATCACTAAGAACTACGAAGCCACCTATGTCATCA
AACAGTCCCAAGGCATCTTGTGGGCCGGAAAGAAAATCATGAAGGCCAACAAAGGCTGGT
CCACCGTTAAAAATCTGATGCTCCTGCTCCAGCACGTCGCCACCGAGTCTATCACCCGCG
TCGGCAAGTGCCCCACCTTCAAAGTTCCCTTCACTAAGGAGCAGGTGATGTGGGAGATGC
AAAAAGGCTGGTACTACTCTTGGCTTCCCGAGATCGTCTACACCCACCAAGTGGTGCACG
ACGACTGGAGAATGAAGCTTGTCGAGGAGCCCACTAGCGGAATTACAATCTATACCGACG
GCGGAAAGCAAAACGGAGAGGGAATCGCTGCATACGTCACATCTAACGGCCGCACCAAGC
AAAAGAGGCTCGGCCCTGTCACTCACCAGGTGGCTGAGAGGATGGCTATCCAGATGGCCC
TTGAGGACACTAGAGACAAGCAGGTGAACATTGTGACTGACAGCTACTACTGCTGGAAAA
ACATCACAGAGGGCCTTGGCCTGGAGGGACCCCAGTCTCCCTGGTGGCCTATCATCCAGA
ATATCCGCGAAAAGGAAATTGTCTATTTCGCCTGGGTGCCTGGACACAAAGGAATTTACG
GCAACCAACTCGCCGATGAAGCCGCCAAAATTAAAGAGGAAATCATGCTTGCCTACCAGG
GCACACAGATTAAGGAGAAGAGAGACGAGGACGCTGGCTTTGACCTGTGTGTGCCATACG
ACATCATGATTCCCGTTAGCGACACAAAGATCATTCCAACCGATGTCAAGATCCAGGTGC
CACCCAATTCATTTGGTTGGGTGACCGGAAAGTCCAGCATGGCTAAGCAGGGTCTTCTGA
TTAACGGGGGAATCATTGATGAAGGATACACCGGCGAAATCCAGGTGATCTGCACAAATA
TCGGCAAAAGCAATATTAAGCTTATCGAAGGGCAGAAGTTCGCTCAACTCATCATCCTCC
AGCACCACAGCAATTCAAGACAACCTTGGGACGAAAACAAGATTAGCCAGAGAGGTGACA
AGGGCTTCGGCAGCACAGGTGTGTTCTGGGTGGAGAACATCCAGGAAGCACAGGACGAGC
ACGAGAATTGGCACACCTCCCCTAAGATTTTGGCCCGCAATTACAAGATCCCACTGACTG
TGGCTAAGCAGATCACACAGGAATGCCCCCACTGCACCAAACAAGGTTCTGGCCCCGCCG
GCTGCGTGATGAGGTCCCCCAATCACTGGCAGGCAGATTGCACCCACCTCGACAACAAAA
TTATCCTGACCTTCGTGGAGAGCAATTCCGGCTACATCCACGCAACACTCCTCTCCAAGG
AAAATGCATTGTGCACCTCCCTCGCAATTCTGGAATGGGCCAGGCTGTTCTCTCCAAAAT
CCCTGCACACCGACAACGGCACCAACTTTGTGGCTGAACCTGTGGTGAATCTGCTGAAGT
TCCTGAAAATCGCCCACACCACTGGCATTCCCTATCACCCTGAAAGCCAGGGCATTGTCG
AGAGGGCCAACAGAACTCTGAAAGAAAAGATCCAATCTCACAGAGACAATACACAGACAT
TGGAGGCCGCACTTCAGCTCGCCCTTATCACCTGCAACAAAGGAAGAGAAAGCATGGGCG
GCCAGACCCCCTGGGAGGTCTTCATCACTAACCAGGCCCAGGTCATCCATGAAAAGCTGC
TCTTGCAGCAGGCCCAGTCCTCCAAAAAGTTCTGCTTTTATAAGATCCCCGGTGAGCACG
ACTGGAAAGGTCCTACAAGAGTTTTGTGGAAAGGAGACGGCGCAGTTGTGGTGAACGATG
AGGGCAAGGGGATCATCGCTGTGCCCCTGACACGCACCAAGCTTCTCATCAAGCCAAACT
GAACCCGGGGCGGCCGCTTCCCTTTAGTGAGGGTTAATGCTTCGAGCAGACATGATAAGA
TACATTGATGAGTTTGGACAAACCACAACTAGAATGCAGTGAAAAAAATGCTTTATTTGT
GAAATTTGTGATGCTATTGCTTTATTTGTAACCATTATAAGCTGCAATAAACAAGTTAAC
AACAACAATTGCATTCATTTTATGTTTCAGGTTCAGGGGGAGATGTGGGAGGTTTTTTAA
AGCAAGTAAAACCTCTACAAATGTGGTAAAATCCGATAAGGATCGATCCGGGCTGGCGTA
ATAGCGAAGAGGCCCGCACCGATCGCCCTTCCCAACAGTTGCGCAGCCTGAATGGCGAAT
GGACGCGCCCTGTAGCGGCGCATTAAGCGCGGCGGGTGTGGTGGTTACGCGCAGCGTGAC
CGCTACACTTGCCAGCGCCCTAGCGCCCGCTCCTTTCGCTTTCTTCCCTTCCTTTCTCGC
CACGTTCGCCGGCTTTCCCCGTCAAGCTCTAAATCGGGGGCTCCCTTTAGGGTTCCGATT
TAGAGCTTTACGGCACCTCGACCGCAAAAAACTTGATTTGGGTGATGGTTCACGTAGTGG
GCCATCGCCCTGATAGACGGTTTTTCGCCCTTTGACGTTGGAGTCCACGTTCTTTAATAG
TGGACTCTTGTTCCAAACTGGAACAACACTCAACCCTATCTCGGTCTATTCTTTTGATTT
ATAAGGGATTTTGCCGATTTCGGCCTATTGGTTAAAAAATGAGCTGATTTAACAAATATT
TAACGCGAATTTTAACAAAATATTAACGTTTACAATTTCGCCTGATGCGGTATTTTCTCC
TTACGCATCTGTGCGGTATTTCACACCGCATACGCGGATCTGCGCAGCACCATGGCCTGA
AATAACCTCTGAAAGAGGAACTTGGTTAGGTACCTTCTGAGGCGGAAAGAACCAGCTGTG
GAATGTGTGTCAGTTAGGGTGTGGAAAGTCCCCAGGCTCCCCAGCAGGCAGAAGTATGCA
AAGCATGCATCTCAATTAGTCAGCAACCAGGTGTGGAAAGTCCCCAGGCTCCCCAGCAGG
CAGAAGTATGCAAAGCATGCATCTCAATTAGTCAGCAACCATAGTCCCGCCCCTAACTCC
 
GCCCATCCCGCCCCTAACTCCGCCCAGTTCCGCCCATTCTCCGCCCCATGGCTGACTAAT
TTTTTTATTTATGCAGAGGCCGAGGCCGCCTCGGCCTCTGAGCTATTCCAGAAGTAGTG
AGGAGGCTTTTTTGGAGGCCTAGGCTTTTGCAAAAAGCTTGATTCTTCTGACACAACAGT
CTCGAACTTAAGGCTAGAGCCACCATGATTGAACAAGATGGATTGCACGCAGGTTCTCCG
GCCGCTTGGGTGGAGAGGCTATTCGGCTATGACTGGGCACAACAGACAATCGGCTGCTCT
GATGCCGCCGTGTTCCGGCTGTCAGCGCAGGGGCGCCCGGTTCTTTTTGTCAAGACCGAC
CTGTCCGGTGCCCTGAATGAACTGCAGGACGAGGCAGCGCGGCTATCGTGGCTGGCCACG
ACGGGCGTTCCTTGCGCAGCTGTGCTCGACGTTGTCACTGAAGCGGGAAGGGACTGGCTG
CTATTGGGCGAAGTGCCGGGGCAGGATCTCCTGTCATCTCACCTTGCTCCTGCCGAGAAA
GTATCCATCATGGCTGATGCAATGCGGCGGCTGCATACGCTTGATCCGGCTACCTGCCCA
TTCGACCACCAAGCGAAACATCGCATCGAGCGAGCACGTACTCGGATGGAAGCCGGTCTT
GTCGATCAGGATGATCTGGACGAAGAGCATCAGGGGCTCGCGCCAGCCGAACTGTTCGCC
AGGCTCAAGGCGCGCATGCCCGACGGCGAGGATCTCGTCGTGACCCATGGCGATGCCTGC
TTGCCGAATATCATGGTGGAAAATGGCCGCTTTTCTGGATTCATCGACTGTGGCCGGCTG
GGTGTGGCGGACCGCTATCAGGACATAGCGTTGGCTACCCGTGATATTGCTGAAGAGCTT
GGCGGCGAATGGGCTGACCGCTTCCTCGTGCTTTACGGTATCGCCGCTCCCGATTCGCAG
CGCATCGCCTTCTATCGCCTTCTTGACGAGTTCTTCTGAGCGGGACTCTGGGGTTCGAAA
TGACCGACCAAGCGACGCCCAACCTGCCATCACGATGGCCGCAATAAAATATCTTTATTT
TCATTACATCTGTGTGTTGGTTTTTTGTGTGAATCGATAGCGATAAGGATCCGCGTATGG
TGCACTCTCAGTACAATCTGCTCTGATGCCGCATAGTTAAGCCAGCCCCGACACCCGCCA
ACACCCGCTGACGCGCCCTGACGGGCTTGTCTGCTCCCGGCATCCGCTTACAGACAAGCT
GTGACCGTCTCCGGGAGCTGCATGTGTCAGAGGTTTTCACCGTCATCACCGAAACGCGCG
AGACGAAAGGGCCTCGTGATACGCCTATTTTTATAGGTTAATGTCATGATAATAATGGTT
TCTTAGACGTCAGGTGGCACTTTTCGGGGAAATGTGCGCGGAACCCCTATTTGTTTATTT
TTCTAAATACATTCAAATATGTATCCGCTCATGAGACAATAACCCTGATAAATGCTTCAA
TAATATTGAAAAAGGAAGAGTATGAGTATTCAACATTTCCGTGTCGCCCTTATTCCCTTT
TTTGCGGCATTTTGCCTTCCTGTTTTTGCTCACCCAGAAACGCTGGTGAAAGTAAAAGAT
GCTGAAGATCAGTTGGGTGCACGAGTGGGTTACATCGAACTGGATCTCAACAGCGGTAAG
ATCCTTGAGAGTTTTCGCCCCGAAGAACGTTTTCCAATGATGAGCACTTTTAAAGTTCTG
CTATGTGGCGCGGTATTATCCCGTATTGACGCCGGGCAAGAGCAACTCGGTCGCCGCATA
CACTATTCTCAGAATGACTTGGTTGAGTACTCACCAGTCACAGAAAAGCATCTTACGGAT
GGCATGACAGTAAGAGAATTATGCAGTGCTGCCATAACCATGAGTGATAACACTGCGGCC
AACTTACTTCTGACAACGATCGGAGGACCGAAGGAGCTAACCGCTTTTTTGCACAACATG
GGGGATCATGTAACTCGCCTTGATCGTTGGGAACCGGAGCTGAATGAAGCCATACCAAAC
GACGAGCGTGACACCACGATGCCTGTAGCAATGGCAACAACGTTGCGCAAACTATTAACT
GGCGAACTACTTACTCTAGCTTCCCGGCAACAATTAATAGACTGGATGGAGGCGGATAAA
GTTGCAGGACCACTTCTGCGCTCGGCCCTTCCGGCTGGCTGGTTTATTGCTGATAAATCT
GGAGCCGGTGAGCGTGGGTCTCGCGGTATCATTGCAGCACTGGGGCCAGATGGTAAGCCC
TCCCGTATCGTAGTTATCTACACGACGGGGAGTCAGGCAACTATGGATGAACGAAATAGA
CAGATCGCTGAGATAGGTGCCTCACTGATTAAGCATTGGTAACTGTCAGACCAAGTTTAC
TCATATATACTTTAGATTGATTTAAAACTTCATTTTTAATTTAAAAGGATCTAGGTGAAG
ATCCTTTTTGATAATCTCATGACCAAAATCCCTTAACGTGAGTTTTCGTTCCACTGAGCG
TCAGACCCCGTAGAAAAGATCAAAGGATCTTCTTGAGATCCTTTTTTTCTGCGCGTAATC
TGCTGCTTGCAAACAAAAAAACCACCGCTACCAGCGGTGGTTTGTTTGCCGGATCAAGAG
CTACCAACTCTTTTTCCGAAGGTAACTGGCTTCAGCAGAGCGCAGATACCAAATACTGTC
CTTCTAGTGTAGCCGTAGTTAGGCCACCACTTCAAGAACTCTGTAGCACCGCCTACATAC
CTCGCTCTGCTAATCCTGTTACCAGTGGCTGCTGCCAGTGGCGATAAGTCGTGTCTTACC
GGGTTGGACTCAAGACGATAGTTACCGGATAAGGCGCAGCGGTCGGGCTGAACGGGGGGT
TCGTGCACACAGCCCAGCTTGGAGCGAACGACCTACACCGAACTGAGATACCTACAGCGT
GAGCTATGAGAAAGCGCCACGCTTCCCGAAGGGAGAAAGGCGGACAGGTATCCGGTAAGC
GGCAGGGTCGGAACAGGAGAGCGCACGAGGGAGCTTCCAGGGGGAAACGCCTGGTATCTT
TATAGTCCTGTCGGGTTTCGCCACCTCTGACTTGAGCGTCGATTTTTGTGATGCTCGTCA
GGGGGGCGGAGCCTATGGAAAAACGCCAGCAACGCGGCCTTTTTACGGTTCCTGGCCTTT
TGCTGGCCTTTTGCTCACATGGCTCGACAGATCT
**pONY8.3G FB29 - (SEQ ID NO:19)**

AGATCTTGAATAATAAAATGTGTGTTTGTCCGAAATACGCGTTTTGAGATTTCTGTCGCC
GACTAAATTCATGTCGCGCGATAGTGGTGTTTATCGCCGATAGAGATGGCGATATTGGAA
AAATTGATATTTGAAAATATGGCATATTGAAAATGTCGCCGATGTGAGTTTCTGTGTAAC
TGATATCGCCATTTTTCCAAAAGTGATTTTTGGGCATACGCGATATCTGGCGATAGCGCT
TATATCGTTTACGGGGGATGGCGATAGACGACTTTGGTGACTTGGGCGATTCTGTGTGTC
GCAAATATCGCAGTTTCGATATAGGTGACAGACGATATGAGGCTATATCGCCGATAGAGG
CGACATCAAGCTGGCACATGGCCAATGCATATCGATCTATACATTGAATCAATATTGGCC
ATTAGCCATATTATTCATTGGTTATATAGCATAAATCAATATTGGCTATTGGCCATTGCA
TACGTTGTATCCATATCGTAATATGTACATTTATATTGGCTCATGTCCAACATTACCGCC
ATGTTGACATTGATTATTGACTAGTTATTAATAGTAATCAATTACGGGGTCATTAGTTCA
TAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAGT
ACATCAAGTGTATCATATGCCAAGTCCGCCCCCTATTGACGTCAATGACGGTAAATGGCC
CGCCTGGCATTATGCCCAGTACATGACCTTACGGGACTTTCCTACTTGGCAGTACATCTA
CGTATTAGTCATCGCTATTACCATGGTGATGCGGTTTTGGCAGTACACCAATGGGCGTGG
ATAGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTGCGATCGCCCGCC
CCGTTGACGCAAATGGGCGGTAGGCGTGTACGGTGGGAGGTCTATATAAGCAGAGCTCGT
TTAGTGAACCGGGCACTCAGATTCTGCGGTCTGAGTCCCTTCTCTGCTGGGCTGAAAAGG
CCTTTGTAATAAATATAATTCTCTACTCAGTCCCTGTCTCTAGTTTGTCTGTTCGAGATC
CTACAGTTGGCGCCCGAACAGGGACCTGAGAGGGGCGCAGACCCTACCTGTTGAACCTGG
CTGATCGTAGGATCCCCGGGACAGCAGAGGAGAACTTACAGAAGTCTTCTGGAGGTGTTC
CTGGCCAGAACACAGGAGGACAGGTAAGATTGGGAGACCCTTTGACATTGGAGCAAGGCG
CTCAAGAAGTTAGAGAAGGTGACGGTACAAGGGTCTCAGAAATTAACTACTGGTAACTGT
AATTGGGCGCTAAGTCTAGTAGACTTATTTCATGATACCAACTTTGTAAAAGAAAAGGAC
TGGCAGCTGAGGGATGTCATTCCATTGCTGGAAGATGTAACTCAGACGCTGTCAGGACAA
GAAAGAGAGGCCTTTGAAAGAACATGGTGGGCAATTTCTGCTGTAAAGATGGGCCTCCAG
ATTAATAATGTAGTAGATGGAAAGGCATCATTCCAGCTCCTAAGAGCGAAATATGAAAAG
AAGACTGCTAATAAAAAGCAGTCTGAGCCCTCTGAAGAATATCTCTAGAACTAGTGGATC
CCCCGGGCTGCAGGAGTGGGGAGGCACGATGGCCGCTTTGGTCGAGGCGGATCCGGCCAT
TAGCCATATTATTCATTGGTTATATAGCATAAATCAATATTGGCTATTGGCCATTGCATA
CGTTGTATCCATATCATAATATGTACATTTATATTGGCTCATGTCCAACATTACCGCCAT
GTTGACATTGATTATTGACTAGTTATTAATAGTAATCAATTACGGGGTCATTAGTTCATA
GCCCATATATGGAGTTCCGCGTTACATAACTTACGGTAAATGGCCCGCCTGGCTGACCGC
CCAACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAGTAC
ATCAAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTACG
TATTAGTCATCGCTATTACCATGGTGATGCGGTTTTGGCAGTACATCAATGGGCGTGGAT
AGCGGTTTGACTCACGGGGATTTCCAAGTCTCCACCCCATTGACGTCAATGGGAGTTTGT
TTTGGCACCAAAATCAACGGGACTTTCCAAAATGTCGTAACAACTCCGCCCCATTGACGC
AAATGGGCGGTAGGCATGTACGGTGGGAGGTCTATATAAGCAGAGCTCGTTTAGTGAACC
GTCAGATCGCCTGGAGACGCCATCCACGCTGTTTTGACCTCCATAGAAGACACCGGGACC

GATCCAGCCTCCGCGGCCCCAAGCTTGTTGGGATCCACCGGTCGCCACCATGGTGAGCAA
GGGCGAGGAGCTGTTCACCGGGGTGGTGCCCATCCTGGTCGAGCTGGACGGCGACGTAAA
CGGCCACAAGTTCAGCGTGTCCGGCGAGGGCGAGGGCGATGCCACCTACGGCAAGCTGAC
CCTGAAGTTCATCTGCACCACCGGCAAGCTGCCCGTGCCCTGGCCCACCCTCGTGACCAC
CCTGACCTACGGCGTGCAGTGCTTCAGCCGCTACCCCGACCACATGAAGCAGCACGACTT
CTTCAAGTCCGCCATGCCCGAAGGCTACGTCCAGGAGCGCACCATCTTCTTCAAGGACGA
CGGCAACTACAAGACCCGCGCCGAGGTGAAGTTCGAGGGCGACACCCTGGTGAACCGCAT
CGAGCTGAAGGGCATCGACTTCAAGGAGGACGGCAACATCCTGGGGCACAAGCTGGAGTA
CAACTACAACAGCCACAACGTCTATATCATGGCCGACAAGCAGAAGAACGGCATCAAGGT
GAACTTCAAGATCCGCCACAACATCGAGGACGGCAGCGTGCAGCTCGCCGACCACTACCA
GCAGAACACCCCCATCGGCGACGGCCCCGTGCTGCTGCCCGACAACCACTACCTGAGCAC
CCAGTCCGCCCTGAGCAAAGACCCCAACGAGAAGCGCGATCACATGGTCCTGCTGGAGTT
CGTGACCGCCGCCGGGATCACTCTCGGCATGGACGAGCTGTACAAGTAAAGCGGCCGCGA
CTCTAGAGTCGACCTGCAGGCATGCAAGCTTCAGCTGCTCGAGGGGGGGCCCGGTACCCA
GCTTTTGTTCCCTTTAGTGAGGGTTAATTGCGCGGGAAGTATTTATCACTAATCAAGCAC
AAGTAATACATGAGAAACTTTTACTACAGCAAGCACAATCCTCCAAAAAATTTTGTTTTT
ACAAAATCCCTGGTGAACATGATTGGAAGGGACCTACTAGGGTGCTGTGGAAGGGTGATG
GTGCAGTAGTAGTTAATGATGAAGGAAAGGGAATAATTGCTGTACCATTAACCAGGACTA
AGTTACTAATAAAACCAAATTGAGTATTGTTGCAGGAAGCAAGACCCAACTACCATTGTC
AGCTGTGTTTCCTGACCTCAATATTTGTTATAAGGTTTGATATGAATCCCAGGGGGAATC
TCAACCCCTATTACCCAACAGTCAGAAAAATCTAAGTGTGAGGAGAACACAATGTTTCAA
CCTTATTGTTATAATAATGACAGTAAGAACAGCATGGCAGAATCGAAGGAAGCAAGAGAC
CAAGAATGAACCTGAAAGAAGAATCTAAAGAAAAAAGAAGAAATGACTGGTGGAAAA
TAGGTATGTTTCTGTTATGCTTAGCAGGAACTACTGGAGGAATACTTTGGTGGTATGAAG
GACTCCCACAGCAACATTATATAGGGTTGGTGGCGATAGGGGGAAGATTAAACGGATCTG
GCCAATCAAATGCTATAGAATGCTGGGGTTCCTTCCCGGGGTGTAGACCATTTCAAAATT
ACTTCAGTTATGAGACCAATAGAAGCATGCATATGGATAATAATACTGCTACATTATTAG
AAGCTTTAACCAATATAACTGCTCTATAAATAACAAAACAGAATTAGAAACATGGAAGTT
AGTAAAGACTTCTGGCATAACTCCTTTACCTATTTCTTCTGAAGCTAACACTGGACTAAT
TAGACATAAGAGAGATTTTGGTATAAGTGCAATAGTGGCAGCTATTGTAGCCGCTACTGC
TATTGCTGCTAGCGCTACTATGTCTTATGTTGCTCTAACTGAGGTTAACAAAATAATGGA
AGTACAAAATCATACTTTTGAGGTAGAAAATAGTACTCTAAATGGTATGGATTTAATAGA
ACGACAAATAAAGATATTATATGCTATGATTCTTCAAACACATGCAGATGTTCAACTGTT
AAAGGAAAGACAACAGGTAGAGGAGACATTTAATTTAATTGGATGTATAGAAAGAACACA
TGTATTTTGTCATACTGGTCATCCCTGGAATATGTCATGGGGACATTTAAATGAGTCAAC
ACAATGGGATGACTGGGTAAGCAAAATGGAAGATTTAAATCAAGAGATACTAACTACACT
TCATGGAGCCAGGAACAATTTGGCACAATCCATGATAACATTCAATACACCAGATAGTAT
AGCTCAATTTGGAAAAGACCTTTGGAGTCATATTGGAAATTGGATTCCTGGATTGGGAGC
TTCCATTATAAAATATATAGTGATGTTTTTGCTTATTTATTTGTTACTAACCTCTTCGCC
TAAGATCCTCAGGGCCCTCTGGAAGGTGACCAGTGGTGCAGGGTCCTCCGGCAGTCGTTA
CCTGAAGAAAAAATTCCATCACAAACATGCATCGCGAGAAGACACCTGGGACCAGGCCCA
ACACAACATACACCTAGCAGGCGTGACCGGTGGATCAGGGGACAAATACTACAAGCAGAA
GTACTCCAGGAACGACTGGAATGGAGAATCAGAGGAGTACAACAGGCGGCCAAAGAGCTG
GGTGAAGTCAATCGAGGCATTTGGAGAGAGCTATATTTCCGAGAAGACCAAAGGGGAGAT
TTCTCAGCCTGGGGCGGCTATCAACGAGCACAAGAACGGCTCTGGGGGGAACAATCCTCA
CCAAGGGTCCTTAGACCTGGAGATTCGAAGCGAAGGAGGAAACATTTATGACTGTTGCAT
TAAAGCCCAAGAAGGAACTCTCGCTATCCCTTGCTGTGGATTTCCCTTATGGCTATTTTG

GGGACTAGTAATTATAGTAGGACGCATAGCAGGCTATGGATTACGTGGACTCGCTGTTAT
AATAAGGATTTGTATTAGAGGCTTAAATTTGATATTTGAAATAATCAGAAAAATGCTTGA
TTATATTGGAAGAGCTTTAAATCCTGGCACATCTCATGTATCAATGCCTCAGTATGTTTA
GAAAAACAAGGGGGAACTGTGGGGTTTTTATGAGGGGTTTTATAAATGATTATAAGAGT
AAAAAGAAAGTTGCTGATGCTCTCATAACCTTGTATAACCCAAAGGACTAGCTCATGTTG
CTAGGCAACTAAACCGCAATAACCGCATTTGTGACGCGAGTTCCCCATTGGTGACGCGTT
AACTTCCTGTTTTTACAGTATATAAGTGCTTGTATTCTGACAATTGGGCACTCAGATTCT
GCGGTCTGAGTCCCTTCTCTGCTGGGCTGAAAAGGCCTTTGTAATAAATATAATTCTCTA
CTCAGTCCCTGTCTCTAGTTTGTCTGTTCGAGATCCTACAGAGCTCATGCCTTGGCGTAA
TCATGGTCATAGCTGTTTCCTGTGTGAAATTGTTATCCGCTCACAATTCCACACAACATA
CGAGCCGGAAGCATAAAGTGTAAAGCCTGGGGTGCCTAATGAGTGAGCTAACTCACATTA
ATTGCGTTGCGCTCACTGCCCGCTTTCCAGTCGGGAAACCTGTCGTGCCAGTGATGCCCG
GGCGGCCGAGGCGGCCTACGTGAACCATCACCCAAATCAAGTTTTTTGCGGTCGAGGTGC
CGTAAAGCTCTAAATCGGAACCCTAAAGGGAGCCCCCGATTTAGAGCTTGACGGGGAAAG
CCGGCGAACGTGGCGAGAAAGGAAGGGAAGAAAGCGAAAGGAGCGGGCGCTAGGGCGCTG
GCAAGTGTAGCGGTCACGCTGCGCGTAACCACCACACCCGCCGCGCTTAATGCGCCGCTA
CAGGGCGCGTCCATTCGCCATTCAGGCTGCGCAACTGTTGGGAAGGGCGATCGGTGCGGG
CCTCTTCGCTATTACGCCAGCCCGGATCGATCCTTATCGGATTTTACCACATTTGTAGAG
GTTTTACTTGCTTTAAAAAACCTCCCACATCTCCCCCTGAACCTGAAACATAAAATGAAT
GCAATTGTTGTTGTTAACTTGTTTATTGCAGCTTATAATGGTTACAAATAAAGCAATAGC
ATCACAAATTTCACAAATAAAGCATTTTTTTCACTGCATTCTAGTTGTGGTTTGTCCAAA
CTCATCAATGTATCTTATCATGTCTGCTCGAAGCATTAACCCTCACTAAAGGGAAGCGGC
CGCCCGGGTCGACTTCACAGGTGTTTGCGGCGTCTTTTGGAGTCTCCGGGCCTCAAGACG
CGGGGGCTGCTCTGCTCGCCCCACAGCCTTTCTTGTGCCCTCTGGTAGCCTCCCCATGCG
GAGAAATCGCCCCTCTGGTCCTCGCGGAAGTAGAGCTCCCTCCAGATGCCGCGATTCACC
TCTCCCAGCTCTTTAGCGGCTTGTTGCACGCCCCTAATTCTCCATTCCAGCCTTTCTTGG
AGGACCTCGGCTTGCAAAATCTGGCCCCTAATCCACCTATCCCTTCTGGAGGGTGTGTGC
TGGGTGGGACCGGGGCCGAGGTGTCTTCTGGCGATGCAGGTCTGGCTAGGAATCTTCTCC
TCGGGCAGGGACTGTCTCAGCACGCGGCACCACTGGTCCCCCTCCAGGGGGCCTTGTGGG
TCGATCTTCCACCAGTCGTTGCGGCGCTTCTCCTCTTTGCTCTCTTCCTTGAGGTTCATC
TCTTGATCCCTGGCCTCCTTGCTCTCAGCCATGGTGGCGAATTCTCGAGGCTAGCCTCCC
GGTGGTGGGTCGGTGGTCCCTGGGCAGGGGTCTCCAGATCCCGGACGAGCCCCCAAATGA
AAGACCCCCGAGACGGGTAGTCAATCACTCTGAGGAGACCCTCCCAAGGAACAGCGAGAC
CACGAGTCGGATGCAACAGCAAGAGGATTTATTGGATACACGGGTACCCGGGCGACTCAG
TCTATCGGAGGACTGGCGCGCCGAGTGAGGGGTTGTGAGCTCTTTTATAGAGCTCGGGAA
GCAGAAGCGCGCGAACAGAAGCGAGAAGCAGGCTGATTGGTTAATTCAAATAAGGCACAG
GGTCATTTCAGGTCCTTGGGGGAGCCTGGAAACATCTGATGGGTCTTAAGAAACTGCTGA
GGGTTGGGCCATATCTGGGGACCATCTGTTCTTGGCCCCGGGCCGGGGCCGAACCGCGGT
GACCATCTGTTCTTGGCCCCGGGCCGGGGCCGAAACTGCTCACCGCAGATATCCTGTTTG
GCCCAACGTTAGCTGTTTTCGTGTACCCGCCCTTGATCTGAACTTCTCTATTCTTGGTTT
GGTATTTTTCCATGCCTTGCAAAATGGCGTTACTGCGGCTATCAGGCTAAGCAATTTGAG
ATCTGGCCGAGGCGGCCTACTCTGCATTAATGAATCGGCCAACGCGCGGGGAGAGGCGGT
TTGCGTATTGGGCGCTCTTCCGCTTCCTCGCTCACTGACTCGCTGCGCTCGGTCGTTCGG
CTGCGGCGAGCGGTATCAGCTCACTCAAAGGCGGTAATACGGTTATCCACAGAATCAGGG
GATAACGCAGGAAAGAACATGTATAACTTCGTATAATGTATGCTATACGAAGTTATACAT
GTGAGCAAAAGGCCAGCAAAAGGCCAGGAACCGTAAAAAGGCCGCGTTGCTGGCGTTTTT
CCATAGGCTCCGCCCCCCTGACGAGCATCACAAAAATCGACGCTCAAGTCAGAGGTGGCG

AAACCCGACAGGACTATAAAGATACCAGGCGTTTCCCCCTGGAAGCTCCCTCGTGCGCTC
TCCTGTTCCGACCCTGCCGCTTACCGGATACCTGTCCGCCTTTCTCCCTTCGGGAAGCGT
GGCGCTTTCTCATAGCTCACGCTGTAGGTATCTCAGTTCGGTGTAGGTCGTTCGCTCCAA
GCTGGGCTGTGTGCACGAACCCCCCGTTCAGCCCGACCGCTGCGCCTTATCCGGTAACTA
TCGTCTTGAGTCCAACCCGGTAAGACACGACTTATCGCCACTGGCAGCAGCCACTGGTAA
CAGGATTAGCAGAGCGAGGTATGTAGGCGGTGCTACAGAGTTCTTGAAGTGGTGGCCTAA
CTACGGCTACACTAGAAGGACAGTATTTGGTATCTGCGCTCTGCTGAAGCCAGTTACCTT
CGGAAAAAGAGTTGGTAGCTCTTGATCCGGCAAACAAACCACCGCTGGTAGCGGTGGTTT
TTTTGTTTGCAAGCAGCAGATTACGCGCAGAAAAAAAGGATCTCAAGAAGATCCTTTGAT
CTTTTCTACGGGGTCTGACGCTCAGTGGAACGAAAACTCACGTTAAGGGATTTTGGTCAT
GAGATTATCAAAAAGGATCTTCACCTAGATCCTTTTAAATTAAAAATGAAGTTTTAAATC
AATCTAAAGTATATATGAGTAAACTTGGTCTGACAGTTACCAATGCTTAATCAGTGAGGC
ACCTATCTCAGCGATCTGTCTATTTCGTTCATCCATAGTTGCCTGACTCCCCGTCGTGTA
GATAACTACGATACGGGAGGGCTTACCATCTGGCCCCAGTGCTGCAATGATACCGCGAGA
CCCACGCTCACCGGCTCCAGATTTATCAGCAATAAACCAGCCAGCCGGAAGGGCCGAGCG
CAGAAGTGGTCCTGCAACTTTATCCGCCTCCATCCAGTCTATTAATTGTTGCCGGGAAGC
TAGAGTAAGTAGTTCGCCAGTTAATAGTTTGCGCAACGTTGTTGCCATTGCTACAGGCAT
CGTGGTGTCACGCTCGTCGTTTGGTATGGCTTCATTCAGCTCCGGTTCCCAACGATCAAG
GCGAGTTACATGATCCCCCATGTTGTGCAAAAAAGCGGTTAGCTCCTTCGGTCCTCCGAT
CGTTGTCAGAAGTAAGTTGGCCGCAGTGTTATCACTCATGGTTATGGCAGCACTGCATAA
TTCTCTTACTGTCATGCCATCCGTAAGATGCTTTTCTGTGACTGGTGAGTACTCAACCAA
GTCATTCTGAGAATAGTGTATGCGGCGACCGAGTTGCTCTTGCCCGGCGTCAATACGGGA
TAATACCGCGCCACATAGCAGAACTTTAAAAGTGCTCATCATTGGAAAACGTTCTTCGGG
GCGAAAACTCTCAAGGATCTTACCGCTGTTGAGATCCAGTTCGATGTAACCCACTCGTGC
ACCCAACTGATCTTCAGCATCTTTTACTTTCACCAGCGTTTCTGGGTGAGCAAAAACAGG
AAGGCAAAATGCCGCAAAAAAGGGAATAAGGGCGACACGGAAATGTTGAATACTCATACT
CTTCCTTTTTCAATATTATTGAAGCATTTATCAGGGTTATTGTCTCATGAGCGGATACAT
ATTTGAATGTATTTAGAAAAATAAACAAATAGGGGTTCCGCGCACATTTCCCCGAAAAGT
GCCACCTAAATTGTAAGCGTTAATATTTTGTTAAAATTCGCGTTAAATTTTTGTTAAATC
AGCTCATTTTTTAACCAATAGGCCGAAATCGGCAAAATCCCTTATAAATCAAAAGAATAG
ACCGAGATAGGGTTGAGTGTTGTTCCAGTTTGGAACAAGAGTCCACTATTAAAGAACGTG
GACTCCAACGTCAAAGGGCGAAAAACCGTCTATCAGGGCGATGGCCCACTACGTGATAAC
TTCGTATAATGTATGCTATACGAAGTTATCACTACGTGAACCATCACCCTAATCAAGTTT
TTTGGGGTCGAGGTGCCGTAAAGCACTAAATCGGAACCCTAAAGGGAGCCCCCGATTTAG
AGCTTGACGGGGAAAGCCAACCTGGCTTATCGAAATTAATACGACTCACTATAGGGAGAC
CGGC

## Claims

1. Use of a nucleotide sequence coding for retroviral gag and pol proteins capable of assembly of a retroviral vector genome into a retroviral particle in a producer cell to generate a replication defective retrovirus in a target cell, wherein the nucleotide sequence is codon optimised for expression in the producer cell, and wherein the nucleotide sequence comprises the sequence shown in SEQ ID NO: 16.

2. A use according to claim 1 wherein the retroviral particle is substantially derived from EIAV.

3. Use of a nucleotide sequence coding for retroviral gag and pol proteins capable of assembly of a retroviral vector genome into a retroviral particle in a producer cell to prevent packaging of the retroviral vector genome in a target cell, wherein the nucleotide sequence is codon optimised for expression in the producer cell, and wherein the retroviral particle is substantially derived from EIAV; and the nucleotide sequence comprises the sequence shown in SEQ ID NO: 16.

4. Use of a nucleotide sequence coding for retroviral gag and pol proteins capable of assembly of a retroviral vector genome, comprising at least part of a gag nucleotide sequence, into a retroviral particle in a producer cell to prevent recombination between said nucleotide sequence coding for retroviral gag and pol proteins and the at least part of a gag nucleotide sequence, wherein the nucleotide sequence coding for retroviral gag and pol proteins is codon optimised for expression in the producer cell, and wherein the nucleotide sequence comprises the sequence shown in SEQ ID NO: 16.

5. A use according to claim 4 wherein the retroviral particle is substantially derived from EIAV.

6. A use according to any preceding claim wherein the retroviral genome further comprises a nucleotide of interest (NOI).

7. A use according to any preceding claim wherein the nucleotide sequence is Rev independent.

8. A use according to any preceding claim wherein the nucleotide sequence does not contain the RRE sequence.

9. A method of producing a replication defective retrovirus comprising transfecting a producer cell with the following:
i) a retroviral genome;
ii) a nucleotide sequence coding for retroviral gag and pol proteins; and
iii) nucleotide sequences encoding other essential viral packaging components not encoded by the nucleotide sequence of (ii);
**characterised in that** the nucleotide sequence coding for retroviral gag and pol proteins is codon optimised for expression in the producer cell, and **in that** the codon optimised nucleotide sequence comprises the sequence shown in SEQ ID NO: 16.

10. A method according to claim 9 wherein the retroviral particle is substantially derived from EIAV.

11. A method of preventing packaging of a retroviral genome in a target cell comprising the steps of:
a. transfecting a producer cell with the following to produce retroviral particles:
i) a retroviral genome;
ii) a nucleotide sequence coding for retroviral gag and pol proteins; and
iii) nucleotide sequences encoding other essential viral Packaging components not encoded by one or more of the nucleotide sequences of (ii); and
b. transfecting a target cell with retroviral particles of step (a);
**characterised in that** the nucleotide sequence coding for retroviral gag and pol proteins is codon optimised for expression in the producer cell, and **in that** the codon optimised nucleotide sequence comprises the sequence shown in SEQ ID NO: 16.

12. A method according to claim 11 wherein the retroviral particle is substantially derived from EIAV.

13. A method to prevent recombination between a retroviral vector genome and a nucleotide sequence encoding a viral polypeptide required for the assembly of the viral genome into retroviral particles comprising transfecting a producer cell with the following:
(i) a retroviral genome comprising at least part of a gag nucleotide sequence;
(ii) a nucleotide sequence coding for retroviral gag and pol proteins; and
(iii) nucleotide sequences encoding other essential viral packaging components not encoded by the nucleotide sequence of (ii);
**characterised in that** the nucleotide sequence coding for retroviral gag and pol proteins is codon optimised for expression in the producer cell, and **in that** the codon optimised nucleotide sequence comprises the sequence shown in SEQ ID NO: 16.

14. A method according to claim 13 wherein the retroviral particle is substantially derived from EIAV,

15. A method according to any one of claims 9 to 14 wherein the retroviral genome further comprises a nucleotide of interest (NOI).

16. A method according to any one of claims 9 to 14, wherein iii) comprises a nucleotide sequence coding for an env protein.

17. A method according to any one of claims 9 to 14 wherein at least one of i) to iii) contains one or more functional accessory genes.

18. A method according to any one of claims 9 to 14 wherein i) to iii) are devoid of any functional accessory genes.

19. A method according to any one of claims 9 to 18 wherein the nucleotide sequence coding for the retroviral gag and pol proteins is Rev independent

20. A method according to any one of claims 9 to 19 wherein the nucleotide sequence coding for the retroviral gag and pol proteins does not contain the RRE sequence.

21. A nucleotide sequence coding for retroviral gag and pol proteins comprising the sequence of SEQ ID NO: 16.

22. A vector comprising a nucleotide sequence according to claim 21, wherein the vector is Rev independent.

23. A viral vector system comprising:
i) a nucleotide sequence of interest; and
ii) a nucleotide sequence encoding a viral polypeptide required for the assembly of viral particles wherein the nucleotide sequence is as defined in claim 21.

24. A viral production system comprising:
i) a viral genome comprising at least one nucleotide sequence of interest; and
ii) a nucleotide sequence encoding a viral polypeptide required for the assembly of the viral genome into viral particles wherein the nucleotide sequence is as defined in claim 21.

25. A system according to claim 23 or claim 24 wherein the viral vector is a retroviral vector.

26. A system according to claim 25 wherein the retroviral vector is a lentiviral vector.

27. A system according to any one of claims 23 to 26 wherein the lentiviral vector is substantially derived from EIAV.

28. A system according to any of claims 23 to 27 wherein the nucleotide sequence defined i-ii) also includes an envelope protein.

29. A system according to claim 28 wherein the envelope gene is codon optimised.

30. A system according to any of claims 23 to 29 wherein the nucleotide of interest is selected from a therapeutic gene, a marker gene and a selection gene.

31. A system according to any one of claims 23 to 30 comprising one or more functional accessory genes.

32. A system according to any of claims 23 to 30 devoid of any functional accessory genes.

33. A system according to any one of claims 23 to 32 wherein the nucleotide sequence (ii) does not contain the RRE sequence.

34. A viral system according to any one of claims 23 to 33 for use in a method of producing viral particles.

35. A method for producing a viral particle which method comprises introducing into a producer cell:
i) a viral genome as defined in any one of claims 24 to 33,
ii) one or more nucleotide sequences as defined in claim 21 and,
iii) nucleotide sequences encoding other essential viral packaging components not encoded by one or more of the nucleotide sequences of (ii).

36. A viral particle produced by the production system of any one of claims 29 to 34 or by the method of claim 35.

37. A viral system according to any one of claims 24 to 34, or a viral particle according to claim 36, for treating a viral infection.

38. A pharmaceutical composition comprising the viral system of any one of claims 24 to 34, or the viral particle of claim 37, together with a pharmaceutically acceptable carrier or diluent.

## Patentansprüche

1. Verwendung einer Nukleotidsequenz, die die Retrovirusproteine gag und pol codiert, welche für die Anordnung eines Retrovirusvektorgenoms in einen Retroviruspartikel in einer Erzeugerzelle geeignet sind, um einen replikationsdefekten Retrovirus in einer Zielzelle zu erzeugen, wobei die Codons der Nukleotidsequenz für die Expression in der Erzeugerzelle optimiert sind, und wobei die Nukleotidsequenz die Sequenz umfasst, die in SEQ ID Nr.: 16 dargestellt ist.

2. Verwendung nach Anspruch 1, wobei der Retroviruspartikel im Wesentlichen von EIAV abgeleitet ist.

3. Verwendung einer Nukleotidsequenz, die die Retrovirusproteine gag und pol codiert, welche für die Anordnung eines Retrovirusvektorgenoms in einen Retroviruspartikel in einer Erzeugerzelle geeignet sind, um das Verpacken des Retrovirusvektorgenoms in einer Zielzelle zu verhindern, wobei die Codons der Nukleotidsequenz für die Expression in der Erzeugerzelle optimiert sind, und wobei der Retroviruspartikel im Wesentlichen von EIAV abgeleitet ist, und wobei die Nukleotidsequenz die Sequenz umfasst, die in SEQ ID Nr.: 16 dargestellt ist.

4. Verwendung einer Nukleotidsequenz, die die Retrovirusproteine gag und pol codiert, welche für die Anordnung eines Retrovirusvektorgenoms, weiches wenigstens einen Teil einer gag-Nukteotidsequenz umfasst, in einen Retroviruspartikel in einer Erzsugerzelle geeignet sind, um die Rekombination zwischen der die Retrovirusproteine gag und pol codierenden Sequenz und wenigstens dem Teil einer gag-Nukleotidsequenz zu verhindern, wobei die Codons der die Retrovirusproteine gag und pol codierenden Sequenz für die Expression in der Erzeugerzelle optimiert sind, und wobei die Nukleotidsequenz die Sequenz umfasst, die in SEQ ID Nr.: 16 dargestellt ist

5. Verwendung nach Anspruch 4, wobei der Retroviruspartikel im Wesentlichen von EIAV abgeleitet ist.

6. Verwendung nach einem der vorangehenden Ansprüche, wobei das Retrovirusgenom außerdem ein interessierendes Nukleotid (NOI) umfasst.

7. Verwendung nach einem der vorangehenden Ansprüche, wobei die Nukleotidsequenz Rev-unabhängig ist.

8. Verwendung nach einem der vorangehenden Ansprüche, wobei die Nukleotidsequenz nicht die Sequenz RRE enthält

9. Verfahren zur Herstellung eines replikationsdefekten Retrovirus, bei dem man eine Erzeugerzelle mit dem Folgenden transfiziert:
i) ein Retrovirusgenom,
ii) eine Nukleotidsequenz, die die Retrovirusproteine gag und pol codiert, und
iii) Nukleotidsequenzen, die andere wesentliche Virusverpackungskomponenten, die nicht von der Nuideotidsequenz von (ii) codiert werden, codieren,
**dadurch gekennzeichnet dass** die Codons der die Retrovirusproteine gag und pol codierende Nukleotidsequenz für die Expression in der Erzeugerzelle optimiert sind, und **dadurch**, dass die Nukleotidsequenz mit den optimierten Codons die Sequenz umfasst, die in SEQ ID Nr.: 16 dargestellt ist

10. Verfahren nach Anspruch 9, wobei der Retroviruspartlkel im Wesentlichen von EIAV abgeleitet ist.

11. Verfahren zum Verhindern der Verpackung eines Retrovirusgenoms in einer Zielzelle mit den Stufen, bei denen man:
a. eine Erzeugerzelle mit den Folgenden transfiziert, um Retroviruspartikel zu erzeugen:
i) ein Retrovirusgenom,
ii) eine Nukleotidsequenz, die die Retrovirusproteine gag und pol codiert, und
iii) Nukleotidsequenzen, die andere wesentliche Virusverpackungskomponenten, die nicht von der Nukleotidsequenz von (ii) codiert werden, codieren, und
b. eine Zielzelle mit dem Retroviruspartikel von Stufe (a) transfiziert,
**dadurch gekennzeichnet, dass** die Codons der die Retrovirusproteine gag und pol codierenden Nukleotidsequenz für die Expression in der Erzeugerzelle optimiert sind und **dadurch**, dass die Nukleotidsequenz mit den optimierten Codons die Sequenz umfasst, die in SEQ ID Nr.: 16 dargestellt ist.

12. Verfahren nach Anspruch 11, wobei das Retroviruspartikel im Wesentlichen von EIAV abgeleitet ist.

13. Verfahren zum Verhindern der Rekombination zwischen einem Retrovirusvektorgenom und einer Nukleotidsequenz, die ein für die Anordnung des Virusgenoms in Retroviruspartikeln erforderliches Viruspolypeptid codiert, bei dem man eine Erzeugerzelle mit dem Folgenden transfiziert:
(i) ein Retrovirusgenom, welches wenigstens einen Teil einer gag-Nukleotidsequenz umfasst,
(ii) eine Nukleotidsequenz, die die Retrovirusproteine gag und pol codiert, und
(iii) Nukleotidsequenzen, die andere wesentliche Virusverpackungskomponenten codiert, die von der Nukleotidsequenz von (ii) nicht codiert werden,
**dadurch gekennzeichnet, dass** die Codons der die Retrovirusproteine gag und pol codierenden Nukleotidsequenz für die Expression in der Erzeugerzelle optimiert sind, und **dadurch**, dass die Nukleotidsequenz mit den optimierten Codons die Sequenz umfasst, die in SEQ ID Nr.: 16 dargestellt **ist.**

14. Verfahren nach Anspruch 13, wobei der Retroviruspartikel im Wesentlichen von EIAV abgeleitet ist

15. Verfahren nach einem der Ansprüche 9 bis 14, wobei das Retrovirusgenom außerdem ein interessierendes Nukleotid (NOI) umfasst.

16. Verfahren nach einem der Ansprüche 9 bis 14, wobei iii) eine ein env-Protein codierenden Nukleotidsequenz umfasst.

17. Verfahren nach einem der Ansprüche 9 bis 14, wobei wenigstens eines von i) bis iii) eines oder mehrere funktionell akzessorische Gene enthält.

18. Verfahren nach einem der Ansprüche 9 bis 14, wobei i) bis iii) frei von jeglichen funktionell akzessorischen Genen sind.

19. Verfahren nach einem der Ansprüche 9 bis 18, wobei die die Retrovirusproteine gag und pol codierende Nukleotidsequenz Rev-unabhängig ist.

20. Verfahren nach einem der Ansprüche 9 bis 19, wobei die die Retrovirusproteine gag und pol codierende Nukleotidsequenz nicht die Sequenz RRE enthält.

21. Nukleotidsequenz, welche die Retrovirusproteine gag und pol codiert und welche die Sequenz von SEQ ID Nr.: 16 umfasst.

22. Vektor, umfassend eine Nukleotidsequenz nach Anspruch 21, wobei der Vektor Rev-unabhängig ist.

23. Virusvektorsystem, umfassend:
i) eine interessierende Nukleotidsequenz und
ii) eine ein für die Herstellung von Viruspartikeln erforderliches Viruspolypeptid codierende Nukleotidsequenz, wobei die Nukleotidsequenz so ist, wie es in Anspruch 21 definiert wird.

24. Virusproduktionssystem, umfassend:
i) ein Virusgenom, umfassend wenigstens eine interessierende Nukleotidsequenz und
ii) eine ein für die Anordnung des Virusgenoms in Viruspartikel erforderliches Viruspolypeptid codierende Nukleotidsequenz, wobei die Nuklootidsequenz so ist, wie es in Anspruch 21 definiert wird.

25. System nach Anspruch 23 oder Anspruch 24, wobei der Virusvektor ein Retrovirusvektor **ist.**

26. System nach Anspruch 25, wobei der Retrovirusvektor ein Lentivirusvektor ist

27. System nach einem der Ansprüche 23 bis 26, wobei der Lentivirusvektor im Wesentlichen von EIAV abgeleitet ist.

28. System nach einem der Ansprüche 23 bis 27, wobei die in i-ii) definierte Nukleotidsequenz auch ein Hüllprotein umfasst.

29. System nach Anspruch 28, wobei die Codons des Hüllgens optimiert sind.

30. System nach einem der Ansprüche 23 bis 29, wobei das interessierende Nukleotid ausgewählt ist unter einem therapeutischen Gen, einem Markergen und einem Selektionsgen.

31. System nach einem der Ansprüche 23 bis 30, umfassend eines oder mehrere funktionell akzessorische Gene.

32. System nach einem der Ansprüche 23 bis 30, welches frei von jeglichen funktionell akzessorischen Genen ist.

33. System nach einem der Ansprüche 23 bis 32, wobei die Nukleotidsequenz (ii) nicht die Sequenz RRE enthält.

34. Virussystem nach einem der Ansprüche 23 bis 33 für die Verwendung bei einem Verfahren der Erzeugung von Viruspartikeln.

35. Verfahren zum Erzeugen von Viruspartikeln, welches Verfahren umfasst, dass man in eine Erzeugerzelle:
i) ein wie in einem Beliebigen der Ansprüche 24 bis 33 definiertes Virusgenom,
ii) eine oder mehrere Nukleotidsequenzen, wie sie in Anspruch 21 definiert sind, und
iii) Nukleotidsequenzen, die andere wesentliche Virusverpackungskomponenten, die nicht von einer oder mehreren der Nukleotidsequenzen von (ii) codiert werden, codieren,
einbringt.

36. Viruspartikel, erzeugt durch das Produktionssystem von einem Beliebigen der Ansprüche 29 bis 34 oder durch das Verfahren von Anspruch 35.

37. Virussystem nach einem der Ansprüche 24 bis 34 oder Viruspartikel nach Anspruch 36 für die Behandlung einer Virusinfektion.

38. Pharmazeutische Zusammensetzung, umfassend das Virussystem nach einem der Ansprüche 24 bis 34 oder den Viruspartikel von Anspruch 37 zusammen mit einem pharmazeutisch verträglichen Träger oder Verdünnungsmittel,

## Revendications

1. Utilisation d'une séquence de nucléotides codant pour des protéines rétrovirales gag et pol qui est capable d'assembler un génome de vecteur rétroviral en une particule rétrovirale dans une cellule productrice afin de générer un rétrovirus déficient en réplication dans une cellule cible, où la séquence de nucléotides est optimisée en codons pour obtenir une expression dans la cellule productrice, et où la séquence de nucléotides comprend la séquence montrée dans SEQ ID NO: 16.

2. Utilisation selon la revendication 1, dans laquelle la particule rétrovirale est essentiellement dérivée de l'EIAV.

3. Utilisation d'une séquence de nucléotides codant pour des protéines rétrovirales gag et pol qui est capable d'assembler un génome de vecteur rétroviral en une particule rétrovirale dans une cellule productrice afin d'empêcher une encapsidation du génome de vecteur rétroviral dans une cellule cible, où la séquence de nucléotides est optimisée en codons pour obtenir une expression dans la cellule productrice, et où la particule rétrovirale est essentiellement dérivée de l'EIAV ; et la séquence de nucléotides comprend la séquence montrée dans SEQ ID NO: 16.

4. Utilisation d'une séquence de nucléotides codant pour des protéines rétrovirales gag et pol qui est capable d'assembler un génome de vecteur rétroviral, comprenant au moins une partie d'une séquence de nucléotides gag, en une particule rétrovirale dans une cellule productrice afin d'empêcher une recombinaison entre ladite séquence de nucléotides codant pour des protéines rétrovirales gag et pol et au moins une partie d'une séquence de nucléotides gag, où la séquence de nucléotides codant pour des protéines rétrovirales gag et pol est optimisée en codons pour obtenir une expression dans la cellule productrice, et où la séquence de nucléotides comprend la séquence montrée dans SEQ ID NO: 16.

5. Utilisation selon la revendication 4, dans laquelle la particule rétrovirale est essentiellement dérivée de l'EIAV.

6. Utilisation selon l'une quelconque des revendications précédentes, dans laquelle le génome rétroviral comprend en outre un nucléotide d'intérêt (NOI).

7. Utilisation selon l'une quelconque des revendications précédentes, dans laquelle la séquence de nucléotides est indépendante de Rev.

8. Utilisation selon l'une quelconque des revendications précédentes, dans laquelle la séquence de nucléotide ne contient pas de séquence RRE.

9. Procédé pour produire un rétrovirus déficient en réplication, qui consiste à transfecter une cellule productrice avec les éléments suivants :
i) un génome rétroviral ;
ii) une séquence de nucléotides codant pour des protéines rétrovirales gag et pol ; et
iii) des séquences de nucléotides codant pour d'autres composants d'encapsidation virale essentiels qui ne sont pas codés par la séquence de nucléotides de (ii) ;
**caractérisé en ce que** la séquence de nucléotides codant pour des protéines rétrovirales gag et pol est optimisée en codons pour obtenir une expression dans la cellule productrice, et **en ce que** la séquence de nucléotides optimisée en codons comprend la séquence montrée dans SEQ ID NO: 16.

10. Procédé selon la revendication 9, dans lequel la particule rétrovirale est essentiellement dérivée de l'EIAV.

11. Procédé pour empêcher une encapsidation d'un génome rétroviral dans une cellule cible, comprenant les étapes consistant à :
a. transfecter une cellule productrice avec les éléments suivants afin de produire des particules rétrovirales :
i) un génome rétroviral ;
ii) une séquence de nucléotides codant pour des protéines rétrovirales gag et pol ; et
iii) des séquences de nucléotides codant pour d'autres composants d'encapsidation virale essentiels qui ne sont pas codés par une ou plusieurs des séquences de nucléotides de (ii) ; et
b. transfecter une cellule cible avec les particules rétrovirales de l'étape
(a) ;
**caractérisé en ce que** la séquence de nucléotides codant pour des protéines rétrovirales gag et pol est optimisée en codons pour obtenir une expression dans la cellule productrice, et **en ce que** la séquence de nucléotides optimisée en codons comprend la séquence montrée dans SEQ ID NO: 16.

12. Procédé selon la revendication 11, dans lequel la particule rétrovirale est essentiellement dérivée de l'EIAV.

13. Procédé pour empêcher une recombinaison entre un génome de vecteur rétroviral et une séquence de nucléotides codant pour un polypeptide viral nécessaire à l'assemblage du génome viral en particules rétrovirales, consistant à transfecter une cellule productrice avec les éléments suivants :
(i) un génome rétroviral comprenant au moins une partie d'une séquence de nucléotides gag ;
ii) une séquence de nucléotides codant pour des protéines rétrovirales gag et pol ; et
iii) des séquences de nucléotides codant pour d'autres composants d'encapsidation virale essentiels qui ne sont pas codés par la séquence de nucléotides de (ii) ;
**caractérisé en ce que** la séquence de nucléotides codant pour des protéines rétrovirales gag et pol est optimisée en codons pour obtenir une expression dans la cellule productrice, et **en ce que** la séquence de nucléotides optimisée en codons comprend la séquence montrée dans SEQ ID NO: 16.

14. Procédé selon la revendication 13, dans lequel la particule rétrovirale est essentiellement dérivée de l'EIAV.

15. Procédé selon l'une quelconque des revendications 9 à 14, dans lequel le génome rétroviral comprend un nucléotide d'intérêt (NOI).

16. Procédé selon l'une quelconque des revendications 9 à 14, dans lequel iii) comprend une séquence de nucléotides codant pour une protéine env.

17. Procédé selon l'une quelconque des revendications 9 à 14, dans lequel au moins l'un de i) à iii) contient un ou plusieurs gènes auxiliaires fonctionnels.

18. Procédé selon l'une quelconque des revendications 9 à 14, dans lequel i) à iii) sont dépourvus de quelconques gènes auxiliaires fonctionnels.

19. Procédé selon l'une quelconque des revendications 9 à 18, dans lequel la séquence de nucléotides codant pour des protéines rétrovirales gag et pol est indépendante de Rev.

20. Procédé selon l'une quelconque des revendications 9 à 19, dans lequel la séquence de nucléotides codant pour des protéines rétrovirales gag et pol ne contient pas la séquence RRE.

21. Séquence de nucléotides codant pour des protéines rétrovirales gag et pol comprenant la séquence de SEQ ID NO: 16.

22. Vecteur comprenant une séquence de nucléotides selon la revendication 21, où le vecteur est indépendant de Rev.

23. Système de vecteur viral comprenant :
i) une séquence de nucléotides d'intérêt ; et
ii) une séquence de nucléotides codant pour un polypeptide viral nécessaire à l'assemblage des particules virales, où la séquence de nucléotides est telle que définie dans la revendication 21.

24. Système de production viral, comprenant :
i) un génome viral comprenant au moins une séquence de nucléotides d'intérêt; et
ii) une séquence de nucléotides codant pour un polypeptide viral nécessaire à l'assemblage du génome viral en particules virales, où la séquence de nucléotides est telle que définie dans la revendication 21.

25. Système selon la revendication 23 ou la revendication 24, dans lequel le vecteur viral est un vecteur rétroviral.

26. Système selon la revendication 25, dans lequel le vecteur rétroviral est un vecteur lentiviral.

27. Système selon l'une quelconque des revendications 23 à 26, dans lequel le vecteur lentiviral est essentiellement dérivé de l'EIAV.

28. Système selon l'une quelconque des revendications 23 à 27, dans lequel la séquence de nucléotides définie en i-ii) comprend également une protéine d'enveloppe.

29. Système selon la revendication 28, dans lequel le gène d'enveloppe est optimisé en codons.

30. Système selon l'une quelconque des revendications 23 à 29, dans lequel le nucléotide d'intérêt est sélectionné parmi un gène thérapeutique, un gène marqueur et un gène de sélection.

31. Système selon l'une quelconque des revendications 23 à 30, comprenant un ou plusieurs gènes auxiliaires fonctionnels.

32. Système selon l'une quelconque des revendications 23 à 30, dépourvu de quelconques gènes auxiliaires fonctionnels.

33. Système selon l'une quelconque des revendications 23 à 32, dans lequel la séquence de nucléotides (ii) ne contient pas la séquence RRE.

34. Système viral selon l'une quelconque des revendications 23 à 33, destiné à être utilisé dans un procédé de production de particules virales.

35. Procédé pour produire une particule virale, lequel procédé consiste à introduire dans une cellule productrice :
i) un génome viral tel que défini dans l'une quelconque des revendications 24 à 33,
ii) une ou plusieurs séquences de nucléotides telles que définies dans la revendication 21 et,
iii) des séquences de nucléotides codant pour d'autres composants d'encapsidation virale essentiels qui ne sont pas codés par une ou plusieurs des séquences de nucléotides de (ii).

36. Particule virale produite par le système de production de l'une quelconque des revendications 29 à 34 ou par le procédé de la revendication 35.

37. Système viral selon l'une quelconque des revendications 24 à 34, ou particule virale selon la revendication 36, destiné(e) au traitement d'une infection virale.

38. Composition pharmaceutique comprenant le système viral de l'une quelconque des revendications 24 à 34, ou la particule virale de la revendication 37, avec un véhicule ou diluant pharmaceutiquement acceptable.
